(19) Europäisches Patentamt
European Patent Office
Office européen des brevets

(11) Publication number: **0 008 888**
**B1**

# (12) EUROPEAN PATENT SPECIFICATION

(45) Date of publication of patent specification: 21.09.83

(21) Application number: 79301646.0

(22) Date of filing: 14.08.79

(51) Int. Cl.³: C 07 D 487/04, A 61 K 31/40 //(C07D487/04, 205/00, 209/00)

## (54) Beta-lactam antibacterial compounds, their preparation and pharmaceutical compositions containing them.

(30) Priority: 23.08.78 GB 3434378

(43) Date of publication of application: 19.03.80 Bulletin 80/6

(45) Publication of the grant of the patent: 21.09.83 Bulletin 83/38

(84) Designated Contracting States: BE CH DE FR GB IT NL SE

(56) References cited:
EP - A - 0 001 627
EP - A - 0 001 628
DE - A - 2 652 675
DE - A - 2 652 676
DE - A - 2 652 680
DE - A - 2 751 597
DE - A - 2 805 701

(73) Proprietor: BEECHAM GROUP PLC
Beecham House Great West Road
Brentford Middlesex (GB)

(72) Inventor: Bateson, John Hargreaves
6 Cedar House Oakfield Drive
Reigate Hill Reigate, Surrey (GB)
Inventor: Roberts, Patricia Margaret
15 Bakehouse Road
Horley, Surrey (GB)

(74) Representative: Hesketh, Alan, Dr. et al,
European Patent Attorney Beecham
Pharmaceuticals Great Burgh Yew Tree Bottom Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 008 888 B1

β-lactam antibacterial compounds, their preparation and pharmaceutical compositions containing them

The present invention relates to β-lactam antibacterials, to compositions containing them, to the process for their preparation and to compounds useful as intermediates in that process.

Belgian Patent No. 860962 discloses a vast group of compounds of the general formula (I):

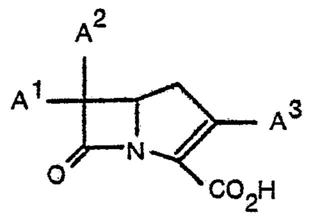

(I)

and their salts and esters wherein $A^1$, $A^2$ and $A^3$ may be hydrogen or various optionally substituted hydrocarbon groups. There have been many other publications on carbapenems; see for example United States Patent 3,950,357 and European Patent Application Nos. 0001264 and 0001265 on thienamycin and derivatives thereof; Belgian Patent No. 865,578, and European Patent Application Nos. 0001567 and 0002058 on PS—5, PS—6, PS—7 and related compounds; Belgian Patent No. 864.570 on naturally occurring compounds; and European Patent Application Nos. 0000828, 0001627, 0001628 and 0002564 on carbapenems prepared by total synthesis.

A further and considerably different group of $\Delta^2$-carbapenems has now been discovered which carry a thioether moiety on the 5-membered ring. These compounds have been found to possess gram-negative and gram-positive antibacterial activity so that they are of interest as broad spectrum antibacterial agents.

The present invention provides the compounds of the formula (II):

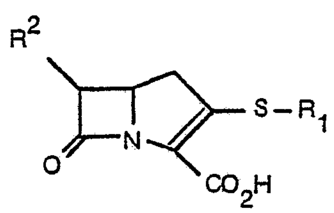

(II)

and salts and esters thereof wherein $R_1$ is a lower alkyl group, an aralkyl group or is a lower alkyl group substituted on other than the carbon atom to which the sulphur atom is attached by a $CO_2R_3$, $OR_4$, $N(R_5)COR_6$, $N(R_5)CO_2R_6$ or $N(R_5)R_7$ group or by a $CO_2R_3$ and a $N(R_5)COR_6$ group wherein $R_3$ is a group such that $CO_2R_3$ is a carboxylic acid group or a salt or lower alkyl or benzyl ester thereof; $R_4$ is a hydrogen atom or a lower alkyl, lower acyl, benzyloxycarbonyl or p-nitrobenzyloxycarbonyl group or a $CONR_8R_9$ group, $R_5$ is a hydrogen atom or a lower alkyl group; $R_6$ is a lower alkyl, benzyl or p-nitrobenzyl group; $R_7$ is a hydrogen atom or a lower alkyl aralkyl, benzhydryl or trityl group; $R_8$ is a hydrogen atom or a lower alkyl or phenyl group; $R_9$ is a hydrogen atom or a lower alkyl group; and $R_2$ is a hydrogen atom or an azidoloweralkyl group or a group $CR_{10}R_{11}R_{12}$ wherein $R_{10}$ is a hydrogen atom or a hydroxyl group; $R_{11}$ is a hydrogen atom or a lower alkyl group; and $R_{12}$ is a hydrogen atom, loweracyloxyloweralkyl group, hydroxyloweralkyl group, lower alkyl group, a benzyl group, a phenyl group or is joined to $R_{11}$ to form part of a $C_{5-7}$ carbocyclic ring or is a group of the formula $CH(OH)R_{13}$ or CHX wherein $R_{13}$ is a hydrogen atom or lower alkyl group and X is an oxygen atom or a $CR_{14}R_{15}$ group where $R_{14}$ is a hydrogen atom or a lower alkyl, phenyl, CN, $CO_2R_{16}$ or $CO.R_{16}$ group where $R_{16}$ is a lower alkyl, phenyl or benzyl group and $R_{15}$ is a hydrogen atom or a lower alkyl group or is joined to $R_{14}$ to form part of a $C_{5-7}$ carbocyclic ring; with the proviso that the following compounds are excluded: wherein $R_2$ is 1-hydroxyethyl or acylated derivatives thereof or hydroxymethyl and $R_1$ is methyl or aminoalkyl of up to 3 carbon atoms: wherein $R_2$ is 1-hydroxyethyl and $R_1$ is benzyl, $NH_2(CH_2)_4$—, $NH_2C(CH_3)_2CH_2$—, $NH_2CH_2C(CH_3)_2$— or N-substituted aminoethyl: wherein $R_2$ is hydroxymethyl and $R_1$ is $NH_2C(CH_3)_2CH_2$—; wherein $R_2$ is $PhCH_2CH(OH)$— or ethyl and $R_1$ is aminoethyl: and wherein $R_2$ is isopropyl and $R_1$ is 2-acetamidoethyl.

When used herein the term "lower" means the group contains up to 4 carbon atoms. When used herein the term "lower alkyl" more suitably means a group of up to 3 carbon atoms which is most suitably a straight chain group.

When used herein the term "aralkyl" means a lower alkyl group substituted by a phenyl, fluorophenyl, chlorophenyl, bromophenyl, methoxyphenyl, methylphenyl, nitrophenyl or aminophenyl group.

Suitably $R_1$ is a lower alkyl group such as a methyl, ethyl or n-propyl group. A favoured lower alkyl group is the ethyl group.

Suitably $R_1$ is an aralkyl group such as a benzyl group.

Suitably $R_1$ is a lower alkyl group substituted on other than the carbon atom to which the sulphur atom is attached by a $CO_2R_3$ group, for example wherein the lower alkyl group is an ethyl group and $R_3$ is a methyl group.

Suitably $R_1$ is a lower alkyl group substituted on other than the carbon atom to which the sulphur atom is attached by a $OR_4$ group, for example wherein the lower alkyl group is an ethyl group and $R_4$ is a hydrogen atom or a methyl or acetyl group.

Suitably $R_1$ is a lower alkyl group substituted on other than the carbon atom to which the sulphur atom is attached by a $NR_5R_7$, for example wherein the lower alkyl group is ethyl and $R_5$ and $R_7$ are both methyl groups.

Suitably also $R_1$ is a lower alkyl group substituted on other than the carbon atom to which the sulphur atom is attached by a $OR_4$ group, for example wherein the lower alkyl group is an ethyl group and $R_4$ is a $CONR_8R_9$ group.

Preferably $R_1$ is a $CH_2CH_2OCONHCH_3$ group.

Suitably $R_1$ is a lower alkyl group substituted by a $N(R_5)COR_6$, $N(R_5)CO_2R_6$ or $N(R_5)R_7$ group where $R_5$, $R_6$ and $R_7$ are as defined in relation to formula (II).

Aptly $R_1$ is a $-CH_2-CH_2-N(R_5)COR_6$, $-CH_2-CH_2-N(R_5)CO_2R_6$ or $-CH_2-CH_2-N(R_5)R_7$ group where $R_5$, $R_6$ and $R_7$ are as above defined.

Aptly $R_5$ is a hydrogen atom or a methyl group.

Aptly $R_6$ is a methyl or ethyl group.

Aptly $R_7$ is a hydrogen atom or a methyl or trityl group. A favoured substituted alkyl group of this kind is the $-CH_2-CH_2-NH_2$ group. A further favoured substituted alkyl group of this kind is the $-CH_2-CH_2-NH.CO.CH_3$ group. Another favoured substituted alkyl group of this kind is the $-CH_2-CH_2-NH-C(C_6H_5)_3$ group. The preceding groups containing a benzyloxycarbonyl p-nitrobenzyloxycarbonyl or trityl group are envisaged as intermediates in the preparation of corresponding $NH_2$ compounds.

The compounds of the formula (II) wherein $R_2$ is a hydrogen atom offer the advantage of favourable activity coupled with a readier synthesis than those where $R_2$ is a substituent.

Thus one favoured sub-group of compounds of the formula (II) is that of the formula (III):

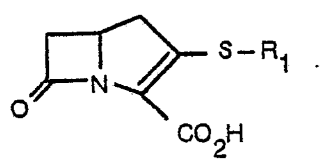

(III)

and salts and esters thereof wherein $R_1$ is as defined in relation to formula (II). In such compounds $R_1$ is most preferably ethyl.

A further suitable sub-group of compounds of the formula (II) is that of the formula (IV):

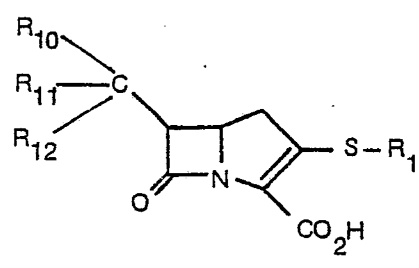

(IV)

and salts and esters thereof wherein $R_1$ $R_{10}$ $R_{11}$ and $R_{12}$ are as defined in relation to formula (II).

Suitably $R_{10}$ is a hydrogen atom. Suitably $R_{10}$ is a hydroxyl group. Suitably values for $R_{12}$ include the hydrogen atom and the methyl, ethyl, hydroxyethyl, acetoxyethyl, n-propyl, phenyl, CHO, $CH{=}CHCO_2R_{17}$, $CH{=}CHCOR_{17}$ and $CH{=}CHR_{18}$ groups wherein $R_{17}$ is a methyl, ethyl, benzyl or p-nitrobenzyl group and $R_{18}$ is a hydrogen atom or a methyl, ethyl or benzyl group.

Suitable values for $R_{11}$ include the hydrogen atom and the methyl, ethyl and n-propyl groups.

Favourably $R_{11}$ is a hydrogen atom or a methyl group.

Favourably $R_{12}$ is a hydrogen atom or a methyl group.

A suitable sub-group of compounds of the formula (IV) is that of the formula (V):

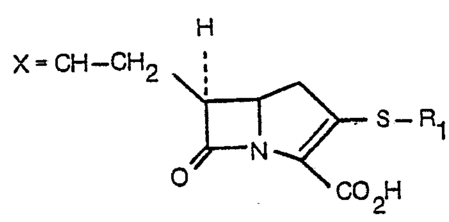

(V)

and salts and esters thereof wherein $R_1$ and X are defined in relation to formula (II).

Suitably X is a $CR_{14}R_{15}$ group where $R_{14}$ and $R_{15}$ are defined as in relation to formula (II).

Favourably $CR_{14}R_{15}$ is a CN, $CHCO_2R_{17}$ or $CHCOR_{17}$ group where $R_{17}$ is as defined in relation to formula (IV).

A further apt sub-group of compounds of the formula (IV) is that of the formula (VI):

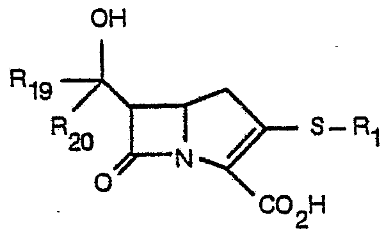

(VI)

and salts and esters thereof wherein $R_1$ is as defined in relation to formula (II), $R_{19}$ is a hydrogen atom or a lower alkyl group and $R_{20}$ is a hydrogen atom or a lower alkyl group.

Suitably $R_{19}$ is a hydrogen atom or a methyl or ethyl group. Suitably $R_{20}$ is a hydrogen atom or a methyl or ethyl group.

Favourably the $C(OH)(R_{19})R_{20}$ moiety is a $C(CH_3)_2OH$, $CH(CH_3)OH$ or $CH(C_2H_5)OH$ group of which the $CH(CH_3)OH$ group is generally preferred.

Yet another apt sub-group of compounds of the formula (II) is that of the formula (VII):

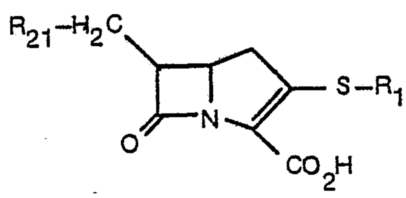

(VII)

and salts and esters thereof wherein $R_1$ is as defined in relation to formula (II) and $R_{21}$ is a hydrogen atom or a methyl, ethyl or hydroxymethyl group.

The compounds of the formulae (II)—(VII) and salts thereof tend to be more active than corresponding esters and are thus particularly suitable. It is most suitable that they are in the form of a carboxylic acid salt. When $R_1$ contains an amino group the compound of the formula (II)—(VII) is zwitterionic. When $R_1$ does not contain an amino group it is preferred that the compound of the formula (II) has its carboxylic acid group salted by a pharmaceutically acceptable cation such as those of the alkali or alkaline earth metals or a non-toxic amine. Favoured salts include the sodium and potassium salts.

Compounds of the formulae (II)—(VII) which are esterified have activity in their own right but less than the corresponding salts so in general it is preferred that esters of this invention are those which are convertible to a corresponding salt by chemical or biological means (i.e. cleavable esters).

Suitably the acid is esterified by a group of the sub-formulae (a), (b), (c) or (d):

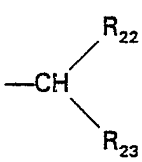 (a) 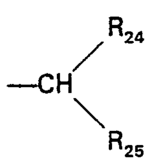 (b) 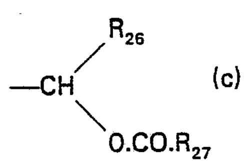 (c) 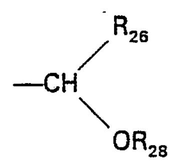 (d)

wherein $R_{22}$ is a hydrogen atom or an alkyl, alkenyl or alkynyl group of up to 3 carbon atoms; $R_{23}$ is a hydrogen atom or a methyl group; $R_{24}$ is a phenyl group or a phenyl group substituted by a fluorine, chlorine or bromine atom or a nitro, methyl or methoxyl group; $R_{25}$ is a hydrogen atom or a phenyl group or a phenyl group substituted by a fluorine, chlorine or bromine atom or a nitro, methyl or methoxyl group; $R_{26}$ is a hydrogen atom or a methyl group and $R_{27}$ is a lower alkyl, phenyl or lower alkoxy group or $R_{26}$ is joined to $R_{27}$ to form a phthalidyl group; and $R_{28}$ is a lower alkyl, phenyl, chlorophenyl or nitrophenyl group; or $CH(R_{22})R_{23}$ is a phenacyl or p-bromophenacyl group.

Favourably $R_{22}$ is a hydrogen atom or a methyl, ethyl, vinyl or ethenyl group. Favourably $R_{23}$ is a hydrogen atom. Favourably $R_{24}$ is a phenyl, p-bromophenyl, p-methoxyphenyl or p-nitrophenyl group. Favourably $R_{25}$ is a hydrogen atom. Favourably $R_{27}$ is a methyl, t-butyl or ethoxyl group or is joined to $R_{26}$. Favourably $R_{28}$ is a methyl group.

Particularly apt groups of the sub-formula (a) include the methyl and ethyl groups.

Particularly apt groups of the sub-formula (b) include the benzyl and p-nitrobenzyl groups.

Particularly apt groups of the sub-formula (c) include the acetoxymethyl, pivaloyloxymethyl, $\alpha$-ethoxycarbonyloxyethyl and phthalidyl groups.

A particularly apt group of the sub-formula (d) is the methoxymethyl group.

A preferred esterifying group is p-nitrobenzyl.

A further preferred esterifying group is the phthalidyl group.

The compounds of the formula (IV)—(VII) may have the cis-configuration about the β-lactam ring. The compounds of the formulae (IV) and (VI)—(VII) may alternatively have the trans-configuration about the β-lactam ring. If desired these compounds may be presented as mixtures of cis- and trans-compounds although it is not normally preferred to so do. The compounds of the formula (II)—(VII) are generally provided as mixtures of 5R and 5S forms.

The compounds of the formula (III) are advantageously provided as the 5S- form substantially free of the 5R- form. Thus, for example, preferred compounds of this invention (such as that of the formula (III) wherein $R_1$ is ethyl and its pharmaceutically acceptable salts and cleavable esters such as its phthalidyl ester) can be provided as 5RS mixtures of the 5S- form substantially free of the 5R- form. The 5S- forms are believed to have the same stereochemistry as (+)-p-nitrobenzyl-3-ethylthio-7-oxo-1-azabicyclo[3.2.0]hept-2-ene-2-carboxylate.

This invention also provides an antibacterial pharmaceutical composition which comprises a compound of the formula (II)—(VII) or a salt or ester thereof and a pharmaceutically acceptable carrier. Most suitably the composition will be in unit dosage form and will comprise 25—1000 mg and more usually 50—500 mg of a compound of the formula (II)—(VII). Favourably the compound of the formula (II)—(VIII) present in such compositions will be in the form of an ester in-vivo hydrolysable to the parent acid or its salt. Preferably the compound of the formula (II)—(VII) present in such compositions will be in the form of a pharmaceutically acceptable salt. The compositions of this invention may beneficially also comprise a penicillin or cephalosporin. Certain particularly suitable penicillins for use in these compositions include amoxycillin trihydrate and sodium amoxycillin.

The compositions of this invention may be used for the treatment of bacterial infections due to susceptible bacteria such as gram positive bacteria such as *Staphylococcus aureus* or gram negative bacteria such as *Escherichia coli* or *Klebsiella aerogenes.*

The compositions may be administered in conventional manner, for example orally or parenterally or in cattle by intramammary administration (for the treatment of mastitis).

The present invention also provides a process for the preparation of the compounds of the invention which process comprises the base catalysed isomerism of the double bond in an ester of a compound of the formula (VIII):

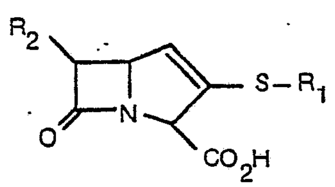

(VIII)

wherein $R_1$ and $R_2$ are as defined in relation to formula (II) and thereafter if desired converting a cleavable ester of the formula (II) into the compound of the formula (II) or a salt thereof.

The base used to bring about this isomerisation will be a strong base of low nucleophilicity such as 1,5-diazabicyclo[5.4.0]undec-5-ene (D.B.U).

The reaction is generally carried out in an aprotic medium such as a halohydrocarbon, for example dichloromethane. The isomerisation may take place at a depressed, ambient or elevated temperature (such as from −80° to +80°C) although it is most convenient to carry out the reaction at ambient temperature.

In general the desired ester of the compound of formula (II) may be isolated by washing with brine to remove the water soluble materials and then evaporating the dried organic solution. The initially isolated product may be purified by chromatography for example over silica or Florisil using a solvent such as ethyl acetate/petroleum ether mixtures. Further purification can sometimes be achieved by recrystallisation for example from the same solvent.

Any convenient ester may be used in the process of this invention. Since it is frequently desirable to form a salt of a compound of the formula (II), the ester employed is preferably one which is readily converted to the parent acid or its salt by mild methods of hydrogenolysis. In a further aspect therefore the invention includes a process for preparing a salt or free acid of a compound (II) which process comprises de-esterifying an ester of a compound of formula (II). Particularly suitable esters for use in this process include benzyl esters, optionally substituted for example in the para position by a lower alkoxy or nitro group or a halogen atom.

A preferred ester for use in this process is the p-nitrobenzyl ester.

An ester of a compound of the formula (II) may be de-esterified by conventional methods of hydrogenolysis.

Suitable methods include hydrogenation in the presence of a transition metal catalyst. The pressure of hydrogen used in the reaction may be low, medium or high but in general an approximately atmospheric or slightly super-atmospheric pressure of hydrogen is preferred. The transition metal

catalyst employed is preferably palladium on charcoal or on calcium carbonate. The hydrogenation may be effected in any convenient solvent in which the ester is soluble such as aqueous tetrahydrofuran, aqueous dioxan or the like. If this hydrogenation is carried out in the presence of a base then a salt of a compound of the formula (II) is produced. Suitable bases for inclusion include $NaHCO_3$, $KHCO_3$, $Na_2CO_3$, $K_2CO_3$, $CaCO_3$, $MgCO_3$, $LiHCO_3$, $NH_4OCOCH_3$ and the like. If no base is present then hydrogenation leads to the preparation of an acid within formula (II) which may then be neutralised if desired to yield a salt. Suitable bases which may be used to neutralise acids within formula (II) include LiOH, NaOH, $NaHCO_3$, KOH, $Ca(OH)_2$ and $Ba(OH)_2$.

The salts of acids (II) may be converted to esters in conventional manner, for example by reaction with a reactive halide such as bromophthalide in solution in dimethylformamide or like solvent.

The substituent group or groups within the groups $R_1$ and $R_2$ in the compounds of formula (II) may be varied by conventional reactions. Thus for example when a substituent is a nitro group it may be reduced in a conventional manner to an amino group, for example by catalysed hydrogenation. Similarly an amino group may be acylated to give a substituted amido group, for example by treatment with an acyl halide in the presence of an organic base. Substituents such as $NHCO_2CH_2C_6H_5$, $NH.CO_2CH_2C_6H_4NO_2$ or $OCO_2CH_2C_6H_5$ may be converted to an amino or hydroxyl group for example by hydrogenolysis.

The ester of the compound of formula (VIII) may be prepared by the reaction of iodobenzene dichloride in the presence of pyridine on an ester of a compound of the formula (IX):

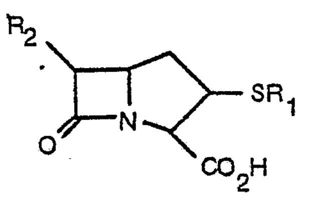

(IX)

where $R_1$ and $R_2$ are defined in relation to formula (II). The reaction may be carried out in an aprotic medium such as benzene or toluene. Most suitably the reaction takes place in benzene at a depressed temperature for example below 10°C.

The ester of the compound of the formula (VIII) may be isolated by conventional methods such as those described for the ester of the compound of the formula (II).

The preceding reaction is sometimes particularly useful in that the derived ester of the compound of the formula (II) may sometimes be isolated from the reaction mixture.

The ester of the compound of the formula (IX) may be prepared by the addition of a thiol $R_1SH$ to an ester of a compound of the formula (X):

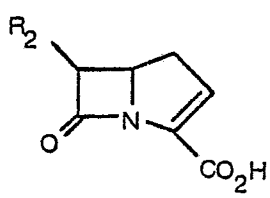

(X)

wherein $R_2$ is as defined in relation to formula (II).

The addition of the thiol usually takes place in an inert organic solvent of relatively high dielectric constant such as dimethylformamide or similar solvents.

Normally the addition reaction is carried out in the presence of a base such as a carbonate or bicarbonate such as those of the alkali or alkaline earth metals, for example potassium carbonate.

The reaction is generally performed at a non-extreme temperature, for example −20° to 40°C and is conveniently performed at ambient temperature.

The ester of the compound of the formula (X) may be prepared by the ring closing elimination of the elements of triphenylphosphine oxide from the corresponding ester of the compound of the formula (XI):

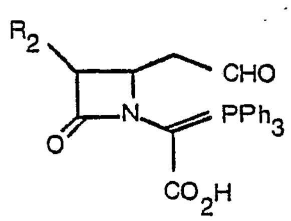

(XI)

wherein $R_2$ is as defined in relation to formula (II).

The solvent used in this process will be an inert organic solvent such as ethyl acetate.

Since the ring closure tends to occur spontaneously even at relatively low temperatures such as −20°C to 0°C, it is generally convenient to prepare and ring-close the phosphorane aldehyde in situ.

The preparation of the ester of the compound of the formula (XI) may be effected by the ozonolysis in the presence of excess trifluoroacetic acid of the corresponding ester of the compound of the formula (XII):

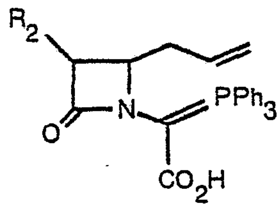

(XII)

wherein $R_2$ is as defined in relation to formula (II) and thereafter neutralising the trifluoroacetic acid.

This ozonolysis is normally carried out in an inert solvent such as ethyl acetate.

The process is normally carried out at a depressed temperature, for example −80° to −20°C more usually at about −70° to −50°C. In general ozone is passed through the solution until a light blue colour is produced. At this point excess ozone may be removed by passing through an inert gas. The intermediate ozonide may now be reduced by the addition of a conventional reducing agent such as triphenylphosphine. The trifluoroacetic acid should now be neutralised for example by the addition of a solution of a base such as sodium bicarbonate solution at about 0°C.

Once the neutralization is complete the reaction is usually allowed to warm to ambient temperature under which conditions the compound spontaneously cyclises.

Suitable reaction sequences leading to the preparation of intermediates such as the esters of the compound of the formula (XII) are shown in the following Schemes:

Scheme 1

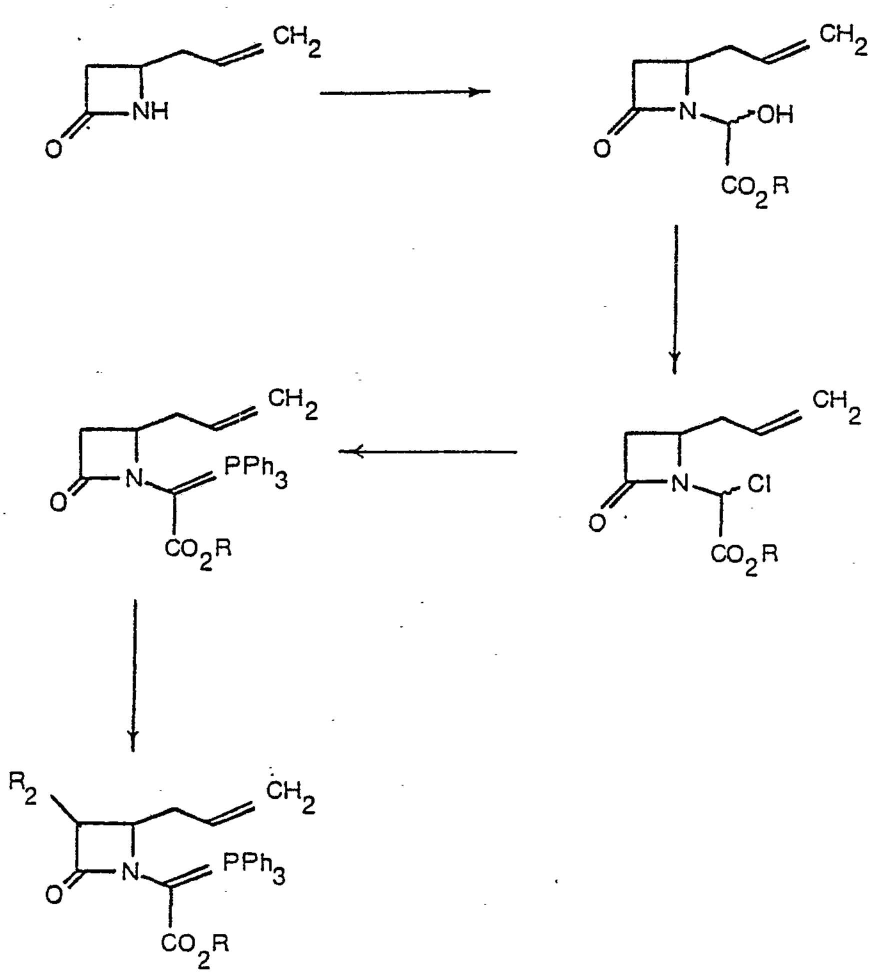

Scheme 2

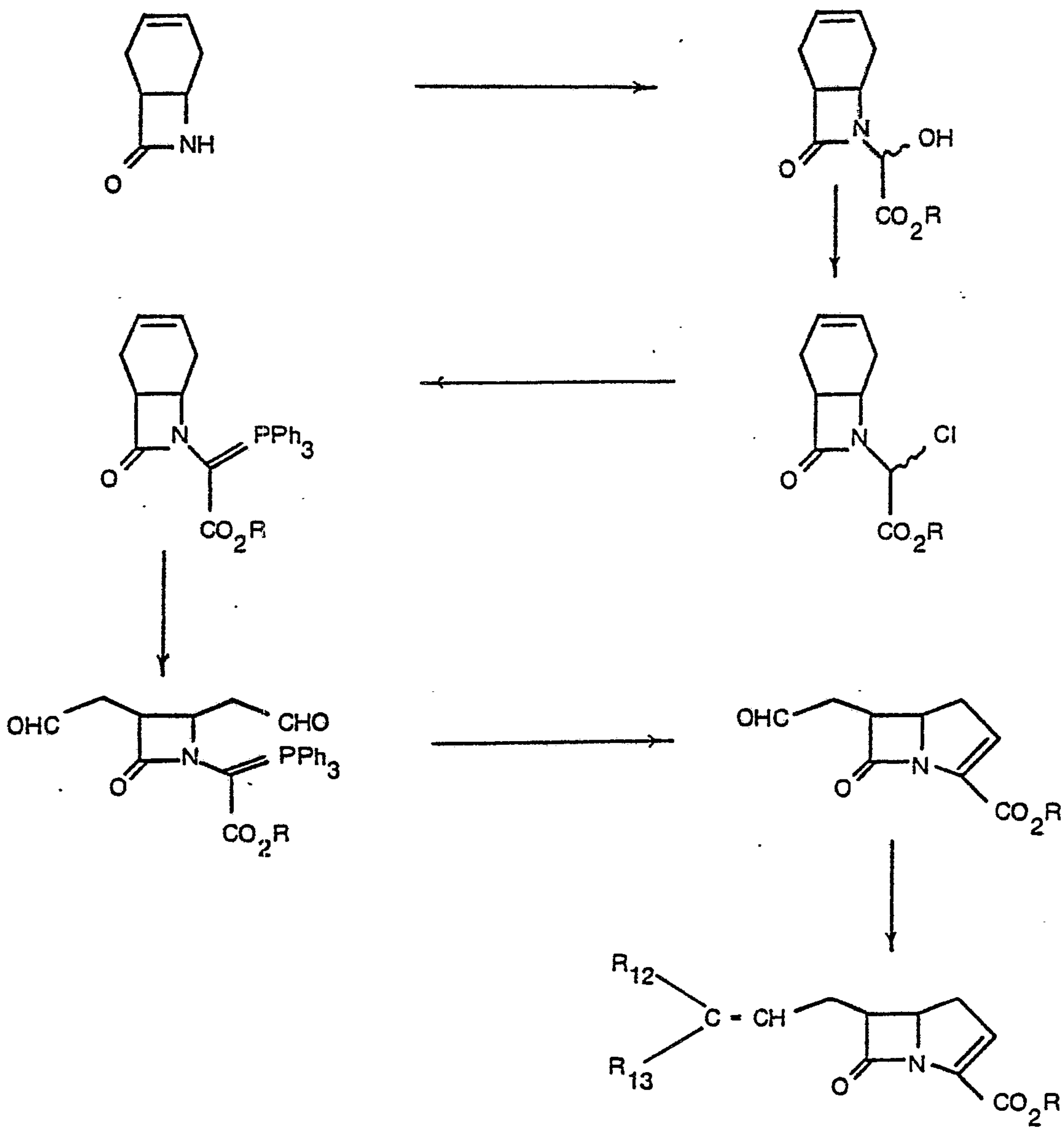

The ester of the compound of the formula (XII) wherein $R_2$ is a group $CR_{10}R_{11}R_{12}$ as defined in relation to formula (II) may be prepared by the reaction of an ester of the compound of the formula (XIII):

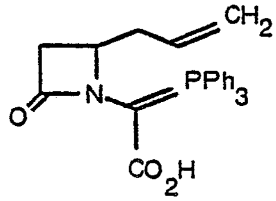

(XIII)

with a base to generate an anion of the ester of the compound of the formula (XIV):

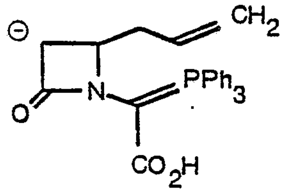

(XIV)

which is then reacted with a compound of the formula (XV) or (XVI):

(XV) 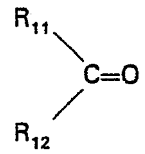 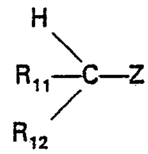 (XVI)

wherein Z is Cl, Br or I.

The generation and reaction of the anion is brought about in an inert organic medium under aprotic conditions. Suitable solvents for the reaction include hydrocarbons such as hexane, tetrahydrofuran, dimethoxyethane, dimethylformamide, hexamethylphosphorus triamide or mixtures thereof.

Since the anion is a highly reactive species its generation and reaction are normally effected at a depressed temperature, for example below 0°C, more suitably —80° to —60°C.

The base used to generate the anion will be a strong base of low nucleophilicity. Suitable bases include lithium alkyls such as lithium butyl, lithium amides such as lithium di-isopropylamide or lithium hexamethyldisilylamide.

Once formed the anion may be quenched by the addition of the dry compound of the formula (XV) or (XVI). The compound of the formula (XV) or (XVI) may be added neat or dissolved in a dry inert solvent.

It is most suitable to carry out the preceding reactions under an inert atmosphere, for example under dry nitrogen or dry argon.

The preceding reaction normally produces a mixture of the cis- and trans- compounds. These may often be separated chromatographically, for example over silica gradiently eluting with solvent mixtures such as ethyl acetate/hexane.

The esters of the compound of the formula (XIII) as hereinbefore define may be prepared by the reaction of triphenylphosphine and the corresponding ester of the compound of the formula (XVII):

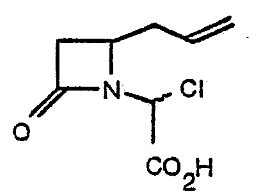

(XVII)

This reaction is normally effected in the presence of at least one equivalent of a base of relatively low nucleophilicity such as 2,6-lutidine at an ambient temperature in a dry solvent such as dioxan, tetrahydrofuran or the like.

The ester of the compound of the formula (XVII) may be prepared from corresponding ester of the carbinol of the formula (XVIII):

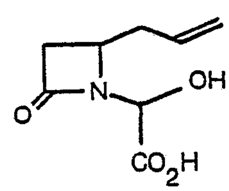

(XVIII)

by reaction with thionyl chloride.

This reaction is also normally effected in the presence of at least one equivalent of a base of relatively low nucleophilicity in a dry solvent such as dioxan or tetrahydrofuran but in this instance the reaction is performed at a depressed temperature, for example —30 to —10°C.

The ester of the preceding carbinol may be prepared by the reaction of the compound of the formula (XIX):

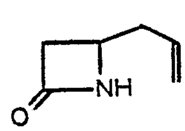

(XIX)

with a glyoxylic acid ester.

Normally this reaction is carried out in an inert solvent at an elevated temperature, for example in dry benzene under reflux.

The esters of the compounds of the formula (XVIII) may also be prepared by esterification of a salt of the compound of the formula (XVIII) in conventional manner. Suitable methods include the reaction of alkali metal salt such as a sodium or potassium salt with a reactive halide or sulphonate ester such as

a bromide, chloride, mesylate, tosylate or the like. Such esterifications may be carried out under conventional conditions, for example in dimethylformamide at room temperature.

The salt of compound of the formula (XVIII) may be prepared by neutralisation of the compound of the formula (XVIII) for example with an alkali metal carbonate or bicarbonate, for example sodium or potassium carbonate.

The compound of formula (XVIII) may be prepared by the reaction of glyoxylic acid with the compounds of the formula (XIX) as hereinbefore defined.

The esters of the compound of the formula (XI) may also be prepared by the ozonolysis of an ester of a compound of the formula (XX):

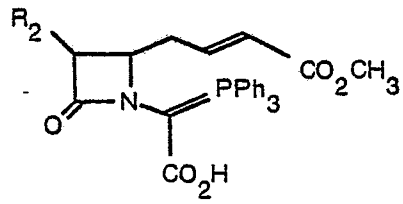

(XX)

wherein $R_2$ is as defined in relation to formula (II).

The ozonolysis is generally performed in the presence of trifluoroacetic acid in methylene chloride or ethyl acetate at −70°C.

The ester of the compound of the formula (XX) may be prepared via reaction of triphenylphosphine and the corresponding chloro compound which may in turn be prepared from the corresponding hydroxy compound which may be prepared from the N—H compound of the formula (XXI):

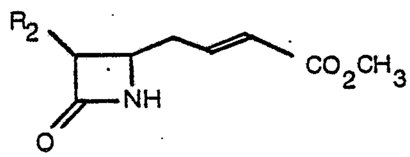

(XXI)

wherein $R_2$ is as defined in relation to formula (II). This sequence may be carried out in analogous manner to the analogous sequence as hereinbefore described.

The compound of the formula (XXI) may be prepared by the oxidation of a compound of the formula (XXII):

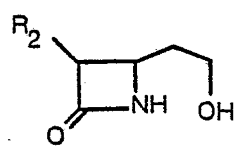

(XXII)

wherein $R_2$ is as defined in relation to formula (II) with pyridinium chlorochromate and thereafter reacting the thus produced aldehyde of the formula (XXIII):

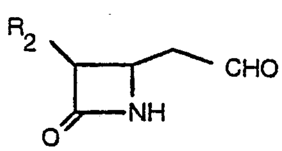

(XXIII)

with $Ph_3P{=}CHCO_2CH_3$.

The oxidation may be carried out in methylene chloride or the like at room temperature. When the oxidation is judged complete (for example by tlc) the reaction may be filtered and the phosphorane may be added to the filtrate for reaction.

The compound of the formula (XXII) may be prepared by the mild acid hydrolysis of a corresponding compound of the formula (XXVI):

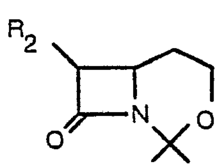

(XXIV)

or the 2,2′-spirocyclohexyl analogue thereof wherein $R_2$ is as defined in relation to formula (II).

The preceding hydrolysis may be carried out in aqueous acetone using small quantities of mineral or sulphuric acid.

The esters of the compounds of the formula (XI) wherein $R_2$ is a group of the formula $CH_2$—$CH{=}CHR_{14}R_{15}$ as defined in relation to formula (II) may be prepared from the corresponding compound wherein $R_2$ is a group $CH_2CHO$ by reaction with a Wittig reagent $R_{14}R_{15}C{=}PPh_3$ in conventional manner.

The ester of the compound of the formula (X) wherein $R_2$ is a $CH_2CHO$ group may be obtained by the ozonolysis of the corresponding ester of a compound of the formula (XXV):

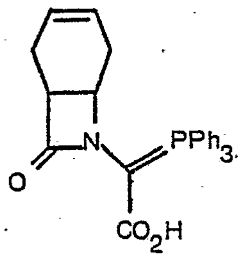

(XXV)

followed by ring closure of the resulting ester of the compound of the formula (XXVI):

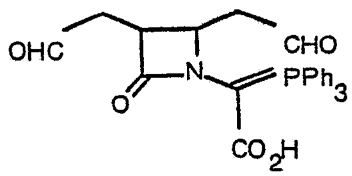

(XXVI)

under conditions similar to those outlined for the analogous conversions described hereinbefore.

The ester of the phosphorane of the formula (XXV) may be obtained from the corresponding chloride which may be obtained from the corresponding hydroxy compound which in turn may be obtained from the corresponding NH compound of the formula (XXVII):

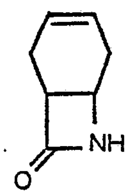

(XXVII)

by a reaction sequence similar to that described hereinbefore for analogous compounds.

The addition of a thiol $R_1SH$ to an ester of a compound of the formula (X) as hereinbefore defined can give rise to a number of isomers such as the esters of the isomers of the formulae (XXVIII)—(XXX):

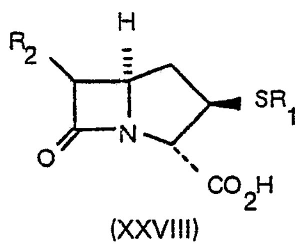

(XXVIII)

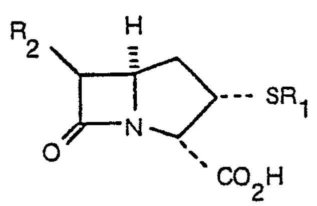

(XXIX)

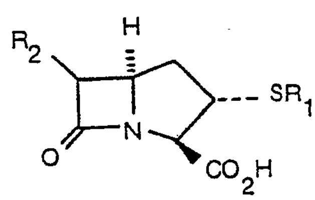

(XXX)

wherein $R_1$ and $R_2$ are as defined in relation to formula (II). The preceding isomers may be utilised in a mixture or they may be separated from each other by chromatography and utilised separately. Suitable chromatographic systems include column chromatography over silica eluting with ethyl acetate/petroleum ether mixtures.

An ester of the compound of the formula (XXX) may be converted to the corresponding ester of the compound of the formula (XXIX) by treatment with a strong base of low nucleophilicity such as 1,5-diazabicyclo[5.4.0]undec-5-ene or the like in ethyl acetate or dichloromethane.

The esters of the hereinbefore described compounds of type (VIII) which are cleavable by hydrogenation may be converted into the compound of the formula (VIII) or its salt by hydrogenation under similar conditions to these described for like reactions for like esters of the compounds of the formula (II). The salts of the compound of the formula (VIII) may be used to prepare esters of the compound of the formula (VIII) under similar conditions to those employed for the esterification of a salt of the compound of the formula (II).

The following may be consulted for the preparation of compositions and of intermediates of use for the preparation of compounds of this invention:

British Patent Publication No. 2013667
European Patent Application Publication No. 0002564
European Patent Application Publication No. 0000828
West German Application No. 2811514.2
Belgian Patent No. 860962
European Patent Application Publication No. 0008497

Unless stated otherwise all synthetic compounds described in the Examples are racemic. In order to define the relative stereochemistry of the assymmetric centres of the racemic compounds, the structural formulae depict one enantiomer thereof and indicate the absolute stereochemistry of the assymmetric carbon atoms for that enantiomer. The stereochemical momenclature refers to that enantiomer which is depicted, as do $^1H$ n.m.r. spectral assignments.

Florisil, Celite, High-Flo, Hy-Flo, Hiflo-Supercel, Microbandapak and Biogel referred to in the Examples are Trade Marks.

The compounds of the formula (III) may be obtained in their resolved forms by chemical or biological means at a suitable stage of the synthesis, for example see Scheme 3.

Scheme 3

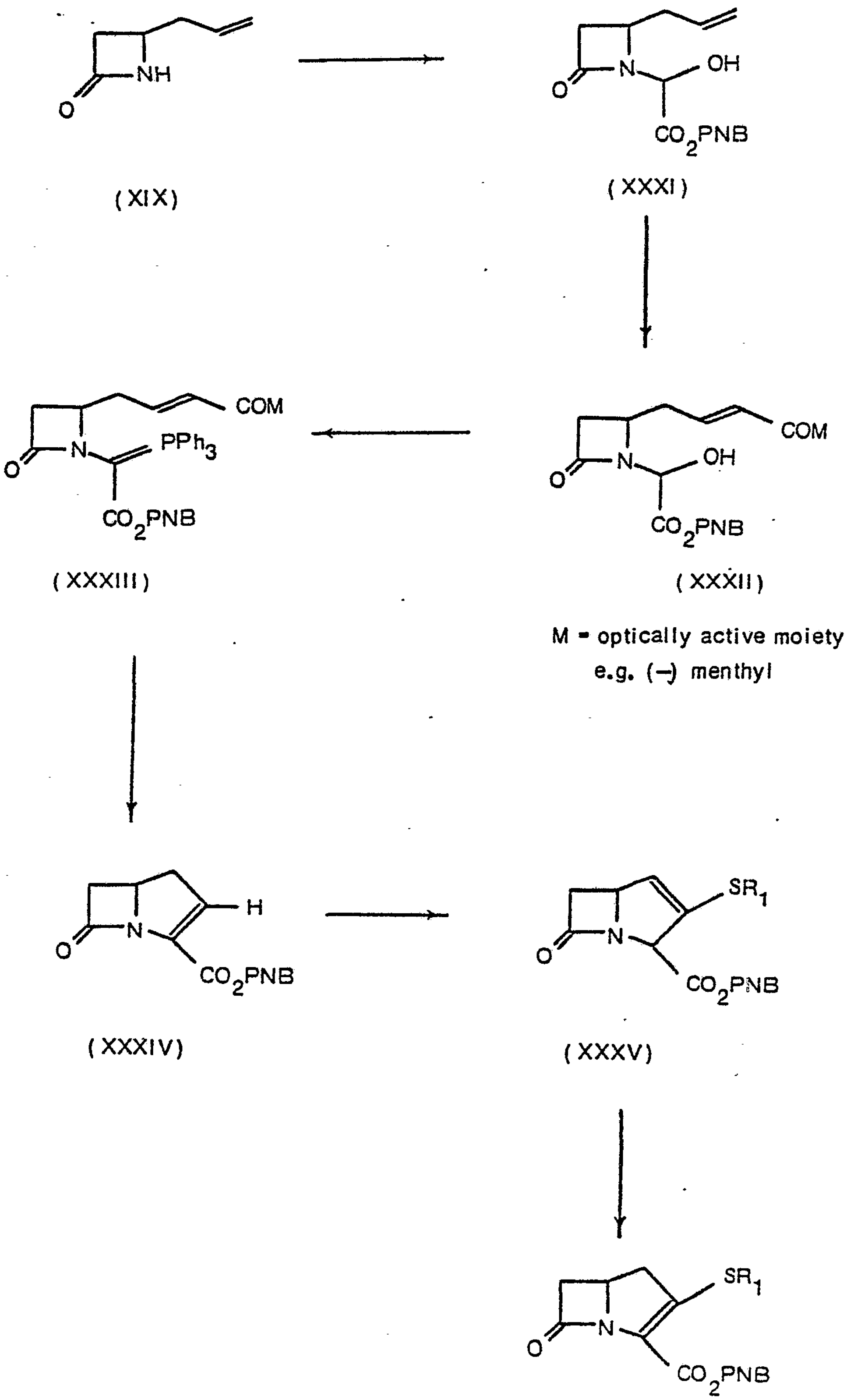

(XIX)

(XXXI)

(XXXIII)

(XXXII)

M = optically active moiety
e.g. (–) menthyl

(XXXIV)

(XXXV)

(XXXVI)

Resolution may be effected at an early stage of the synthesis as by so doing a common optically active intermediate such as that of the formula (XXXIV) is prepared which may be subjected to the hereinbefore described processes to provide the compounds of the formula (III) in resolved form.

A suitable stage for chemical resolution to be effected is that wherein a compound of the formula

(XIX) is separated into pure optical isomers, for example according to the process of British Patent No. 1273278.

A further suitable stage at which chemical resolution may be effected in that wherein the glyoxylate ester of the formula (XXXI) is treated sequentially with ozone, triphenylphosphine and an optically active phosphorane for example (—)carbomenthoxymethylenetriphenylphosphorane to afford the (+) and (—) isomers in resolved form.

A further suitable stage at which chemical resolution may be effected is that wherein the racemic compound of the formula (XXXV) is converted to a carboxylic acid or salt thereof, which is reacted with an optically active amine to form diastereoisomeric salts which are separated by crystallisation. These are independently re-esterified in conventional manner and isomerised to provide the optically active compound of the formula (III) for example the compound of the formula (XXXVI).

A suitable method for the resolution of compounds of the formula (III) by biological means is that of the dissolution of said compounds, when in the form of a phthalide ester, in mouse plasma. The phthalide ester of the desired isomer is hydrolysed to afford the biologically preferred salt of the compound of the formula (III).

This may be detected by h.p.l.c. assay using a reverse phase column eluting with an aqueous acetonitrile buffer of suitable pH linked with a u.v. detector monitoring at a suitable wavelength for example 300 nm.

Preferably the h.p.l.c. assay uses a $C_{18}$ reversed phase column eluting with 0.05 M ammonium phosphate buffer (pH 4.7) containing 5% acetonitrile. It has been found using this system that 3-ethylthio-7-oxo-1-azabicyclo[3.2.0]hept-2-ene-2-carboxylate has a retention time of 4.6 minutes with a flow rate of 3 ml $min^{-1}$. Collection of the appropriate fractions and solvent removal in conventional manner affords the active isomer of the formula (III).

The more desired isomers have the same stereochemistry at $C_5$ as (+)-p-nitrobenzyl 3-ethylthio-7-oxo-1-azabicyclo[3.2.0]hept-2-ene-2-carboxylate.

The following examples illustrate the invention.

Example 1

p-Nitrobenzyl 3-ethylthio-7-oxo-1-azabicyclo[3,2,0]hept-3-ene-2α-carboxylate

*Method (i)*

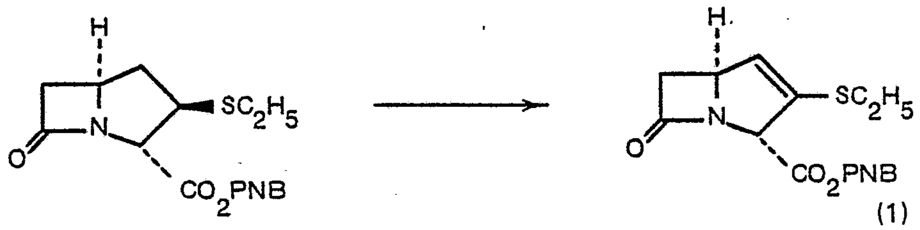

(1)

A solution of *p*-nitrobenzyl 3β-ethylthio-7-oxo-1-azabicyclo[3,2,0]heptane-2α-carboxylate (0.28 g) in sodium dried benzene (12 ml), under argon at ambient temperature was treated with dry pyridine (0.14 g). The solution was cooled to its freezing point and iodobenzene dichloride (0.24 g) was added in one portion. The mixture was stirred rapidly to give a clear solution which was stood in the refrigerator (+8°) for 2.25 hours. The reaction mixture was filtered through Kieselguhr to remove the solid which had come out of solution, and the orange filtrate was reduced in volume to about 2 ml, then chromatographed on silica 60 (230—400 Mesh) eluting with 50% ethyl acetate in petroleum ether 60°—80°, to give the product (1) (0.159 g) contaminated with a trace of starting material. Addition of ether gave (1) as white crystals (0.13 g) m.p. 82—82.5°, $\nu_{max}$ ($CHCl_3$) 1780, 1760, 1575, 1530, 1355 $cm^{-1}$; δ ppm ($CDCl_3$) 1.31 (3H, t, J 7Hz, $CH_3$), 2.83 (2H, q, J 7Hz, $CH_2CH_3$ + 1H, m, C6—H), 3.42 (1H, dd, J 16, 5Hz, C6—H), 4.54—4.70 (1H, m, C5—H), 5.17 (1H, dd, J 3.5, 1.5Hz); 5.30 (2H, s, $CH_2$ Ar), 5.80 (1H, d, d, J 1.5, 1.5Hz), 7.55 (2H, d, J 8.5Hz, Ar), 8.24 (2H, d, J 8.5Hz, Ar) (Found: $M^+$ at 348.0737. $C_{16}H_{16}N_2O_5S$ requires $M^+$ at 348.0779) (Found: C, 55.14; H, 4.80; N, 8.03%. $C_{16}H_{16}N_2SO_5$ requires C, 55.14; H, 4.60; N, 8.05%).

*Method (ii)*

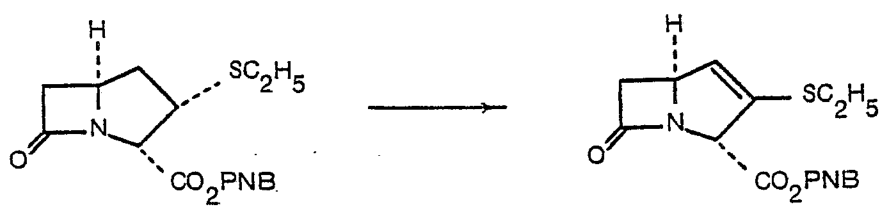

(1)

A solution of *p*-nitrobenzyl 3α-ethylthio-7-oxo-1-azabicyclo[3,2,0]heptane-2α-carboxylate (0.06 g) in dry benzene (3 ml), under argon at ambient temperature was treated with dry pyridine (0.03 g), cooled to its freezing point and treated with iodobenzene dichloride (0.051 g) as in (i). After standing at 8° for 2 hours the pale orange solution was decanted from the buff coloured solid and chromatographed on silica 60 (230—400 Mesh). Elution with 50% ethyl acetate in petroleum ether 60°—80° gave the product (1) initially as a gum (0.031 g) contaminated with traces of less polar materials. Trituration of this gum with ether gave (1) as a white crystalline solid (0.025 g).

*Method (iii)*

(Also isolating *p*-nitrobenzyl-3-ethylthio-7-oxo-1-azabicyclo[3,2,0]hept-2-ene-2-carboxylate)

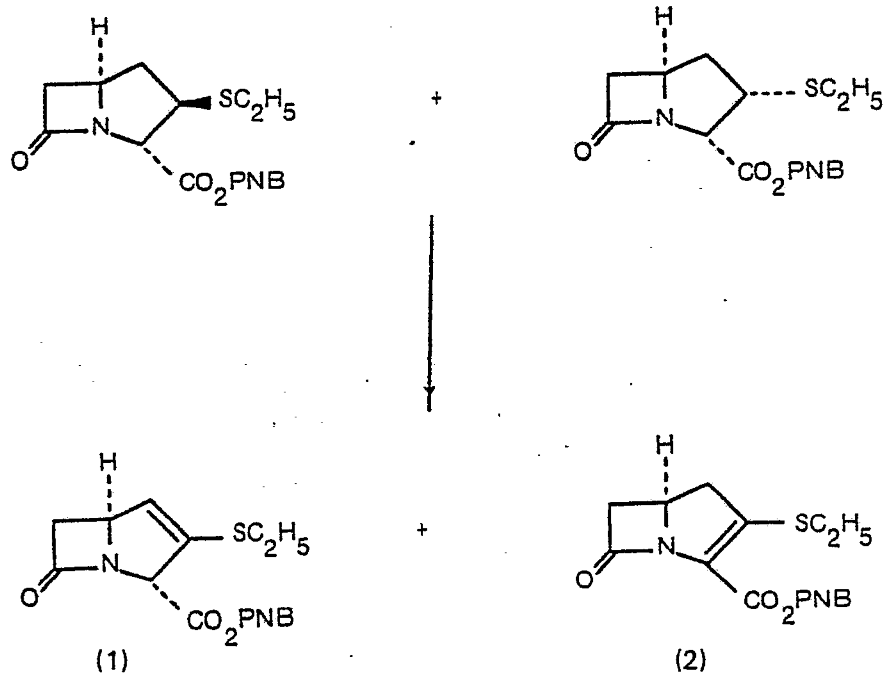

(1) (2)

a mixture of the 3β- and 3α-isomers (0.11 g, 2:1 ratio) in dry benzene (5 ml) was treated with pyridine (0.055 g) and iodobenzene dichloride (0.094 g) as in (ii). Chromatography as in (ii) gave (1) as a gum (0.042 g) contaminated with traces of the 3β-isomer and less polar material. Trituration with ether gave crystalline (1) (0.022 g). The mother liquors were shown by TLC to be predominantly (1).

Further elution gave 7 mg of material identical to (2) by u.v. TLC and i.r. (see Example 4).

*Method (iv)*

(Also isolating p-nitrobenzyl-4-chloro-3-ethylthio-7-oxo-1-azabicyclo[3,2,0]hept-3-ene-2α-carboxylate

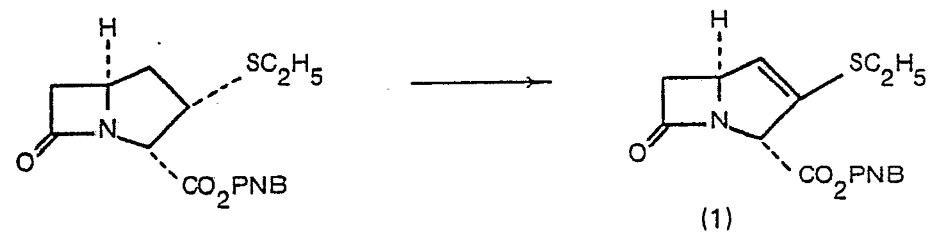

(1)

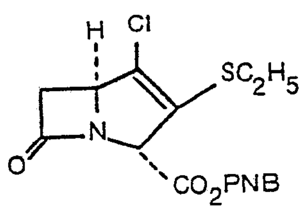

(3)

A solution of *p*-nitrobenzyl 3α-ethylthio-7-oxo-1-azabicyclo[3,2,0]heptane-2α-carboxylate (0.220 g) in chloroform (25 ml), which had been passed through basic alumina, under argon was treated with dry pyridine (0.15 ml). The solution, cooled in a bath at −20° was treated with

iodobenzene dichloride (0.347 g), added in one portion. The stirred mixture was allowed to reach 0° over 20 minutes and after a further 1.5 hours at 0° the brown reaction mixture obtained was reduced in volume and chromatographed on 20 g silica 60 (<230 Mesh), eluting with 30% ethyl acetate in peotroleum ether. The first product obtained was *p*-nitrobenzyl-4-chloro-3-ethylthio-7-oxo-1-aza-bicyclo[3,2,0]hept-3-ene-2α-carboxylate (3) (0.011 g) as white crystals ex. ether m.p. 114—119°, $\nu_{max}$ ($CHCl_3$) 1785, 1755, 1522, 1345 $cm^{-1}$; δ ppm ($CDCl_3$) 1.20 (3H, t, J 8Hz, $CH_3$), 2.80 (1H, q, J 8Hz) and 2.85 (1H, q, J 8Hz, $CH_2CH_3$), 3.00 (1H, dd, J 16.5, 2.5Hz, C6—H), 3.45 (1H, dd, J 16.5, 5Hz C6—H), 4.25 (1H, ddd, J 5, 2.5 2.5Hz, C5—H), 5.15 (1H, d, J 2.5Hz, C2—H), 5.26 (2H, s, $CH_2$ Ar), 7.48 (2H, d, J 8.5Hz, Ar), 8.25 (2H, d, J 8.5Hz, Ar) (Found: $M^+$ at 382.0396. $C_{16}H_{15}N_2O_5SCl$ requires $M^+$ at 382.0390).

The second product obtained was (1) (0.034 g).

*Method (v)*

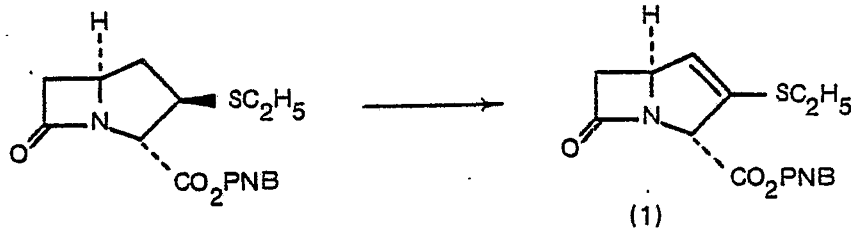

(1)

A solution of *p*-nitrobenzyl 3β-ethylthio-7-oxo-1-azabicyclo[3,2,0]heptane-2α-carboxylate (0.070 g) in dry benzene (5 ml), under argon at ambient temperature was treated with pyridine (0.018 g) followed by N-chlorosuccinimide (0.027 g). The clear solution obtained was stirred at ambient temperature when a gummy solid gradually appeared. After 1 hour the pale yellow solution was decanted off, evaporated and the residue chromatographed on 3 g silica 60 (230—400 Mesh) eluting with 50% ethyl acetate in petroleum ether to give the required product (1) (0.03 g), initially as a gum which crystallised from ether.

*Method (vi)*

The procedure was silimar to method (v) except that the N-chlorosuccinimide was added at the freezing point of the benzene solution and the clear solution obtained was stood in the refrigerator (+8°) for 24 hours, and then at room temperature for 4 hours. Work up as in (v) gave (1) as a crystalline solid (0.032 g).

*Method (vii)*

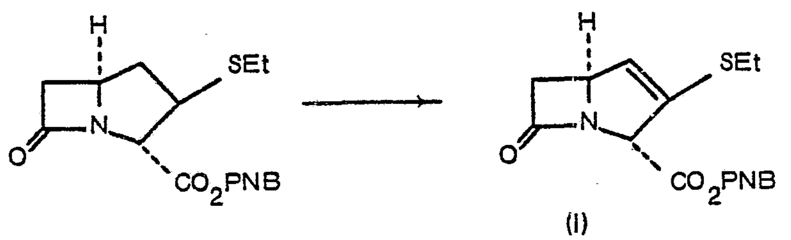

(I)

p-Nitrobenzyl 3β-ethylthio-7-oxo-1-azabicyclo[3.2.0]heptane-2α-carboxylate (80 mg) in dry methylene chloride (6 ml) was treated with dry pyridine (44 mg). The stirred solution under argon was cooled in a bath at 70° and then treated with a solution of chlorine (18 mg) in dry carbon tetrachloride (0.25 ml) added in one aliquot via a syringe through a septum. A clear colourless solution was immediately obtained and the reaction vessel was transferred to an ice-bath. After a further 20 minutes the reaction vessel was transferred to the refrigerator (+8°) for a further 1.5 hour, and then the still clear solution was reduced in volume for chromatography on silica gel (230—400 Mesh) (5.5 x 2 cm.). Elution with 50% ethyl acetate in petroleum ether (60°—80°) gave the required product (1) (42 mg).

Example 2

p-Nitrobenzyl-3-ethylthio-7-oxo-1-azabicyclo[3,2,0]hept-3-ene-2β-carboxylate

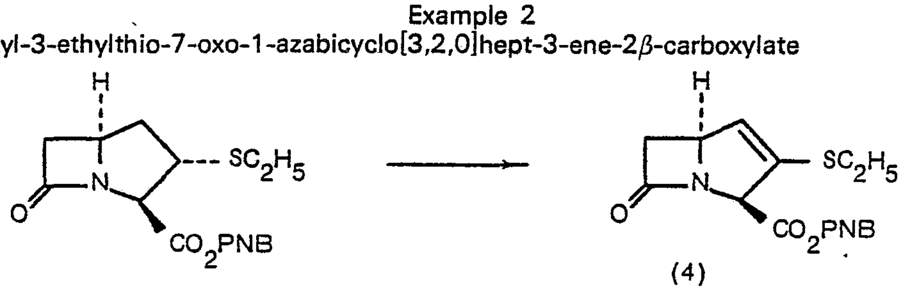

(4)

A solution of *p*-nitrobenzyl 3$\alpha$-ethylthio-7-oxo-1-azabicyclo[3,2,0]heptane-2$\beta$-carboxylate (0.07 g) in dry benzene (3 ml), under argon at ambient temperature was treated with dry pyridine (0.035 g). The stirred solution cooled to its freezing point was treated with iodobenzene dichloride (0.06 g), and after 10 minutes was stood in the refrigerator (+8°) for 2 hours. The brown solution was decanted from some solid and chromatographed on silica 60 (230—400 Mesh) eluting with 50% ethyl acetate in petroleum ether (60°—80°) to give (4), initially as a gum (0.03 g) which crystallised on trituration with ether. Recrystallisation of (4) from ethyl acetate/petroleum ether 60°—80° gave a micro-crystalline solid, m.p. 106—7°. $\nu_{max}$ ($CHCl_3$) 1778, 1750, 1575 (wk), 1522, 1350 $cm^{-1}$; $\delta$ ppm ($CDCl_3$) 1.30 (3H, t, J 7Hz, $CH_3$), 2.82 (2H, q, J 7Hz, $CH_2CH_3$), 2.93 (1H, d, d, J 15.5, 2.5Hz, C6—H), 3.18 (1H, ddd, J 15.5, 4.5, 1.5 Hz, C6—H), 4.34—4.62 (2H, C5—H and C2—H), 5.19 and 5.38 (2H, centres ABq, J 15 Hz, $CH_2$ Ar), 5.77 (1H, dd, J's <1.5Hz, collapsing to s on irradiation at $\delta$ 4.50, =CH), 7.55 (2H, d, J 8.5Hz, Ar), 8.50 (2H, d, J 8.5Hz, Ar) (Found: C, 54.95; H, 4.63; N, 7.92%. $C_{16}H_{16}N_2SO_5$ requires: C, 55.17; H, 4.60; N, 8.05%).

Further elution gave recovered starting-material (10 mg) followed by a trace of material identical by TLC and u.v. to the 1-azabicyclo[3.2.0]hept-2-ene (2) (See Example 4).

## Example 3

Conversion of p-nitrobenzyl 3$\alpha$-ethylthio-7-oxo-1-azabicyclo[3,2,0]-heptane-2$\beta$-carboxylate to its 2$\alpha$ isomer

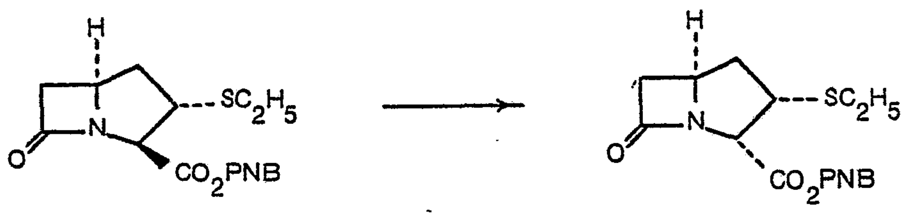

p-Nitrobenzyl 3$\alpha$-ethylthio-7-oxo-1-azabicyclo[3,2,0]heptane-2$\beta$-carboxylate (0.048 g) in ethyl acetate (2 ml) was treated with 1,5-diazabicyclo[5.4.0]undec-5-ene (D.B.U.) (0.0048 g). The solution darkened. After leaving overnight at ambient temperature the solution was diluted with ethyl acetate, washed with water, brine, dried over magnesium sulphate, filtered and evaporated. The residue, shown by TLC to contain more 2$\beta$-carboxylate than 2$\alpha$-carboxylate was taken up in dry methylene chloride (2 ml) and treated with D.B.U. (0.004 g). After standing for 5 hours at ambient temperature the solution was diluted with more methylene chloride, washed with water, then brine, filtered through "Phase-Sep" paper and evaporated to a gum (0.029 g), identical to the 2$\alpha$ isomer by TLC and NMR.

## Example 4

p-Nitrobenzyl 3-ethylthio-7-oxo-1-azabicyclo[3,2,0]hept-2-ene-2-carboxylate

*Method (i)*

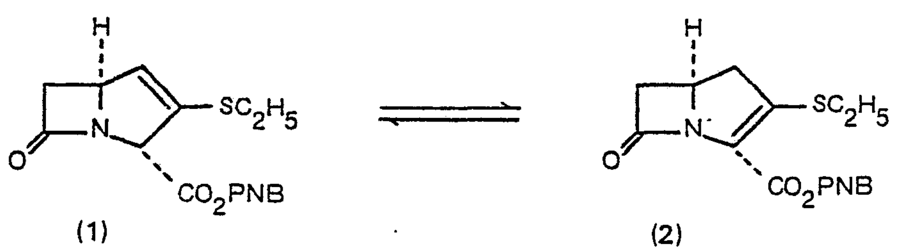

(1) (2)

*p*-Nitrobenzyl 3-ethylthio-7-oxo-1-azabicyclo[3,2,0]hept-3-ene-2$\alpha$-carboxylate (1) (0.208 g) in dry methylene chloride (10 ml) at ambient temperature was treated with D.B.U. (0.034 g, 2 drops) for 2.5 hours. The yellow solution was washed with brine, filtered through "Phase-Sep" paper and evaporated. Chromatography on 15 g Florisil (200—300 Mesh), eluting with 50% ethyl acetate in petroleum ether 60°—80°, gave recovered starting material (1) (0.10 g) followed by the required product (2) as yellow crystals (0.06 g) (ex. ether). Recrystallisation of (2) from ethyl acetate/petroleum ether 60°—80°, gave yellow plates m.p. 134.5—139°, $\nu_{max}$ ($CHCl_3$) 1780, 1700 (broad, weak), 1520, 1345 $cm^{-1}$; $\lambda_{max}$ (ethanol) 320 nm, ($\varepsilon$ 12,200) 268 nm ($\varepsilon$ 10,300); $\delta$ ppm ($CDCl_3$) 1.32 (3H, t, J 7.5Hz, $CH_3$), 2.85 (2H, q, J 7.5Hz, $CH_2CH_3$), 2.93 (1H, dd, J 16, 2.5 Hz; C6—H), 2.99 (1H, dd, J 18, 8.5Hz, C4—H), 3.28 (1H, dd, J 18, 8.5Hz, C4—H), 3.49 (1H, dd, J 16, 5.5Hz, C6—H), 4.23 (1H, tdd, J 8.5, 5.5, 2.5Hz, C5—H), 5.22 and 5.48 (2H, centres ABq, J 14Hz, $CH_2$ Ar), 7.62 (2H, d, J 8Hz, Ar), 8.20

(2H, d, J 8Hz, Ar) (Found: C, 54.93; H, 4.77; N, 7.96%. $C_{16}H_{16}N_2SO_5$ requires C, 55.17; H, 4.60; N, 8.05%).

*Method (ii)*

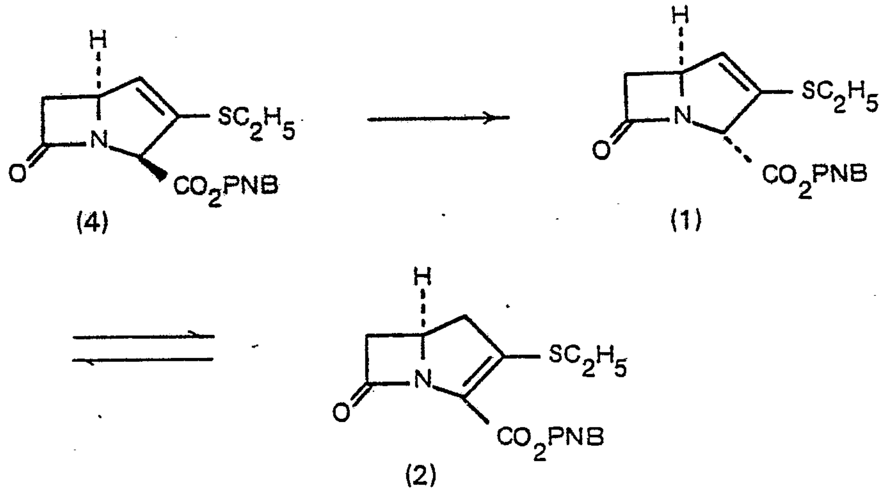

(4) (1)

(2)

*p*-Nitrobenzyl 3-ethylthio-7-oxo-1-azabicyclo[3,2,0]hept-3-ene-2β-carboxylate (4) (4 mg) in dry methylene chloride (10 drops) was treated with D.B.U. (0.1 ml of a solution of 1 drop in 5 ml methylene chloride). TLC after 5 minutes showed the complete conversion of the 2β-isomer (4) to the 2α-isomer (1) and after 4 hours an approximately 2:1 ratio of (1):(2) could be observed.

Example 5

Sodium 3-ethylthio-7-oxo-1-azabicyclo[3,2,0]hept-3-ene-2α-carboxylate

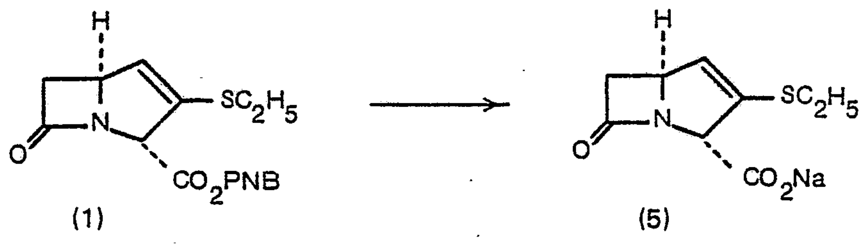

(1) (5)

*Method (i)*

A catalyst of 5% palladium on charcoal (Engelhard 4526, 55 mg) suspended in 2:1 dioxan:water (6 ml) was prehydrogenated for 20 minutes at room temperature/atmospheric pressure. A solution of *p*-nitrobenzyl 3-ethylthio-7-oxo-1-azabicyclo[3,2,0]hept-3-ene-2α-carboxylate (1) (50 mg) in dioxan (2 ml) was added to the catalyst mixture. After hydrogenation for 40 minutes sodium bicarbonate (12 mg) dissolved in a minimum of water was added and the catalyst removed by filtration through "High-Flo". The filtrate was evaporated to remove dioxan and the cloudy aqueous phase washed with ethylacetate (X3), then evaporated almost to dryness. Re-evaporation from ethanol (X3) then toluene (X3) gave the required salt (5) as a pale yellow solid (24 mg), $\nu_{max}$ (KBr) 1762, 1612 $cm^{-1}$; δ ppm ($D_2O$) 1.25 (3H, t, J 7.5Hz, $CH_3$), 2.85 (2H, q, J 7.5Hz, $CH_2CH_3$), 2.86 (1H, dd, J 2.5, 17Hz, C6—H), 3.40 (1H, dd, J 5, 17Hz, C6—H), 4.80 (1H centre of m, C2—H), 5.85 (1H, C4—H), C5—H obscured by $H_2O$ at δ 4.6 ppm.

*Method (ii)*

A catalyst of 5% palladium on charcoal (Engelhard 4526, 400 mg) suspended in 2:1 dioxan:water (45 ml) was pre-hydrogenated at ambient temperature and pressure for 20 minutes. A solution of the ester (1) (400 mg) in dioxan (30 ml) was added to the catalyst mixture along with sodium bicarbonate (94.5 mg) dissolved in water (2 ml). After hydrogenation for one hour at ambient temperature and pressure a further 150 mg of catalyst was added, and hydrogenation continued for 30 minutes. The mixture was filtered through Celite and the filtrate worked up as in i) to give the required salt (5) as a white solid (243 mg).

*Method (iii)*

The ester (1) (240 mg) in dioxan (20 ml) and sodium bicarbonate (57.5 mg) in water (9 ml) were added to prehydrogenated 5% palladium on charcoal (288 mg) in 2:1 dioxan:water (24 ml). After

hydrogenation at ambient temperature and pressure for 1 hour 15 minutes, work up as in ii) gave the salt (5) as an off-white solid (162 mg).

Example 6

Sodium 3-ethylthio-7-oxo-1-azabicyclo[3,2,0]hept-3-ene-2β-carboxylate

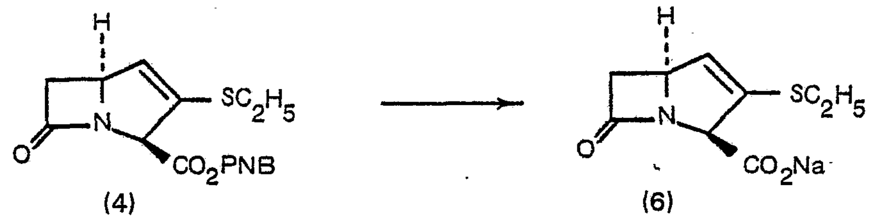

(4) (6)

A catalyst of 5% palladium on charcoal (28 mg), suspended in 2:1 dioxan-water (3 ml) was prehydrogenated for 20 minutes at room temperature/atmospheric pressure. A solution of *p*-nitrobenzyl 3-ethylthio-7-oxo-1-azabicyclo[3,2,0]hept-3-ene-2β-carboxylate (4) (25 mg) in dioxan (2 ml) was added to the catalyst mixture. Hydrogenation and work-up as in Example 5; gave the salt (6) as a pale yellow solid (6 mg) $\nu_{max}$ (KBr), 1760—1750, 1620—1610 $cm^{-1}$; δ ($D_2O$) 1.29 (3H, t, J 7.5Hz, $CH_3$), 2.65—3.40 [4H, including:— 2.87 (2H, q, J 7.5Hz, $CH_2CH_3$) +2C6—H], 5.88 (1H, broad s, =CH), C2, C5—H obscured by $H_2O$ at 4.5—4.6 ppm.

Example 7

Sodium 3-ethylthio-7-oxo-1-azabicyclo[3,2,0]hept-2-ene-2-carboxylate

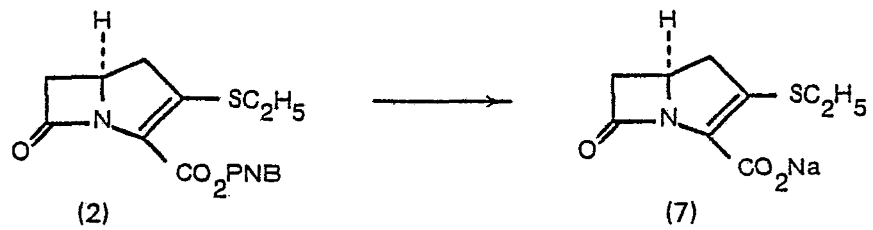

(2) (7)

A catalyst of 5% palladium on charcoal (73 mg), suspended in 2:1 dioxan-water (10 ml) was prehydrogenated for 20 minutes at room temperature/atmospheric pressure. A solution of *p*-nitrobenzyl 3-ethylthio-7-oxo-1-azabicyclo[3,2,0]hept-2-ene-2-carboxylate (2) (60 mg) in dioxan (4 ml) was added to the catalyst mixture. After hydrogenation for 3 hours at room temperature/atmospheric pressure, sodium bicarbonate (14.4 mg) dissolved in a minimum of water was added and the catalyst removed by filtration through "High-Flo", washing with water. The filtrate was evaporated to remove dioxan and the cloudy yellow aqueous phase shaken with ethyl acetate. An emulsion formed which was broken up by centrifugation. The separated aqueous solution was re-washed with ethyl acetate, then reduced in volume by evaporation and chromatographed on Bio-Gel P2, eluting with neutralised de-ionised water containing 1% n-butanol. The fractions containing the required product (7) could be detected by u.v. ($\lambda_{max}$ 295 n.m.). Evaporation of these fractions to reduced volume followed by re-evaporation from ethanol (X3) then toluene gave the title salt (7) as a pale yellow hgroscopic solid (20 mg), $\nu_{max}$ (KBr) 1750 (broad), 1600 $cm^{-1}$; $\lambda_{max}$ (ethanol) 295 nm.

Example 8

Phthalidyl 3-ethylthio-7-oxo-1-azabicyclo[3.2.0]hept-3-ene-2α-carboxylate

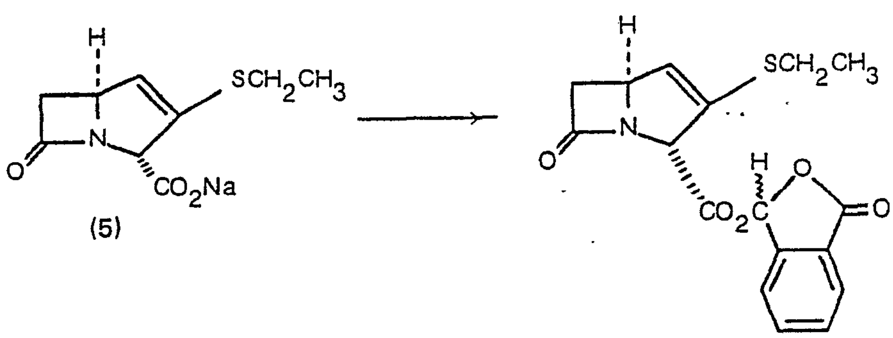

(5) (8)

Sodium 3-ethylthio-7-oxo-1-azabicyclo[3.2.0]hept-3-ene-2α-carboxylate (5) (114 mg) prepared as in Example 5(i) in dry dimethylformamide (3 ml) was treated with bromophthalide (103 mg). After

stirring at ambient temperature for 1.5 hours the reaction mixture was diluted with ethyl acetate (100 ml), washed with water, brine, dried over magnesium sulphate, filtered and evaporated. Chromatography on silica gel (<230 mesh) (6 g) eluting with 30% ethyl acetate in petroleum ether (60°—80°) gave a gum (108 mg) shown by the n.m.r. spectrum to consist mainly of the required ester (8). The isomers of (8) were present in almost equal amounts and had $\nu_{max}$ ($CH_2Cl_2$) 1790, 1780 $cm^{-1}$; $\delta$ ppm ($CDCl_3$) 1.28 and 1.30 (3H, 2 triplets, J 7.5Hz, $CH_3$), 2.81 and 2.82 (2H, 2 quartets, J 7.5Hz, $CH_2CH_3$), 2.83 (1H, dd, J 16 and 2.5Hz, C6—H), 3.39 (1H, dd, J 16 and 5.5Hz, C6—H), 4.60 (1H, centre of m; C5—H), 5.12 (1H, dd, J 3.0 and 2Hz, C2—H), 5.80 (1H, dd, J. 3.0 and 2Hz, C4—H), 7.40 (1H, s, $CO_2CH$), 7.40—8.00 (4H, Ar) (Found: $M^+$ at 345.0667 $C_{17}H_{15}NO_5S$ requires $M^+$ at 345.0669).

Example 9

Phthalidyl 3-ethylthio-7-oxo-1-azabicyclo[3.2.0]hept-2-ene-2-carboxylate

*Method (i)*

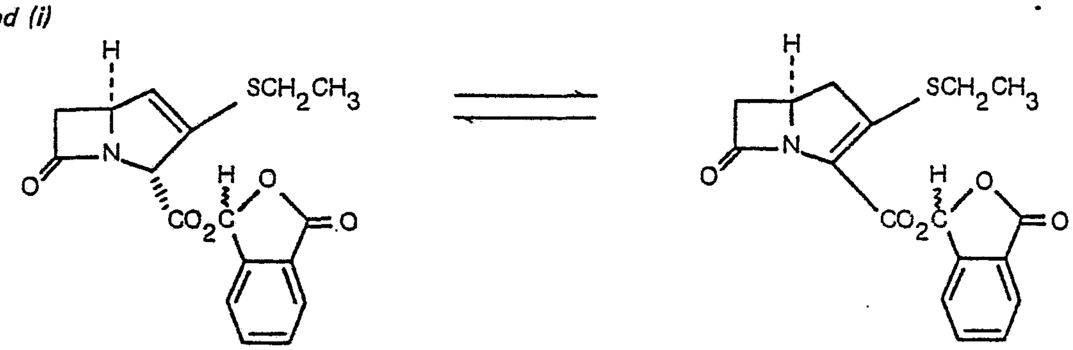

(8) (9)

Phthalidyl 3-ethylthio-7-oxo-1-azabicyclo[3.2.0]hept-3-ene-2$\alpha$-carboxylate (8) (90 mg) in dry methylene chloride (4 ml) was treated with D.B.U. (17 mg). After standing at ambient temperature for 2.5 hours the yellow solution was washed with brine, filtered through "Phase-Sep" paper and evaporated. Chromatography on 5 g silica gel (230—400 Mesh) eluting with 50% ethyl acetate in petroleum ether (60°—80°) followed by neat ethyl acetate gave in the first fractions recovered starting material (8) as a gum (21 mg). The later fractions contained the required product (9) contaminated with a small amount of starting material (8) (T.L.C. Analysis). Trituration of the gum obtained from these later fractions with ether gave the two phthalide esters of (9) as a white solid (22.5 mg). The ethereal washings on evaporation gave a further 6.7 mg of recovered starting material (8).

On other occasions the phthalide esters (9) were separated on chromatography. The least polar isomer was obtained as white crystals (ex ethyl acetate) m.p. 175—185° dec. $\lambda_{max}$ (ethanol) 329 ($\varepsilon$, 13,500), 274 ($\varepsilon$ 2,780) and 230 ($\varepsilon$, 11,800) nm; $\nu_{max}$ ($CHCl_3$) 1790 (strong), 1720 $cm^{-1}$; $\delta$ ppm ($CDCl_3$) 1.35 (3H, t, J 7.5Hz, $CH_3$), 2.88 (2H, q, J 7.5Hz, $CH_2CH_3$), 2.90 (1H, dd, J 16 and 2.5Hz, C6—H), 2.98 (1H, dd, J 18 and 8Hz, C4—H), 3.32 (1H, dd, J 18 and 8.5Hz, C4—H), 3.45 (1H, dd, J 16 and 6Hz), 4.00—4.35 (1H, m, C5—H), 7.44 (1H, s, O—CH—O), 7.37—7.90 (4H, Ar) (Found: $M^+$ at 345.0665. $C_{17}H_{15}NO_5S$ requires $M^+$ at 345.0669). (Found: C, 59.08; H, 4.42; N, 3.99% $C_{17}H_{15}NO_5S$ requires: C, 59.13; H, 4.35; N, 4.06%). The more polar isomer of (9) was obtained as white crystals (ex ethyl acetate) m.p. 172—5°, $\lambda_{max}$ (ethanol) 328 ($\varepsilon$, 10,470), 282 ($\varepsilon$, 2,400), 230 ($\varepsilon$, 10,050) nm; $\nu_{max}$ ($CHCl_3$) 1790 (stront), 1720 $cm^{-1}$; $\delta$ ppm ($CDCl_3$) 1.30 (3H, t, J 7.5Hz, $CH_3$), 2.84 (2H, q, J 7.5Hz, $CH_2CH_3$), 2.88 (1H, dd, J 16 and 3Hz, C6—H), 2.99 (1H, dd, J 18 and 8.5Hz, C4—H), 3.28 (1H, dd, J 18 and 8.5Hz, C4—H), 3.45 (1H, dd, J 16 and 5.5Hz, C6—H), 4.00—4.35 (1H, m, C5—H), 7.49 (1H, s, O—CH—O) 7.40—7.97 (4H, m, Ar) (Found: C, 58.77; H, 4.26; N, 4.01%. $C_{17}H_{15}NO_5S$ requires C, 59.13; H, 4.35; N, 4.06%).

*Method (ii)*

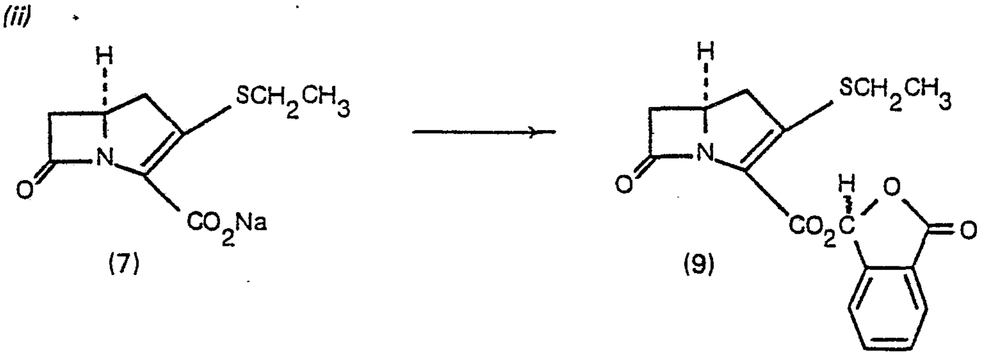

(7) (9)

Sodium 3-ethylthio-7-oxo-1-azabicyclo[3.2.0]hept-2-ene-2-carboxylate (7) (5.6 mg) was taken

up in dry dimethylformamide (2 ml) and treated with bromophthalide (5 mg). After stirring at ambient temperature for 1.5 hours the reaction mixture was diluted with ethyl acetate, washed with water, brine and filtered through 'Phase-Sep' paper. The filtrate was evaporated then chromatographed on 2 g Florisil (200—300 Mesh), eluting with 50% ethyl acetate in petroleum ether (60°—80°) followed by ethyl acetate to give the phthalide esters (9) as a white solid (ex ether) (2.8 mg) identical by T.L.C. u.v. and I.R. to the product from (i).

Example 10

Pivaloyloxymethyl 3-ethylthio-7-oxo-1-azabicyclo[3.2.0]hept-3-ene-2$\alpha$-carboxylate

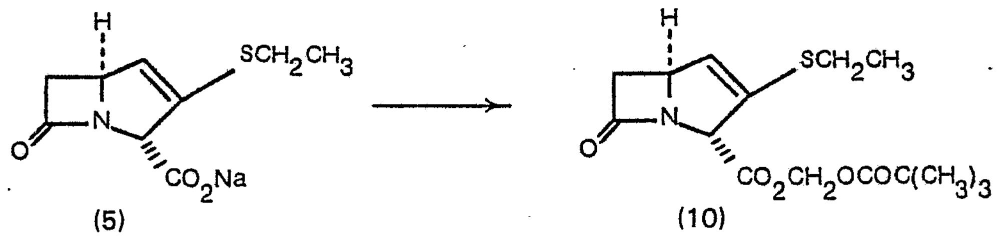

(5) (10)

Sodium 3-ethylthio-7-oxo-1-azabicyclo[3.2.0]hept-3-ene-2$\alpha$-carboxylate (5) (162 mg), prepared as in Example (5 iii), in dry dimethylformamide (5 ml) was treated with bromomethyl pivalate (134 mg). After stirring for 30 minutes at ambient temperature the yellow reaction solution was diluted with ethyl acetate, washed with water, brine, dried over magnesium sulphate and filtered. The filtrate was evaporated to dryness, then taken up in toluene and chromatographed on silica gel (230—400 M, 4 x 3 cm). Elution with 30% ethyl acetate in petroleum ether (60°—80°) gave the required product (10) as a gum (89 mg), $\nu_{max}$ ($CHCl_3$) 1778, 1765, 1575 $cm^{-1}$; $\delta$ ppm ($CDCl_3$) 1.21 (9H, s, ${}^tBu$), 1.30 (3H, t, J 7.3Hz, $CH_2CH_3$), 2.81 (2H, q, J 7.3Hz, $CH_2CH_3$), 2.83 (1H, dd, J 16.1 and 2.8Hz, 1 C6—H), 3.38 (1H, dd, J 16.1 and 5.4 Hz, 1 C6—H), 4.60 (1H, m, collapsing to ddd, J 5.4, 3.2 and 2.8Hz on irradiation at $\delta$ 5.80, C5—H), 5.09 (1H, dd, J 3.2 and 2.0Hz, C2—H), 5.79 (3H, $OCH_2O$ and C4—H) (Found: $M^+$ at 327.1135 $C_{15}H_{21}NO_5S$ requires $M^+$ at 327.1140).

Example 11

Pivaloyloxymethyl 3-ethylthio-7-oxo-1-azabicyclo[3.2.0]hept-2-ene-2-carboxylate

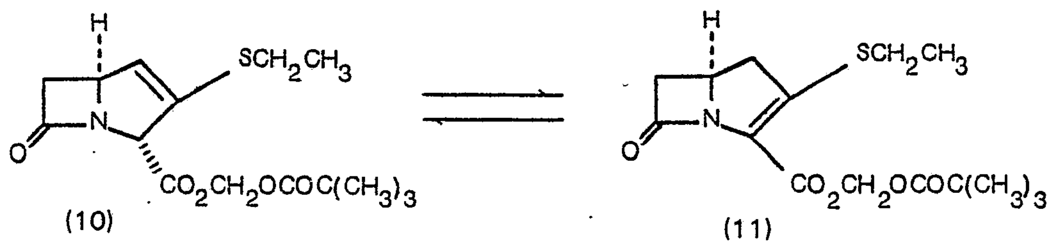

(10) (11)

Pivaloyloxymethyl 3-ethylthio-7-oxo-1-azabicyclo[3.2.0]hept-3-ene-2$\alpha$-carboxylate (10) (77 mg) in dry methylene chloride (4 ml) was treated with D.B.U. (11 mg). After standing at ambient temperature for 3 hours the yellow solution was chromatographed on silica gel (230—400 M, 5 x 2 cm). Elution with 50—70% ethyl acetate in petroleum ether (60°—80°) gave recovered starting material (10) (43 mg) followed by the required product (11) as a gum (20 mg) $\lambda_{max}$ (ethanol 323 nm; $\nu_{max}$ ($CHCl_3$) 1779, 1745, 1710, 1540 $cm^{-1}$; $\delta$ ppm ($CDCl_3$), 1.21 (9H, s, ${}^tBu$), 1.32 (3H, t, J 7Hz, $CH_2CH_3$), 2.86 (2H, q, J 7Hz, $CH_2CH_3$), 2.88 (1H, dd, J 16 and 2.5Hz, 1 C6—H), 2.98 (1H, dd, J 18 and 8.5Hz, 1 C4—H), 3.28 (1H, dd J 18 and 8.5Hz, 1 C4—H), 3.47 (1H, dd, J 16 and 5Hz, 1 C6—H), 4.20 (1H, tdd, J 8.5, 5 and 2.5 Hz, C5—H), 5.83 and 5.94 (2H, ABq, J 5Hz, $OCH_2O$).

Example 12

Conversion of p-nitrobenzyl 3$\alpha$-(2-p-nitrobenzyloxycarbonylaminoethylthio)-7-oxo-1-azabicyclo-[3.2.0]heptane-2$\beta$-carboxylate to its 2$\alpha$ isomer

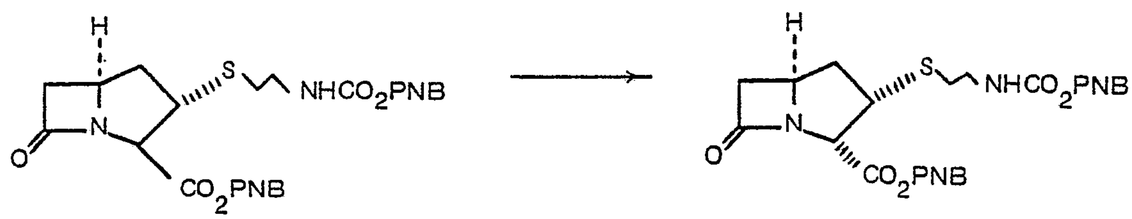

p - Nitrobenzyl 3$\alpha$ - (2 - p - nitrobenzyloxycarbonylaminoethylthio) - 7 - oxo - 1 - azabicyclo[3.2.0]-heptane-2$\beta$-carboxylate (157 mg) in dry methylene chloride (5 ml) was treated with D.B.U. (17.5 mg). After standing at room temperature for 4 hours the solution was reduced in volume by evaporation and chromatographed on 10 g silica gel (<230 Mesh). Elution with 50—60% ethyl acetate in petroleum ether (60°—80°) gave the 2$\alpha$ isomer as a gum (116 mg), $\nu_{max}$ ($CHCl_3$) 3450, 1770, 1745 (wk), 1730, 1522, 1350 $cm^{-1}$; $\delta$ ppm ($CDCl_3$) 1.63 (1H, ddd, J 12, 8 and 7.5Hz, 1 C4—H), 2.48—2.95 (4H, overlapping m's, 1 C6—H, side chain $CH_2$, 1 C4—H), 3.21—3.47 (3H, overlapping m's, side chain $CH_2$, 1 C6—H), 3.66—3.97 (2H, overlapping m's, C3—H, C5—H), 4.36 (1H, d, J 6Hz, C2—H), 5.17 and 5.25 (4H, 2 singlets, benzyl $CH_2$'s) obscuring 1H, N*H*; 7.45 and 7.49 (4H, 2d's, J 8Hz, Ar) 8.15 and 8.18 (4H, 2 doublets, J 8Hz, Ar) (Found: m/e at 391 $M^+$ -p-nitrobenzylalcohol).

## Example 13

p-Nitrobenzyl 3-(2-p-nitrobenzyloxycarbonylaminoethylthio)-7-oxo-1-azabicyclo[3.2.0]hept-3-ene-2$\alpha$-carboxylate

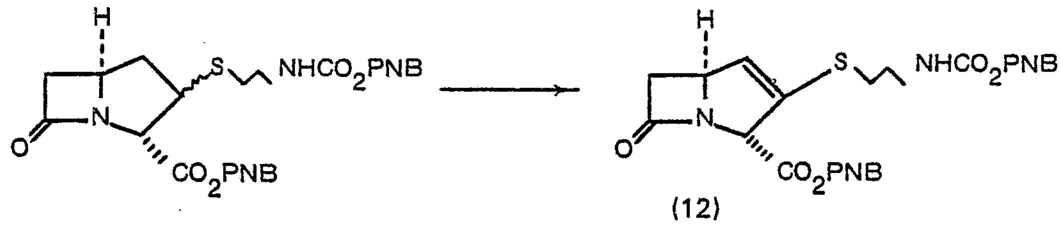

(12)

A mixture of the 3$\alpha$ and 3$\beta$ isomers of p-nitrobenzyl 3-(2-p-nitrobenzyloxycarbonylaminoethylthio)-7-oxo-1-azabicyclo[3.2.0]heptane-2$\alpha$-carboxylate (545 mg) in dry benzene (20 ml) was treated with pyridine (175 mg). The stirred colourless solution under argon was cooled to its freezing point and then iodobenzene dichloride (300 mg) was added in one portion. After stirring vigorously for a few minutes the clear yellow solution was stood at +8°. After thirty minutes the benzene solution was decanted from the solid which had come out of solution and placed on a column of 20 g silica gel (230—300 Mesh), followed by the solid taken up in dry methylene chloride. Elution of the column with 50% ethyl acetate in petroleum ether (60°—80°) gave the required product (12) initially as a sticky foam (218 mg) which crystallised from ethyl acetate/petroleum ether (60°—80°). A further 30 mg of the product (12) contaminated by a trace of starting material was then obtained, followed by almost pure starting material (16 mg).

The product (12) had m.p. 11.5—112.5°, $\nu_{max}$ ($CH_2Cl_2$) 3450, 1780, 1750, 1730, 1575 (wk), 1520, 1345 $cm^{-1}$; $\delta$ ppm ($CDCl_3$) 2.70—3.18 [3H, including 2.84 (1H, dd J 16 and 2.5Hz, C6—H) + sidechain $CH_2$], 3.40 (1H, dd, J 16 and 5Hz, C6—H), 3.37 (2H, t, J 6Hz, sidechain $CH_2$) 4.50—4.68 (1H, m, C5—H), 5.10—5.30 [6H, including 5.19 and 5.26 (4H, 2 singlets, benzyl $CH_2$'s), NH and C2—H obscured], 5.98 (1H, C4—H), 7.48—7.50 (4H, 2d, J 8Hz, Ar), 8.18 (4H, 2 d, J 8Hz, Ar) (Found:— C, 53.18; H, 4.16; N, 10.24% $C_{24}H_{22}N_4SO_9$ requires: C, 53.14; H, 4.06; N, 10.33%).

## Example 14

p-Nitrobenzyl 3-(2-p-nitrobenzyloxycarbonylaminoethylthio)-7-oxo-1-azabicyclo[3.2.0]hept-2-ene-2-carboxylate

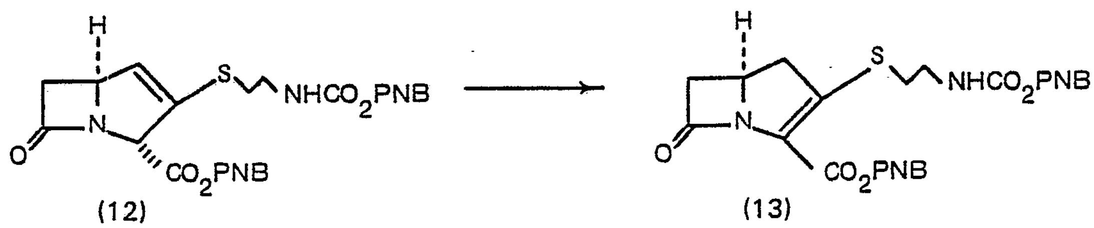

(12) (13)

p-Nitrobenzyl 3-(2-p-nitrobenzyloxycarbonylaminoethylthio)-7-oxo-1-azabicyclo[3.2.0]hept-3-ene-2$\alpha$-carboxylate (12) (200 mg) in dry methylene chloride (10 ml) was treated with D.B.U. (30 mg). After standing at room temperature for 2 hours the dark solution was reduced in volume and chromatographed on 15 g silica gel (230—400 Mesh). Elution with 50—60% ethyl acetate in petroleum ether (60°—80°) gave recovered starting material (12) (100 mg). Further elution with ethyl acetate gave the required product (13) as a white solid (50 mg) after washing with ether. The product (13) was obtained as white crystals (ex ethyl acetate) m.p. 154—9°, $\lambda_{max}$ (ethanol) 314 nm ($\varepsilon$, 9,800) and 265 nm.($\varepsilon$ 15,500); $\nu_{max}$ ($CHCl_3$) 3450, 1782, 1725, 1520, 1350 $cm^{-1}$; $\delta$ ppm ($CDCl_3$) 2.75—3.65 [8H, including 2.91 (1H, dd, J 17 and 3Hz, C6—H), 3.36 (2H, t, J 6Hz, sidechain $CH_2$), + 1C6—H, C4—$H_2$ and sidechain $CH_2$], 4.00—4.38 (1H, m, C5—H), 5.00—5.55 [5H, including 5.16 (2H, s, $NHCO_2CH_2$), 5.20 and 5.46 (2H, ABq, J 14 Hz, $\alpha\beta$ unsaturated $CO_2CH_2$), NH obscured] 7.45 and 7.65 (4H, 2d's, J 8Hz,

Ar), 8.18 (4H, 2d's, J 8Hz, Ar) (Found: C, 53.51; H, 3.98; N, 10.44% $C_{24}H_{22}N_4SO_9$ requires: C, 53.14; H, 4.06; N, 10.33%).

Example 15

3-Aminoethylthio-7-oxo-1-azabicyclo[3.2.0]hept-2-ene-2-carboxylic acid

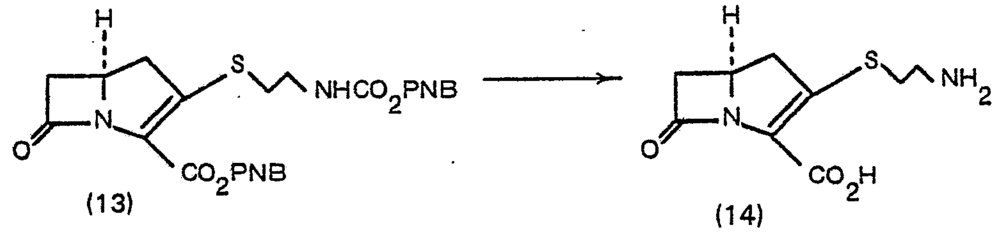

(13) (14)

p-Nitrobenzyl 3-(2-p-nitrobenzyloxycarbonylaminoethylthio)-7-oxo-1-azabicyclo[3.2.0]hept-2-ene-2-carboxylate (13) (50 mg) was taken up in dioxan (9 ml) heating slightly to give a clear solution. To this solution was added ethanol (0.5 ml), de-ionised water (1.6 ml), M/20 pH7 phosphate buffer (2 ml and 10% palladium on charcoal (50 mg). The mixture was hydrogenated at ambient temperature and pressure for 2.75 hours, and the catalyst then removed by filtration through 'HY—FLO', washing with water (10 ml). The clear filtrate was extracted with ether (5 x 10 ml). After reducing the aqueous phase by evaporation to about 5 ml it was chromatographed on Biogel P—2 (20 x 130 mm) eluting with de-ionised water. The fractions were examined by u.v. Those containing chromophores at 295 and 243 n.m. were combined, reduced in volume and re-chromatographed on XAD—2 (200 x 13 mm) eluting with de-ionised water. The earlier fractions which absorbed at 253 n.m. were discarded. Two intermediate fractions, which although absorbing at 293 nm did not appear by u.v. to be as pure as the later fractions were kept to one side for rechromatography. The last fractions $\lambda_{max}$ ($H_2O$) (295 nm) were calculated to contain 2.8 mg of the required product (14) assuming $\varepsilon_{max}$ 10,000; $\delta$ ppm ($D_2O$) showed a complex pattern of signals between 3.00 and 5.10.

Example 16

Conversion of p-nitrobenzyl 3$\alpha$-(2-triphenylmethylaminoethylthio)-7-oxo-1-azabicyclo[3.2.0]heptane-2$\beta$-carboxylate to its 2$\alpha$ isomer

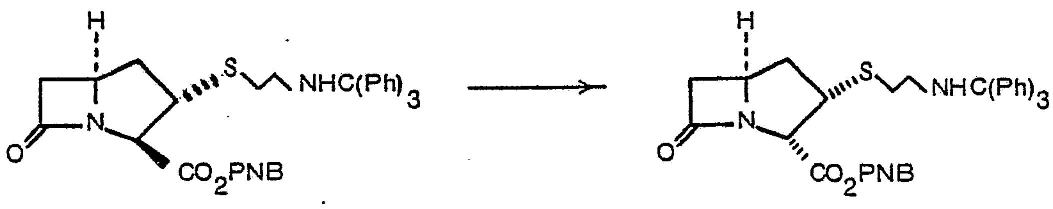

p-Nitrobenzyl 3$\alpha$-(2-triphenylmethylaminoethylthio)-7-oxo-1-azabicyclo[3.2.0]heptane-2$\beta$-carboxylate (64 mg) in dry methylene chloride (2 ml) was treated with D.B.U. (6 mg). After leaving overnight at ambient temperature the solution was chromatographed on silica gel (<230 Mesh) (15 g) eluting with 30—40% ethyl acetate in petroleum ether (60°—70°) to give the 2$\alpha$ isomer as a gum (38 mg).

Example 17

p-Nitrobenzyl 3-(2-triphenylmethylaminoethylthio)-7-oxo-1-azabicyclo[3.2.0]hept-3-ene-2$\alpha$-carboxylate

*Method (i)*

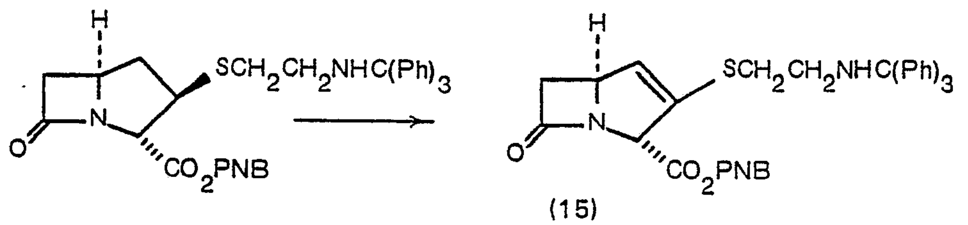

(15)

p-Nitrobenzyl 3$\beta$-(2-triphenylmethylaminoethylthio)-7-oxo-1-azabicyclo[3.2.0]heptane-2$\alpha$-carboxylate (186 mg) in dry benzene (5 ml) was treated with dry pyridine (53.5 mg). The stirred solution under argon was cooled to its freezing point and then iodobenzene dichloride (93 mg) was

added in one portion with vigorous stirring to give a clear solution which was stood at +8. After 1.5 hours the solution was decanted off from some solid which had come out of solution, then chromatographed on silica gel (<230 Mesh, 10 g). Elution with 20% to 30% ethyl acetate in petroleum ether (60°—80°) gave a gum (97 mg) from which the required product (15) was obtained as white crystals (71 mg) [Ex ethyl acetate/petroleum ether (60°—80°)]. Recrystallisation gave a micro-crystalline solid m.p. 167—9°, $\nu_{max}$ ($CHCl_3$) 1778, 1755, 1575 (wk), 1522, 1345 $cm^{-1}$; the n.m.r. spectrum showed 0.5 M ethyl acetate, $\delta$ ppm ($CDCl_3$) inter alia 1.82 (1H, broad signal exch. $D_2O$, NH), 2.41 (2H, t, J 6Hz, sidechain $CH_2$), 2.72 (1H, dd, J 16 and 2.5Hz, C6—H), 2.93 (2H, t, J 6Hz, sidechain $CH_2$), 3.33 (1H, dd, J 16 and 5Hz, C6—H), 4.42—4.60 (1H, m, C5—H, 5.11 (1H, dd, J 2.5 and 1.5Hz, C2—H), 5.20 (2H, s, benzyl $CH_2$), 5.68 (1H, dd, J 1.5 and 1.5Hz, C4—H), 7.10—7.60 (17H, Ar), 8.14 (2H, d, J 8.5Hz, p-nitrobenzyl Ar) (Found: C, 68.44; H, 5.42; N, 6.86% $C_{35}H_{31}N_3O_5S$. $\frac{1}{2}$ EtOAC requires C, 68.41; H, 5.39; N, 6.47%).

*Method (ii)*

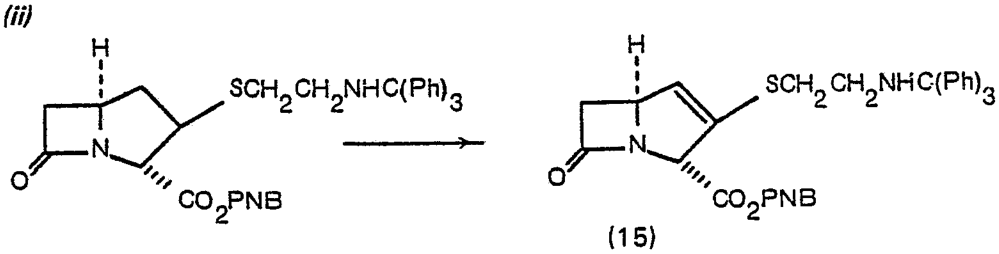

(15)

In a similar manner to that described in (17 i) p-nitrobenzyl 3$\alpha$-(2-triphenylmethylaminoethylthio)-7-oxo-1-azabicyclo[3.2.0]heptane-2$\alpha$-carboxylate (561 mg) prepared as in Example (16) was converted to the required product (15) (97 mg).

Example 18

p-Nitrobenzyl 3-(2-triphenylmethylaminoethylthio)-7-oxo-1-azabicyclo[3.2.0]hept-2-ene-2-carboxylate

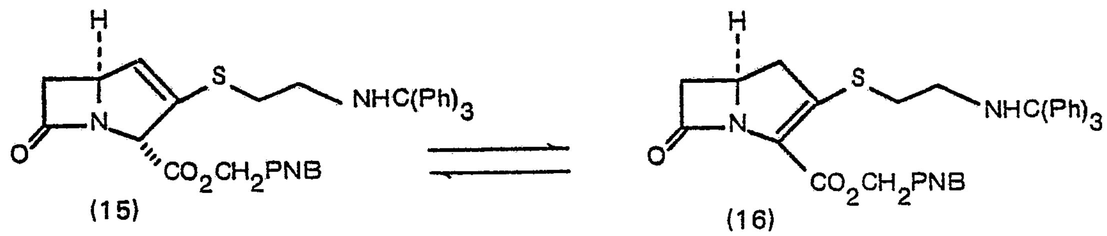

(15) (16)

p-Nitrobenzyl 3-(2-triphenylmethylaminoethylthio)-7-oxo-1-azabicyclo[3.2.0]hept-3-ene-2$\alpha$-carboxylate (15) (175 mg) in dry methylene chloride (10 ml) was treated with D.B.U. (13 mg). After standing at ambient temperature for three hours the solution was washed with water then brine, filtered through Phase-Sep paper, reduced in volume by evaporation and chromatographed on Florisil (200—300 Mesh, 10 g). Elution with 30% ethyl acetate in petroleum ether (60°—80°) gave recovered (15) (88 mg) followed by the required product (16). Some (16) crystallised out of the chromatography fractions and was obtained with 1 molar equivalent or ethyl acetate of crystallisation as fine white needles (7.8 mg) m.p. 97—103°, $\lambda_{max}$ (ethanol) 322 ($\varepsilon$ 12,100) and 266 ($\varepsilon$ 10,800) nm; $\nu_{max}$ ($CHCl_3$) 1780, 1700 (weak, broad), 1522, 1350 $cm^{-1}$; $\delta$ ppm ($CDCl_3$) inter alia 1.65 (1H, broad signal exch. $D_2O$, NH), 2.43 (2H, t, J 6Hz, sidechain $CH_2$), 2.65—3.65 [6H, including 2.95 (2H, t, J 6Hz, sidechain $CH_2$), 3.48 (1H, dd, J 16 and 5.5Hz, C6—H) C4—$H_2$ and 1 C6—H partially obscured], ca 4.10 (1H, m, partially obscured by EtOAC, C5—H), 5.20 and 5.46 (2H, ABq, J 14Hz, benzyl $CH_2$), 7.05—7.70 (17H, Ar), 8.15 (2H, d, J 8Hz, p-nitrobenzyl Ar) (Found: C, 67.53; H, 5.33; N, 6.56% $C_{35}H_{31}N_3SO_5$. $C_4H_8O_2$ requires C, 67.53; H, 5.63; N, 6.06%).

A further 10.9 mg of (16) was obtained as a white solid on evaporation of the mother liquors.

Example 19

p-Nitrobenzyl 3-(2-methoxycarbonylethylthio)-7-oxo-1-azabicyclo[3.2.0]heptane-2-carboxylate

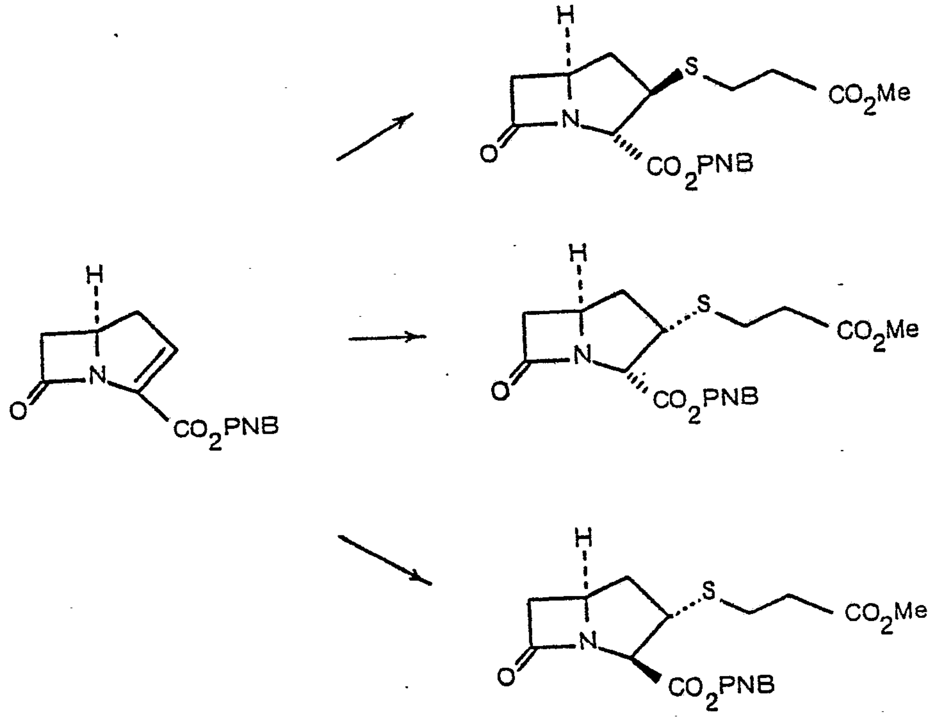

(17)

(18)

(19)

To p-nitrobenzyl 7-oxo-1-azabicyclo[3.2.0]hept-2-ene-2-carboxylate (518 mg) in dry dimethylformamide (10 ml) was added with stirring methyl 3-mercaptopropionate (216 mg) followed by potassium carbonate (25 mg). The starting material dissolved almost immediately. Stirring was continued at ambient temperature and after 45 minutes a further two drops of the thiol were added. After a total of 1 hour the yellow solution was diluted with ethyl acetate, washed with water, dried over magnesium sulphate and filtered. The filtrate was evaporated to dryness then chromatographed on silica gel (<230 Mesh). Elution with 30—50% ethyl acetate in petroleum ether (60°—80°) gave the products (17), (18) and (19).

The first fractions contained some of the least polar isomer (17) as a gum (66 mg), $\nu_{max}$ ($CHCl_3$), 1768, 1745, 1522, 1348 $cm^{-1}$; $\delta$ ppm ($CDCl_3$) 2.18 (2H, dd, J 9 and 6Hz, C4—$H_2$), 2.38—2.95 [5H, including 2.72 (1H, dd, J 16 and 3Hz, C6—H) and (4H, m, $SCH_2CH_2$)], 3.36 (1H, dd, J 16 and 5Hz, C6—H), 3.62 (1H, td, J 9 and 7Hz, C3—H), 3.66 (3H, s, Me), 4.10 (1H, tdd, J 6, 5 and 3Hz, C5—H), 4.78 (1H, d, J 7Hz, C2—H), 5.25 (2H, s, benzyl $CH_2$), 7.53 and 8.20 (4H, 2d's, J 8Hz, Ar) (Found: $M^+$ at 408.0978 $C_{18}H_{20}N_2O_7S$ requires $M^+$ at 408.0991).

The next fractions contained a mixture of isomers (17) and (18) as a gum (339 mg). The ratio of (17) to (18) was estimated to be 13:8 from the n.m.r. spectrum. [See Example (20) for the data on a pure sample of the C-2$\alpha$, 3$\alpha$ isomer (18)].

The final fractions gave the C-2$\beta$, 3$\alpha$ isomer (19) as white crystals (ex ether) (244 mg). A portion of (19) was recrystallised from ethyl acetate/petroleum ether (60°—80°) to give crystals m.p. 113—115°, $\nu_{max}$ ($CHCl_3$) 1765, 1740, 1522, 1345 $cm^{-1}$; $\delta$ ppm ($CDCl_3$) 1.90 (1H, ddd, J 12, 12 and 10Hz, 1 C4—H), 2.14—2.93 [6H including 2.31 (1H, ddd, J 12, 6 and 6Hz, C4—H), and (5H, overlapping m's, 1 C6—H and $SCH_2CH_2$)], 3.13 (1H, dd, J 16 and 4Hz, 1 C6—H), 3.67 (3H, s, Me) — partially obscuring 3.60—3.93 (2H, overlapping m's, C5 and C3—H's), 4.16 (1H, d, J 7Hz, C2—H), 5.29 (2H, S, benzyl $CH_2$), 7.57 and 8.21 (4H, 2d's, J 8Hz, Ar) (Found: C, 52.90; H, 4.98; N, 6.76% $C_{18}H_{20}N_2O_7S$ requires C, 52.94; H, 4.90; N, 6.86%).

Example 20

The conversion of p-nitrobenzyl 3α-(2-methoxycarbonylethylthio)-7-oxo-1-azabicyclo[3.2.0]heptane-2β-carboxylate to its C-2α isomer

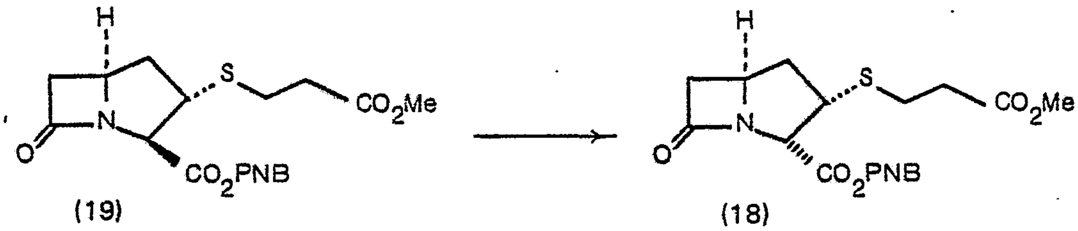

(19) (18)

p-Nitrobenzyl 3α-(2-methoxycarbonylethylthio)-7-oxo-1-azabicyclo[3.2.0]heptane-2β-carboxylate (19) (102 mg) was treated with D.B.U. (7.5 mg) in dry methylene chloride (4 ml). After 5 hours at ambient temperature the solution was chromatographed on silica gel (<230 Mesh). Elution with 30—50% ethyl acetate in petroleum ether (60°—80°) gave the required isomer (18) as a gum (91 mg) $\nu_{max}$ ($CHCl_3$) 1768, 1742, 1522, 1348 $cm^{-1}$; δ ppm ($CDCl_3$) 1.62 (1H, ddd, J 13.5, 9 and 8Hz, 1 C4—H), 2.46—2.90 (6H, overlapping m's, $SCH_2CH_2$, 1 C4—H and 1 C6—H), 3.31 (1H, dd, J 16 and 4.5Hz, 1 C6—H), 3.66 (3H, s, Me) partially obscuring 3.63—3.98 (2H, overlapping m's, C3 and C5—H's), 4.36 (1H, d, J 6.5Hz, C2—H), 5.27 (2H, s, benzyl $CH_2$), 7.52 and 8.20 (4H, 2d's, J 9Hz, Ar) (Found: $M^+$ at 408.1000 $C_{18}H_{20}N_2O_7S$ requires $M^+$ at 408.0989.

Example 21

p-Nitrobenzyl 3-(2-methoxycarbonylethylthio)-7-oxo-1-azabicyclo[3.2.0]hept-3-ene-2α-carboxylate

*Method (i)*

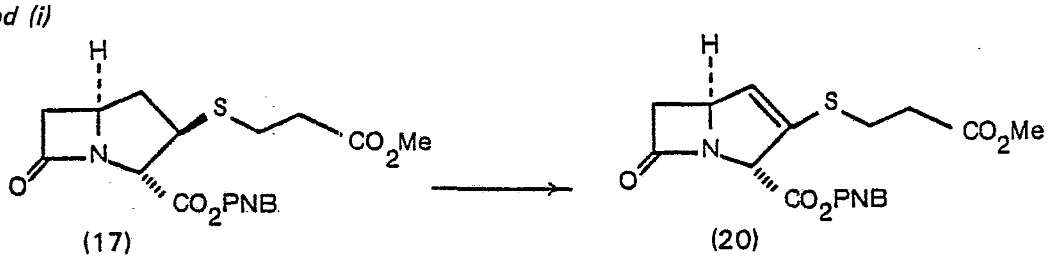

(17) (20)

p-Nitrobenzyl 3β-(2-methoxycarbonylethylthio)-7-oxo-1-azabicyclo[3.2.0]heptane-2α-carboxylate (17) (73 mg) was taken up in dry benzene and the solution obtained evaporated to dryness. The residual gum was then dissolved in benzene (5 ml) containing pyridine (35 mg). The stirred solution, under argon, was cooled to its freezing point and iodobenzene dichloride (60 mg) was added in one portion to give a pale yellow solution. This solution was transferred to the refrigerator (+8°). After 10 minutes some white crystals were observed to have come out of the solution which by now was colourless, and after a further 20 minutes the reaction mixture was stood at room temperature for 15 minutes. The benzene solution was decanted off and put on a column of silica gel (230—400 Mesh, 6 g). The residual crystals dissolved in chloroform were placed on the same column. Elution with 50% ethyl acetate in petroleum ether (60°—80°) gave the required product (20) as white crystals (ex. ether) (27 mg). Recrystallization from ethyl acetate/petroleum ether (60°—80°) gave white needles m.p. 94.5—97.5°, $\nu_{max}$ ($CHCl_3$) 1778, 1750 (shoulder), 1740, 1570 (wk), 1522, 1348 $cm^{-1}$; δ ppm ($CDCl_3$) 2.50—3.15 [5H, including 2.83 (1H, dd, J 16 and 3Hz, 1 C6—H), and (4H, m, $SCH_2CH_2$)], 3.40 (1H, dd, J 16 and 5Hz, 1 C6—H), 3.68 (3H, s, Me), 4.59 (1H, dddd, J 5, 3, 3 and 2Hz, C5—H), 5.14 (1H, dd, J 3 and 2Hz, C2—H), 5.26 (2H, s, benzyl $CH_2$), 5.85 (1H, dd, J 2 and 2Hz, C4—H), 7.51 and 8.21 (4H, 2d's, J 9Hz, Ar) (Found:— C, 53.11; H, 4.49; N, 6.80% $C_{18}H_{18}N_2O_7S$ requires C, 53.20; H, 4.43; N, 6.90%).

*Method (ii)*

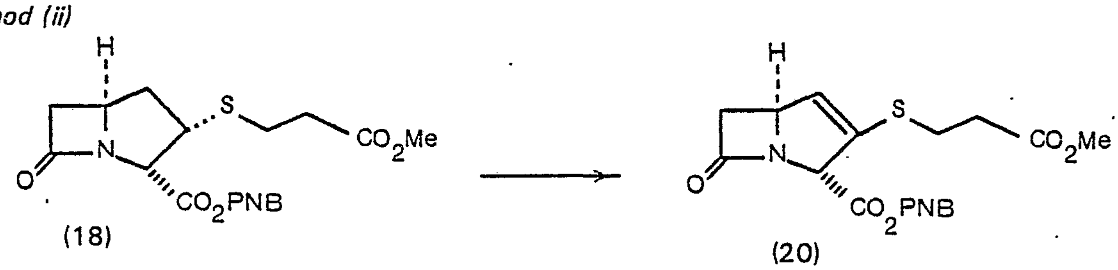

(18) (20)

In a similar manner to that described in Example (21 i) p-nitrobenzyl 3α-(2-methoxycarbonyl-

ethylthio)-7-oxo-1-azabicyclo[3.2.0]heptane-2α-carboxylate (18) (77 mg) prepared as in Example (20) was converted to (20) (40 mg).

Example 22

p-Nitrobenzyl 3-(2-methoxycarbonylethylthio)-7-oxo-1-azabicyclo[3.2.0]hept-2-ene-2-carboxylate

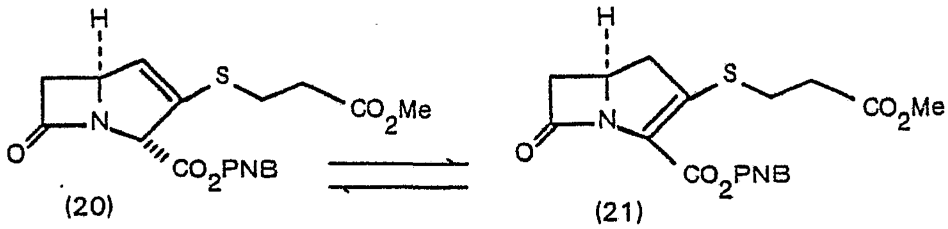

(20) (21)

p-Nitrobenzyl 3-(2-methoxycarbonylethylthio)-7-oxo-1-azabicyclo[3.2.0]hept-3-ene-2α-carboxylate (20) (161 mg) in dry methylene chloride (10 ml) was treated with D.B.U. (18.1 mg). The yellow solution obtained was stood at ambient temperature for 2.5 hours then reduced in volume by evaporation and chromatographed on florisil (200—300 Mesh) (10 g). Elution with 50% ethyl acetate in petroleum ether (60°—80°) gave recovered (20) (71 mg) followed by the required product (21) as a yellow solid (40 mg) (ex ether). Recrystallisation of this solid from ethyl acetate/petroleum ether (60°—80°) gave crystals m.p. 117—119.5°, $\lambda_{max}$ (ethanol) 316 (ε, 13,200) and 265 (ε, 11,300) nm; $\nu_{max}$ ($CHCl_3$) 1780, 1738, 1700 (shoulder), 1520, 1345 $cm^{-1}$; δ ppm ($CDCl_3$) 2.63 (2H, t, J 6Hz, sidechain $CH_2$), 2.80—3.65 [6H, including 2.94 (1H, dd, J 16 and 3Hz, 1 C6—H), 3.50 (1H, dd, J 16 and 5Hz, 1 C6—H), partially obscuring (4H, overlapping m's, sidechain $CH_2$ and C4—$H_2$)], 2.70 (3H, s, Me), 4.23 (1H, tdd, J 8.5, 5 and 3Hz, C5—H), 5.22 and 5.48 (2H, ABq, J 13Hz, benzyl $CH_2$), 7.62 and 8.20 (4H, 2d's J 8Hz, Ar) (Found: C, 52.96; H, 4.48; N, 6.83% $C_{18}H_{18}N_2O_7S$ requires C, 53.20; H, 4.43; N, 6.90%).

Example 23

Potassium 3-(2-methoxycarbonyl ethylthio)-7-oxo-1-azabicyclo[3.2.0]hept-2-ene-2-carboxylate

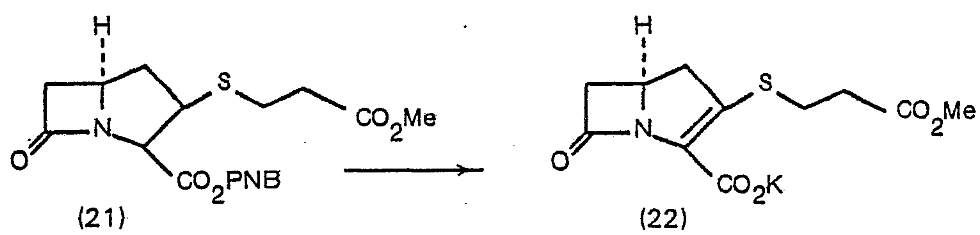

(21) (22)

To p-nitrobenzyl 3-(2-methoxycarbonylethylthio)-7-oxo-1-azabicyclo[3.2.0]hept-2-ene-2-carboxylate (21) (42 mg) in dioxan (6 ml)/de-ionised water (1.6 m) and 0.05 M pH7 phosphate buffer (2.07 ml) was added a catalyst of 10% palladium on charcoal (84 mg). The mixture was hydrogenated for 4 hours at ambient temperature and pressure. After filtration through Celite washing with water, ethyl acetate was added to the yellow filtrate. The emulsion obtained was broken down by centrifugation and the aqueous phase separated off, rewashed with ethyl acetate, then reduced in volume by evaporation and chromatographed on a column of XAD—2 (22 × 1 cm), eluting with de-ionised water. The fractions were examined by u.v. and those containing the required salt (22) $\lambda_{max}$ (297 nm) were combined. The yield of (22) was estimated to be 3.3 mg assuming $\varepsilon_{max}$ of 10,000 at 297 nm.

Example 24

p-Nitrobenzyl 3-(2-hydroxyethylthio)-7-oxo-1-azabicyclo[3.2.0]heptane-2-carboxylate

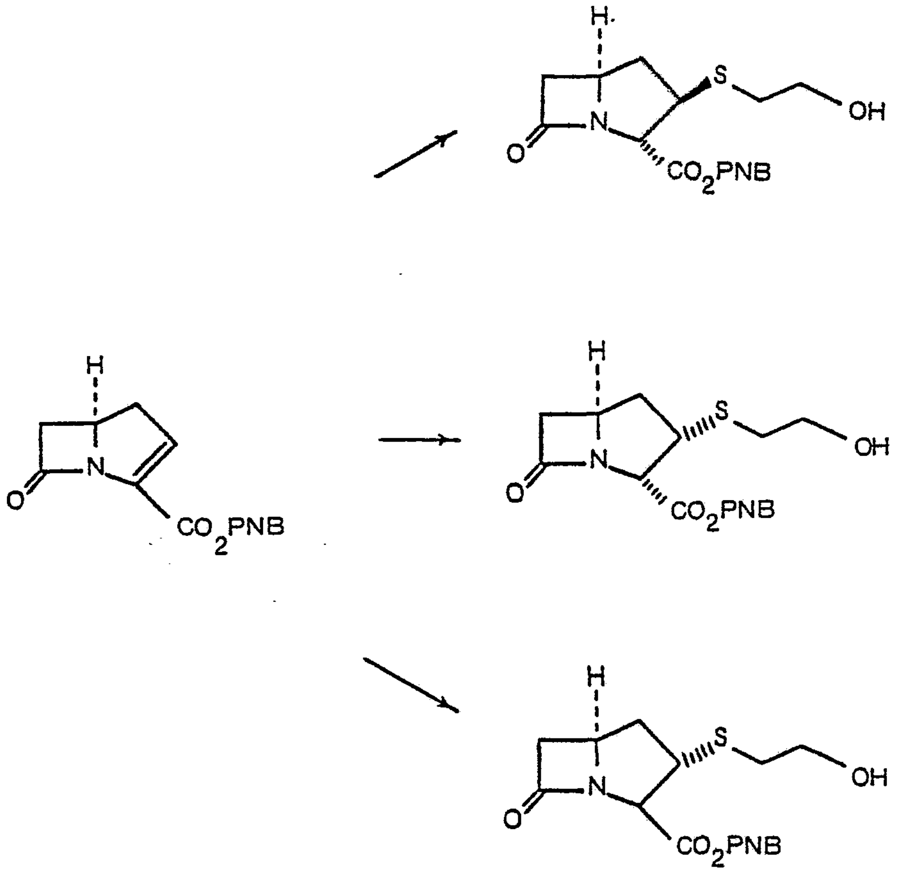

(23)

(24)

(25)

To p-nitrobenzyl 7-oxo-1-azabicyclo[3.2.0]hept-2-ene-2-carboxylate (576 mg) in dry dimethylformamide was added with stirring 2-mercaptoethanol (172 mg) followed by potassium carbonate (28 mg). After stirring at ambient temperature for 45 minutes the red reaction solution was reduced in volume by evaporation, diluted with ethyl acetate (100 ml), washed with water, brine, dried over magnesium sulphate, filtered and evaporated to dryness. The residual oil was chromatographed on silica gel (<230 Mesh, 20 g), eluting with 50% to 70% ethyl acetate in petroleum ether (60°—80°) followed by neat ethyl acetate to give a total yield of 555 mg of the three isomers (23), (24) and (25) of the required product. A mixture of the least polar isomers (23) and (24) was obtained from the first fractions as an oil (476 mg) $\nu_{max}$ ($CHCl_3$) 1770, 1750, 1522, 1345 $cm^{-1}$; the n.m.r. spectrum showed the ratio of the C-2$\alpha$, 3$\beta$ isomer (23) and the C-2$\alpha$, 3$\alpha$ isomer (24) to be about 1:2; $\delta$ ppm ($CDCl_3$) 1.67 ($\frac{2}{3}$H, ddd, J 13, 8.5 and 7.5Hz, 1 C4—H of C—3a isomer) 2.22 ($\frac{1}{3}$H, m, C4—$CH_2$ of C-3$\beta$ isomer), 2.40—2.99 [$4\frac{2}{3}$H, 1H, i.e. *OH* exch. $D_2$ leaving 2.49—2.99 ($3\frac{2}{3}$H, overlapping m's, $SCH_2$, 1 C6—H and 1 C4—H of C-3$\alpha$ isomer)], 3.33 and 3.38 (1H, 2 overlapping dd's, J 16 and 5Hz, cis C6—H of both isomers), 3.56—4.20 (5H, C3—H, C5—H and $CH_2$OH which on $D_2$O exch. sharpened to t, J 6Hz), 4.48 ($\frac{2}{3}$H, d, J 6Hz, C2—H of C-3$\alpha$ isomer), 4.82 ($\frac{1}{3}$H, d, J 7Hz, C2—H of C-3$\beta$ isomer), 5.28 (2H, s, benzyl $CH_2$), 7.52 and 7.56 (2H, 2 overlapping d's J 8Hz, Ar), 8.20 (2H, d, J 8Hz, Ar).

The next fractions contained a mixture of all three isomers (17 mg) and then the most polar isomer (25) was obtained as a gum (61 mg) $\nu_{max}$ ($CHCl_3$ 1765, 1742, 1522, 1345 $cm^{-1}$; $\delta$ ppm ($CDCl_3$) 1.90 (1H, ddd, J 12, 12 and 10Hz, C4—H), 2.20—2.50 [2H, 1H exch. in $D_2$O leaving (1H, ddd, J 12, 5 and 5Hz, C4—H], 2.62—2.93 [3H, including 2.80 (1H, dd, J 16 and 2.5Hz, C6—H) and S$CH_2$], 3.13 (1H, ddd, J 16, 4 and <1Hz, C6—H), 3.58—3.92 [4H C3—H, C5—H and $CH_2$OH (which on $D_2$O exch. sharpened to t, J 6Hz, at $\delta$ 3.70)], 4.19 (1H, dd, J 7.5 and <1Hz, C2—H), 5.30 (2H, s, benzyl $CH_2$), 7.58 and 8.30 (4H, 2d's J 8.5Hz, Ar) (Found: $M^+$ at 366.0868 $C_{16}H_{18}N_2O_6S$ required $M^+$ at 366.0885).

Example 25

p-Nitrobenzyl 3-(2-acetoxyethylthio)-7-oxo-1-azabicyclo[3.2.0]heptane-2-carboxylate

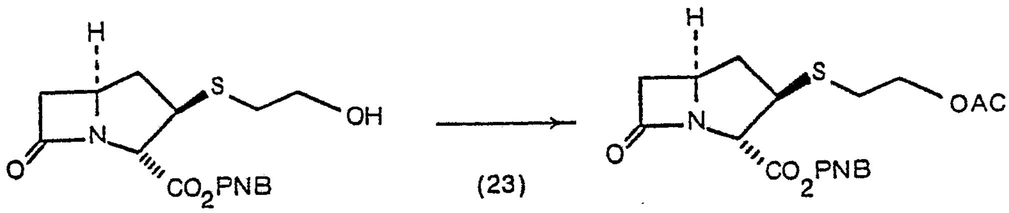

(23) (26)

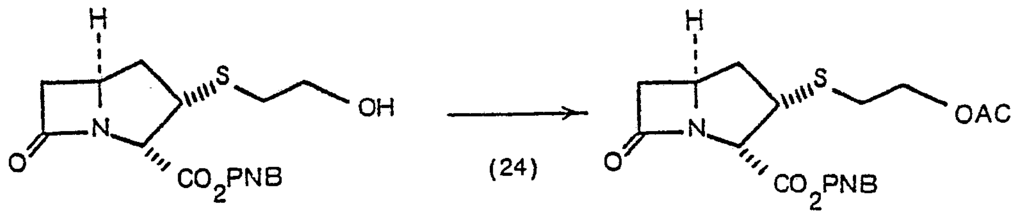

(24) (27)

The mixture of the C-3$\beta$ and C-3$\alpha$ isomers of p-nitrobenzyl 3-(2-hydroxyethylthio)-7-oxo-1-azabicyclo[3.2.0]heptane-2$\alpha$-carboxylate (23) and (24) prepared as in Example (24) (330 mg) was taken up in dry pyridine (3 ml). To the stirred solution were added three 3 A molecular sieves followed by acetic anhydride (0.2 ml). After stirring at ambient temperature for 4 hours the reaction solution was diluted with ethyl acetate and washed successively with water, dilute citric acid and brine. The organic phase was dried over magnesium sulphate, filtered and evaporated. Chromatography (x2) on silica gel (<230 Mesh) eluting with 30%—50% ethyl acetate in petroleum ether (60°—80°) partially separated the two isomers (26) and (27) of the required product.

The first fractions contained the C-2$\alpha$, 3$\beta$ isomer (26) as a gum (52 mg) $\nu_{max}$ ($CHCl_3$) 1765, 1742 (strong), 1522, 1350 $cm^{-1}$; ($CDCl_3$) 2.04 (3H, s, Me), 2.22 (2H, centre of m, C4—$CH_2$), 2.61-3.01 (3H, overlapping m's, 1 C6—H and $SCH_2$), 3.39 (1H, dd, J 16 and 5Hz, C6—H), 3.71 (1H, td, J 9 and 7.5Hz, C3—H), 3.98—4.38 (3H, overlapping m's, C5—H and $CH_2OAC$), 4.83 (1H, d, J 7Hz, C2—H), 5.27 (2H, s, benzyl $CH_2$), 7.55 and 8.22 (4H, 2d's, J 8.5Hz, Ar) (Found: $M^+$ at 408.0989 as required for $C_{18}H_{20}N_2O_7S$).

The intermediate fractions contained both the C-3$\beta$ and C-3$\alpha$ isomers (26) and (27) [(27)>(26) by T.L.C. analysis] (97 mg). The final fractions contained the major more polar isomer (27) also as a gum (94 mg), $\nu_{max}$ ($CHCl_3$) 1768, 1742 (strong), 1522, 1350 $cm^{-1}$; $\delta$ ppm ($CDCl_3$) 1.64 (1H, ddd, J 13, 9 and 8Hz, C4—H), 2.04 (3H, s, Me), 2.47—2.95 [4H, including 2.80 (2H, t, J 6Hz, $SCH_2$), 1 C6—H and 1 C4—H partially obscured], 3.33 (1H, dd, J 16 and 5Hz, C6—H), 3.72—4.00 (2H, overlapping m's, C3—H and C5—H), 4.18 (2H, t, J 6Hz, $CH_2OAC$), 4.37 (1H, d, J 6Hz, C2—H), 5.30 (2H, s, benzyl $CH_2$), 7.54 and 8.22 (4H, 2d's, J 9Hz, Ar) (Found: $M^+$ at 408.0992 $C_{18}H_{20}N_2O_7S$ requires $M^+$ at 408.0987).

Example 26

p-Nitrobenzyl 3-(2-acetoxyethylthio)-7-oxo-1-azabicyclo[3.2.0]hept-3-ene-2$\alpha$-carboxylate and p-nitrobenzyl 3-(2-acetoxyethylthio)-7-oxo-1-azabicyclo[3.2.0]hept-2-ene-2-carboxylate

*Method (i)*

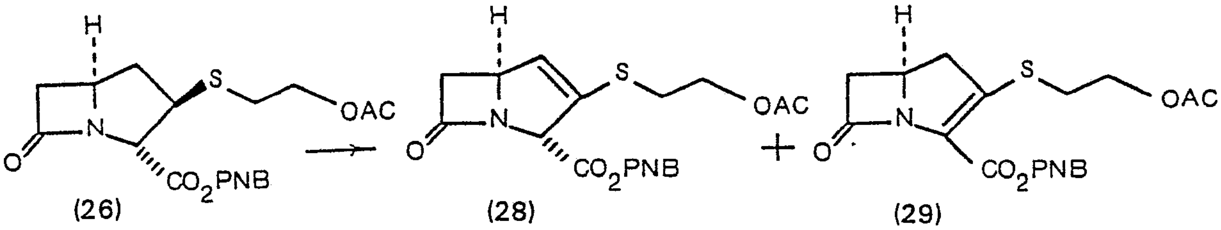

(26) (28) (29)

p-Nitrobenzyl 3$\beta$-(2-acetoxyethylthio)-7-oxo-1-azabicyclo[3.2.0]heptane-2$\alpha$-carboxylate, prepared as in Example (25) (54 mg) was taken up in dry benzene (3 ml) then evaporated to dryness. The residual gum was re-taken up in dry benzene (3 ml) and treated with dry pyridine (25 mg). The stirred solution, under argon was cooled to its freezing point and then iodobenzene dichloride (44 mg) was added in one portion. The reaction vessel was removed from the cooling bath and after shaking vigorously to give an almost clear solution was stood at +8° for 1.5 hours. A white solid came out of solution. Chromatography of the total reaction mixture on silica gel (6 g) eluting with 50% ethyl acetate in petroleum ether (60°—80°) followed by neat ethyl acetate gave the product (28) as a gum (22 mg), followed by a 50% pure (u.v. Analysis) sample of the product (29) (5 mg).

The major product (28) had $\nu_{max}$ ($CHCl_3$) 1778, 1750 (shoulder), 1742, 1570 (weak), 1520, 1345 $cm^{-1}$; $\delta$ ppm ($CDCl_3$) 2.05 (3H, s, $CH_3$) 2.70—3.20 [3H, including 2.86 (1H, dd, J 16 and 3Hz, C6—H) and C2H, m, $SCH_2$)], 3.42 (1H, dd, J 16 and 5Hz, C6—H), 4.22 (2H, t, J 6Hz, $CH_2$OAC), 4.60 (1H, m, C5—H), 5.18 (1H, dd, J 3.5 and 2Hz, C2—H), 5.27 (2H, s, benzyl $CH_2$), 5.97 (1H, dd, J 2 and 2Hz, C4—H), 7.52 and 8.21 (4H, 2d's, J 8.5Hz, Ar) (Found: $M^+$ at 406.0842 $C_{18}H_{18}N_2O_7S$ requires $M^+$ at 406.0834).

*Method (ii)*

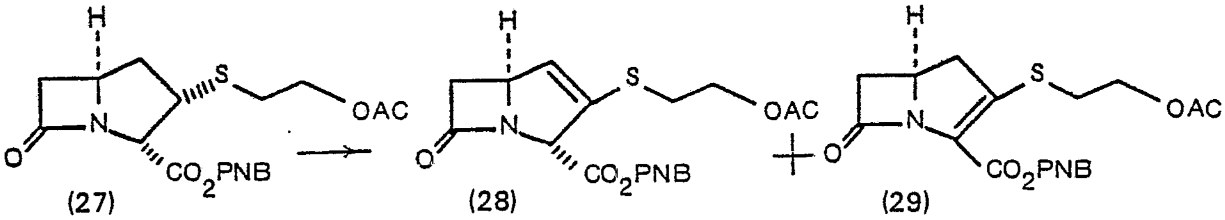

(27) (28) (29)

In a similar manner to that described in (26 i) p-nitrobenzyl 3$\alpha$-(2-acetoxyethylthio)-7-oxo-1-azabicyclo[3.2.0]heptane-2$\alpha$-carboxylate (27) (107 mg) prepared as in Example (25) was converted to (28) (48 mg) and (29) (6 mg).

## Example 27

The isomerisation of p-nitrobenzyl 3-(2-acetoxyethylthio)-7-oxo-1-azabicyclo[3.2.0]hept-3-ene-2$\alpha$-carboxylate to p-nitrobenzyl 3-(2-acetoxyethylthio)-7-oxo-1-azabicyclo[3.2.0]hept-2-ene-2-carboxylate

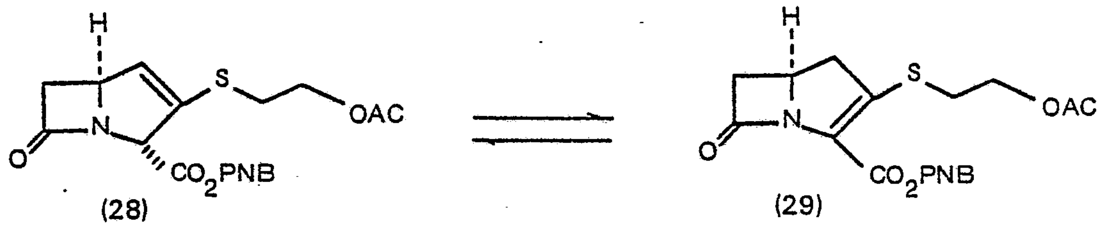

(28) (29)

p-Nitrobenzyl 3-(2-acetoxyethylthio)-7-oxo-1-azabicyclo[3.2.0]hept-3-ene-2$\alpha$-carboxylate (28) (32 mg) was treated with D.B.U. (4 mg) in dry methylene chloride (3 ml). After 2 hours chromatography on silica gel (230—400 Mesh) (6 g) eluting with 50% ethyl acetate in petroleum ether (60°—80°) followed by neat ethyl acetate gave recovered starting material (28) (16 mg) followed by the required product (29) (8 mg). The product (29) was obtained as white crystals (ex ethyl acetate/petroleum ether (60°—80°)], mp. 125—126.5°, $\lambda_{max}$ (ethanol) 314 and 267 nm; $\nu_{max}$ ($CHCl_3$) 1781, 1739, 1705 (weak), 1521, 1348 $cm^{-1}$; $\delta$ ppm ($CDCl_3$) 2.05 (3H, s, $CH_3$), 2.82—3.20 (4H, overlapping m's, $SCH_2$, 1 C6—H and 1 C4—H), 3.37 (1H, dd, J 18 and 9Hz, 1 C4—H), 3.51 (1H, dd, J 17 and 5Hz, 1 C6—H), 4.21 (2H, t, J 6Hz, $CH_2$ OAC)- obscuring (1H, C5—H), 5.23 and 5.48 (2H, ABq, J 13.5Hz, benzyl $CH_2$), 7.63 and 8.20 (4H, 2d's, J 8Hz, Ar) (Found:— C, 53.45; H, 4.36; N, 6.86% $C_{18}H_{18}N_2O_7S$ requires C, 53.20; H, 4.43; N, 6.90%).

Example 28

p-Nitrobenzyl 3-(2-methoxyethylthio)-7-oxo-1-azabicyclo[3.2.0]heptane-2-carboxylate

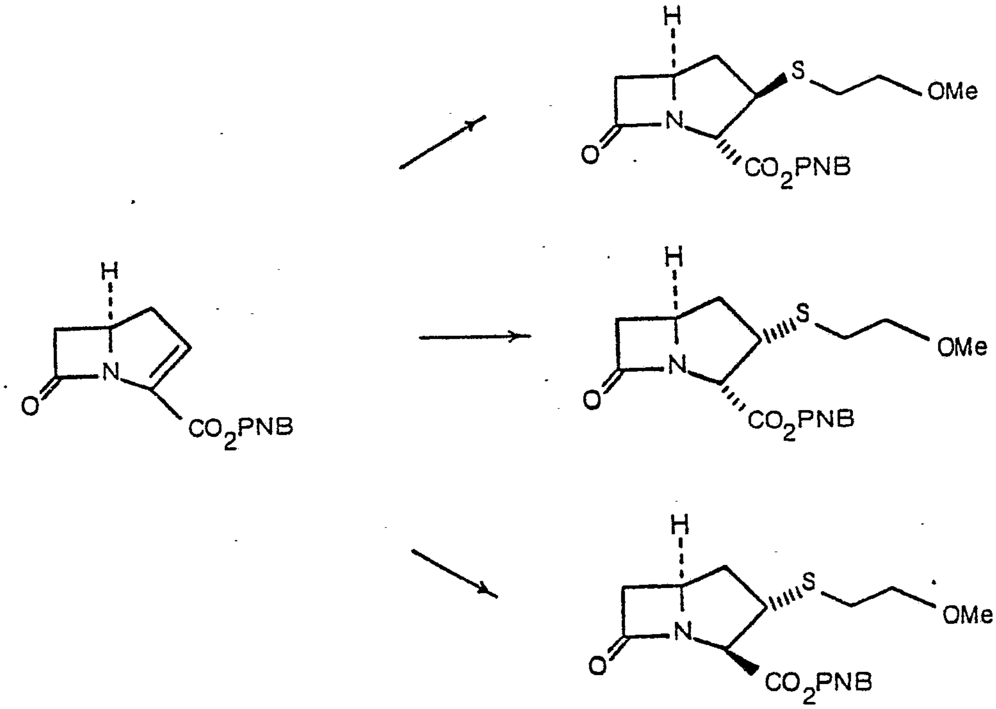

(30)

(31)

(32)

An ethereal solution of 2-methoxyethane thiol (Chapman and Owen J.C.S. 1950 p. 579) estimated to contain 3.44 g/100 ml was prepared and stored over basic alumina. A portion (8 ml) of this solution was added to p-nitrobenzyl 7-oxo-1-azabicyclo[3.2.0]hept-2-ene-2-carboxylate (576 mg) in dry dimethylformamide (10 ml), followed by potassium carbonate (27.6 mg). After stirring at ambient temperature for 15 minutes the ether was blown off to give a yellow solution. A further 1 ml of the thiol solution was added followed by after one hour a further 9 mg of potassium carbonate and 1 ml of the thiol solution. After a total of 1.5 hours the reaction solution was diluted with ethyl acetate, washed with water, brine, dried over magnesium sulphate and filtered. This ethyl acetate extract was combined with that from a similar preparation from 29 mg of the starting material. The combined extracts were evaporated to dryness and the residual oil taken up in toluene for chromatography on silica gel (<230 Mesh, 30 g). Elution with 30—50% ethyl acetate in petroleum ether followed by ethyl acetate gave the products (30), (31) and (32).

The first fractions contained the C-2$\alpha$, 3$\beta$ isomer (30) as a gum (162 mg), $\nu_{max}$ ($CHCl_3$), 1762, 1750, 1520, 1342 $cm^{-1}$; $\delta$ ppm ($CDCl_3$) 2.13 (1H, ddd, J 13, 8 and 6Hz, 1C4—H), 2.27 (1H, ddd, J 13,10 and 5Hz, 1C4—H), 2.60 (1H, dd, J 14 and 6Hz, SC*H*), 2.73 (1H, dd, J 16 and 2.5Hz, 1C6—H), 2.82 (1H, dd, J 14 and 6Hz, SCH), [3.29 (3H, s, Me) and 3.50 (2H, t, J 6Hz, $CH_2O$) 1C6—H obscured], 3.72 (1H, ddd, J 10, 8 and 7Hz, C3—H), 4.11 (1H, dddd, J 6, 5, 5 and 2.5Hz, C5—H), 4.83 (1H, d, J 7Hz, C2—H), 5.27 (2H, s, benzyl $CH_2$), 7.55 and 8.20 (4H, 2d's, J 8Hz, Ar) (Found: $M^+$ at 380.1039 $C_{17}H_{20}N_2O_6S$ requires $M^+$ at 380.1040).

The next fractions contained a mixture of the C-2$\alpha$, 3$\beta$ isomer (30) and the C-2$\alpha$, 3$\alpha$ isomer (31) in the ratio 3:4 [N.M.R. analysis] as a gum (193 mg), and the final fractions gave the most polar isomer (32) as a gum (260 mg), $\nu_{max}$ ($CHCl_3$) 1762, 1742, 1520, 1343 $cm^{-1}$; $\delta$ ppm ($CDCl_3$) 1.90 (1H, ddd, J 12, 12 and 10Hz, 1C4—H), 2.33 (1H, ddd, J 12, 5.5 and 5.5Hz), 1C4—H), 2.70 (2H, t, J 6Hz, $SCH_2$), 2.80 (1H, dd, J 16 and 2.5Hz, C6—H), 3.12 (1H, ddd, J 16, 4 and <1Hz, 1C6—H), 3.29 (3H, s, Me), 3.50 (2H, t, J 6Hz, $OCH_2$), 3.58—4.00 (2H, overlapping m's, C3 and C5—H's), 4.20 (1H, dd, J 7.5 and <1Hz, C2—H), 5.29 (2H, s, benzyl $CH_2$), 7.60 and 8.20 (4H, 2d's, J 8.5Hz, Ar) (Found: $M^+$ at 380.1041 as required for $C_{17}H_{20}N_2O_6S$).

## Example 29

The conversion of p-nitrobenzyl 3$\alpha$-(2-methoxyethylthio)-7-oxo-1-azabicyclo[3.2.0]heptane-2$\beta$-carboxylate to its 2$\alpha$ isomer

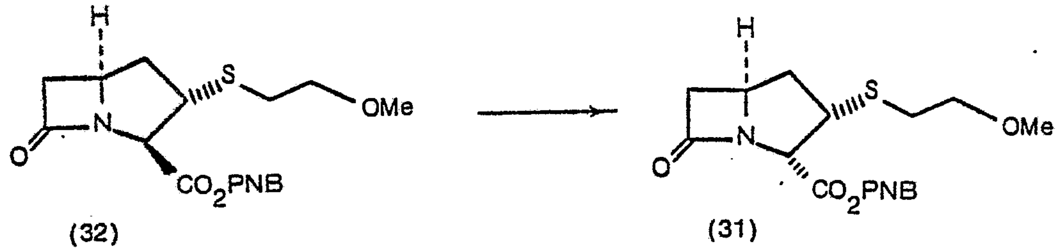

(32) (31)

p-Nitrobenzyl 3$\alpha$-(2-methoxyethylthio)-7-oxo-1-azabicyclo[3.2.0]heptane-2$\beta$-carboxylate (32) in dry methylene chloride (15 ml) at ambient temperature was treated with D.B.U. (25 mg) for 4 hours. The reaction solution was then reduced in volume and chromatographed on silica gel (<230 Mesh, 10 g). Elution with 40% ethyl acetate in petroleum ether (60°—80°) gave the required product (31) as a gum (159 mg), $\nu_{max}$ ($CHCl_3$) 1762, 1745 (shoulder), 1520, 1352 $cm^{-1}$; $\delta$ppm ($CDCl_3$) 1.63 (1H, ddd, J 12, 9 and 8Hz, 1C4—H), 2.50—2.88 [4H, including 2.77 (1H, dd, J 16 and 2.5Hz, 1C6—H), 1C4—H and $SCH_2$ overlapping m's], 3.30 (3H, s, Me), 3.32 (1H, dd, J 16 and 5Hz, 1C6—H), 3.52 (2H, t, J 6Hz, $OCH_2$), 3.75—4.00 (2H, overlapping m's, C3 and C5—H's), 4.41 (1H, d, J 6Hz, C2—H), 5.28 (2H, s, benzyl $CH_2$), 7.54 and 8.22 (4H, 2d's, J 8Hz, Ar) (Found: $M^+$ at 380.1019 $C_{17}H_{20}N_2O_6S$ requires $M^+$ at 380.1040).

## Example 30

p-Nitrobenzyl 3-(2-methoxyethylthio)-7-oxo-1-azabicyclo[3.2.0]hept-3-ene-2$\alpha$-carboxylate

*Method (i)*

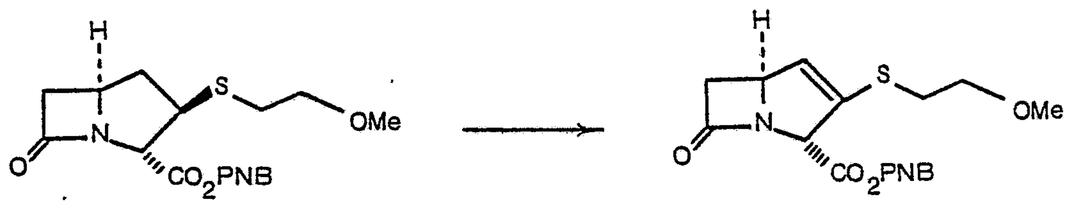

(30) (33)

p-Nitrobenzyl 3$\beta$-(2-methoxyethylthio)-7-oxo-1-azabicyclo[3.2.0]heptane-2$\alpha$-carboxylate (30) (114 mg) in dry benzene (10 ml) was treated with dry pyridine (57 mg). The solution, under argon was cooled to its freezing point and iodobenzene dichloride (99 mg) added in one portion with vigorous stirring. The clear solution was stood at +8° for 1 hour. The reaction mixture containing some white solid was then filtered through celite washing with toluene and methylene chloride. The combined filtrate and washings were reduced in volume by evaporation and chromatographed on silica gel (<230 Mesh, 10 g). Elution with 30—40% ethyl acetate in petroleum ether (60°—80°) gave the required product (33) as a gum (54 mg), $\nu_{max}$ ($CHCl_3$) 1778, 1755, 1570 (wk), 1522, 1345 $cm^{-1}$; $\delta$ ppm ($CDCl_3$) 2.84 (1H, dd, J 16 and 2.5Hz, 1C6—H), 3.00 (2H, m, $SCH_2$), 3.32 (3H, s, Me), 3.42 (1H, dd, J 16 and 5Hz, 1C6—H), 3.57 (2H, t, J 6Hz, $OCH_2$), 4.51—4.68 (1H, m, C5—H), 5.23 (1H, dd, J 2.5 and 1.5Hz, C2—H), 5.28 (2H, s, benzyl $CH_2$), 5.88 (1H, dd J 1.5 and 1.5Hz, C4—H), 7.54 and 8.21 (4H, 2d's J 8.5Hz, Ar) (Found: $M^+$ at 378.0895 $C_{17}H_{18}N_2O_6S$ requires $M^+$ at 378.0883).

*Method (ii)*

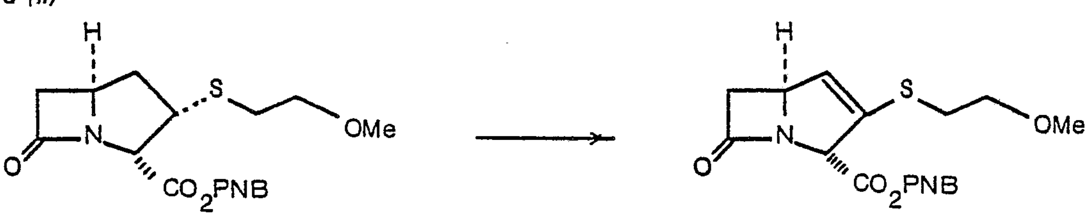

(31) (33)

In a similar manner to that described in Example 30 i) p-nitrobenzyl 3$\alpha$-(2-methoxyethylthio)-7-oxo-1-azabicyclo[3.2.0]heptane-2$\alpha$-carboxylate (31) (88 mg) was converted to the required product (33) (36 mg).

## Example 31

p-Nitrobenzyl 3-(2-methoxyethylthio)-7-oxo-1-azabicyclo[3.2.0]hept-2-ene-2-carboxylate

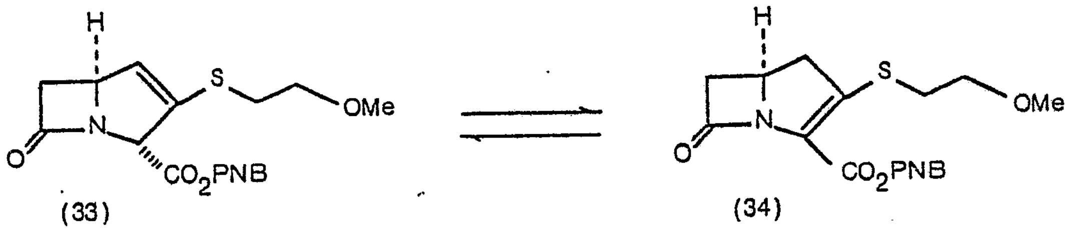

(33) (34)

p-Nitrobenzyl 3-(2-methoxyethylthio)-7-oxo-1-azabicyclo[3.2.0]hept-3-ene-2$\alpha$-carboxylate (196 mg) in dry methylene chloride (10 ml) at room temperature was treated with D.B.U. (24 mg). After standing for 2 hours the yellow solution was reduced in volume and chromatographed on Florisil (200—300 Mesh) (10 g). Elution with 50% ethyl acetate in petroleum ether (60°—80°) gave recovered starting material (33) (118 mg) followed by the required product (34) as a yellow solid (38 mg) (ex ether). Recrystallisation of this solid [ex ethyl acetate/petroleum ether (60°—80°)] gave yellow crystals m.p. 92—4°, $\lambda_{max}$ (ethanol) 317 ($\varepsilon$ 13,200) and 265 ($\varepsilon$ 11,300) nm; $\nu_{max}$ ($CHCl_3$) 1780, 1700, 1522, 1345 $cm^{-1}$; $\delta$ ppm ($CDCl_3$) 2.80—3.66 [11H, including 2.92 (1H, dd, J 16 and 2.5Hz, 1C6—H), 3.34 (3H, s, Me), 3.57 (2H, t, J 6Hz, $OCH_2$), partially obscuring $SCH_2$ C4—$H_2$ and 1C6—H], 4.23 (1H, ddd, J 8.5, 5 and 2.5Hz C5—H), 5.23 and 5.49 (2H, ABq, J 13.5Hzx, benzyl $CH_2$), 7.64 and 8.20 (4H, 2d's, J 8.5Hz, Ar) (Found: C, 53.96; H, 4.95; N, 7.25: $C_{17}H_{18}N_2O_6S$ requires C, 53.97; H, 4.76; N, 7.41%).

## Example 32

Sodium 3-(2-methoxyethylthio)-7-oxo-1-azabicyclo[3.2.0]hept-2-ene-2-carboxylate

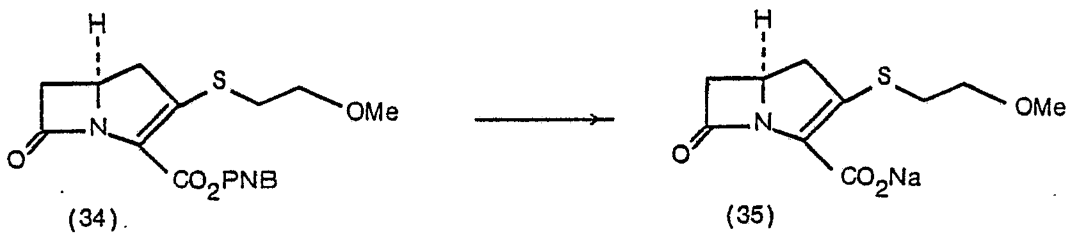

(34). (35)

A catalyst of 5% palladium on charcoal (60 mg) suspended in dioxan (6 ml)/deionised water (3 ml) was prehydrogenated at ambient temperature and pressure for 20 minutes. To this catalyst mixture was then added p-nitrobenzyl 3-(2-methoxyethylthio)-7-oxo-1-azabicyclo[3.2.0]hept-2-ene-2-carboxylate (34) (44 mg) in dioxan (6 ml). After hydrogenation for a further 2 hours 45 minutes sodium bicarbonate (9.8 mg) dissolved in water (2 ml) was added to the hydrogenation flask. The catalyst was then removed by filtration through celite washing with de-ionised water. The filtrate and washings were reduced in volume by evaporation and the cloudy aqueous solution obtained was extracted (x2) with ethyl acetate. The aqueous extract was reduced to 5 ml by evaporation, then chromatographed on biogel P—2 (15 x 2.5 cm) eluting with de-ionized water. The fractions were examined by u.v. and those with $\lambda_{max}$ ($H_2O$) 298 nm combined. It was estimated that 5 mg of the required salt (35) was present in those fractions assuming $\varepsilon_{max}$ 8,000 at 298 nm.

Example 33

p-Nitrobenzyl 3-(2-methylcarbamoyloxyethylthio)-7-oxo-1-azabicyclo[3.2.1]heptane-2-carboxylate

(23) 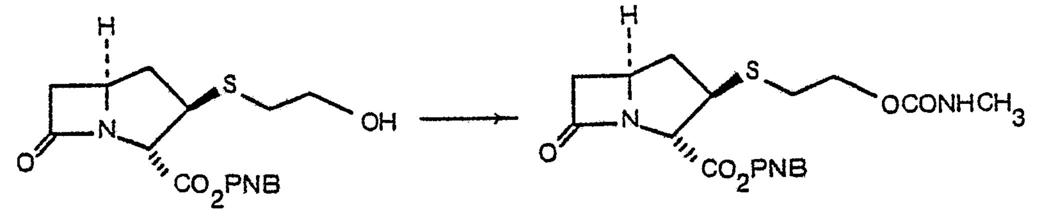 (36)

(24) 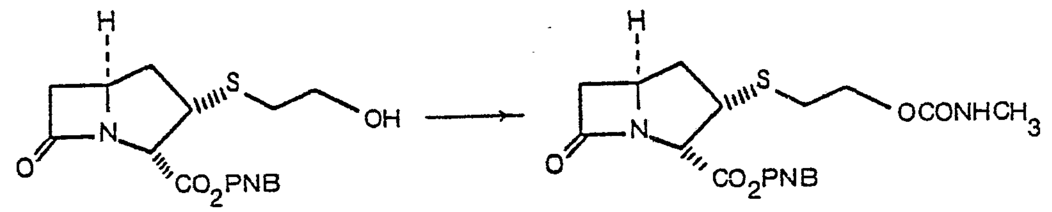 (37)

(25) 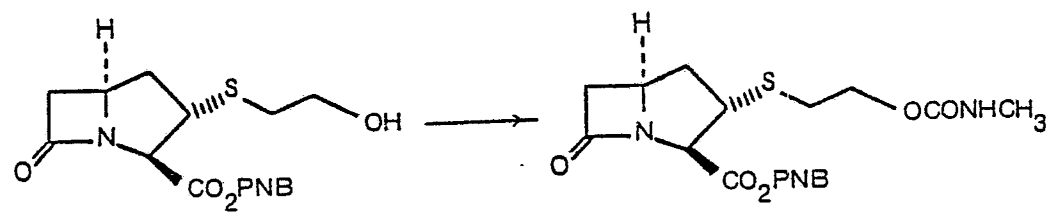 (38)

The mixture of isomers of p-nitrobenzyl 3-(2-hydroxyethylthio)-7-oxo-1-azabicyclo[3.2.0]heptane-2-carboxylate (23), (24) and (25) prepared as in Example (24) (107 mg) was treated with dry pyridine (1 ml) and methyl isocyanate (1 ml). The solution obtained after standing at ambient temperature overnight was diluted with toluene (3 ml) and evaporated to dryness (twice). The residual oil re-taken up in toluene was chromatographed on silica gel (<230 Mesh, 9 x 2 cm). Elution with 50—70% ethyl acetate in petroleum ether (60°—80°) followed by neat ethyl acetate gave the three isomers (36), (37) and (38).

The first fractions gave the least polar isomer (36) as a gum (21 mg), $\nu_{max}$ ($CHCl_3$) 3460 (wk), 1765, 1745, 1725, 1520, 1345 $cm^{-1}$; $\delta$ ppm ($CDCl_3$), 2.20 (2H, m, C4—$H_2$), 2.51—3.07 [6H, including 2.78 (3H, d, J 5Hz, collapsing to s on irradiation at $\delta$ 4.95, Me), partially obscuring (3H, m's, $SCH_2$ and 1C 6—H)], 3.40 (1H, dd, J 16 and 5.5Hz, 1C6—H), 3.75 (1H, td, J 9 and 6Hz, C3—H, 4.02—4.45 (3H, overlapping m's, $OCH_2$ and C5—H), 4.87 (1H, d, J 7Hz, C2—H) obscuring (1H, NH), 5.28 (2H, s, benzyl $CH_2$), 7.55 and 8.22 (4H, 2d's, J 8Hz, Ar) (Found: $M^+$ at 423.1090 $C_{18}H_{21}N_3O_7S$ requires $M^+$ at 423.1098).

The next fractions contained the C—2$\alpha$,3$\alpha$ isomer (37), contaminated with a trace of the C—2$\alpha$,3$\beta$ isomer (36), (18 mg) and further elution gave pure (T.L.C. analysis) (37) again as a gum (57 mg), $\nu_{max}$ ($CHCl_3$) 3470 (wk), 1765, 1745, 1725, 1522, 1350 $cm^{-1}$; $\delta$ ppm ($CDCl_3$) 1.64 (1H, ddd, J 14, 9.5 and 7.5Hz, 1C4—H), 2.50—2.95 [7H including 2.77 (3H, d, J 5Hz, Me) partially obscuring (4H, m's, 1C6—H, 1C4—H and $SCH_2$)], 3.34 (1H, dd, J 16 and 4.5Hz, 1C6—H), 3.75—4.03 (2H, overlapping m's, C3 and C5—H's), 4.19 (2H, t, J 6Hz, $OCH_2$), 4.43 (1H, d, J 6Hz, C2—H), 4.90 (1H, broad signal, NH), 5.30 (2H, s, benzyl $CH_2$), 7.55 and 8.26 (4H, 2d's, J 8.5Hz, Ar) (Found: $M^+$ at 423.1099 $C_{18}H_{21}N_3O_7S$ requires $M^+$ at 423.1100).

The last fractions contained the C—2$\beta$,3$\alpha$ isomer (38) as a white solid (29 mg) (ex ether) which was recrystallised from ethyl acetate/petroleum ether (60°—80°) m.p. 105—6°, $\nu_{max}$ ($CHCl_3$) 3470 (wk), 1770, 1742, 1725, 1522, 1350 $cm^{-1}$; $\delta$ ppm ($CDCl_3$) 1.89 (1H, ddd, J 12, 12 and 9.5 Hz, 1C4—H), 2.32 (1H, ddd J 12, 5.5 and 5.5Hz, 1C4—H), 2.63—2.90 [6H, including 2.73 (3H, d, J 5Hz collapsing to s on irradiation at 4.77, Me) partially obscuring 1C6—H and $SCH_2$], 3.12 (1H, ddd, J 16, 4.5 and ca 1Hz), 3.58—3.90 (2H, overlapping m's, C3 and C5—H's), 4.13 (2H, t, J 6.5Hz, $OCH_2$) — obscuring C2—H, 4.77 (1H, broad, NH), 5.28 (2H, s, benzyl $CH_2$), 7.55 and 8.19 (4H, 2d's, J 8.5Hz, Ar) (Found: m/e at 348.0778. $C_{16}H_{16}N_2O_5S$ (M—HOCONHMe) requires m/e at 348.0777).

Example 34

The conversion of p-nitrobenzyl 3α-(2-methylcarbamoyloxyethylthio)-7-oxo-1-azabicyclo[3.2.0] heptane-2β-carboxylate to its 2α isomer

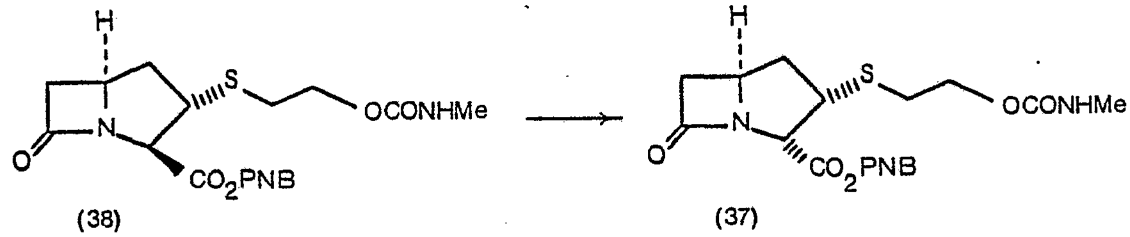

(38) (37)

p-Nitrobenzyl 3α-(2-methylcarbamoyloxyethylthio)-7-oxo-1-azabicyclo[3.2.0]heptane-2β-carboxylate (78 mg) was treated with D.B.U. (8.4 mg) in dry methylene chloride (2.5 ml). After standing at ambient temperature for 4 hours the yellow solution was chromatographed on silica gel (<230 Mesh), 5 g). Elution with 50% ethyl acetate gave the required isomer (37) as a gum (39 mg).

Example 35

p-Nitrobenzyl 3-(2-methylcarbamoyloxyethylthio)-7-oxo-1-azabicyclo[3.2.0]hept-3-ene-2α-carboxylate

*Method (i)*

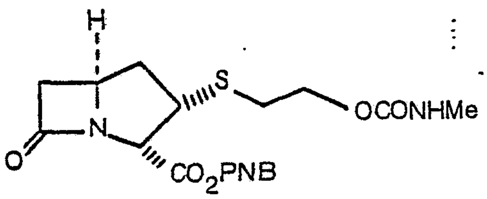

(37)

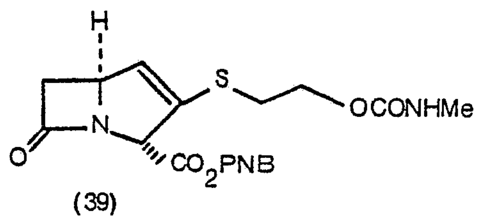

(39)

p-Nitrobenzyl 3α-(2-methylcarbamoyloxyethylthio)-7-oxo-1-azabicyclo[3.2.0]heptane-2α-carboxylate (37) (109 mg) was taken up in dry benzene (10 ml). The solution was evaporated to dryness and the residual gum re-taken up in dry benzene (10 ml) was treated with dry pyridine (61 mg). The stirred solution, under argon was cooled to its freezing-point and then iodobenzene dichloride (83.5 mg) was added in one portion. The clear pale yellow solution obtained was stood at 8° for 2 hours. A reddish-brown solid precipitated. The benzene solution was decanted off and placed on a column of silica gel (<230 Mesh, 2.5 x 7 cm). The residual solid dissolved in methylene chloride was then put on the same column. Elution of this column with 50—70% ethyl acetate in petroleum ether (60°—80°) followed by neat ethyl acetate gave the product (39) initially as a gum (43 mg) followed by recovered starting material (37) (12 mg). The product (39) was obtained as white crystalline plates [ex ethyl acetate/petroleum ether (60°—80°)] which at about 80° changed to fine needles m.p. 109.5—111.5°, $\nu_{max}$ ($CHCl_3$) 3470 (wk), 1775, 1755, 1720, 1575 (wk), 1522, 1350 $cm^{-1}$; δ ppm ($CDCl_3$) 2.77 (3H, d, J 5Hz, Me) partially obscuring 2.71—3.33 (3H, overlapping m's, 1C6—H and $SCH_2$), 3.41 (1H, dd, J 16 and 5.5Hz, 1C6—H), 4.23 (2H, m, $OCH_2$), 4.53—4.70 (1H, m, C5—H), 4.95 (1H, broad, NH), 5.29 (2H, s, benzyl $CH_2$) obscuring C2—H, 6.07 (1H, dd, J 2 and 2Hz, C4—H), 7.55 and 8.24 (4H, 2d's, J 9Hz, Ar) (Found: C, 51.21; H, 4.56; N, 10.01% $C_{18}H_{19}N_3O_7S$ requires C, 51.31; H, 4.51; N, 9.98%).

*Method (ii)*

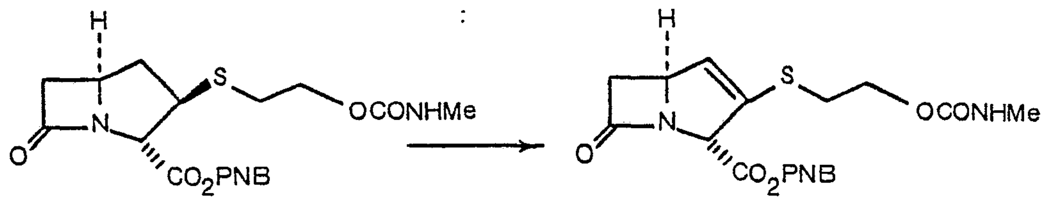

(36) (39)

In a similar manner to that described in (35 i) p-nitrobenzyl 3β-(2-methylcarbamoyloxyethylthio)-7-oxo-1-azabicyclo[3..2.0]heptane-2α-carboxylate (36) (43 mg) was converted to the required product (39) (14 mg).

Example 36

p-Nitrobenzyl 3-(2-methylcarbamoyloxyethylthio)-7-oxo-1-azabicyclo[3.2.0]hept-2-ene-2-carboxylate

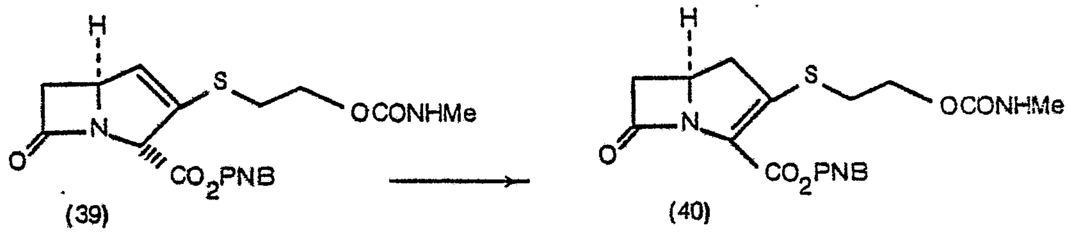

(39) (40)

p-Nitrobenzyl 3-(2-methylcarbamoyloxyethylthio)-7-oxo-1-azabicyclo[3.2.0]hept-3-ene-2$\alpha$-carboxylate (39) (128 mg) was treated with D.B.U. (13.8 mg) in dry methylene chloride (4 ml). After standing for 2 hours at ambient temperature the dark yellow solution was chromatographed on silica gel (230—400 Mesh). Elution with 50% ethyl acetate in petroleum ether (60°—80°) then neat ethyl acetate gave recovered starting material (39) (53 mg) followed by the required product (40) as a white solid (28 mg) (ex ether). Recrystallisation of a portion of this solid from ethyl acetate/petroleum ether (60°—80°) gave cream coloured crystals m.p. 126—132°, $\lambda_{max}$ (ethanol) 315 ($\varepsilon$, 12,400) and 265 ($\varepsilon$, 10,700) nm; $\nu_{max}$ ($CHCl_3$) 3470 (wk), 1782, 1720 (strong), 1522, 1350 $cm^{-1}$; $\delta$ ppm ($CDCl_3$) 2.70—3.64 [9H, including 2.78 (3H, d, J 5Hz, collapsing to a s on irradiation at 4.66, Me), 2.93 (1H, dd, J 16 and 2Hz, 1C6—H), 3.50 (1H, dd, J 16 and 5.5Hz, 1C6—H), partially obscuring C4H, overlapping m's, $SCH_2$ and C4—$H_2$)], 4.19 (2H, t, J 6Hz, $OCH_2$) obscuring C5—H, 4.66 (1H, broad, NH), 5.23 and 5.49 (2H, ABq, J 14Hz, benzyl $CH_2$), 7.63 and 8.20 (4H, 2d's, J 8.5Hz, Ar) (Found: C, 51.19; H, 4.51; N, 9.91% $C_{18}H_{19}N_3O_7S$ requires C, 51.31; H, 4.51; N, 9.98%).

Example 37

Sodium 3-(2-methylcarbamoyloxyethylthio)-7-oxo-1-azabicyclo[3.2.0]hept-2-ene-2-carboxylate

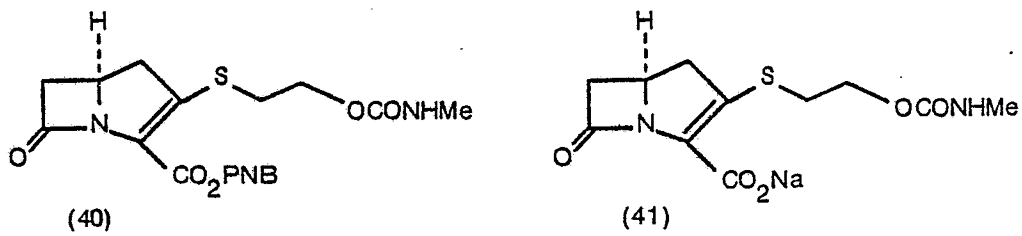

(40) (41)

A catalyst of 5% palladium on charcoal (80 mg) suspended in dioxan (8 ml)/de-ionised water (4 ml) was hydrogenated at ambient temperature and pressure for 20 minutes. p-Nitrobenzyl 3-(2-methylcarbamoyloxyethylthio)-7-oxo-1-azabicyclo[3.2.0]hept-2-ene-2-carboxylate (53 mg) dissolved in dioxan (8 ml) was added to the hydrogenation flask. After hydrogenation for a further 2.5 hours sodium bicarbonate (16 mg) dissolved in water was added and the catalyst then removed by filtration through celite washing with water. The clear yellow filtrate and washings were reduced in volume until cloudy then washed with ethyl acetate (twice). Each time an emulsion formed with was broken down by centrifugation. The aqueous phase was reduced to about 5 ml by evaporation and then chromatographed on Biogel P—2 (15 x 2.5 cm), eluting with de-ionised water, to give the required salt (41), $\lambda_{max}$ 297 nm. It was estimated that the yield of (41) was 4.3 mg assuming $\varepsilon_{max}$ of 8,000 at 297 nm.

Example 38

p-Nitrobenzyl 3-methylthio-7-oxo-1-azabicyclo[3.2.0]heptane-2-carboxylate

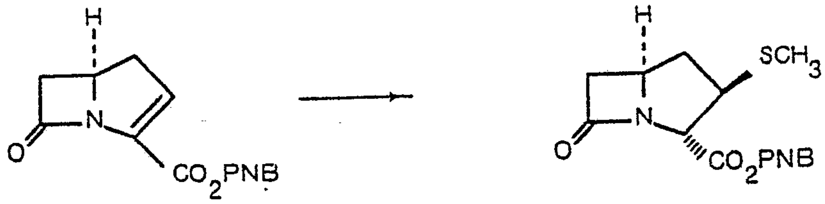

(42)

+

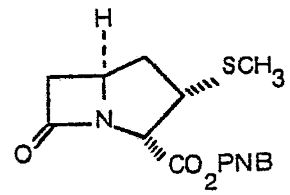

(43)

+

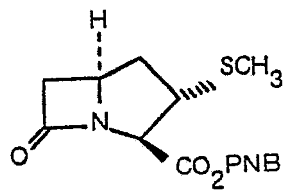

(44)

A "co-crystalline" mixture of p-nitrobenzyl 7-oxo-1-azabicyclo[3.2.0]hept-2-ene-2-carboxylate and triphenylphosphine oxide (700 mg, estimated to contain 300 mg triphenylphosphine oxide), in dry dimethylformamide (10 ml) was cooled, with stirring in a bath at −20°. Methanethiol was passed into the mixture for 10 minutes. Potassium carbonate (28 mg) was then added and the cooling bath removed. After stirring for 20 minutes at ambient temperature the pale orange solution was diluted with ethyl acetate (200 ml) washed with water, brine, dried over magnesium sulphate filtered and evaporated. The residual semi-solid was taken up in toluene and chromatographed on silica gel (<230 Mesh, 40 g). Elution with 500 ml each of 30%, 40% and 50% ethyl acetate in petroleum ether (60°—80°) gave the products (42), (43) and (44).

The first fractions gave (42) as a white solid (131 mg), obtained as white needles m.p. 113.5—114° [ex ethyl acetate/petroleum ether (60°—80°)], $\nu_{max}$ ($CHCl_3$) 1770, 1755, 1525, 1352 $cm^{-1}$; $\delta$ ppm ($CDCl_3$) 2.13 (3H, s, Me) partially obscuring (2H, dd, J 8.5 and 6Hz, C4—$H_2$), 2.75 (1H, dd, J 16 and 2.5Hz, 1C6—H), 3.39 (1H, dd, J 16 and 5.5 Hz, 1C6—H), 3.56 (1H, td, J 8.5 and 7Hz, C3—H), 4.13 (1H, tdd, J 6, 5.5 and 2.5Hz, C5—H), 4.83 (1H, d, J 7.5Hz, C2—H), 5.28 (2H, s, benzyl $CH_2$), 7.55 and 8.22 (4H, 2d's, J 8.5Hz, Ar) (Found: C, 53.66; H, 5.04; N, 8.35% $C_{15}H_{16}N_2O_5S$ requires C, 53.57; H, 4.76; N, 8.33%).

The next fractions gave a gum (65 mg) shown by N.M.R. to be a mixture of (42) and (43) in the ratio 1:2. The final fractions gave the C—2$\beta$, 3$\alpha$ isomer (44) (113 mg) obtained as microcrystalline needles from ethyl acetate/petroleum ether (60°—80°) m.p. 95—6°, $\nu_{max}$ ($CHCl_3$) 1770, 1745, 1522, 1350 $cm^{-1}$; $\delta$ ppm ($CDCl_3$) 1.88 (1H, ddd, J 12, 12 and 9.5Hz, 1C4—H), 2.11 (3H, s, Me), 2.32 (1H, ddd, J 12, 5.5 and 5.5Hz, 1C4—H), 2.82 (1H, dd, J 16 and 2.5Hz, 1C6—H), 3.13 (1H, ddd, J 16, 4.5 and <1Hz, 1C6—H), 3.50—3.87 (2H, overlapping m's, C3 and C5—H's) 4.20 (1H, dd, J 7.5 and <1Hz), 5.30 (2H, s, benzyl $CH_2$), 7.58 and 8.22 (4H, 2d's, J 8.5Hz, Ar) (Found: C, 53.69; H, 4.81; N, 8.37% $C_{15}H_{16}N_2O_5S$ requires C, 53.57; H, 4.76; N, 8.33%).

Example 39

The conversion of p-nitrobenzyl 3$\alpha$-methylthio-7-oxo-1-azabicyclo[3.2.0]heptane-2$\beta$-carboxylate to its 2$\alpha$ isomer

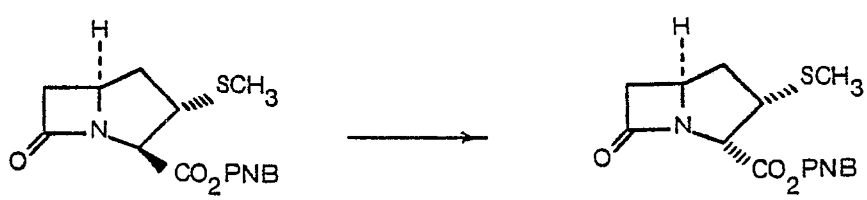

(44) (43)

p-Nitrobenzyl 3$\alpha$-methylthio-7-oxo-1-azabicyclo[3.2.0]heptane-2$\beta$-carboxylate (44) (97) mg) in

dry methylene chloride (3.8 ml) was treated with D.B.U. (13 mg). After standing at ambient temperature for 5 hours the solution was chromatographed on silica gel (<230 Mesh, 5 g). Elution with 50% ethyl acetate in petroleum ether (60°—80°) gave the required product (43) initially as a gum (89 mg) which crystallised from ethyl acetate/petroleum ether (60°—80°) to give white crystals m.p. 66—7°, $\nu_{max}$ ($CHCl_3$) 1762, 1750, 1522, 1348 $cm^{-1}$; $\delta$ ppm ($CDCl_3$) 1.64 (1H, ddd, J 13, 9 and 7.5Hz, 1C4—H), 2.13 (3H, s, Me), 2.62 (1H, ddd, J 13, 6 and 6Hz, 1C4—H), 2.78 (1H, dd, J 16 and 2Hz, 1C6—H), 3.83 (1H, dd, J 16 and 4.5Hz, 1C6—H), 3.59—4.02 (2H, overlapping m's, C—3 and C—5 H's), 4.38 (1H, d, J6Hz, C2—H), 5.29 (2H, s, benzyl $CH_2$), 7.53 and 8.22 (4H, 2d's, J 8.5Hz, Ar) (Found: C, 53.51; H, 4.99; N, 8.38% $C_{15}H_{16}N_2O_5S$ requires C, 53.57; H, 4.76; N, 8.33%).

Example 40

p-Nitrobenzyl 3-methylthio-7-oxo-1-azabicyclo[3.2.0]hept-3-ene-2$\alpha$-carboxylate

*Method (i)*

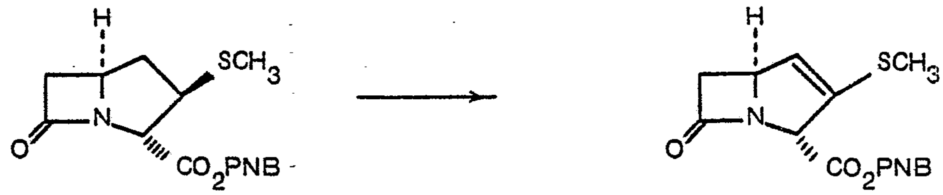

(42) (45)

p-Nitrobenzyl 3$\beta$-methylthio-7-oxo-1-azabicyclo[3.2.0]heptane-2$\alpha$-carboxylate (42) (99 mg) in dry benzene (10 ml) was treated with dry pyridine (51.2 mg). The solution, under argon, was cooled to its freezing point and then treated with iodobenzene dichloride (89 mg) added in one portion with vigorous stirring. The clear solution obtained was stood at 8° for one hour and then filtered through celite washing with toluene to remove the white solid which had precipitated. The combined filtrate and washings were reduced in volume and chromatographed on silica gel (230—400 Mesh, 9.5 x 2.5 cm). Elution with ethyl acetate/petroleum ether (60°—80°) mixtures gave the required product (45) as a white solid (34 mg), obtained as white microcrystalline plates [ex ethyl acetate/petroleum ether (60°—80°)] m.p. 126—130°, $\nu_{max}$ ($CH_2Cl_2$) 1778, 1755, 1575 (wk), 1522, 1350 $cm^{-1}$; $\delta$ ppm ($CDCl_3$) 2.36 (3H, s, Me), 2.83 (1H, dd, J 16 and 2.5Hz, 1C6—H), 3.40 (1H, dd, J 16 and 5Hz, 1C6—H), 4.53—4.70 (1H, m, C5—H), 5.14—5.22 (1H, m, C2—H), 5.28 (2H, s, benzyl $CH_2$), 5.70 (1H, m, C4—H), 7.55 and 8.22 (4H, 2d's, J 8.5Hz, Ar) (Found: C, 53.73; H, 3.89; N, 8.24% $C_{15}H_{14}N_2O_5S$ requires C, 53.89; H, 4.19; N, 8.38%).

*Method (ii)*

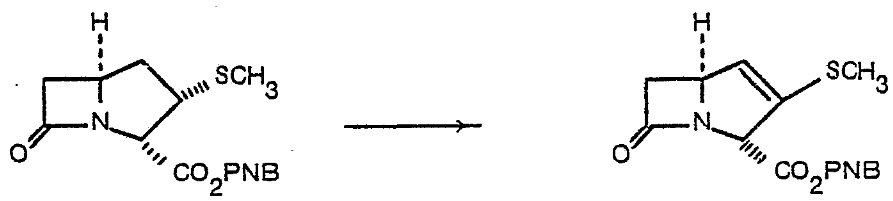

(43) (45)

In a similar manner to that described in (40 i) p-nitrobenzyl 3$\alpha$-methylthio-7-oxo-1-azabicyclo[3.2.0]heptane-2$\alpha$-carboxylate (43) (67 mg) was converted to the required product (45) (25 mg). Some recovered starting material (43) (12 mg) was also isolated.

Example 41

p-Nitrobenzyl 3-methylthio-7-oxo-1-azabicyclo[3.2.0]hept-2-ene-2-carboxylate

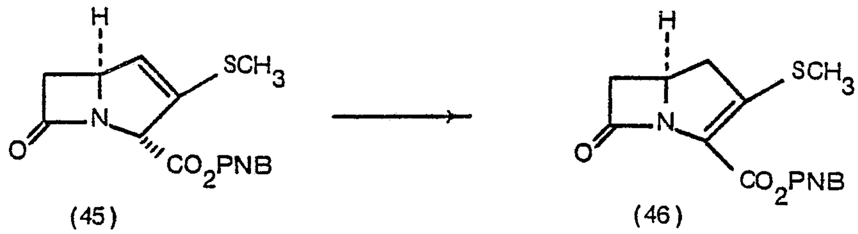

(45) (46)

p-Nitrobenzyl 3-methylthio-7-oxo-1-azabicyclo[3.2.0]hept-3-ene-2$\alpha$-carboxylate (45) (79) was treated with D.B.U. (10.8 mg) in dry methylene chloride (3.2 ml). The reaction solution was stood at

room temperature for 2 hours then chromatographed on Florisil (200—300 Mesh), 5 x 2.5 cm). Elution with 50% ethyl acetate in petroleum ether (60°—80°) gave recovered starting material (45) (34 mg), followed by the required product (46) as yellow crystals (25 mg). The product (46) was recrystallised from ethyl acetate/petroleum ether (60°—80°) to give yellow needles m.p. 140—8°, $\lambda_{max}$ (ethanol) 318 ($\varepsilon$, 12,900) and 266 ($\varepsilon$, 11,202) nm; $\nu_{max}$ ($CHCl_3$) 1780, 1700, 1522, 1350 $cm^{-1}$; $\delta$ ppm ($CDCl_3$) 2.39 (3H, s, Me), 2.94 (1H, dd, J 16 and 2.5Hz, 1C6—H), 3.10 (1H, dd, J 18 and 8.5Hz, 1C4—H), 3.30 (1H, dd, J 18 and 8.5 Hz, 1C4—H), 3.51 (1H, dd, J 16 and 5Hz, 1C6—H), 4.25 (1H, m, C5—H), 5.23 and 5.49 (2H, ABq, J 14Hz, benzyl $CH_2$), 7.65 and 8.20 (4H, 2d's, J 8.5Hz, Ar) (Found: C, 53.81; H, 4.02; N, 8.32% $C_{15}H_{14}N_2O_5S$ requires C, 53.89; H, 4.19; N, 8.38%).

Example 42

Sodium 3-methylthio-7-oxo-1-azabicyclo[3.2.0]hept-2-ene-2-carboxylate

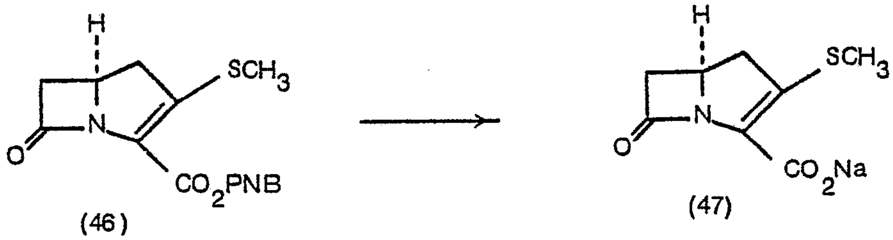

(46) (47)

A catalyst of 5% palladium on charcoal (120 mg) suspended in dioxan (12 ml)/de-ionised water (6 ml) was prehydrogenated at ambient temperature and pressure for 20 minutes. p-Nitrobenzyl 3-methylthio-7-oxo-1-azabicyclo[3.2.0]hept-2-ene-2-carboxylate (80 mg) in dioxan (12 ml) was then added to the hydrogenation flask and hydrogenation continued for a further 2.5 hours. Sodium bicarbonate (20 mg) in de-ionised water (1 ml) was added and the catalyst removed by filtration through Celite washing with water (3 ml). The almost colourless filtrate was evaporated to remove dioxan and the clear yellow solution obtained was washed with ethyl acetate (x2). The aqueous extract was evaporated to remove residual ethyl acetate then chromatographed on Biogel P—2 (16 x 2 cm), eluting with de-ionised water. The fractions were examined by u.v. and those with $\lambda_{max}$ ($H_2O$) 300 nm combined. These were estimated to contain 3.5 mg of the required product (47), assuming $\varepsilon_{max}$ of 8,000 at 300 nm. It was also estimated that a further 2 mg of the salt (47) was present in some less pure (u.v. analysis) fractions.

Example 43

Preparation of p-nitrobenzyl 3-benzylthio-7-oxo-1-azabicyclo[3.2.0]heptane-2-carboxylate

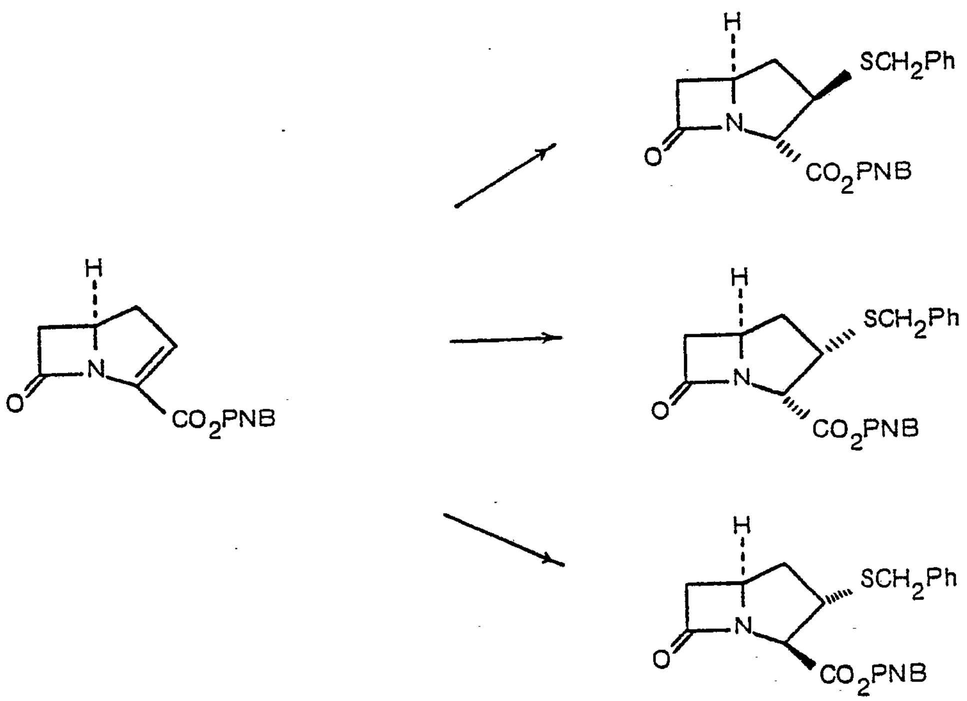

(48)

(49)

(50)

To p-nitrobenzyl 7-oxo-1-azabicyclo[3.2.0]hept-2-ene-2-carboxylate (576 mg) in dry dimethylformamide was added with stirring benzyl mercaptan (248 mg) followed by powdered potassium

carbonate (28 mg). Stirring was continued at ambient temperature and the starting material dissolved over a few minutes. After 25 minutes the orange solution was diluted with ethyl acetate (250 ml), washed with water then brine, dried over magnesium sulphate, filtered and evaporated. The residual oil was taken up in toluene and chromatographed on 30 g silica gel (<230 Mesh). Elution with 30% then 40% ethyl acetate in petroleum ether (60°—80°) gave a total yield of 623 mg of the three isomers of p-nitrobenzyl 3-benzyl thio-7-oxo-1-azabicyclo[3.2.0]heptane-2-carboxylate.

The first fractions contained the 2α, 3β isomer (48) (223 mg) which crystallised from ethyl acetate/petroleum ether (60°—80°) to give crystalline plates m.p. 86—7°, $\nu_{max}$ ($CHCl_3$) 1765, 1755, 1522, 1345 $cm^{-1}$; δ ppm ($CDCl_3$) 2.11 (2H, centre of m, C4—$H_2$), 2.67 (1H, dd, J 16 and 3Hz, C6—H), 3.33 (1H, dd, J 16 and 5.5Hz, C6—H), 3.45 (1H, td, J 8.5 and 7Hz, C3—H), 3.71 (2H, s, S$CH_2$ Ph), 3.96—4.18 (1H, m, C5—H), 4.73 (1H, d, J 7Hz, C2—H), 5.26 (2H, s, $OCH_2Ar$), 7.23 (5H, s, Ph), 7.54 and 8.20 (4H, 2 d's, J 8.5Hz, p-nitrobenzyl Ar's) (Found: C, 61.19; H, 4.81; N, 6.78% $C_{21}H_{20}N_2O_5S$ requires C, 61.17; H, 4.85; N, 6.80%).

The next fractions contained a mixture of the 2α, 3β isomer (48) and the more polar 2α, 3α isomer (49) as a gum (137 mg). The ratio of (48) to (49) was 5:4 was estimated from the N.M.R. spectrum. [See Example (44) for the data on (49)].

The next fraction contained all three isomers (23 mg) and the final fractions contained the most polar 2β, 3α isomer (50) contaminated with 10% each of (48) and (49) [N.M.R. Analysis], as a gum (240 mg) $\nu_{max}$ ($CHCl_3$) 1765, 1745, 1522, 1345 $cm^{-1}$; δ ppm ($CDCl_3$) inter alia 1.87 (1H, ddd, J 12, 12 and 10Hz, C4—H), 2.17 (1H, ddd, J 12, 6 and 6Hz, C4—H), 2.27 (1H, dd, J 16 and 3Hz, C6—H), 3.09 (1H, ddd, J 16, 4.5 and <1Hz, C6—H), 3.36—3.75 (4H, including 3.70 (2H, s, $SCH_2$) + (2H, m's, C3 and C5—H's), 4.03 (1H, dd J 7.5 and <1Hz, C2—H), 5.29 (2H, s, $OCH_2$ Ar), 7.26 (5H, s, Ph), 7.58 and 8.20 (4H, 2d's, J 8.5Hz, p-nitrobenzyl Ar's) (Found: $M^+$ at 412.1102 $C_{21}H_{20}N_2O_5S$ requires $M^+$ at 412.1092).

Example 44

The Conversion of p-Nitrobenzyl 3α-benzylthio-7-oxo-1-azabicyclo[3.2.0]heptane-2β-carboxylate to its 2α-isomer

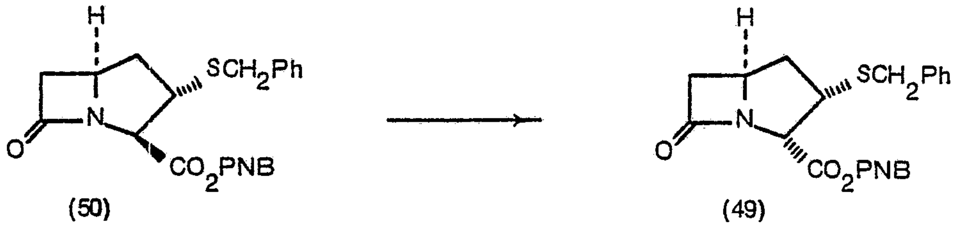

(50) (49)

p-Nitrobenzyl 3α-benzylthio-7-oxo-1-azabicyclo[3.2.0]heptane-2β-carboxylate (50) (200 mg) obtained in Example 42 i.e. contaminated with 10% each of the 2α, 3α and 2α, 3β isomers (49) and (48), was taken up in dry methylene chloride (10 ml) and treated with D.B.U. (22 mg). After standing for 4 hours at ambient temperature the solution was evaporated to dryness and the residual oil taken up in toluene for chromatography on silica gel (<230 Mesh, 10 g). Elution with 30% ethyl acetate in petroleum ether (60°—80°) gave the 2α, 3β isomer (48) (8 mg), followed by a 1:1 mixture (T.L.C. analysis) of the 2α, 3β and 2α, 3α isomers (48) and (49) (18 mg), and finally the 2α, 3α isomer (49) as a gum (140 mg), $\nu_{max}$ ($CHCl_3$), 1765, 1755, 1522, 1345 $cm^{-1}$; δ ppm ($CDCl_3$) 1.57 (1H, ddd, J 13, 8.5 and 7.5Hz, C4—H), 2.46 (1H, ddd, J 13, 6, and 6Hz, C4—H), 2.74 (1H, dd, J 15.5 and 2Hz, C6—H), 3.29 (1H, dd, J 15.5 and 4.5Hz, C6—H, 3.50—3.93 [4H, including 3.76 (2H, s, $SCH_3$) partially obscuring (2H, m's, C3 and C5—H)] 4.40 (1H, d, J 6Hz, C2—H), 5.19 (2H, s, p-nitrobenzyl $CH_2$), 7.26 (5H, s, Ph), 7.48 and 8.20 (4H, 2d's J 8.5 Hz p-nitrobenzyl Ar's) (Found: $M^+$ at 412.1102 $C_{12}H_{20}N_2O_5S$ requires $M^+$ at 412.1092).

Example 45

*Method (i)*

p-Nitrobenzyl 3-benzylthio-7-oxo-1-azabicyclo[3.2.0]hept-3-ene-2α-carboxylate

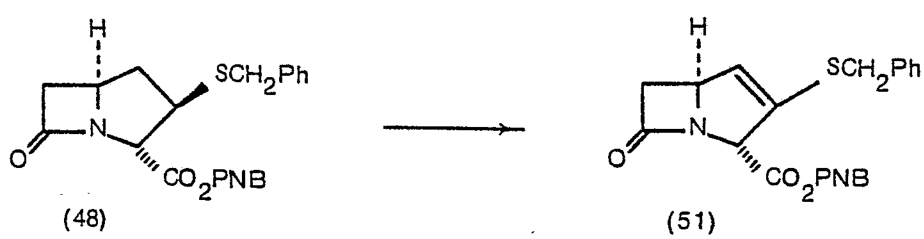

(48) (51)

p-Nitrobenzyl 3β-benzylthio-7-oxo-1-azabicyclo[3.2.0]heptane-2α-carboxylate (48), prepared as

in Example (43) (82 mg) in dry benzene (7 ml) was treated with dry pyridine (38 mg). The stirred solution, under argon was cooled to its freezing point and then iodobenzene-dichloride (66 mg) was added in one portion with vigorous stirring, to give a clear, almost colourless solution, which was stood at +8°. After 1.5 hours the benzene solution was decanted off, from crystals which had been precipitated, then reduced in volume for chromatography on silica gel (<230 Mesh) (4 g). Elution with 20% to 30% ethyl acetate in petroleum ether (60°—80°) gave the required product (51) as a gum (40 mg) which crystallised on trituration with ether. Recrystallisation gave white crystals m.p. 116—7°, $\nu_{max}$ ($CHCl_3$) 1775, 1750, 1522, 1345 $cm^{-1}$; $\delta$ ppm ($CDCl_3$) 2.77 (1H, dd, J 16 and 2.5Hz, C6—H), 3.36 (1H, dd, J 16 and 5.5Hz, C6—H), 4.00 (2H, s, $SCH_2$), 4.49—4.66 (1H, m, C5—H), 5.17 (1H, dd, J 3 and 2Hz, C2—H), 5.27 (2H, s, $OCH_2$), 5.82 (1H, dd, J 2 and 2Hz, C4—H), 7.29 (5H, s, Ph), 7.52 and 8.20 (4H, 2d's, J 8.5Hz, p-nitrobenzyl Ar) (Found: C, 61.27; H, 4.17; N, 6.65% $C_{21}H_{18}N_2O_5S$ requires C, 61.46; H, 4.39; N, 6.83%).

*Method (ii)*

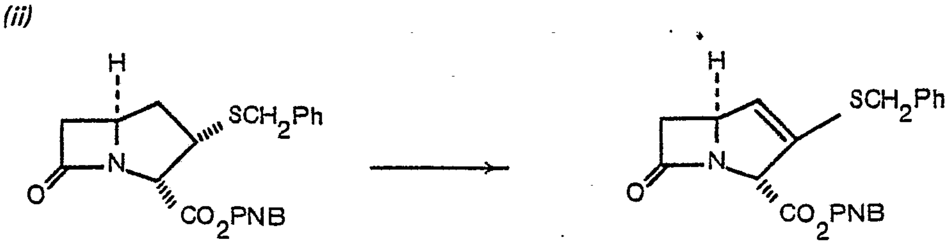

(49) (51)

In a similar manner to that described in (45 i) p-nitrobenzyl 3$\alpha$-benzylthio-7-oxo-1-azabicyclo[3.2.0]heptane-2$\alpha$-carboxylate (49) (134 mg) prepared as in Example (44), was converted to the required product (51) (48 mg).

Example 46

p-Nitrobenzyl 3-benzylthio-7-oxo-1-azabicyclo[3.2.0]hept-2-ene-2-carboxylate

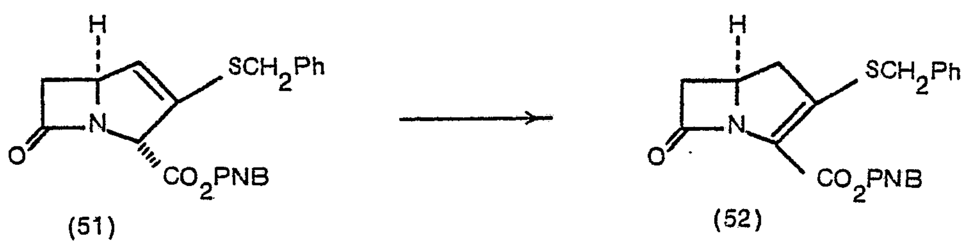

(51) (52)

p-Nitrobenzyl 3-benzylthio-7-oxo-1-azabicyclo[3.2.0]hept-3-ene-2$\alpha$-carboxylate (51) (183 mg) was treated with D.B.U. (20.4 mg) in dry methylene chloride (6 ml). After 1 hour 45 minutes the solution was chromatographed on Florisil (200—300 Mesh) (8.5 x 2.5 cm). Elution with 30%—40% ethyl acetate in petroleum ether (60°—80°) gave recovered starting material (51) as a white solid (99 mg). Further elution with neat ethyl acetate gave the required product (52). Some of (52) crystallised out from the chromatography fractions and was obtained as pale yellow crystalline plates (22 mg) m.p. 160—170°, $\lambda_{max}$ (ethanol) 319 ($\varepsilon$, 12,200) and 264 ($\varepsilon$, 9,700 nm; $\nu_{max}$ ($CHCl_3$) 1780, 1700, 1520, 1350 $cm^{-1}$; $\delta$ ppm ($CDCl_3$) 2.93 (1H, dd, J 16 and 2.5Hz, C6—H), 3.06 (1H, dd, J 18 and 8.5Hz, C4—H), 3.28 (1H, dd, J 18 and 8.5Hz, C4—H), 3.50 (1H, dd, J 16 and 5.5Hz, C6—H), 4.00—4.25 [3H, including 4.10 (2H, s, $SCH_2$), 1H, C5—H partially obscured], 5.23 and 5.50 (2H, ABq, J 14Hz, $OCH_2$), 7.33 (5H, s, Ph) 7.63 and 8.20 (4H, 2d's, J 8.5Hz, p-nitrobenzyl Ar) (Found: C, 61.04: H, 4.09; N, 6.62% $C_{21}H_{18}N_2O_5S$ requires C, 61.46; H, 4.39; N, 6.83%).

A further 20 mg of (52) was obtained as a white solid on evaporation of the chromatography fractions.

Example 47

Sodium 3-benzylthio-7-oxo-1-azabicyclo[3.2.0]hept-2-ene-2-carboxylate

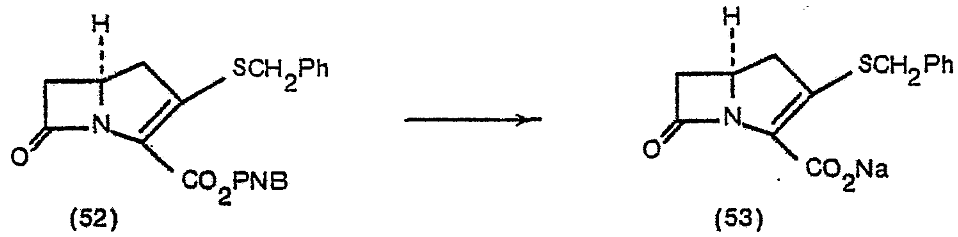

(52) (53)

A catalyst of 5% palladium on charcoal (105 mg) suspended in dioxan (10.5 ml)/de-ionised water (5.25 ml) was prehydrogenated at ambient temperature and pressure for 20 minutes. p-Nitrobenzyl 3-benzylthio-7-oxo-1-azabicyclo[3.2.0]hept-2-ene-2-carboxylate (70 mg) dissolved in dioxan (10.5 ml) was then added to the catalyst mixture and hydrogenated at ambient temperature and pressure for 2 hours 15 minutes. Sodium bicarbonate (14.3 mg) dissolved in de-ionised water (2 ml) was added to the hydrogenation flask and the catalyst removed by filtration of the contents through Celite, washing with de-ionised water. The pale, clear filtrate was reduced in volume by evaporation and the yellow cloudy aqueous solution obtained was extracted (x2) with ethyl acetate. The aqueous phase was reduced by evaporation to about 5 ml, then chromatographed on biogel P—2 (14 x 2.5 cm), eluting with de-ionised water. The fractions were examined by u.v. and the first ones with $\lambda$max ($H_2O$) $\leqslant$294 nm were discarded. The next fractions had $\lambda_{max}$ ($H_2O$) 300 nm and were combined. The total volume was 10 ml, H.P.L.C. showed one component, and it was estimated that 7 mg of the required salt (53) was present, assuming $\varepsilon_{max}$, 10,000 at 300 nm. Evaporation of an aliquot of this solution, azeotroping off the water with ethanol gave an off-white solid, $\lambda_{max}$ ($H_2O$) 299 and 265 (slight inflexion) nm; $\nu_{max}$ (KBr) 1750 (shoulder) $cm^{-1}$. One further fraction had $\lambda_{max}$ ($H_2O$) 300 nm but also showed a slightly stronger absorbance than the previous fractions in the 250 nm region and it was estimated that 2 mg of the required salt (53) was present.

Example 48

p-Nitrobenzyl 3$\alpha$-(2-dimethylaminoethylthio-7-oxo-1-azabicyclo[3.2.0]heptane-2$\alpha$-carboxylate

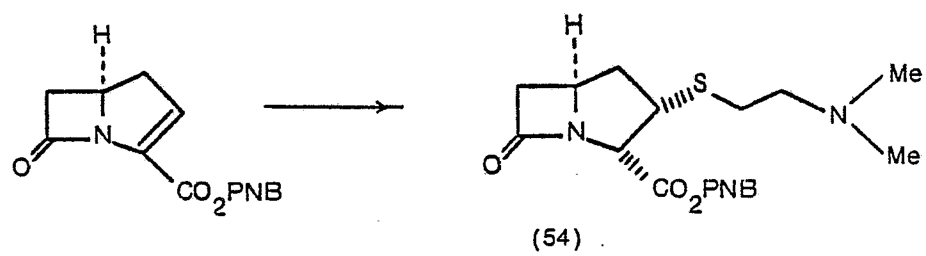

(54)

2-Dimethylaminoethanethiol hydrochloride (1.415 g) suspended in dry methylene chloride (20 ml) was treated with triethylamine (1.44 ml). On stirring at ambient temperature an almost clear pink solution was obtained which gradually became cloudy. After twenty minutes the mixture was filtered through Celite.

Some of the filtrate (6 ml) was added to a stirred suspension of p-nitrobenzyl 7-oxo-1-azabicyclo[3.2.0]hept-2-ene-2-carboxylate (576 mg) in dry dimethylformamide (10 ml). Potassium carbonate (56 mg) was added and after stirring for thirty minutes at ambient temperature the reaction mixture was diluted with ethyl acetate (125 ml), washed with water (3 x 100 ml), then brine, dried over magnesium sulphate, filtered and evaporated to dryness. The residual gum was combined with material obtained from a previous reaction (28 mg), taken up in chloroform and chromatographed on silica gel (<230 Mesh, 40 g). Elution with chloroform followed by 5 to 10% ethanol in chloroform gave the product (54) (349 mg) as a gum, $\nu_{max}$ ($CHCl_3$) 2870, 2830, 2790, 1770, 1750, 1522, 1350 $cm^{-1}$; $\delta$ ppm ($CDCl_3$) 1.63 (1H, ddd, J 14, 9 and 7.5Hz, 1C4—H), 2.19 (6H, $NMe_2$) 2.35—2.88 [6H, including 2.79 (1H, dd, J 16 and 2.5Hz, 1C6—H), $SCH_2CH_2N$ and 1C4—H— complex overlapping m's], 3.31 (1H, dd, J 16 and 4.5Hz, 1C6—H), 3.68—4.00 (2H, overlapping m's, C—5 and C—3H's), 4.39 (1H, d, J 6Hz, C2—H), 5.27 (2H, s, benzyl $CH_2$), 7.54 and 8.21 (4H, 2d's, J 8.5Hz, Ar) (Found: $M^+$ at 393.1355 as required for $C_{18}H_{23}N_3O_5S$).

Example 49

p-Nitrobenzyl 3-(2-dimethylaminoethylthio-7-oxo-1-azabicyclo[3.2.0]hept-3-ene-2$\alpha$-carboxylate

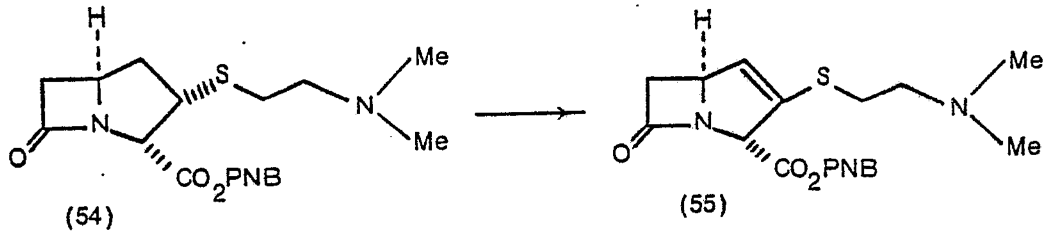

(54) (55)

p-Nitrobenzyl 3$\alpha$-(dimethylaminoethylthio)-7-oxo-1-azabicyclo[3.2.0]heptane-2$\alpha$-carboxylate (54) (232 mg) was taken up in dry benzene (15 ml) and evaporated to dryness. The residual gum redissolved in dry benzene (15 ml) was treated with dry pyridine (112 mg). The stirred solution, under argon was cooled to its freezing point and then iodobenzene dichloride (194 mg) was added in one portion. Stirring was continued for a few minutes with no external cooling until the benzene had melted. The reaction mixture containing some white solid was then stood at +8° for 1.5 hours, and then filtered through Celite washing with benzene. The filtrate/washings were discarded. The Celite was then washed with chloroform (50 ml), and these chloroform washings were reduced in volume by evaporation then chromatographed on silica gel (<230 Mesh, 20 g). Elution with chloroform followed by chloroform/ethanol mixtures gave the required product (55) as a gum (33 mg), followed by an approximately 1:1 mixture (T.L.C. analysis) of product (55) and starting material (54) (51 mg). The product (55) had: $\nu_{max}$ ($CHCl_3$) 2850 (wk), 2820 (wk), 2780 (wk), 1775, 1750, 1570 (wk), 1520, 1345 $cm^{-1}$; $\delta$ ppm ($CDCl_3$) 2.20—3.03 [11H, including 2.25 (6H, s, $NMe_2$), 1C6—H and $SCH_2CH_2N$ overlapping m's], 3.38 (1H, dd, J 16 and 5.5Hz, 1C6—H), 4.51—4.69 (1H, m, C5—H), 5.16 (1H, dd, J 3.5 and 2Hz, C2—H), 5.26 (2H, s, benzyl $CH_2$), 5.82 (1H, dd, J2 and 2Hz, C4—H), 7.53 and 8.19 (4H, 2d's, J 8.5Hz, Ar) (Found: $M^+$ at 391.1218 $C_{18}H_{21}N_3O_5S$ requires $M^+$ at 391.1199).

Example 50

p-Nitrobenzyl 3-(2-dimethylaminoethylthio)-7-oxo-1-azabicyclo[3.2.0]hept-2-ene-2-carboxylate

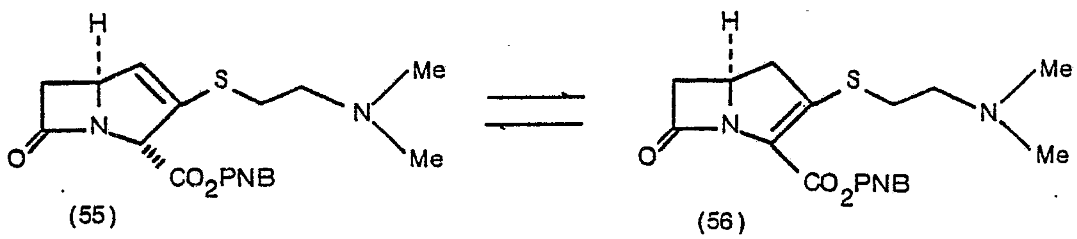

(55) (56)

p-Nitrobenzyl 3-(2-dimethylaminoethylthio-7-oxo-1-azabicyclo[3.2.0]hept-3-ene-2$\alpha$-carboxylate (55) (30 mg) was treated with D.B.U. (3.2 mg) in dry methylene chloride (1 ml). After standing at ambient temperature for 3 hours the solution was chromatographed on Florisil (200—300 Mesh) (1 x 4 cm). Elution with ethyl acetate followed by 10% ethanol in ethyl acetate gave recovered starting material (15 mg) followed by the required product (56) as yellow crystals (4.4 mg) (ex ether) m.p. 105—9°, $\lambda_{max}$ (ethanol) 318 ($\varepsilon$, 11,700) and 265 ($\varepsilon$, 10,200) nm; $\nu_{max}$ ($CHCl_3$) 2860 (wk), 2820 (wk), 2780 (wk), 1780, 1700, 1520, 1348 $cm^{-1}$; $\delta$ ppm ($CDCl_3$) 2.15—3.68 [14H, including 2.28 (6H, s, $NMe_2$), 3.34 (1H, dd, J 18 and 8.5Hz, 1C4—H), 3.54 (1H, dd, J 16 and 5Hz, 1C6—H), $SCH_2CH_2N$, 1C6—H and 1C4—H — overlapping m's], 4.05—4.43 (1H, m C5—H), 5.25 and 5.51 (2H, ABq, J 14Hz, benzyl $CH_2$), 7.66 7.66 and 8.25 (4H, 2d's, J 9Hz, Ar) (Found: $M^+$ at 391.1229 $C_{18}H_{21}N_3O_5S$ requires $M^+$ at 391.1199).

Example 51

Benzyl 3-(2-acetamidoethylthio)-7-oxo-1-azabicyclo[3.2.0]heptane-2-carboxylate

(i)

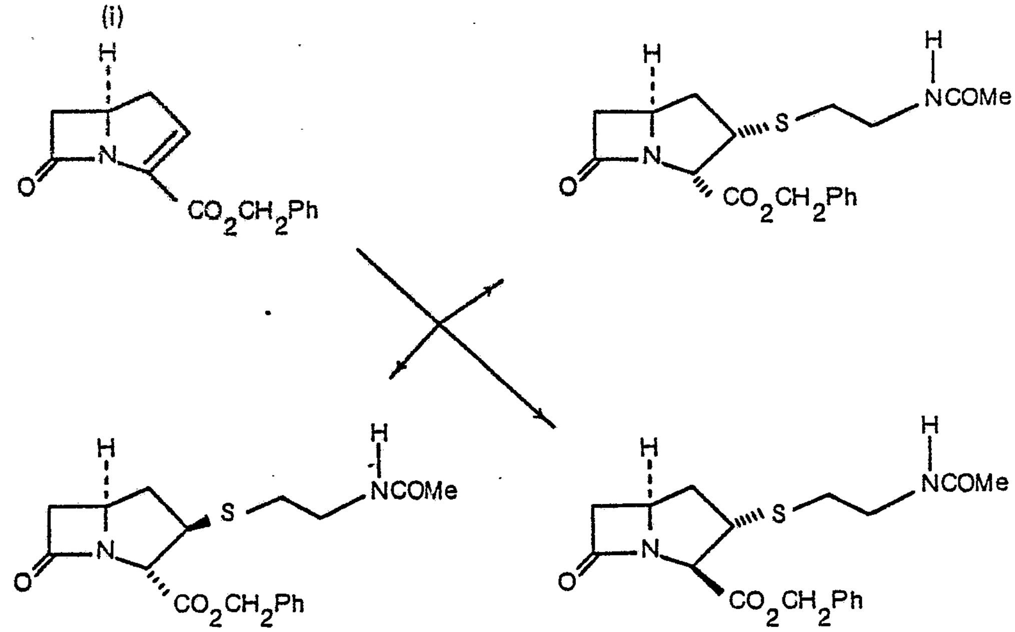

Benzyl 7-oxo-1-azabicyclo[3.2.0]hept-2-ene-2-carboxylate (0.560 g) dissolved in dry dimethylformamide (5 ml), was treated with 2-acetamidoethanethiol (0.302 g) and potassium carbonate (0.159 g). After stirring for 1 hour at room temperature, the solution was concentrated under high vacuum and the residue partitioned between ethylacetate and brine. The organic phase was dried over sodium sulphate, concentrated, and chromatographed on silica gel 60 (<230 Mesh) eluting with chloroform/ethanol 19:1 to give three isomers of benzyl 3-(2-acetamidoethylthio)-7-oxo-1-azabicyclo[3.2.0]heptane-2-carboxylate. The $2\alpha,3\alpha$-isomer was isolated as a gum (0.361 g); $\nu_{max}$ ($CHCl_3$) 3480, 3000, 1765, 1745 sh, 1670 and 1520 $cm^{-1}$; $\tau$ ppm ($CDCl_3$) 2.71 (5H, s, phenyl), 3.78 (1H, br, NH), 4.87 (2H, s, benzyl $CH_2$), 5.66 (1H, d, J 5Hz, C2—H), 6.0—6.4 (1H, m, C5—H), 6.6—6.9 (3H, m, $NCH_2$ and C6—H), 7.1—7.6 (5H, m, $SCH_2$, C6—H, C4—H and C3—H), 8.10 (3H, s, $COCH_3$) and 8.41 (1H, dt, J 13 and 7Hz, C4—H) ($M^+$ at m/e 362.1294. $C_{18}H_{22}N_2O_4S$ requires 362.1300). The $2\alpha,3\beta$-isomer was also a gum (0.192 g); $\nu_{max}$ 3460, 3000, 1765, 1745 sh, 1665 and 1515 $cm^{-1}$; $\tau$ ($CDCl_3$) 2.71 (5H, s, phenyl), 3.80 (1H, br, NH), 4.86 (2H, s, benzyl $CH_2$), 5.30 (1H, d, J 7Hz, C2—H), 5.97 (1H, qd, J 5 and $2\frac{1}{2}$Hz, C5—H), 6.43 (1H, brq, J 8Hz, C3—H), 6.5—6.8 (3H, m, $NCH_2$ and C6—H), 7.1—7.5 (3H, m, $SCH_2$ and C6—H), 7.84 (2H, dd, J 8 and 5Hz, C4—$H_2$) and 8.10 (3H, s, $COCH_3$) ($M^+$ at m/e 362.1315. $C_{18}H_{22}N_2O_4S$ requires 362.1300). The most polar isomer was the $2\beta,3\alpha$-compound (0.145 g); m.p. 117—118° (chloroform/60—80° petroleum ether); $\nu_{max}$ ($CHCl_3$) 3460, 3000, 1765, 1740, 1665 and 1510 $cm^{-1}$; $\tau$ ($CDCl_3$) 2.63 (5H, brs, phenyl), 4.00 (1H, vbrs, NH), 4.80 (2H, s, benzyl $CH_2$), 5.89 (1H, d, J 7Hz, C2—H), 6.1—6.5 (2H, m, C3—H and C5—H, 6.5—6.8 (2H, m, $NCH_2$), 6.89 (1H, dd, J 16 and 4Hz, C6—H), 7.23 ((1H, dd, J 16 and 3Hz, C6—H), 7.2—7.5 (2H, m, $SCH_2$), 7.5—8.1 (2H, m, C4—$H_2$) and 8.08 (3H, s, $COCH_3$) (Found: C, 59.6; H, 6.1; N, 7.7% $C_{18}H_{22}N_2O_4S$ requires C, 59.7; H, 6.1; N, 7.7%).

(ii)

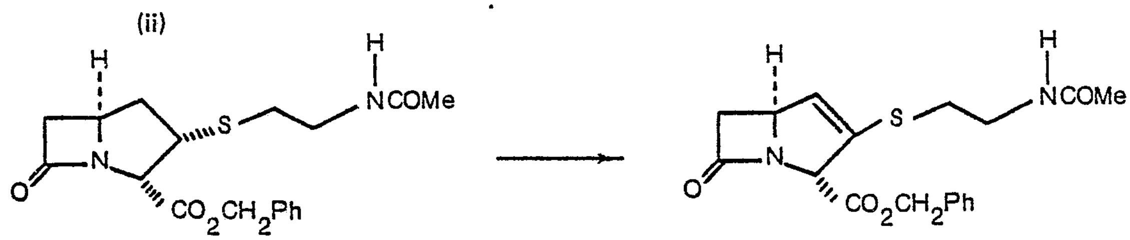

(57)

The $2\alpha,3\alpha$-isomer of benzyl 3-(2-acetamidoethylthio)-7-oxo-1-azabicyclo[3.2.0]heptane-2-carboxylate (0.054 g) was dissolved in dry benzene (4 ml) and treated with pyridine (0.026 g). The solution was stirred under argon and cooled at 5°C, when iodobenzene dichloride (0.045 g) was added. After 5 minutes the mixture was set aside in the refrigerator for 2 hours. The solution was decanted from a sticky residue, which was then extracted with ethyl acetate. Combined solutions were concentrated and chromatographed on silica gel 60 (<230 Mesh) eluting with ethyl acetate/ethanol 19:1 to give benzyl 3-(2-acetamidoethylthio)-1-azabicyclo[3.2.0]hept-3-ene-2-carboxylate (57) (0.012

g); m.p. 104—6° (ethyl-acetate/60—80° petroleum ether); $\nu_{max}$ ($CHCl_3$) 3450, 2990, 2920, 1780, 1745, 1670, 1575 and 1510 $cm^{-1}$; $\tau$ ($CDCl_3$) 2.67 (5H, s, phenyl), 4.02 (1H, t, J 2Hz, C4—H), 4.20 (1H, brs, NH), 4.83 (2H, s, benzyl $CH_2$), 4.92 (1H, dd, J 3 and 2Hz, C2—H), 5.3—5.6 (1H, m, C5—H), 6.4—6.8 (3H, m, C6—H and $NCH_2$), 7.0—7.3 (3H, m, C6—H and $SCH_2$) and 8.06 (3H, s, $COCH_3$) (Found: C, 59.7; H, 5.6; N, 7.6% $C_{18}H_{20}N_2O_4S$ requires C, 60.0; H, 5.6; N, 7.8%).

Example 52

Benzyl 3-(2-acetamidoethylthio)-7-oxo-1-azabicyclo[3.2.0]hept-2-ene-2-carboxylate

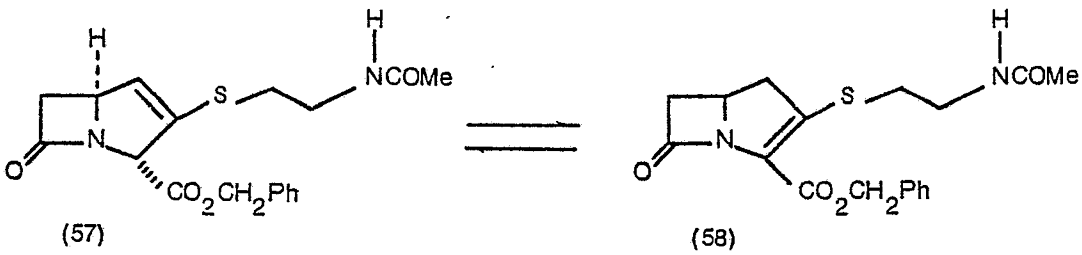

(57) (58)

A solution of benzyl 3-(2-acetamidoethylthio)-7-oxo-1-azabicyclo[3.2.0]hept-3-ene-2-carboxylate (57) (0.050 g) in dry methylene chloride (5 ml) was treated with 1,5-diazabicyclo[5.4.0]undec-5-ene (0.005 g). It was stirred under argon for 5 hours at room temperature and then concentrated. Chromatography on silica gel 60 (1:1 mixture of 230—400 mesh and <230 mesh) eluting with ethylacetate/ethanol 4:1 gave recovered Δ-3 compound (57) (0.031 g) plus benzyl 3-(2-acetamidoethylthio)-7-oxo-1-azabicyclo[3.2.0]hept-2-ene-2-carboxylate (58) (0.012 g); $\nu_{max}$ ($CHCl_3$) 3450, 2990, 1785, 1690 sh, 1670, 1550 and 1510 $cm^{-1}$; $\lambda$max (ethanol) 316 nm; $\tau$ ($CDCl_3$) 2.5—2.9 (5H, m, phenyl), 4.08 (1H, br, NH), 4.74 (2H, s, benzyl $CH_2$), 5.6—6.0 (1H, m, C5—H), 6.4—7.3 (8H, m, C4—$H_2$, C6—$H_2$, $NCH_2CH_2S$) and 8.06 (3H, s, $COCH_3$) ($M^+ - 42$ at m/e 318).

Example 53

p-Nitrobenzyl 3-(2-acetamido-2-methoxycarbonylethylthio)-7-oxo-1-azabicyclo[3.2.0]heptane-2-carboxylate

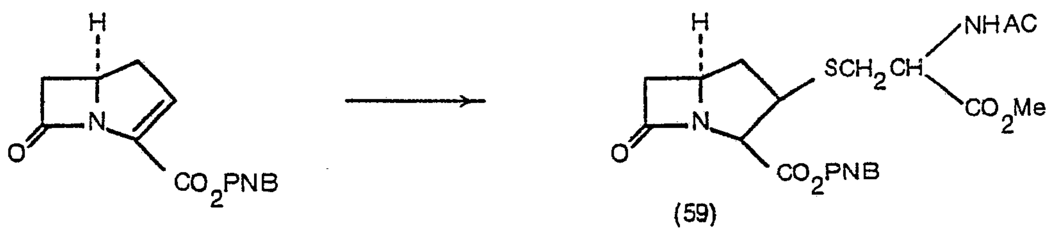

(59)

p-Nitrobenzyl 7-oxo-1-azabicyclo[3.2.0]hept-2-ene-2-carboxylate (557 mg) in dry dimethylformamide (10 ml) was treated with the methyl ester of N-acetyl L-cysteine (342 mg) and potassium carbonate (27.5 mg). After stirring at ambient temperature for 1 hour the orange solution was diluted with ethyl acetate (150 ml) then washed with water and brine, dried over magnesium sulphate and tiltered. The filtrate was evaporated to dryness then chromatographed on silica gel (<230 Mesh, 30 g), eluting with ethyl acetate.

The first material isolated (Fraction 7) was a single isomer (I) (N.M.R. and T.L.C. analysis) of the required product (59). This isomer was initially obtained as a gum (155 mg) which solidified on trituration with ether. Recrystallisation (x2) from ethyl acetate/petroleum ether (60°—80°) gave white crystals m.p. 130—5°, $[\alpha]_D^{25°}$ +89° in chloroform; $\nu_{max}$ ($CHCl_3$) 3430 (wk), 1765, 1745 (st), 1678, 1522, 1348 $cm^{-1}$; $\delta$ ppm ($CDCl_3$) 1.63 (1H, ddd, J 13.6, 8.3 and 7.1Hz, 1C4—H), 2.03 (3H, s, $NHCOCH_3$), 2.64 (1H, ddd, J 13.6, 6.6 and 6.6Hz, 1C4—H), 2.83 (1H, dd, J 16 and 2.3 Hz, 1C6—H), 2.94 (1H, dd, J 13.8 and 4.7Hz, SCH), 3.15 (1H, dd, J 13.8 and 4.7Hz, SCH), 3.34 (1H, dd, J 16 and 4.9Hz, 1C6—H), 3.74 (3H, s, OMe), — partially obscuring 3.70—4.00 (2H, m's, C3 and C5—H's), 4.38 (1H, d, J 5.8Hz, C2—H), 4.88 (1H, dt, J 8.0 and 4.7Hz), 5.30 (2H, s, benzyl $CH_2$, 6.55 (1H, broad d, J 8.0Hz, NH), 7.54 and 8.23 (4H, 2d's, J 8.5Hz, Ar) (Found: C, 51.74; H, 4.66; N, 8.92% $C_{20}H_{23}N_3O_8S$ requires C, 51.61; H, 4.95; N, 9.03%).

The next fractions (8—9) gave a gum (327 mg), which was stood under ether at +8° for 3 days. The ethereal solution was decanted off and the gummy material remaining crystallised from ethylacetate/petroleum ether (60°—80°) to give white crystals (146 mg), $[\alpha]_D^{25°}$ = −2° in chloroform. The n.m.r. spectrum showed 30—40% of isomer (I) mixed with another isomer (II) of (59). Rechromatography on silica gel (<230 M) eluting with chloroform then 5% ethanol in chloroform partially separated (I) and (II). The last fractions shown by T.L.C. to contain a predominance of (II) were combined and evaporated. Crystallisation from ethyl acetate/petroleum ether (60°—80°) (x3) gave (II)

as white crystals m.p. 112—117°, $[\alpha]_D^{25°}$ −51° in chloroform; $\nu_{max}$ ($CHCl_3$) 3430 1765, 1745, 1678, 1522, 1350 $cm^{-1}$; δ ppm ($CDCl_3$) 1.62 (1H, ddd, J 13.5, 8.7 and 7.3Hz, 1C4—H), 2.02 (3H, s, $NHCOCH_3$), 2.63 (1H, ddd, J 13.5, 6.6 and 6.6Hz, 1C4—H), 2.78 (1H, dd, J 16 and 2.3Hz, 1C6—H), 2.90 (1H, dd, J 13.8 and 6.2Hz, SCH), 3.20 (1H, dd, J 13.8 and 4.9Hz, SCH), 3.33 (1H, dd, J 16 and 5Hz, 1C6—H), 3.74 (3H, s, OMe) — partially obscuring ca 3.70—4.00 (2H, m's, C3 and C5—H's), 4.38 (1H, d, J 5.9Hz, C2—H), 4.79 (1H, ddd, J 7.6, 6.2 and 4.9Hz, CHNH), 5.29 (2H, s, benzyl $CH_2$), 6.31 (1H, broad d, J 7.6Hz, NH), 7.55 and 8.23 (4H, 2d's, J 8.5Hz, Ar) (Found: C, 51.78; H, 4.81; N, 8.80% $C_{20}H_{23}N_3O_8S$ requires C, 51.61; H, 4.95; N, 9.03%).

Fraction (10) gave a gum (74 mg) shown by TLC and N.M.R. to contain about 50% of (59) isomer (II).

Fractions (12—16) gave two more isomers III and (IV) of (59) in the ratio 2:1 (N.M.R. analysis) as a white solid (100 mg) (ex ether), $\nu_{max}$ ($CHCl_3$) 3420 (wk), 1765, 1742, 1678, 1521, 1345 $cm^{-1}$; δ ppm ($CDCl_3$) 2.01 (3H, s, $NHCOCH_3$) — partially obscuring (1H, m, 1C4—H), 2.33 (1H, ddd, J 12, 5 and 5Hz, 1C4—H), 2.68—3.30 [4H, including 2.78 (1H, dd, J 16 and 2.5Hz, 1C6—H), 3.13 (1H, dd, J 16 and 4.5Hz, 1C6—H) — partially obscuring (2H, m, $SCH_2$)], 3.74 (3H, s, OMe) — obscuring (2H, C3 and C5—H's), 4.13 (1H, d, J 7.5Hz, C2—H), 4.78 (1H, dt, J 7 and 5Hz, CHNH), 5.12—5.47 (2H, m, benzyl $CH_2$), 6.35 and 6.51 (1H, 2d's J 7Hz, NH, major isomer δ 6.51) 7.60 and 8.22 (4H, 2d's, J 8.5Hz, Ar).

Rechromatography of the material (75 mg) from fraction (11) gave a further 35 mg of (III) and (IV) as a white solid, which was combined with the solid from (12—16) $[\alpha]_D^{25°}$ +87° in chloroform (Found: $M^+$ at 465.1231 $C_{20}H_{23}N_3O_8S$ requires $M^+$ at 465.1203).

Treatment of this mixture of (III) and (IV) (90 mg) in dry methylene chloride (5 ml) with D.B.U. (8.8 mg) at ambient temperature for 4 hours, followed by chromatography on silica gel (<230 Mesh) eluting with ethyl acetate gave isomers (I) and (II) of (59), as a white solid (39 mg). $[\alpha]_D^{25°}$ +41° in chloroform. T.L.C. and N.M.R. showed a predominance of the less polar isomer (I).

## Example 54

p-Nitrobenzyl 3-(2-acetamido-2-methoxycarbonylethylthio)-7-oxo-1-azabicyclo[3.2.0]hept-3-ene-2-carboxylate

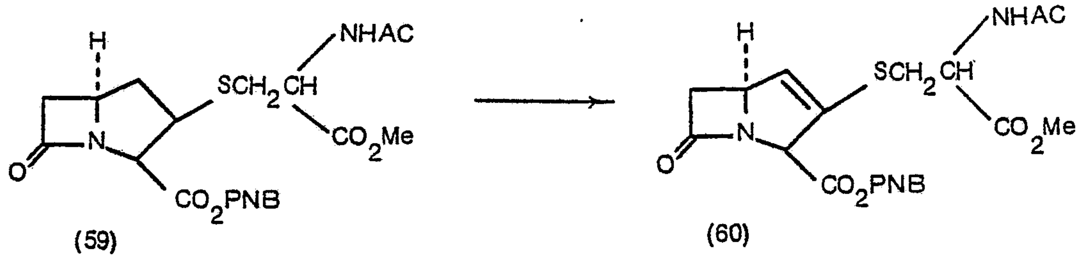

(59) (60)

p-Nitrobenzyl 3-(2-acetamido-2-methoxycarbonylethylthio)-7-oxo-1-azabicyclo[3.2.0]heptane-2-carboxylate (59) (1:1 mixture of isomers (I) and (II) by T.L.C.) (100 mg) in dry benzene (8 ml) was treated with pyridine (41 mg). The almost clear solution obtained was stirred under argon, cooled to its freezing point and iodobenzene dichloride (71 mg) added in one portion. The clear yellow solution was stood at +8°. After 1 hour the by now colourless solution was decanted off from the yellow gum which had formed. The gum was then taken up in dry methylene chloride. Both the benzene and methylene chloride solutions were chromatographed on a column of silica gel (230—400 Mesh, 4 x 2 cm). Elution with ethyl acetate gave the required product (60) as a white solid (34 mg) m.p. 151—160°, $\nu_{max}$ ($CHCl_3$) 3420 (wk), 1778, 1745, 1678, 1570 (wk), 1520, 1342 $cm^{-1}$; δ ppm (D.M.F. $-D_7$) 1.92 (3H, s, NHCO $CH_3$), 2.70—3.55 [4H, including 2.93 (1H, dd, J 16 and 2.5Hz 1C6—H), 3.40 (1H, dd, J 16 and 5.5Hz, 1C6—H), partially obscuring (2H, m, $SCH_2$)], 3.67 (3H, s, OMe) 4.54—4.76 (2H, overlapping m's, C5—H and CHNH), 5.10—5.20 (1H, m, C2—H), 5.40 (2H, s, benzyl $CH_2$), 6.24 (1H, m, C4—H), 7.75 (2H, d, J 8Hz, Ar), 8.30 (2H, d, J 8Hz, Ar) — obscuring (1H, NH).

## Example 55

p-Nitrobenzyl 3-(2-acetamidoethylthio)-7-oxo-1-azabicyclo[3.2.0]hept-3-ene-2-carboxylate

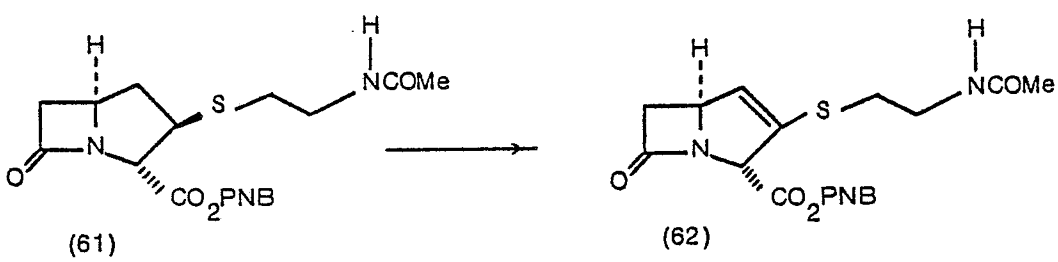

(61) (62)

A solution of p-nitrobenzyl 3-(2-acetamidoethylthio)-7-oxo-1-azabicyclo[3.2.0]heptane-2-carboxylate (61) (0.315 g) in dry benzene (60 ml) was stirred under argon and treated with pyridine (0.142 g). It was cooled to 5°C and treated with iodobenzene dichloride (0.296 g). After a period of 4 hours at 5°C, the solution was filtered and the residue washed with ethyl acetate. Combined organic solutions were concentrated and then chromatographed on silica gel 60 (<230 Mesh) eluting with ethyl acetate/ethanol 9:1 to give p-nitrobenzyl 3-(2-acetamidoethylthio)-7-oxo-1-azabicyclo[3.2.0]hept-3-ene-2-carboxylate (62) (0.087 g), m.p. 149—150°C (ethyl acetate); $\nu_{max}$ ($CHCl_3$) 3420, 2980, 2930, 1775, 1750, 1670, 1605, 1570, 1520 and 1350 $cm^{-1}$; $\tau$ ($CDCl_3$) 1.75 and 2.45 (4H, 2d, J 9Hz, phenyl) 3.91 (1H, t, J 2Hz, C4—H), 4.15 (1H, broad, NH), 4.71 (2H, s, benzyl $CH_2$), 4.84 (1H, dd, J 3 and 2Hz, C2—H), 5.3—5.5(1H, m, C5—H), 6.54 (2H, q, J 7Hz, $NCH_2$), 6.58 (1H, dd, J 16 and 5Hz, C6—H), 6.8—7.2 (2H, m, $SCH_2$), 7.13 (1H, dd, J 16 and 3Hz, C6—H) and 8.02 (3H, s, $COCH_3$).

## Example 56

p-Nitrobenzyl 3-(2-acetamidoethylthio)-7-oxo-1-azabicyclo[3.2.0]hept-2-ene-2-carboxylate

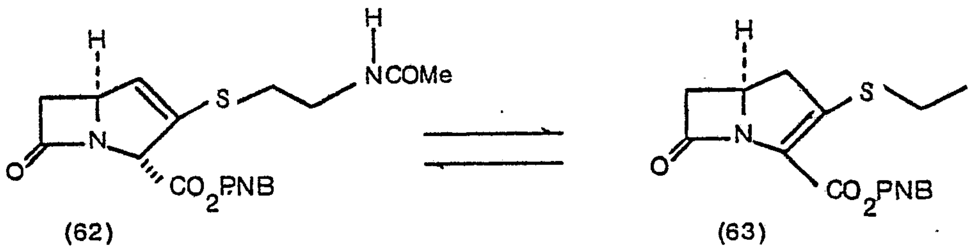

(62) (63)

p-Nitrobenzyl 3-(2-acetamidoethylthio)-7-oxo-1-azabicyclo[3.2.0]hept-3-ene-2-carboxylate (62) (0.091 g) was stirred in dry methylene chloride (10 ml) under argon, and treated with D.B.U. (0.009 g). The solution was concentrated after a period of 4 hours at room temperature, and then chromatographed on silica gel 60 (230—400 Mesh) eluting with ethyl acetate/ethanol 4:1. This gave recovered Δ-3 compound (62) (0.050 g) followed by p-nitrobenzyl 3-(2-acetamidoethylthio)-7-oxo-1-azabicyclo[3.2.0]hept-2-ene-2-carboxylate (63) (0.033 g) m.p. 130—133° (ethyl acetate) $\lambda_{max}$ (ethanol) 316 nm ($\varepsilon$ 12,400), $\nu_{max}$ ($CHCl_3$) 3400, 2970, 1780, 1700, 1670, 1605, 1550, 1520 and 1350 $cm^{-1}$; $\tau$ ($CDCl_3$) 1.75 and 2.34 (4H, 2d, J 9Hz, phenyl), 4.10 (1H, broad, NH), 4.48 and 4.76 (2H, ABq, J 14 Hz, benzyl $CH_2$), 5.73 (1H, tdd, J 9, 6 and 3Hz, C5—H), 6.3—7.2 (8H, m, C6—$H_2$, C4—$H_2$ and $NCH_2CH_2S$) and 8.00 (3H, s, $COCH_3$).

## Example 57

Sodium 3-(2-acetamidoethylthio)-7-oxo-1-azabicyclo[3.2.0]hept-2-ene-2-carboxylate

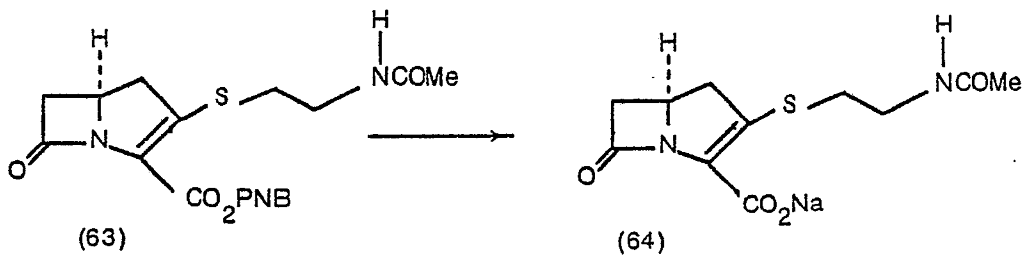

(63) (64)

5% Palladium on charcoala catalyst (0.045 g) was prehydrogenated for 20 minutes in a mixture of dioxan (5 ml) and water (2.5 ml). A solution of p-nitrobenzyl 3-(2-acetamidoethylthio)-7-oxo-1-azabicyclo[3.2.0]hept-2-ene-2-carboxylate (63) (0.030 g) in dioxan (5 ml) was then added and it was hydrogenated at atmospheric pressure for 2.5 hours. The product was treated with sodium bicarbonate (0.006 g) in water (0.5 ml) and then filtered through Celite. The filtrate was concentrated to the point of turbidity and then extracted twice with ethyl acetate (5 ml). The resulting aqueous solution was applied to a column of Biogel P—2 (200—400 Mesh) (25 x 100 mm) and eluted with water to give an aqueous solution of sodium 3-(2-acetamidoethylthio)-7-oxo-1-azabicyclo[3.2.0]hept-2-ene-2-carboxylate (64) (0.008 g) $\lambda_{max}$ (water) 300 nm.

Example 58

p-Nitrobenzyl 6-(oxo-ethan-2-yl)-7-oxo-1-azabicyclo[3.2.0]hept-2-ene-2-carboxylate

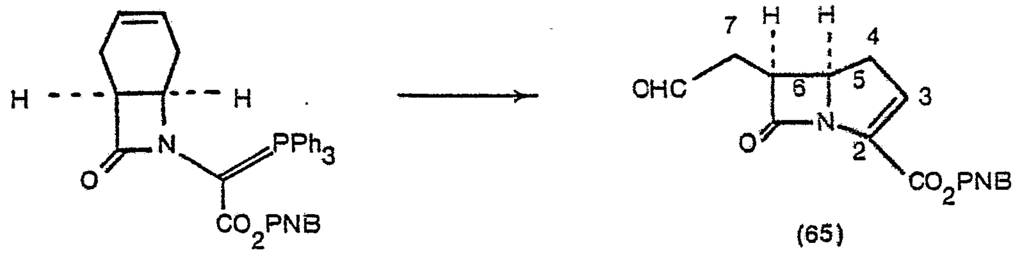

(65)

7-(1-p-Nitrobenzyloxycarbonyl-1-triphenylphosphoranylidenemethyl)-8-oxo-7-azabicyclo[4,2,0]oct-3-ene (10.0 g) suspended in ethyl acetate (650 ml) was treated with trifluoroacetic acid (65 ml) and the clear solution was stired at room temperature for 20 min. The solution was cooled to −70° and a stream of ozonised oxygen was passed (45 min). The solution was then purged with argon gas at this temperature (15 min), triphenylphosphine (4.8 g) added, and the solution was allowed to warm to −5°. Saturated aqueous sodium hydrogen carbonate solution (500 ml) was added and neutralisation to pH7 was completed by the addition of solid sodium hydrogen carbonate at 0°. The ethyl acetate was separated, and the aqueous phase extracted with further portions of ethyl acetate (3 x 100 ml). The organic layers were combined and dried ($Na_2SO_4$). Recovery gave a pale-orange gum, which was chromatographed on Kieselgel 60 (20 x 6 cm; 1:1 mixture of 230—400 mesh, and <230 mesh grades). The material was applied to the column in ethyl acetate-toluene l;l; 20 ml). Elution with ethyl acetate-light petroleum (3:1; 20 ml fractions) afforded triphenyl phosphine, followed by the title compound (65) (fractions 9—14; 1.58 g). Rechromatography of fractions 15—23, which contained triphenylphosphine oxide, led to the isolation of a further quantity of aldehyde (65) (670 mg) (yield 2.25 g; 37%). Recrystallisation from ethylacetate-diethylether afforded prisms m.p. 132—133° (bulk 128—131°), $\nu_{max}$ ($CHCl_3$) 1780, 1730, 1610, 1525 and 1350 $cm^{-1}$; $\delta$ ($CDCl_3$) 2.5—3.2 (4H, m, C4—$H_2$ and C8—$H_2$), 4.05 (1H, dt, J 11 and 6Hz, C6$\alpha$—H), 4.53 (1H, td, J 10 and 6Hz, C5$\alpha$—H), 5.24 and 5.45 (2H, ABq, J 14Hz), 6.57 (1H, t, J 3Hz, C3—H), 7.58 and 8.20 (4H, AA'BB', J 8Hz), and 9.79 (1H, s) (Found: C, 58.0; H, 4.5; N, 8.4. $C_{16}H_{14}N_2O_6$ requires C, 58.2; H, 4.3; N, 8.5%).

Example 59

p-Nitrobenzyl-6$\beta$-(2-oxoethyl)-3-ethylthio-7-oxo-1-azabicyclo[3.2.0]heptane-2-carboxylate

*Method (i)* From the isolated aldehyde (65)

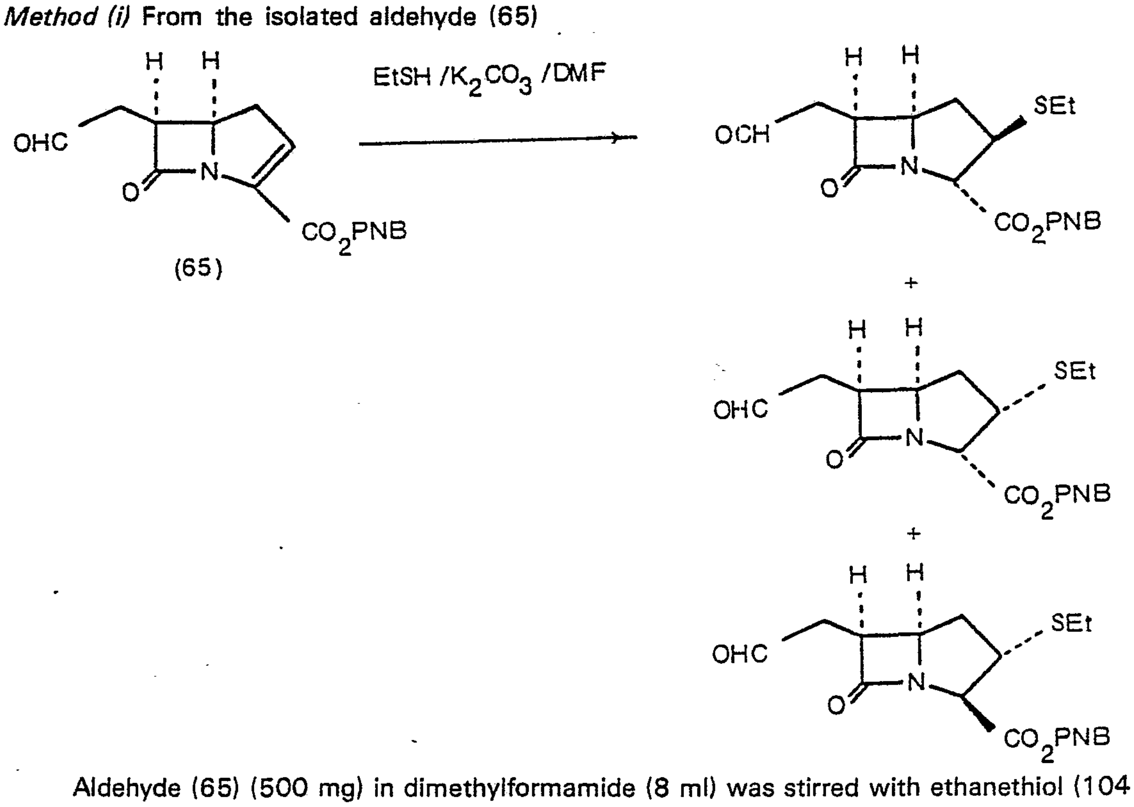


(65)

(66)

\+

(67)

\+

(68)

Aldehyde (65) (500 mg) in dimethylformamide (8 ml) was stirred with ethanethiol (104 mg; 87

μl; 1.1 equiv.) in the presence of finely-ground potassium carbonate (52 mg; 0.25 equiv.) at room temperature for 10 min. The solution was diluted with ethyl acetate (45 ml), extracted with saturated aqueous sodium chloride solution (3 x 10 ml), and dried ($Na_2SO_4$). Recovery gave an oil which was chromatographed on Kieselgel 60 (15 x 4 cm; 1:1 mixture 230—400 mesh and <230 mesh grades). Elution with ethyl acetate-light petroleum (1:1; 25 ml fractions) led to the isolation of isomers (66—68) of the title compound as gums (86%). Isomers (66) and (67) (0.352 g; 59%) (fractions 14—21) were obtained as a mixture (*ca* 2:1 ratio; nmr spectrum).

Rechromatography afforded the pure materials.

*C2α,C3β-Isomer (66):* $\nu_{max}$ ($CHCl_3$) 2735 wk, 1765, 1755, 1730, 1610, 1525 and 1350 $cm^{-1}$; δ ($CDCl_3$) 1.18 (3H, t, J 7Hz, $SCH_2$ $CH_3$), 1.9—2.2 (2H, m, C8—$H_2$), 2.57 (2H, q, J 7Hz, $SCH_2CH_3$), 2.5—3.0 (2H, m, C4—$H_2$), 3.43 (dt, J 10, 7Hz, C6α—H), 3.7—4.25 (2H, m, C3α—H and C5α—H), 4.73 (1H, d, J 7Hz, C2β—H), 5.26 (2H, s), 7.54 and 8.20 (4H, AA'BB', J 9Hz), 9.76 (1H, s, C9—H); C1 ($NH_3$ gas) m/e 410 (40%) $(M + NH_4)^+$, m/e 393 (100%) $(M + H)^+$ (Found: $M^+$, 392.1022. $C_{18}H_{20}N_2O_6S$ requires M, 392.1042).

*C2α,C3α Isomer (67):* $\nu_{max}$ ($CHCl_3$) 2730 wk, 1770, 1750, 1730, 1610, 1525 and 1350 $cm^{-1}$; δ ($CDCl_3$) 1.23 (3H, t, J 7Hz $SCH_2CH_3$), 1.5—2.25 (4H, m, C4—$H_2$ and C8—$H_2$), 2.58 (2H, q, J 7Hz, $SCH_2CH_3$), 3.4—4.15 (3H, m, C3β—H, C5α—H and C6α—H), 4.25 (1H, d, J 7Hz, (2β—H), 5.26 (2H, s), 7.52 and 8.21 (4H, AA'BB', J 9Hz), and 9.77 (1H, s, C9—H). Continued elution of the original column (fractions 22—33) gave *C2β,C3α-Isomer (68)* as a gum (0.160 g; 27%) $\nu_{max}$ ($CHCl_3$) 2730 wk, 1768, 1745, 1728, 1610, 1525 and 1350 $cm^{-1}$; δ ($CDCl_3$) 1.20 (3H, t, J 7Hz $SCH_2CH_3$), 1.7—2.35 (4H, m, C4—$H_2$ and C8—$H_2$), 2.57 (2H, q, J 7Hz, $SCH_2CH_3$), 3.35—4.25 (3H, m, C3β—H, C5α—H and C6α—H), 4.20 (1H, J 8Hz, C2α—H), 5.31 (2H, s), 7.60 and 8.25 (4H, AA'BB', J 9Hz), and 9.77 (1H, s, C9—H) (Found: $M^+$ 392.1031. $C_{18}H_{20}N_2O_6S$ requires M, 392.1042).

*Method (ii)* 'In situ' preparation from phosphorane

The title compound was prepared in improved yield without isolation and purification of aldehyde (65). Ozonolysis of 7-(1-p-nitrobenzyloxycarbonyl-1-triphenylphosphoranylidenemethyl)-8-oxo-7-aza-bicyclo[4.2.0]oct-3-ene (20 g) was carried out as described above. The crude aldehyde (65) in dimethylformamide (15 ml) was stirred with ethanethiol (2.27 g; 2.75 ml; 1 equiv. based on phosphorane) in the presence of potassium carbonate (100 mg) at room temperature for 10 min. Recovery and chromatography as described in Method (i) led to the isolation of the C2α,C3β-isomer (66) together with the C2α,C3α-isomer. (67) (ratio 5:2; nmr spectrum) (8.5 g; 59% overall from phosphorane). Only traces of the corresponding C2β,C3α-isomer (68) could be detected (tlc analysis).

Example 60

p-Nitrobenzyl 3-ethylthio-6β-(2-hydroxyethyl)-7-oxo-1-azabicyclo[3.2.0]heptane-2-carboxylate (69—71)

Each isomer of the aldehyde (66—67) (example 59), in turn, was converted to the corresponding primary alcohol as follows:

a) From isomer (66) (example 59).

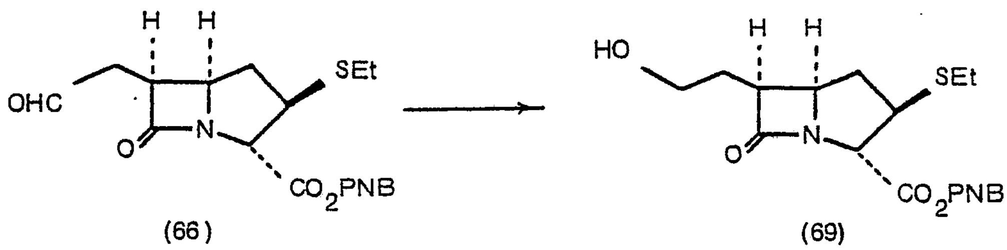

(66) (69)

The aldehyde (66) (48 mg) in tetrahydrofuran (0.5 ml) was treated with a solution of sodium borohydride (1.16 mg) 0.25 equiv.) in water (1 ml of a solution containing 11.6 mg; of reagent in 10 ml water), dropwise at 0°. The solution was stirred for 5 min. and pH 7 sodium phosphate buffer (0.5 ml) added. The solution was diluted with ethyl acetate (10 ml) and washed with saturated aqueous sodium chloride solution (3 x 1 ml). Evaporation gave the corresponding C2α-C3β-substituted alcohol (69) (48 mg) in quantitative yield as a gum, $\nu_{max}$ ($CHCl_3$) 3470 br, 1750 st, 1610, 1525, 1350 and 1180 $cm^{-1}$; δ ($CDCl_3$) 1.19 (3H, t, J 7Hz, $SCH_2CH_3$), 1.26 br (1H $D_2O$ exch., OH) 1.73 (1H, t, J 6Hz) and 1.83 (1H, t, J 6Hz) C8—$H_2$), 1.95—2.4 (2H, m, C4—$H_2$), 2.57 (2H, q, 7 Hz, $SCH_2CH_3$), 3.27—3.64 (2H, m, C3α—H and C6α—H), 3.74 (2H, t, J6 Hz, C9—$H_2$), 4.22 (1H, ddd, J 9,6 and 4Hz, C5α—H), 4.73 (1H, d, J 7Hz, C2β—H), 5.26 (2H, s), 7.54 and 8.20 (4H, AA'BB', J 9Hz) (Found: $M^+$, 394.1218. $C_{18}H_{22}N_2O_6S$ requires M, 394.1018); m/e (C1; $NH_3$ gas) 395 (100%) $(M + H)^+$.

b) From isomer (67) (example 59).

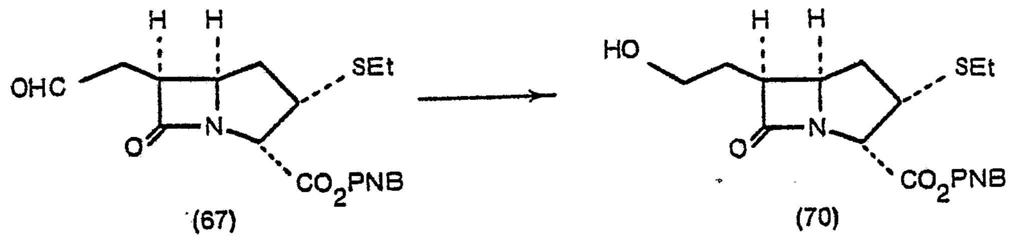

(67) (70)

The aldehyde (67) (150 mg) in tetrahydrofuran (2 ml) was treated with sodium borohydride (3.62 mg; 0.25 equiv.) in water (1 ml of a solution containing 36.2 mg of reagent in 10 ml water), dropwise at 0°. Recovery as described in a) gave the corresponding C2$\alpha$,C3$\alpha$-substituted alcohol (70) as a gum (140 mg) $\nu_{max}$ ($CHCl_3$) 3470 br, 1750 st, 1610, 1525, 1350 and 1170 $cm^{-1}$; $\delta$ ($CDCl_3$) 0.86 br (1H, $D_2O$ each, OH), 1.22 (3H, t, J 7Hz, $SCH_2CH_3$), 1.5—2.25 (4H, m, C8—$H_2$ and C4—$H_2$), 2.58 (2H, q, J 7Hz, $SCH_2CH_3$), 3.4—4.1 (3H, m, C3$\beta$—H, (5$\alpha$—H and C6$\alpha$—H), 3.74 (2H, t, J 6Hz, C9—$H_2$), 4.27 (1H, d, J 6.5 Hz, C2$\beta$—H), 5.26 (2H, s), 7.54 and 8.20 (4H, AA'BB', J 9Hz).

c) From isomer (68) (example 59)

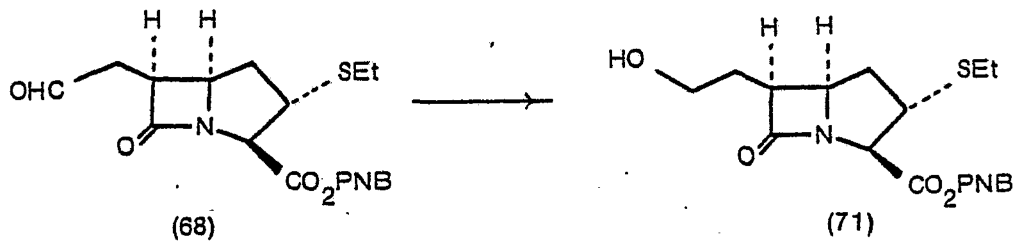

(68) (71)

The aldehyde (68) (230 mg) in tetrahydrofuran (3 ml) was stirred during the dropwise addition of a solution of sodium borohydride (6.1 mg; 0.25 equiv.) in water (2 ml) at 0°. After 5 min. the product was recovered as described under section a) to give the C2$\beta$,C3$\alpha$-isomer (71) of the title compound as a gum (215 mg) $\nu_{max}$ ($CHCl_3$) 3470 br, 1760, 1740, 1610, 1525, 1350 and 1175 $cm^{-1}$; $\delta$ ($CDCl_3$) 1.21 (3H, t, J 7Hz, $SCH_2CH_3$), 1.7—2.4 (5H, m, C8—$H_2$, C4—$H_2$ and OH), 2.57 (2H, q, J 7Hz), 3.35 (1H, m, $W\frac{1}{2}$ 11Hz, C6$\alpha$—H), *ca* 3.6 (1H, m, C3$\beta$—H), 3.68 (2H, t, J 6Hz, C9—$H_2$), 3.86 (1H, m, C5$\alpha$—H), 4.17 (1H, d, J 8Hz, C2$\alpha$—H), 5.28 (2H, s), 7.58 and 8.20 (4H, AA'BB', J 9Hz).

The alcohols (69—71) were generated from the aldehydes when required, dried and converted to a carboxylate or sulphonate ester.

p-Nitrobenzyl 6β-(2-acetoxyethyl)-3-ethylthio-7-oxo-1-azabicyclo[3.2.0]hept-2-ene-2-carboxylate

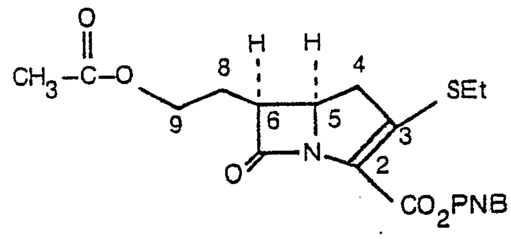

(76)

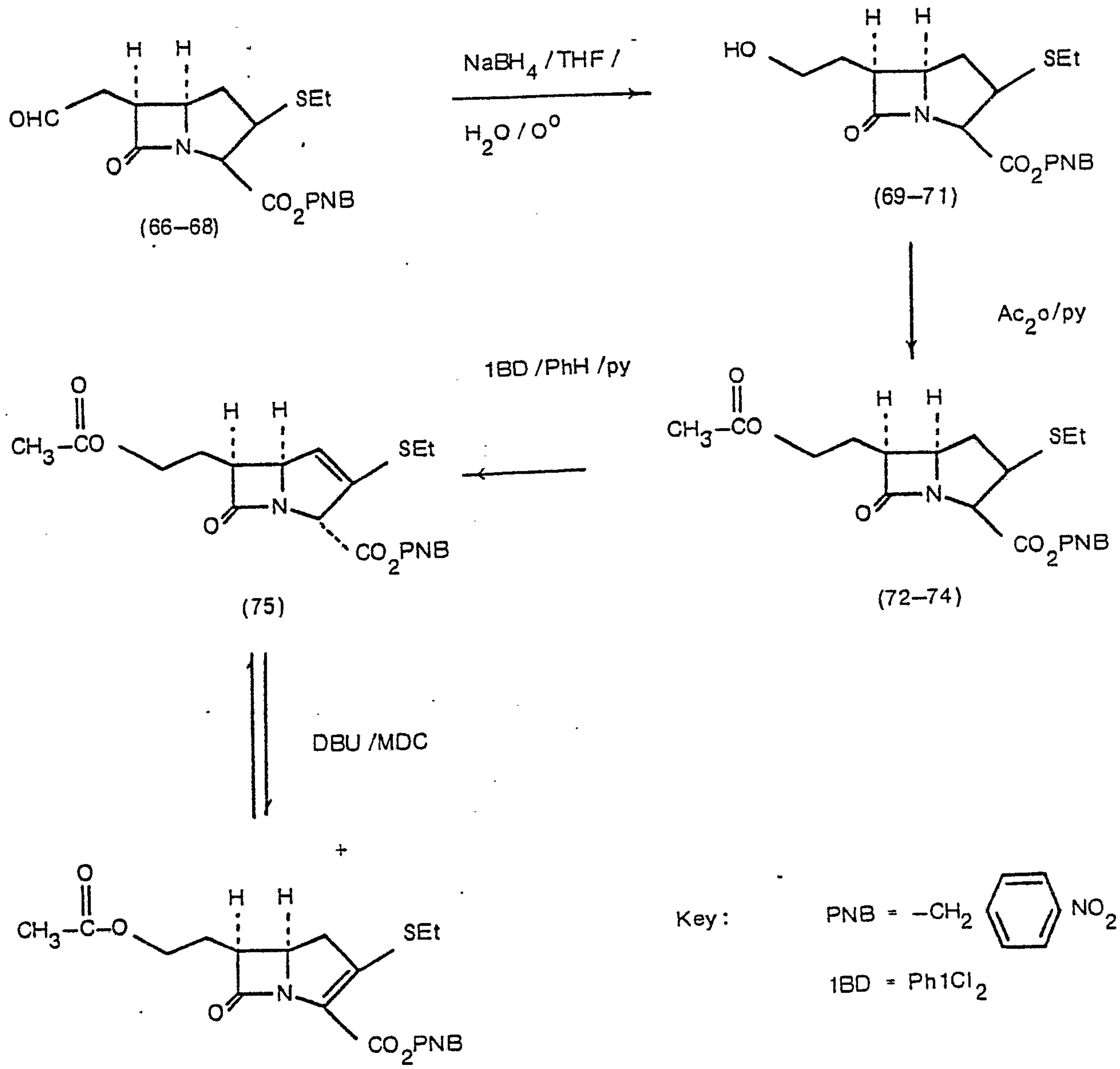

Example 61

p-Nitrobenzyl 6β-(2-acetoxyethyl)-3-ethylthio)-7-oxo-1-azabicyclo[3.2.0]heptane-2-carboxylate

Each isomer of the primary alcohol (69—71) (example 60) was converted, in turn, to the corresponding acetate.

a) From isomer (69) (example 60)

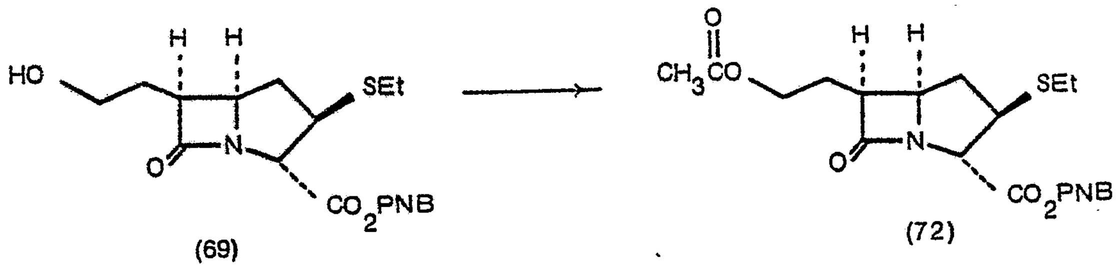

(69) (72)

The freshly prepared alcohol (69) (450 mg) in anhydrous pyridine (5 ml) was stirred in the presence of an excess of acetic anhydride (200 μl) at room temperature for 4 h. The reaction mixture was diluted with ethyl acetate (50 ml), extracted with saturated aqueous citric acid solution (20 ml) followed by saturated aqueous sodium chloride solution (2 x 10 ml), and dried ($Na_2SO_4$). Evaporation and chromatography of the residue on Kieselgel 60 (11 x 3 cm) [elution with ethyl acetate-light petroleum (1:4)] afforded the C2α,C3α-isomer (72) of the title compound as a gum (400 mg), $\nu_{max}$ ($CHCl_3$) 1765, 1745 st, 1610, 1525, 1350, and 1220 $cm^{-1}$; δ ($CDCl_3$) 1.20 (3H, t, J 7Hz, $SCH_2CH_3$), 1.7—2.0 (2H, m, C8—$H_2$), 2.04 (3H, s), 2.10—2.26 (2H, m, C4—$H_2$), 2.58 (2H, q, J 7Hz, $SCH_2CH_3$), 3.44 (1H, dt, J 11 and 6.5Hz, C6α—H), 3.53 (1H, q, J 7Hz, C3α—H), 4.16 (2H, t, J 6Hz, C9—$H_2$), 4.2 (1H, m, $W\frac{1}{2}$ 13Hz, C5α—H), 4.74 (1H, d, J 7Hz, C2β—H), 5.27 (2H, s), 7.55 and 8.23 (4H, AA'BB', J 9Hz) (Found: $M^+$, 436.1308. $C_{20}H_{24}N_2O_7S$ requires M, 436.1302).

b) From isomer (70) (example 60)

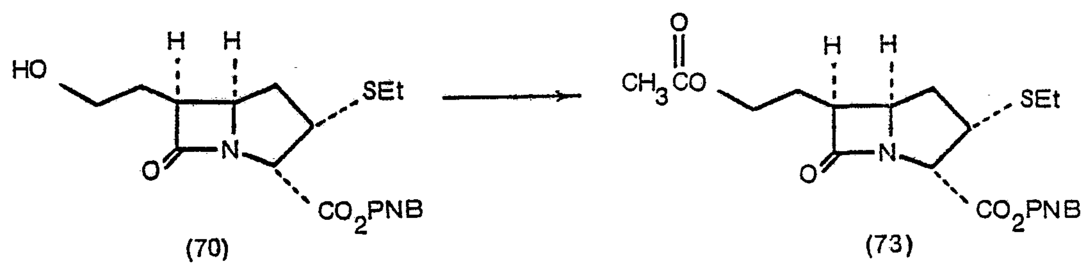

(70) (73)

The freshly-prepared alcohol (70) (40 mg) in anhydrous pyridine (0.25 ml) was acetylated with an excess of acetic anhydride as described above (example 61; a). Recovery and chromatography gave the C2α,C3α-isomer (73) of the title compound as a gum (35 mg), $\nu_{max}$ ($CDCl_3$) 1765, 1740 st, 1610, 1525, 1350 and 1240 $cm^{-1}$; δ ($CDCl_3$) 1.23 (3H, t, J 7Hz, — $SCH_2CH_3$), 1.5—1.8 (1H, m, one C4—H), 1.8—2.2 (2H, m, C8—$H_2$, 2.05 (3H, s), 2.3—2.6 (1H, m, other C4—H) 2.58 (2H, q, J 7Hz, — $SCH_2CH_3$), 3.52 (1H, ddd, J 9.5, 7, and 6Hz, C6α—H), 3.78 (1H, dt, J 10 and 7Hz, C3β—H), 3.97 (1H, dt, J 9 and 6Hz, C5α—H), 4.13 (2H, t, J 6Hz, C9—$H_2$), 4.27 (1H, d, J 7Hz, C2β—H), 5.29 (2H, s), 7.54 and 8.23 (4H, AA'BB', J 9Hz); INDOR irradiation at the frequency of the lower-field transition of the C2β—H doublet confirmed the position of the C3β—H resonance. Assignments were confirmed by irradiation at the frequency of the C8—$H_2$ resonance. This caused the C6α—H signal to simplify to a doublet (J 6Hz) and that due to the C9—$H_2$ to collapse to a singlet. Similarly, irradiation close to the frequency of the C3- and C5- proton resonances simplified the C4—$H_2$ signals at δ 1.6 and 2.4 (Found: $M^+$, 436.1312. $C_{20}H_{24}N_2O_7S$ requires M, 436.1302).

c) From isomer (71) (example 60)

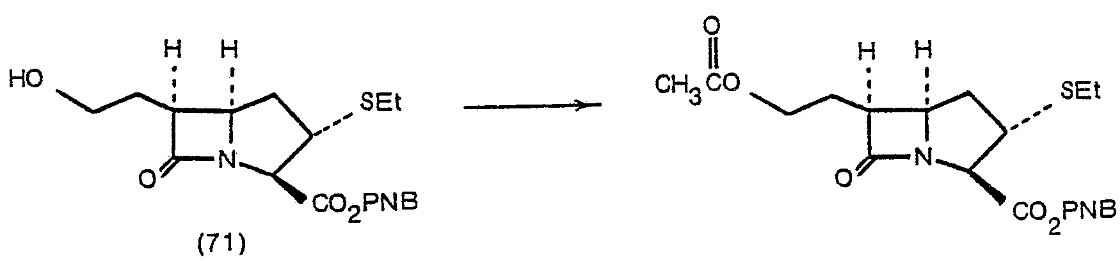

(71)

The freshly-prepared alcohol (71) (330 mg) in anhydrous pyridine (4 ml) was acetylated with an

excess of acetic anhydride as described above (example 61; a). Recovery and chromatography gave the C2$\beta$,C3$\alpha$-isomer (74) of the title compound as a gum (260 mg) $\nu_{max}$ (CDCl$_3$) 1768, 1740, 1610, 1525, 1350 and 1220 cm$^{-1}$; $\delta$ (CDCl$_3$) 1.20 (3H, t, J 7Hz, — SCH$_2$C$H_3$), 1.65—2.2 (4H, m, C8—H$_2$ + C4—H$_2$, 2.01 (3H, s), 2.57 (2H, q, J 7Hz, —SC$H_2$CH$_3$), 3.27 (1H, ddd, J 8, 6 and 7Hz, C6$\alpha$—H), 3.65 (1H, q, J 8Hz, C3$\beta$—H), 3.80 (1H, m, W$\frac{1}{2}$, 12Hz, C5$\alpha$—H), 4.07 (2H, t, J 6Hz, C9—H$_2$), 4.16 (1H, d, J 8Hz, C2$\alpha$—H, 5.28 (2H, s), 7.57 and 8.21 (4H, AA'BB', J 9Hz); irradiation at a frequency close to the centre of the C8—H$_2$ and C4—H$_2$ multiplets reduced the C9—H$_2$ resonance to a singlet, and simplified the signals due to the C3$\beta$— and C5$\alpha$-protons (Found: M$^+$, 436.1305. $C_{20}H_{24}N_2O_7S$ requires M, 436.1302).

Example 62

Isomerisation of isomer (74)

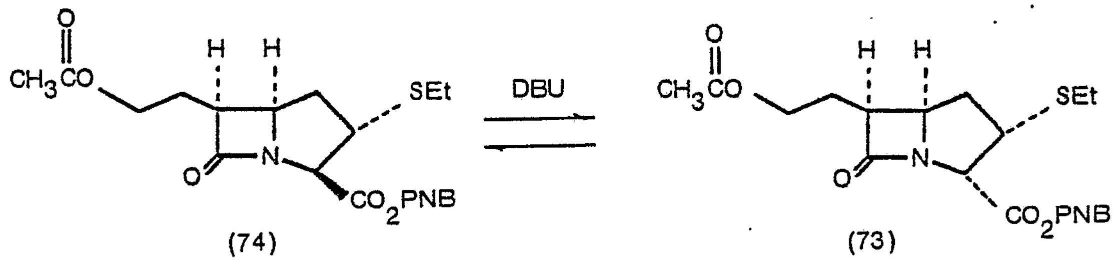


(74) (73)

p-Nitrobenzyl 6$\beta$ - (2 - acetoxyethyl) - 3$\alpha$ - ethylthio) - 7 - oxo - 1 - azabicyclo[3.2.0]heptane-2$\beta$-carboxylate (74) (example 61c) (210 mg) in ethyl acetate (5 ml) was treated with 1,5-diazabicyclo[5.4.0]undec-5-ene-(15 mg) at room temperature in an atmosphere of argon for 7 h. The mixture was chromatographed on Kieselgel 60 (230—400 mesh; 7 x 3 cm), eluting with ethyl acetate-light petroleum (1:1) (3 ml fractions). Fractions 16—20 contained the 2$\alpha$,3$\alpha$-isomer (73) (example 61c) (120 mg), identical (t.l.c. analysis, i.r. spectrum) with the previously-prepared sample.

Example 63

Iodobenzene dichloride-mediated dehydrogenation of isomer (72) (example 61a)

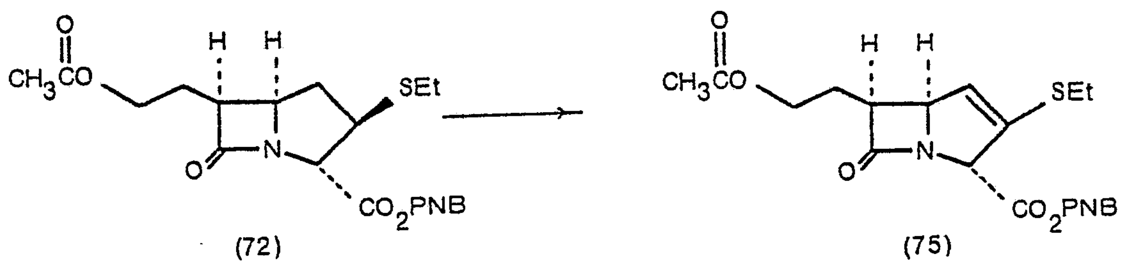

(72) (75)

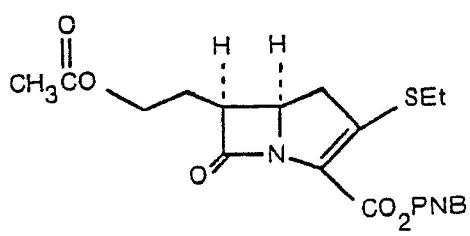

(76)

p-Nitrobenzyl 6$\beta$(2-acetoxyethyl)-3$\beta$-ethylthio-7-oxo-1-azabicyclo[3.2.0]heptane-2$\alpha$-carboxylate (72) (example 61a) (112 mg) in anhydrous benzene (3 ml) was treated successively with anhydrous pyridine (45 mg, 45 $\mu$l; 2 equiv.) and iodobenzene dichloride (78 mg, 1 equiv.) at 0—5° for 1 h. The reaction mixture was chromatographed on Kieselgel 60 (230—400 mesh) (7 x 2 cm) eluting with ethyl acetate-light petroleum (2:3) (5 ml fractions). Fractions 11—29 gave p-nitrobenzyl 6$\beta$-(2-acetoxyethyl)-3-ethylthio-7-oxo-1-azabicyclo[3.2.0]hept-3-ene-2$\alpha$-carboxylate (75) as a gum (64 mg), $\nu_{max}$ (CDCl$_3$) 1770, 1740 st, 1610, 1570 (vinyl thioether), 1525, 1350 and 1220 br cm$^{-1}$; $\delta$ (CDCl$_3$) 1.29 (3H, t, J 7 Hz, SCH$_2$C$H_3$), 1.7—2.2 (2H, m, C8—H$_2$), 2.04 (3H, s), 2.74 (2H, q, J 7Hz, SC$H_2$CH$_3$), 3.60 (1H, m, W$\frac{1}{2}$ 11Hz, C6$\alpha$—H), 4.17 (2H, t, J 6Hz, C9—H$_2$), 4.77 (1H, m, W$\frac{1}{2}$ 6.5Hz, C5$\alpha$—H), 5.12 (1H, dd, J 4 and 2Hz, C2$\beta$—H), 5.30 (2H, s), 5.73 (1H, t, J 2Hz, C4—H), 7.57 and 8.25 (4H, AA'BB', J 9Hz).

Continued elution of the column (fractions 37—41) afforded p-nitrobenzyl 6$\beta$-(2-acetoxyethyl)-3-ethylthio-7-oxo-1-azabicyclo[3.2.0]hept-2-ene-2-carboxylate (76) (9 mg), $\gamma_{max}$ (EtOH), 317, 267 nm;

$\nu_{max}$ ($CDCl_3$) 1780, 1740, 1700 br, 1610, 1550 (vinyl thioether), 1525, 1350 and 1210 $cm^{-1}$; $\delta$ ($CDCl_3$) 1.36 (3H, t, J 7Hz, $SCH_2CH_3$), 1.75—2.25 (2H, m, C8—$H_2$), 2.07 (3H, s), 2.90 (2H, q, J 7Hz, $SCH_2CH_3$), 3.06 (2H, d, J 9Hz, C4—$H_2$, 3.69 (1H, dt, J 9.5 and 6.5Hz, C6$\alpha$—H), 4.16 (2H, t, J 6Hz, C9—$H_2$), 4.31 (1H, dt, J 10 and 6.5Hz, C5$\alpha$—H), 5.20 and 5.48 (2H, ABq, J 14Hz), 7.60 and 8.19 (4H, AA'BB', J 9Hz) (Found: $M^+$, 434.1145. $C_{20}H_{22}N_2O_7S$ requires M, 434.1144).

## Example 64

Isomerisation of (75) (example 63)

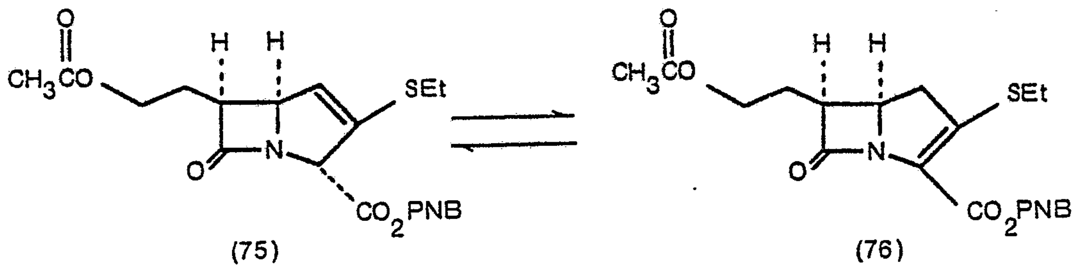

(75) (76)

p-Nitrobenzyl 6$\beta$ - (2) - acetoxyethyl) - 3 - ethylthio) - 7 - oxo-1-azabicyclo[3.2.0]hept-3-ene-2$\alpha$-carboxylate (75) (40 mg) in anhydrous dichloromethane (2 ml) was treated with 1,5-diazabicyclo[5.4.0]undec-5-ene (3 mg) at room temperature in an atmosphere of argon for 4 hr. The reaction mixture was chromatographed rapidly on a column of Keiselgel 60 (5 x 2 cm; 230—400 mesh). Elution with ethyl acetate-light petroleum (7:3) gave recovered Δ3 compound (75) (26 mg). Later fractions from the column gave p-nitrobenzyl-6$\beta$-(2-acetoxyethyl)-3-ethylthio-7-oxo-1-azabicyclo[3.2.0]hept-2-ene-2-carboxylate (76) (5 mg). The material was identical (t.l.c. analysis; i.r. and u.v. spectra) with the sample previously described (example 63).

Sodium 6β-ethyl-3-ethylthio-7-oxo-1-azabicyclo[3.2.0]hept-2-ene-2-carboxylate

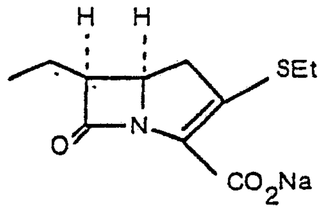

(82)

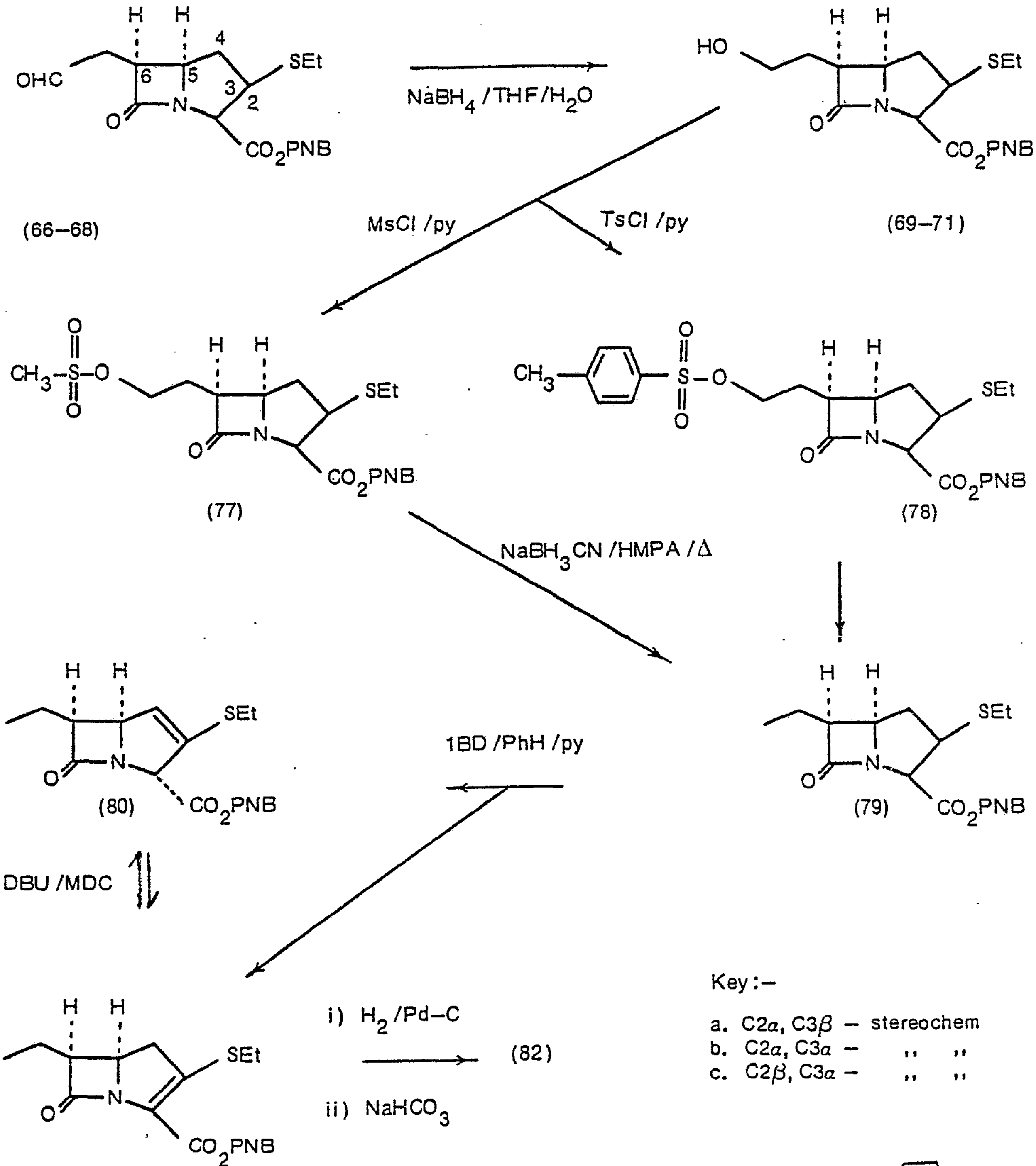


(66–68)

(69–71)

(77)

(78)

(80)

(79)

(81)

(82)

Key:–

a. C2α, C3β – stereochem
b. C2α, C3α – „ „
c. C2β, C3α – „ „

PNB = 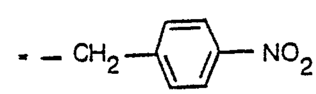

Example 65

p-Nitrobenzyl 6β-(2-methane sulphonyloxyethyl)-3-ethylthio-7-oxo-1-azabicyclo[3.2.0]heptan-2-carboxylate (77).

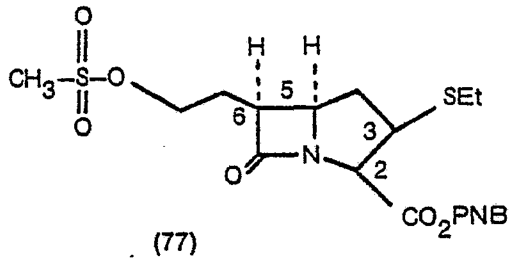

(77)

a: C2α,C3β-stereochemistry

b: C2α,C3α-stereochemistry

c: C2β,C3α-stereochemistry

The aldehydes (66—68) were converted via the alcohols (69—71) into their corresponding methanesulphonate esters, isomers (77; a—c). More conveniently, separation of the individual isomers (a—c) was effected at the last stage of the sequence by column chromatography.

Accordingly, a mixture of isomers (66—68) (example 59) of the aldehyde (3.0 g) in tetrahydrofuran (30 ml) was treated with a solution of sodium borohydride (73 mg; 1 equiv.; 0.25 molar ratio) in water (4 ml) dropwise at 0° over 5 min. The solution was stirred at 0° for a further 25 min. Recovery in ethyl acetate as previously described (example 60) gave the crude alcohols (69—71) (ca. 3.0 g).

The dry alcohols, in anhydrous pyridine (12 ml) were stirred in the presence of an excess of methanesulphonyl chloride (970 mg; 0.65 ml; 1.1 equiv.) at room temperature for 2 hr. The reaction mixture was diluted with ethyl acetate (75 ml) and washed with saturated aqueous citric acid solution (2 x 20 ml) and brine (2 x 10 ml). Evaporation afforded a gum (4.2 g) which was chromatographed on a column (8 x 7 cm) of Kieselgel 60 (1:1 mixture of 230—400 mesh, and <230 mesh grades), eluting with ethyl acetate-light petroleum (1:1) (25 ml fractions). Fractions 16—50 gave the title compound as a mixture of the C2α,C3β-isomer (77; a) together with the C2α,C3α-isomer (77; b) (2.299 g; 56%) (ca1:1 ratio; nmr spectrum).

Rechromatography of an aliquot of the mixture (200 mg) on a column of Kieselgel 60 (<230 mesh) (10 x 2 cm), eluting with ethyl acetate-light petroleum (2:3) (10 ml fractions) separated the individual isomers. The leading fractions gave the C2α,C3β-isomer (77; a) as a gum (48 mg), $\nu_{max}$ ($CDCl_3$) 1765, 1750, 1610, 1525, 1345 and 1175 $cm^{-1}$; δ ($CDCl_3$) 1.20 (3H, t, J 7Hz, $SCH_2CH_3$) 1.9—2.4 (4H, m, C4—$H_2$ and C8—$H_2$, 2.57 (2H, 1, J 7Hz, $SCH_2CH_3$), 3.02 (3H, s), 3.3—4.2 (3H, m, C3α—H and C6α—H), 4.35 (2H, t, J 6Hz, C9—$H_2$), 4.75 (1H, d, J 7Hz, C2β—H), 5.28 (2H, s) 7.55 and 8.23 (4H, J 9Hz, AA'BB'). Later fractions contained isomer (77; b) (42 mg), $\nu_{max}$ ($CHCl_3$) 1765, 1750, 1610, 1525, 1345 and 1170 $cm^{-1}$; δ ($CDCl_3$) 1.22 (3H, t, J 7Hz $SCH_2CH_3$), 1.64 (1H, dt, J 14 and 9Hz, C4—H), 1.9—2.25 (2H, m, C8—$H_2$), *ca* 2.45 (1H, m, C4—H), 2.57 (2H, q, J 7Hz, $SCH_2CH_3$), 3.02 (3H, s), 3.47—4.22 (3H, m, C3β—H, C5α—H and C6α—H), 4.28 (1H, d, J 7Hz, C2β—H), 5.33 (2H, t, J 6Hz, C9—$H_2$), 5.29 (2H, s), 7.54 and 8.22 (4H, J 9Hz, AA'BB').

*Decoupling experiments located the centres of these resonances at 3.78, 4.04 and 3.60 respectively.

Continued elution of the original column gave (fractions 54—60) the pure C2β,C3α-isomer (77; c) of the title compound (551 mg) (13%) as microcrystals, m.p. 132—135°, $\nu_{max}$ ($CHCl_3$) 1765, 1740, 1610, 1520, 1350 sh, 1345 and 1175 $cm^{-1}$; δ ($CDCl_3$) 1.20 (3H, t, J 7Hz, $SCH_2CH_3$), 1.90—2.25 (4H, m, C4—$H_2$ and C8—$H_2$, 2.57 (2H, q, J 7Hz, $SCH_2CH_3$), 3.00 (3H, s), 3.35 (1H, dt, J 9, 5Hz, C6α—H), 3.71 (1H, m, C3β—H), 3.90 (1H, m, C5α—H), 4.17 (1H, d, J 7Hz, C2α—H), 4.28 (2H, t, J 6Hz, C9—$H_2$), 5.29 (2H, s) 7.58 and 8.22 (4H, AA'BB', J 9Hz). (Found: C, 48.1; H, 5.2; N, 5.8; S, 13.3. $C_{19}H_{24}N_2O_8S_2$ requires C, 48.3; H, 5.1; N, 5.9; S, 13.6%). Total yield, overall from aldehydes (66—68), was 66%.

Most conveniently, the mixtures of isomeric methanesulphonates (77; a—c) were reduced with sodium cyanoborohydride (example 67) and the C2, C3-stereoisomers separated at that stage.

Example 66

p-Nitrobenzyl 6β-(2-toluene-p-solphonyloxyethyl-3-ethylthio)-7-oxo-1-azabicyclo[3.2.0]heptane-2-carboxylate (78)

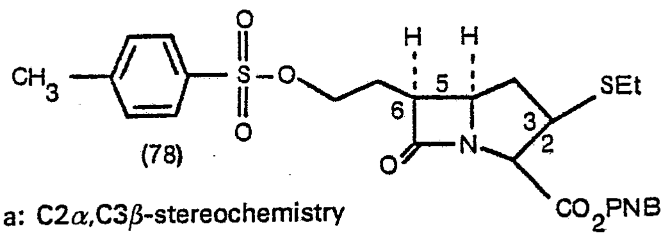

(78)

a: C2α,C3β-stereochemistry

b: C2α,C3α-stereochemistry

c: C2β,C3α-stereochemistry

The individual alcohol isomers (69—71) were dried and treated with toluene-p-sulphonyl chloride (1.1) equiv.) in a small volume of anhydrous pyridine at 0° — room temperature for 2 h. Recovery and chromatography of the products gave the toluene-p-sulphonate derivatives (78; a—c) (55—68%), together with variable amount of a by-product (85) (ca 20%). Unless the reaction conditions were kept rigorously anhydrous this pyrrolidine-lactone became the major product.

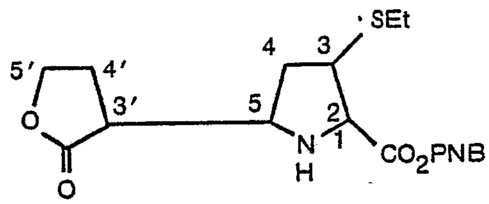

(85)

More conveniently, a mixture of the aldehyde isomers (66—68) (660 mg) in tetrahydrofuran (5 ml) was treated with an excess of sodium borohydride (20 mg) in water (2 ml) dropwise over 5 min. The reaction mixture was stirred at 0° for a further 25 min. Recove;ry in ethyl acetate as previously described (example 60) afforded the crude alcohols (69—71) (580 mg).

The alcohols (69—71) in pyridine (5 ml) were treated with an excess of toluene-p-sulphonylchloride (365 mg, 1.1 equiv.) at 0°. The reaction mixture was allowed to warm to room temperature, and stirring continued for 4 h. The reaction mixture was diluted with ethyl acetate (40 ml) and extracted with saturated aqueous citric acid solution (2 x 10 ml) and brine (2 x 10 ml). Evaporation gave the crude toluene-p-sulphonates (530 mg). Chromatography on Kieselgel 60 (1:1 mixture of 230—400 mesh, and <230 mesh grades) (15 x 4 cm), eluting with ethyl acetate-light petroleum (1:1) (10 ml fractions) gave the pure isomers. Fractions 11—20 gave the C2α,C3β-isomer (78; a) of the title compound (180 mg) as an oil $\nu_{max}$ ($CHCl_3$) 1765, 1755, 1610, 1600, 1525, 1360 sh, 1350 and 1175 $cm^{-1}$; δ ($CDCl_3$) 1.18 (3H, t, J 7Hz, $SCH_2CH_3$), 1.8—2.4 (4H, m, C8—$H_2$ and C4—$H_2$), 2.43 (3H, s), 2.56 (2H, q, J 7Hz, $SCH_2CH_3$), 3.25—3.75 (2H, m, C6α—H and C3α—H, 4.14 (2H, t, J 6Hz, C9—$H_2$), *ca* 4.2 (1H, m, C5α—H), 4.70 (1H, d, J 8Hz, C2β—H), 5.27 (2H, s), 7.35 and 7.77 (4H, J 9Hz, AA'BB' of toluene-p-sulphonate) and 7.55 and 8.23 (4H, J 9Hz, AA'BB' of PNB ester).

Further elution of the column (fractions 21—30) gave the C2α,C3α-isomer (78:b) (167 mg) $\nu_{max}$ ($CHCl_3$) 1765, 1750, 1610, 1600, 1525, 1370, 1350 and 1180 $cm^{-1}$. This sample was contaminated with *ca* 20% isomer (78; a) (nmr spectrum).

Continued elution (fractions 31—34) afforded the C2β,C3α-isomer (78; C) (73 mg) as a gum, $\nu_{max}$ ($CHCl_3$) 1765, 1740, 1610, 1600, 1525, 1350 st and 1175 $cm^{-1}$; δ ($CDCl_3$) 1.23 (3H, t, J 7Hz, $SCH_2CH_3$) 1.8—2.3 (4H, m, C8—$H_2$ and C4—$H_2$), 2.47 (3H, s), 2.57 (2H, q, J 7Hz, $SCH_2CH_3$), 3.25—4.1 (1H, m, C3—H, C5—H and C6—H), 4.10 (2H, t, J 6.5 Hz, C9—$H_2$), 4.21 (1H, d, J 8Hz, C2α—H) 5.28 (2 H, s), 7.34 and 7.77 (4H, J 9Hz, AA'BB' of toluene-p-sulphonate) and 7.57 and 8.24 (4H, J 9Hz, AA'BB' of PNB ester).

Total yield of toluene-p-sulphonates — 420 mg (55% overall from aldehydes).

Later fractions (68—79) contained the pyrrolidine-lactone by-product (85) (160 mg) which was an oil $\nu_{max}$ ($CHCl_3$) 1765 (γ-lactone), 1745, 1610, 1525, 1350 and 1180 $cm^{-1}$; δ ($CDCl_3$) 1.19 (3H, t, J 7Hz, $SCH_2CH_3$), 1.8—2.8 (8H, m, NH, C4—$H_2$, C3'—H, C4'—$H_2$, and q, J 7Hz, $SCH_2CH_3$), 3.50 (1H, q, J *ca* 7Hz, C3α—H), 4.0—4.5 (4H, m, C5'—$H_2$, C5—H, and C2—H), 5.27 (2H, s), 7.55 and 8.20 (4H, J 9Hz, AA'BB') (Found: M + 1, 395.1257. $C_{18}H_{23}N_2O_6S$ requires M, 395.1276).

Most conveniently, the mixture of toluene-p-sulphonate isomers (78; a—c) was reduced with sodium cyanoborohydride and the C2,C3-stereoisomers separated at that stage (example 68).

Example 67

p-Nitrobenzyl 6β-ethyl-3-ethylthio-7-oxo-1-azabicyclo[3.2.0]heptan-2 carboxylate (79)

a: C2α,C3β-stereochemistry

b: C2α,C3α-stereochemistry

c: C2β,C3α-stereochemistry

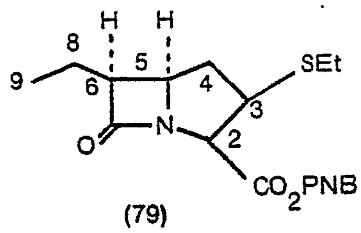

(79)

*Method 1*

From p-Nitrobenzyl 6β-(2-methanesulphonyloxyethyl)-3-ethylthio-7-oxo-1-azabicyclo[3.2.0]heptan-2-carboxylate (77)

a) From isomers (77; a and b) (example 65)

A mixture of the methanesulphonates (77; a and b) of "natural" (C2α) stereochemistry (2.00 g) (ca 1:1 ratio) (example 65) in hexamethylphosphoramide (12 ml) was heated with an excess of sodium cyanoborohydride (2.00 g) at 95° in an atmosphere of argon for 3 h. The reaction mixture was diluted with ethyl acetate (100 ml) and washed with water (2 x 10 ml) and brine (3 x 10 ml). Evaporation gave an oil (2.3 g) which was chromatographed on Kieselgel 60 (1:1 mixture of <230 and 230—400 mesh grades; 8 x 4 cm), eluting with ethyl acetate-light petroleum (1:1) (25 ml fractions). Fractions 2—6 gave the corresponding isomers of the title compound (79; a,b) (1.12 g; 72%) in the ratio *ca.* 1:1 (nmr spectrum). Rechromatography of the aliquot of the material (*ca* 100 mg) on Kieselgel 60 (230—400 mesh; 8 x 2 cm) eluting with ethyl acetate-light petroleum (2:3) effected separation of the pure isomers. The C2α,C3β-isomer (79; a) was a gum, $\nu_{max}$ ($CHCl_3$) 1765, 1755, 1610, 1525 and 1350 cm$^{-1}$; δ ($CDCl_3$) 1.02 (3H, t, J 7Hz, C9—$H_3$), 1.20 (3H, t, J 7Hz, $SCH_2CH_3$), 1.45—1.7 (2H, m, C8—$H_2$), 2.0—2.3 (2H, m, C4—$H_2$), 2.57 (2H, q, J 7Hz, $SCH_2CH_3$), 3.35 (1H, ddd, J 7,6 and 4Hz, C6α—H), 3.44 (1H, q, J 7Hz, C3α—H), 4.17 (1H, ddd, J 5,6 and 4Hz, C5α—H), 4.73 (2H, d, J 7Hz, C2β—H), 5.27 (2H, s), 7.56 and 8.23 (4H, J 9Hz, AA'BB'). (Found: M$^+$, 378.1233. $C_{18}H_{22}N_2O_5S$ requires M, 378.1249).

The C2α,C3α-isomer (79; b), also a gum had $\nu_{max}$ ($CHCl_3$) 1765, 1750, 1610, 1525 and 1350 cm$^{-1}$; δ ($CDCl_3$) 0.97 (3H, t, J 7Hz, C9—$H_3$), 1.22 (3H, t, J 7Hz, $SCH_2CH_3$), 1.45—1.9 (3H, m, C8—$H_2$ and one C4—H), 2.38 (1H, dd, J 12 and 6.5Hz, C4—H), 2.56 (2H, q, J 7Hz, $SCH_2CH_3$), 3.35 (1H, ddd, J 8, 7 and 6Hz, C6α—H), 3.76 (1H, dt, J 10 and 7Hz, C3β—H), 3.93 (1H, dt, J 7 and 6Hz, C5α—H), 4.28 (1H, d, J 7Hz, C2β—H), 5.28 (2 H, s), 7.53 and 8.22 (4 H J 9Hz, AA'BB') (Found: M$^+$, 378.1283. $C_{18}H_{22}N_2O_5S$ requires M, 378.1249).

b) From isomer (77; c) (example 65)

The methane sulphonate of "unnatural" (C2β) stereochemistry (77; c) (550 mg) in hexamethylphosphoramide (5 ml) was treated with an excess of sodium cyanoborohydride (500 mg) at 95° in an atmosphere of argon for 4 h. Recovery in ethyl acetate as described above (section a) gave an oil (680 mg) which was chromatographed on Kieselgel 60 (<230 mesh grade) (8 x 2 cm). Elution with ethyl acetate-light petroleum (2:3) gave the C2α,C3α-isomer (79; b) of the title compound (135 mg), identical (tlc analysis; ir and nmr spectra) with the sample described above (section a). Further elution of the compound gave the C2β,C3α-isomer (79; c) (188 mg) as a gum, $\nu_{max}$ ($CHCl_3$) 1765, 1745, 1610, 1525 and 1350 cm$^{-1}$; δ ($CDCl_3$) 0.97 (3H, t, J 7Hz, C9—$H_3$), 1.23 (3H, t, J 7Hz, $SCH_2CH_3$), 1.5—2.3 (4H, m, C8 $H_2$ and C4—$H_2$), 2.64 (2H, q, J 7Hz, $SCH_2CH_3$), 3.35—4.1 (3H, m, C3—H, C5—H and C6—H), 4.25 (1H, d, J 7Hz, C2α—H), 5.35 (2H, s), 7.67 and 8.31 (4H, J 9Hz, AA'BB') (Found: M$^+$, 378.1258. $C_{18}H_{22}N_2O_5S$ requires M, 378.1249). Total yield of reduced products: 323 mg (74%). Clearly, the reaction conditions caused some isomerization of isomer (79; c) to (79; b).

This sequence allows the synthesis of (79) in yields of 55% overall from aldehydes (66—68).

*Method 2*

From p-nitrobenzyl 6β-(2-toluene-p-sulphonyloxyethyl)-3-ethylthio-7-oxo-1-azabicyclo[3.2.0]heptan-2-carboxylate (78) (example 66)

A mixture of isomers (a—c) of the toluene-p-sulphonate (78) (3.01 g) in hexamethylphosphoramide (12 ml) were heated in the presence of an excess of sodium cyanoborohydride (2.7 g) at 85° in an atmosphere of argon for 7 h. Recovery in ethyl acetate and chromatography as described in Method 1 gave (fractions 2—7) a mixture of the C2α,C3β-isomer (79; a) and the C2α,C3α-isomer (79: b) of the title compound (984 mg; 45%).

Further elution of the column (fractions 8—13) afforded the C2β,C3α-isomer (79; c) (290 mg; 13%).

The products were identified (tlc analysis, ir, nmr spectra) by comparison with the samples described in Method 1.

Example 68

Isomerisation of isomer (79; c)

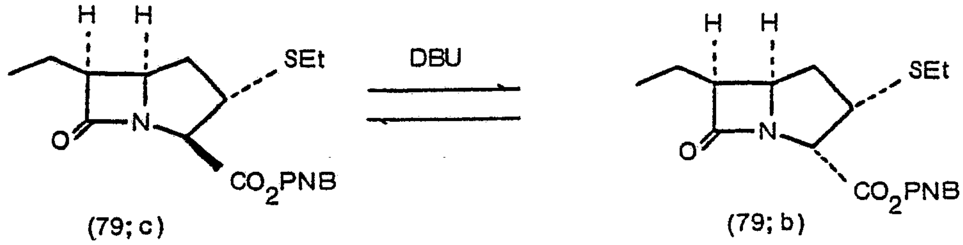


(79; c) (79; b)

p-Nitrobenzyl 6β-ethyl-3α-ethylthio-7-oxo-1-azabicyclo[3.2.0]heptan-2β-carboxylate (79; c) (example 67) (150 mg) in ethyl acetate (5 ml) was treated with 1,5-diazabicyclo[5.4.0]undec-5-ene (4 mg) at room temperature in an atmosphere of argon for 7 h. Chromatography of the reaction mixture on Kieselgel 60 (<230 mesh grade) (5 x 1 cm) (elution with ethyl acetate-light petroleum (1:1) (5 ml fraction)) gave (fractions 3—7) the pure C2α,C3α-isomer (79; b) (105 mg), identical tlc analysis; ir spectrum) with the samples previously prepared (example 67).

Example 69

Iodobenzene dichloride-mediated dehydrogenation of (79; a)

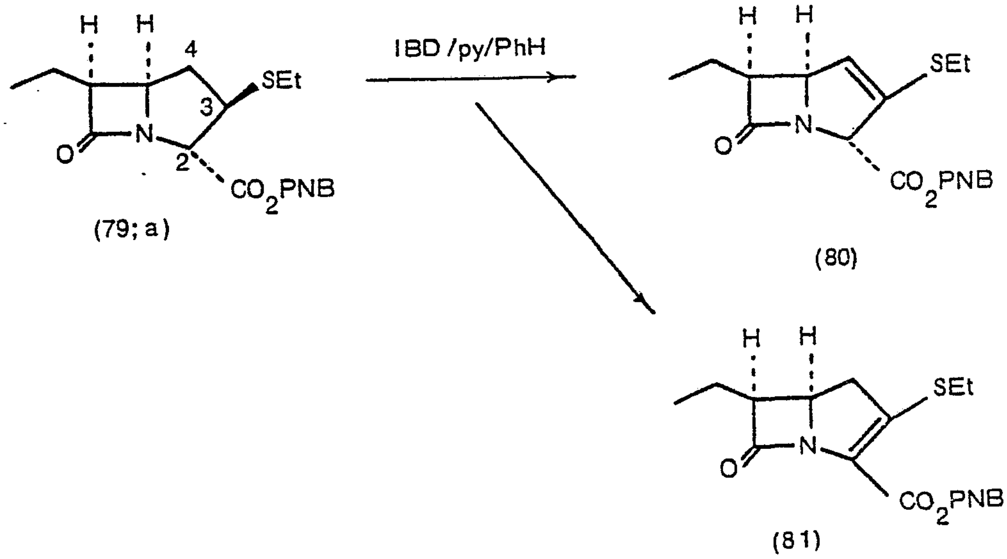


(79; a) (80) (81)

p-Nitrobenzyl 6β-ethyl-3β-ethylthio-7-oxo-1-azabicyclo[3.2.0]heptan-2α-carboxylate (79; a) (example 67) (360 mg) in anhydrous benzene (8 ml) was cooled to 5°. Anhydrous pyridine (152 mg; 2 equiv.) was added, followed by freshly-prepared iodobenzene dichloride (IBD) (276 mg, 1 equiv.). The solution was stirred until homogeneous and then stored at 0—5° in an atmosphere of argon for 1 h. The reaction mixture was chromatographed rapidly on a column of Kieselgel 60 (230—400 mesh grade) (15 x 3 cm) eluting with ethyl acetate-light petroleum (1:1) (5 ml fractions). Fractions 5—11 gave p-nitrobenzyl 6β-ethyl-3-ethylthio-7-oxo-1-azabicyclo[3.2.0]hept-3-ene-2α-carboxylate (80) as a gum (182 mg; 50%) $\nu_{max}$ ($CHCl_3$) 1770, 1750, 1610, 1570 (vinylthioether) 1525 and 1350 $cm^{-1}$; δ ($CDCl_3$) 1.00 (3H, t, J 7Hz, C9—$H_3$), 1.30 (3H, t, J 7Hz, $SCH_2CH_3$), 1.4—1.85 (2H, m, C8—$H_2$), 2.83 (2H, q, J 7Hz, $SCH_2CH_3$), 3.44 (1H, ddd, J 8, 7, and 6Hz, C6α—H) 4.70 (1H, 7 lines, J 6, 4, and 2Hz, C5α—H), 5.08 (1H, dd, J 4 and 2Hz, C2β—H), 5.27 (2H, s), 5.72 (1H, t, J 2Hz, C4—H), 7.54 and 8.21 (4H, AA'BB', J 9Hz); irradiation at the frequency of the C8—$H_2$ resonance collapsed the C6α—H multiplet to a doublet (J 6Hz), and conversely, irradiation at the frequency of the C6α—H resonance simplified the C8—$H_2$ signal. (Found: $M^+$, 376.1117. $C_{18}H_{20}N_2O_5S$ requires M, 376.1093).

Further elution of the column (fractions 12—24) gave p-nitrobenzyl 6β-ethyl-3-ethylthio-7-oxo-1-azabicyclo[3.2.0]hept-2-ene-2-carboxylate (81) as a gum which crystallised (chloroform-acetone) as pale-cream needles (54 mg; 15%), mp 144—146°, $\nu_{max}$ ($CHCl_3$) 1780, 1705, 1610, 1550 (vinylthioether), 1525 and 1350 $cm^{-1}$; $\lambda_{max}$ (EtOH) 317 nm (ε 10,800), 264 (10,900); δ ($CDCl_3$) 1.02 (3H, t, J 7Hz, C9—$H_3$), 1.33 (3H, t, J 7Hz, $SCH_2CH_3$), 1.45—1.95 (2H, m, C8—$H_2$), 2.88 (2H, q, J 7Hz,

SC$H_2CH_3$), 3.01 (2H, d, J 10Hz, C4—$H_2$), 3.52 (1H, ddd, J 8, 7 and 6Hz, C6$\alpha$—H), 4.25 (1H, td, J 10 and 6Hz, C5$\alpha$—H), 5.17 and 5.47 (2H, AB q, J 14Hz), 7.60 and 8.17 (4H, AA'BB', J 9Hz) (Found: C, 57.0; H, 5.3; N 7.1%; $M^+$, 376.1107. $C_{18}H_{20}N_2O_5S$ requires C, 57.4; H, 5.4; N, 7.4%; M, 376.1093).

Example 70

Iodobenzene dichloride-mediated dehydrogenation of (79; b)

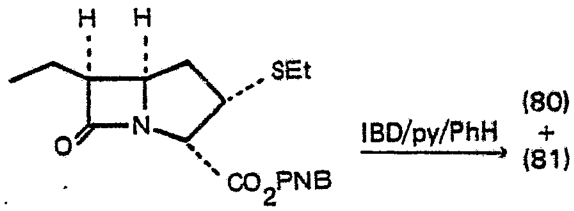


(79; b)

p-Nitrobenzyl 6$\beta$-ethyl-3$\alpha$-ethylthio-7-oxo-1-azabicyclo[3.2.0]heptan-2$\alpha$-carboxylate (79; b) (example 67) (310 mg) in anhydrous benzene (8 ml) was treated with anhydrous pyridine (131 mg; 2 equiv.) and iodobenzene dichloride (238 mg; 1 equiv.) at 0—5° for 1 h. Chromatography of the products was conducted as described in example 69. Fractions 6—11 gave p-nitrobenzyl 6$\beta$-ethyl-3-ethylthio-7-oxo-1-azabicyclo[3.2.0]hept-3-ene-2$\alpha$-carboxylate (80) as a gum (152 mg; 49%), identical (tlc analysis, ir, nmr spectra) with the sample described below (example 69).

Further elution of the column (fractions 12—22) gave p-nitrobenzyl 6$\beta$-ethyl-3-ethylthio-7-oxo-1-azabicyclo[3.2.0]hept-2-ene-2-carboxylate (81) (38 mg; 12%) identical (tlc analysis, ir and uv spectra) with the previous sample (example 69).

Example 71

Isomerisation of isomer (80)

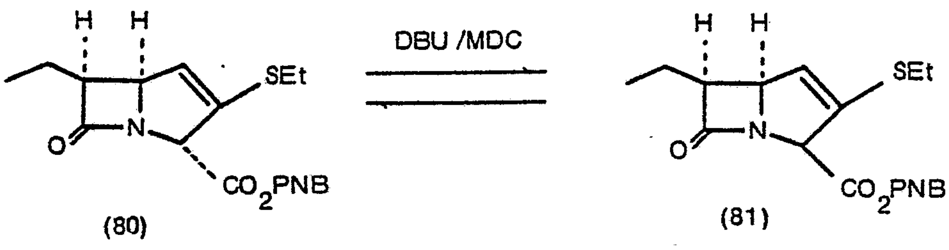


(80) (81)

p-Nitrobenzyl 6$\beta$-ethyl-3-ethylthio-7-oxo-1-azabicyclo[3.2.0]hept-3-ene-2$\alpha$-carboxylate (80) (example 69) in anhydrous dichloromethane (3 ml) was treated with 1,3-diazabicyclo[5.4.0]undec-5-ene-(20 mg) in an atmosphere of argon at 0°. The solution was allowed to warm to room temperature and stirred for 8 h. The reaction mixture, at equilibrium, was chromatographed on Kieselgel 60 (230—400 mesh grade) (8 x 3 cm). Elution with ethyl acetate-light petroleum (1:1) gave (fractions 2—6) recovered starting material (80) (80 mg). Evaporation of fractions 10—16 gave a gum (42 mg) which crystallised to give p-nitrobenzyl 6$\beta$-ethyl-3-ethylthio-7-oxo-1-azabicyclo[3.2.0]hept-2-ene-2-carboxylate (81) (31 mg).

Example 72

Sodium 6$\beta$-ethyl-3-ethylthio-7-oxo-1-azabicyclo[3.2.0]hept-2-ene-2-carboxylate (82)

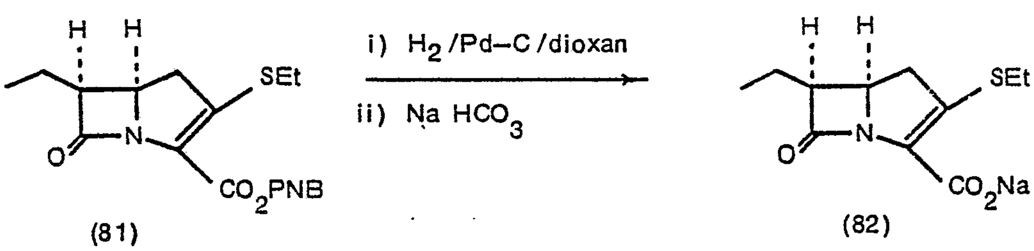


(81) (82)

A catalyst of 5% palladium on carbon (81 mg) in dioxan-water (2:1) (10 ml) was prehydrogenated at room temperature for 20 min. Freshly recrystallised p-nitrobenzyl 6$\beta$-ethyl-3-ethylthio-7-oxo-1-azabicyclo[3.2.0]hept-2-ene-2-carboxylate (81) (81 mg) (example 69) in dioxan (8 ml) was added, and the suspension was shaken in an atmosphere of hydrogen for 2.25 h. Sodium hydrogen carbonate (14.5 mg; 1 equiv.) in water (2 ml) was added, and the solution filtered through "Hiflo-Supercell". Water (10 ml) was added, and the dioxan removed *in vacuo*. The aqueous solution (ca 15 ml) was

extracted with ethyl acetate (3 x 5 ml; centrifugation). The aqueous solution was evaporated *in vacuo* to a volume of 4 ml and applied to a column of 'Biogel P2', eluting with water (5 ml fractions). Fractions 8—16 contained the U.V. chromophore characteristic of the sodium salt ($\lambda_{max}$ 300 nm). Evaporation of these fractions gave the title compound as a solid (12.25 mg) (27%) $\lambda_{max}$ (EtOH) 291 nm; $\lambda_{max}$ ($H_2O$) 300 nm; $\nu_{max}$ ($CHCl_3$) 1760, 1740 sh, and 1600 $cm^{-1}$; $\nu$ (KBr) 1750, 1590 br $cm^{-1}$.

The material exhibited solution stability; an aqueous solution (ca 2 mg $ml^{-1}$) was stored at 5° for 2 months without deterioration (uv spectrum).

Phthalidyl 6β-ethyl-3-ethylthio 7-oxo-1-azabicyclo[3.2.0]hept-2-ene-2-carboxylate

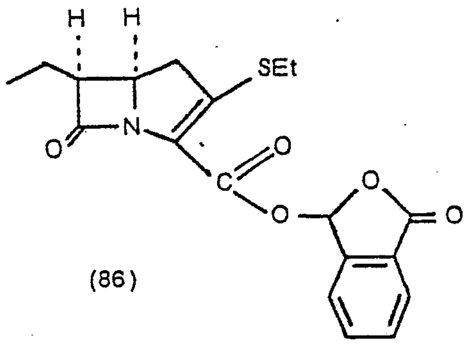

(86)

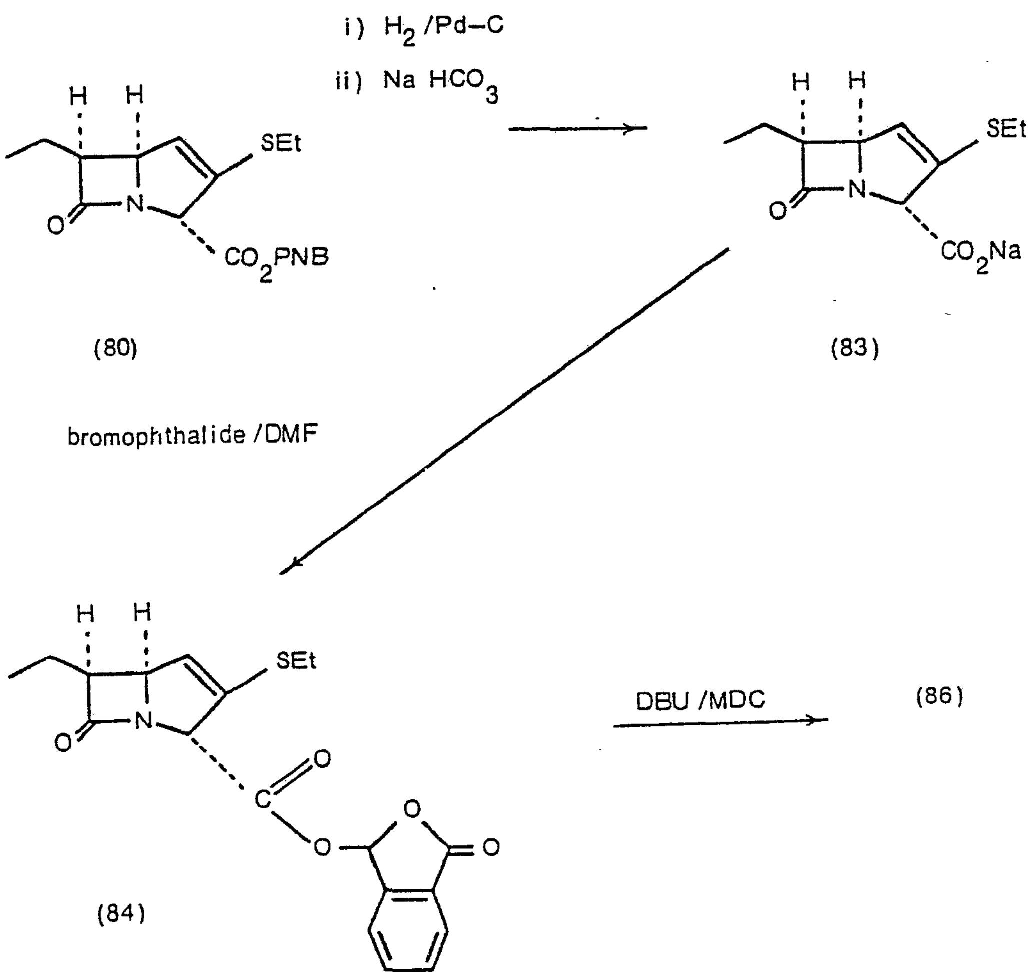


(80) (83) (84) (86)

Example 73

Sodium 6β-ethyl-3-ethylthio-7-oxo-1-azabicyclo[3.2.0]hept-3-ene-2α-carboxylate (83)

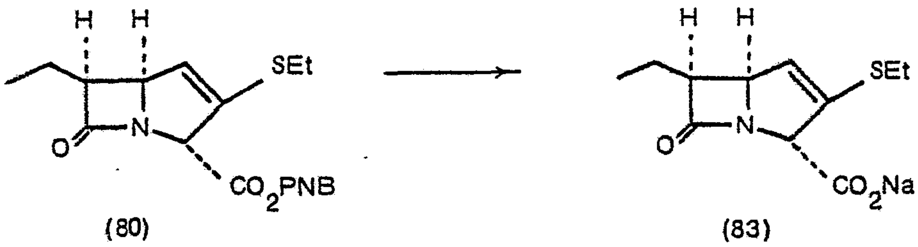

(80) (83)

p-Nitrobenzyl 6β-ethyl-3-ethylthio-7-oxo-1-azabicyclo[3.2.0]hept-3-ene-2α-carboxylate (80) (example 69) (385 mg) in dioxan (15 ml) was added to a suspension of 5% palladium-carbon catalyst (385 mg) in dioxan-water (2:1) (25 ml), that had previously been shaken in an atmosphere of hydrogen for 25 min. Sodium hydrogen carbonate (86 mg; 1 equiv.) in water (3 ml) was also added, and the suspension shaken in an atmosphere of hydrogen at room temperature for 1 h. A further quantity of palladium-carbon catalyst (193 mg) was added, and hydrogenation continued for 0.5 h. The catalyst was removed by filtration through "Hiflo-Supercell" and the catalyst washed well with portions of dioxan. Water (10 ml) was added to the filtrate, and the dioxan removed *in vacuo*. The aqueous solution was extracted with ethyl acetate (2 × 5 ml), and the aqueous solution evaporated *in vacuo*. Trituration and evaporation successively with ethanol and toluene gave the title compound as a pale-cream solid (180 mg), $\nu_{max}$ ($CHCl_3$) 1760 and 1610 $cm^{-1}$; $\nu_{max}$ (KBr) 1765 and 1610 br $cm^{-1}$.

Example 74

Phthalidyl 6β-ethyl-3-ethylthio-7-oxo-1-azabicyclo[3.2.0]hept-3-ene-2α-carboxylate (84)

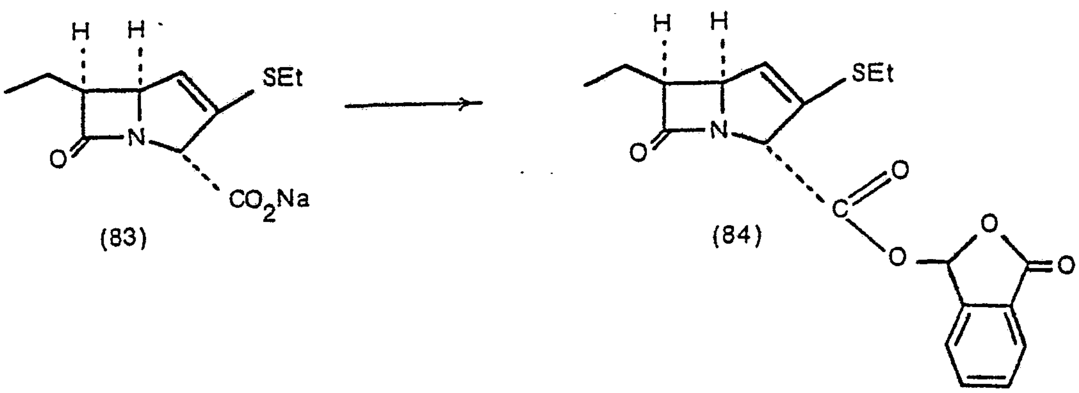

(83) (84)

Sodium 6β-ethyl-3-ethylthio-7-oxo-1-azabicyclo[3.2.0]hept-3-ene-2α-carboxylate (83) (180 mg) (example 73) in dimethylformamide (6 ml) was stirred with bromophthalide (218 mg) at room temperature for 45 min. Ethyl acetate (40 ml) was added and the solution extracted with water (2 × 10 ml) and brine (2 × 10 ml). The solution was dried ($Na_2SO_4$) and evaporated to give an oil (255 mg) which was chromatographed on Kieselgel 60 (230—400 mesh grade) (12 × 3 cm) eluting with ethyl acetate-light petroleum (2:3) (8 ml fractions). Fractions 11—23 gave the title compound as a gum (224 mg) (yield: 58% overall from p-nitrobenzyl ester (80) (example 69), $\nu_{max}$ ($CHCl_3$) 1790, 1775 sh, 1600, 1570, (vinyl thioether) and 980 $cm^{-1}$; δ ($CDCl_3$) 1.30 (3H, t, J 7Hz, $SCH_2CH_3$), 1.4—1.8 (2H, m, C8—$H_2$), 2.85 (2H, q, J 7Hz, $SCH_2CH_3$), 3.54 (1H, m, $W\frac{1}{2}$ 11Hz, C6α—H), 4.70 (1H, 7 lines J 6, 4 and 2Hz, C5α—H), 5.09 (1H, dd, J 4 and 2Hz, C2β—H), 5.75 (1H, t, J 2Hz, C4—H), 7.45 (1H, phthalidyl methine H), 7.6—8.0 (4H, m) (Found: $M^+$, 373.0988 $C_{19}H_{19}NO_5S$ requires M, 373.0984).

Example 75

Phthalidyl 6β-ethyl-3-ethylthio-7-oxo-1-azabicyclo[3.2.0]hept-2-ene-2-carboxylate (86)

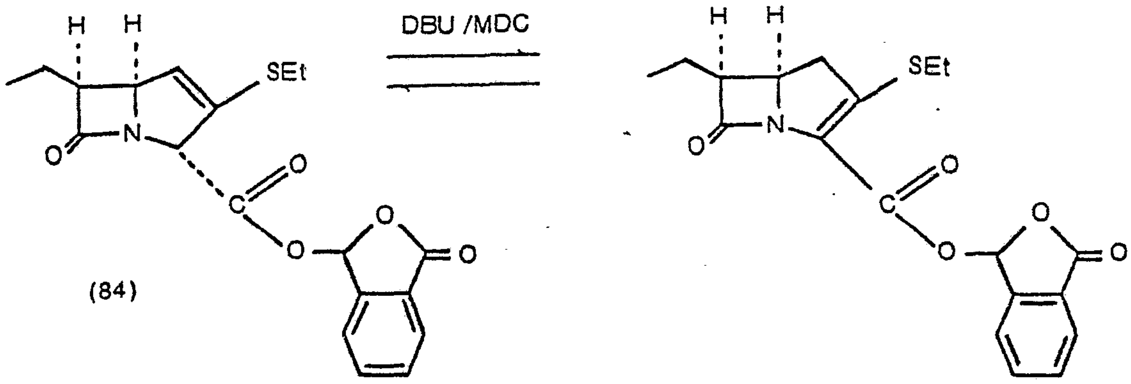


(84)

Phthalidyl 6β-ethyl-3-ethylthio-7-oxo-1-azabicyclo[3.2.0]hept-3-ene-2α-carboxylate (84) (example 74) (210 mg) in anhydrous dichloromethane (5 ml) was treated with 1,5-diazabicyclo[5.4.0]undec-5-ene (DBU) (26 mg; 0.3 equiv.) at room temperature for 3 h. The equilibrium mixture was chromatographed on Kieselgel 60 (230—400 mesh) (12 x 2.5 cm), elution with ethyl acetate-light petroleum (3:2) (5 ml fractions). Fractions 8—16 gave recovered starting material (84) (98 mg), identified by its ir spectrum. Further elution of the column (fractions 17—34) gave the title compound as a white, gummy solid (55 mg). Crystallisation (ethyl acetate-light petroleum) gave needles, m.p. 132—138°; $\lambda_{max}$ (EtOH) 325 nm (ε 8,700) 280 (2,700), 270 (2,500) and 227 (10,800); $\nu_{max}$ ($CHCl_3$) 1785 st, 1725 br, 1600, 1545 (vinyl thioether), and 975 $cm^{-1}$; δ ($CDCl_3$) 1.01 (3H, t, J 7Hz, C9—$H_3$), 1.37 (3H, t, J 7Hz, $SCH_2CH_3$), 1.5—2.1 (2H, m, C8—$H_2$)2.7—3.2 (4H, m, $SCH_2CH_3$ and C4—$H_2$), 3.52 (1H, ddd, J 8, 7 and 6Hz, C6α—H), 4.25 (1H, dt, J 10 and 6Hz, C5α—H), 7.45 (major) and 7.50 (minor) (together 1H, ratio ca 3:2, phthalidyl methine H); 7.6—8.0 (4H, m) (Found: $M^+$, 373.0983. $C_{19}H_{19}NO_5S$ requires M, 373.0984).

Reequilibration of the recovered starting material (84) afforded a further amount of the title compound (20 mg); total yield (86) was 75 mg (36%).

Sodium 3-ethylthio-6β-methyl-7-oxo-1-azabicyclo[3.2.0]hept-2-ene-2-carboxylate

(91)

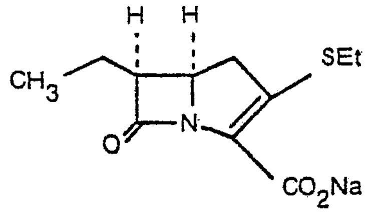

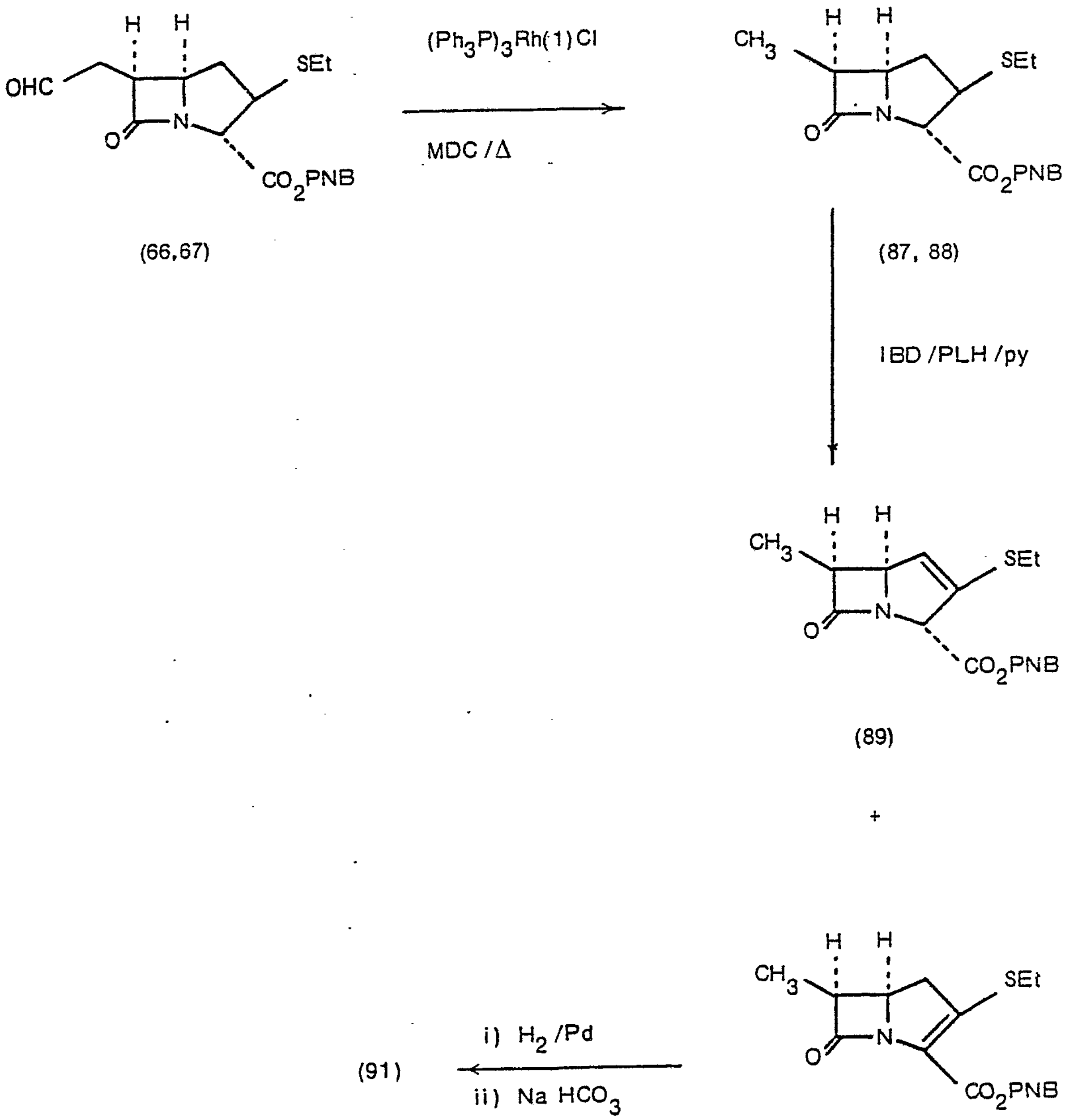


(66,67)

(87, 88)

(89)

+

(90)

(91)

Example 76

p-Nitrobenzyl 3-ethylthio-6β-methyl-7-oxo-1-azabicyclo[3.2.0]heptan-2-carboxylate (87, 88)

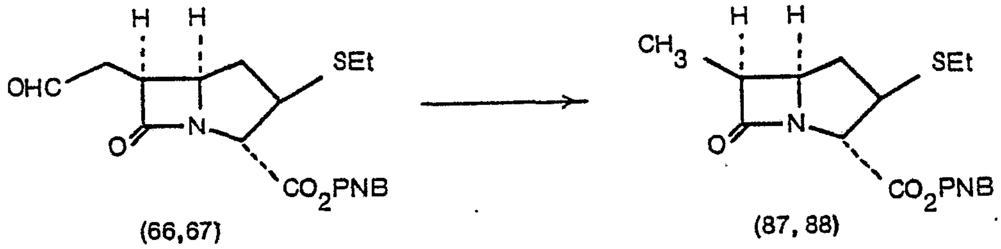

(66,67) (87, 88)

A mixture of the isomers of "natural" C (2) stereochemistry (66, 67) of p-nitrobenzyl 6β-(2-oxoethyl)-3-ethylthio-7-oxo-1-azabicyclo[3.2.0]heptan-2-carboxylate (example 59) (814 mg) in anhydrous dichloromethane (15 ml) was heated in the presence of *tris*-triphenylphosphine rhodium (1) chloride (1.90 g; 1 equiv.) at reflux temperature in an atmosphere of argon for 20 h. The mixture was cooled, filtered, and the solid washed well with further portions of dichloromethane, to give $(Ph_3P)_2$ Rh (CO) Cl (1.02 g) as a green-yellow powder. The filtrate was evaporated to give a dark-red gum (525 mg) which was chromatographed on Kieselgel 60 (1:1 mixture of 230—400 mesh and 230 mesh grades) (14 x 3 cm), (elution with ethyl acetate-light petroleum (1:3)) (10 ml fractions). Fractions 1—25 contained the red pigments. Fractions 35—56 afforded the C2α,C3β-isomer (87) of the title compound as a gum (266 mg) (35%), $\nu_{max}$ ($CHCl_3$) 1765, 1745, 1610, 1520, 1345 and 1180 $cm^{-1}$; δ ($CDCl_3$) 1.15 (3H, d, J 7.5Hz, C8—$H_3$), 1.19 (3H, t, J 8Hz, $SCH_2CH_3$), 1.94 (1H, ddd, J 14, 10.5 and 7.5Hz, C4—H), 2.24 (1H, ddd, J 14,8 and 3.5Hz, C4—H), 2.57 (2H, q, J 8Hz, $SCH_2CH_3$, 3.39 (1H, ddd, J 10.5, 8 and 7.5Hz, C3α—H), 3.53 (1H, d, q, J 7.5 and 6Hz, C6α—H, 4.17 (1H, ddd, J 7.5, 6 and 3.5Hz, C5α—H), 4.72 (1H, d, J 7.5Hz, C2β—H), 5.27 (2H, s), 7.55 and 8.21 (4H, AA'BB', J 9Hz); irradiation at the frequency of the C2- and C—5 proton resonances, simplified the signals assigned to the C3-proton and C4-protons, respectively (Found: $M^+$, 364.1092. $C_{17}H_{20}N_2O_5S$ requires M, 364.1091).

Continued elution of the column (fractions 57—78) gave the C2α,C3α-isomer (88) of the title compound as a gum (189 mg) (25%), $\nu_{max}$ ($CHCl_3$) 1765, 1745, 1610, 1520, 1345 and 1170 $cm^{-1}$; δ ($CDCl_3$) 1.20 (3H, d, J 7.5Hz, C8—$H_3$), 1.24 (3H, t, J 8Hz, $SCH_2CH_3$), 1.66 (1H, ddd, J 13.5, 10 and 8.5Hz, C4—H), 2.40 (1H, dt, J 13.5 and 6.5Hz, C4—H), 2.57 (2H, q, J 8Hz, $SCH_2CH_3$), 3.53 (1H, dq, J 7.5 and 5.5Hz, C6α—H), 3.78 (1H, dt, J 10 and 7Hz, C3β—H), 3.96 (1H, dt, J 8.5 and 6.0Hz, C5α—H), 4.26 (1H, d, J 7Hz, C2β—H), 5.29 (2H, s), 7.54 and 8.22 (4H, J 9Hz, AA'BB'). (Found: $M^+$, 364.1109. $C_{17}H_{20}N_2O_5S$ requires M, 364.1091).

Attempts to transform the C2β,C3α-isomer (68) of the aldehyde did not result in the formation of the expected C2β,C3α-product but led to the recovery of C2α,C3α-isomer (88). This arises as a consequence of the basic nature of the reagent; base-catalysed epimerisation at C2 occurs during the course of the decarbonylation reaction.

Total yield of decarbonylated products (87, 88): 455 mg (60%).

Example 77

Iodobenzene dichloride-mediated dehydrogenation of isomer (87, 88)

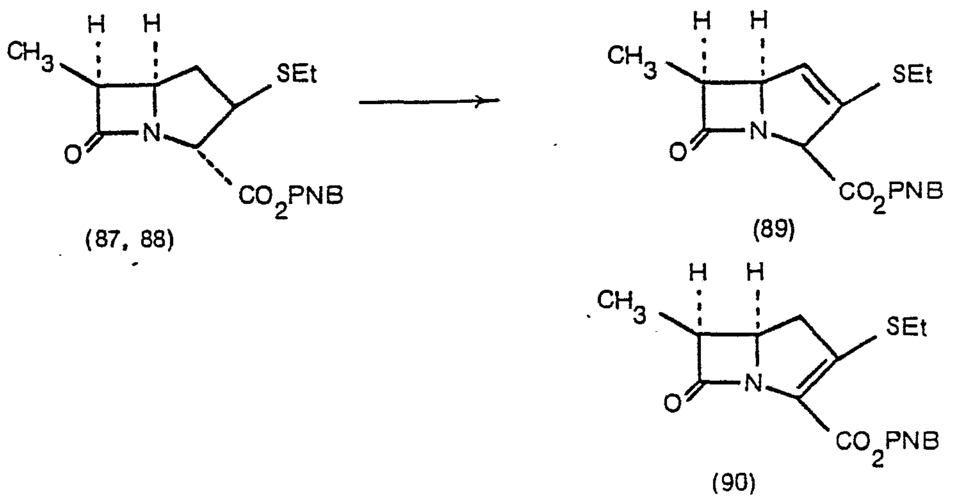

(87, 88) (89)

(90)

A mixture of the C2α,C3β- (87) and C2α,C3α- (88) isomer of p-nitrobenzyl 3-ethylthio-6β-methyl-7-oxo-1-azabicyclo[3.2.0]heptan-2-carboxylate (example 76) (320 mg) in anhydrous benzene

(8 ml) was treated successively with pyridine (144 mg; 2 equiv.) and iodobenzene dichloride (266 mg; 1.1 equiv.) at 0—5° for 2 hr. The reaction mixture was chromatographed rapidly on Kieselgel 60 (230—400 mesh grade) (12 x 3 cm) eluting with ethyl acetate-light petroleum (1:1) (5—8 ml fractions). Fractions 14—23 afforded p-nitrobenzyl 3-ethylthio-6β-methyl-7-oxo-1-azabicyclo[3.2.0]hept-3-ene-2α-carboxylate (89) as a gum (177 mg; 55%), $\nu_{max}$ ($CHCl_3$) 1775, 1755, 1610, 1570 (vinyl thioether), 1525 and 1350 $cm^{-1}$; δ ($CDCl_3$) 1.14 (3H, d, J 7.5Hz, C8—$H_3$), 1.29 (3H, t, J 7Hz, $SCH_2CH_3$), 2.83 (2H, q, J 7Hz, $SCH_2CH_3$), 3.59 (1H, dq, J 7.5 and 5.5 Hz, C6α—H), 4.71 (1H, 7 lines, J 5.5, 3.5 and 2Hz, C5α—H), 5.10 (1H, dd, J 3.5 and 2Hz, C2β—H), 5.27 (2H, s) 5.70 (1H, t, J 2Hz, C4—H), 7.54 and 8.22 (4H, J 9Hz, AA'BB'). (Found: $M^+$ 362.0953. $C_{17}H_{18}N_2O_5S$ requires M, 362.0936).

Continued elution of the column (fractions 34—46) gave p-nitrobenzyl 3-ethylthio-6β-methyl-7-oxo-1-azabicyclo[3.2.0]hept-2-ene-2-carboxylate (90) (40 mg) which crystallised (ethyl acetate-light petroleum) as pale-yellow needles (28 mg; 18%) m.p. 136°, $\lambda_{max}$ (EtOH) 318 nm (ε 11,900), 266 (11,700) and 216 infl; $\nu_{max}$ ($CHCl_3$) 1780, 1700, 1605, 1550 (vinyl thioether), 1520 and 1345 $cm^{-1}$; δ ($d_6$-acetone) 1.24 (3H, d, J 7.5Hz, C8—$H_3$), 1.29 (3H, t, J 7Hz, $SCH_2CH_3$), 2.97 (2H, q, J 7Hz, $SCH_2CH_3$), 2.19 (2H, d, J 10Hz, C4—$H_2$), 3.74 (1H, dt, J 7.5 and 6Hz, C6α—H), 4.33 (1H, dt, J 10 and 6Hz, C5α—H), 5.25 and 5.52 (2H, J 14Hz, ABq), 7.78 and 8.24 (4H, J 9Hz, AA'BB') (Found: C, 56.0; H, 5.2; N, 7.6%; $M^+$ 362.0915. $C_{17}H_{18}N_2O_5S$ requires C, 56.3; H, 5.1; N, 7.7%; M, 362.0963).

Example 78

Isomerisation of isomer (89)

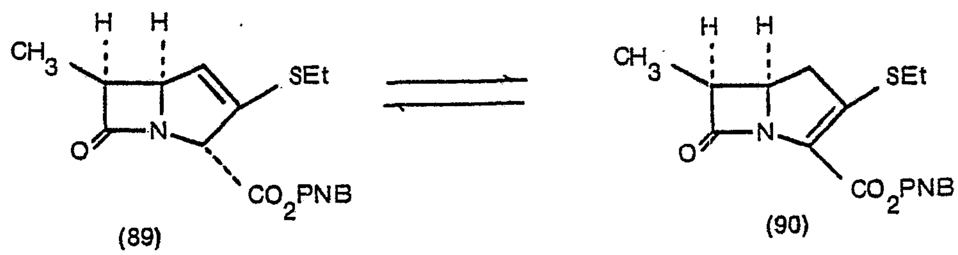

(89) (90)

p-Nitrobenzyl 3-ethylthio-6β-methyl-7-oxo-1-azabicyclo[3.2.0]hept-3-ene-2α-carboxylate (89) (example 77) (160 mg) in anhydrous dichloromethane (1.5 ml) was stirred with 1,5-diazabicyclo[5.4.0]undec-5-ene (20 mg; 0.3 equiv.) at room temperature in an atmosphere of argon for 2 hr. The equilibrium mixture was chromatographed on Kieselgel 60 (230—400 mesh grade) (10 x 3 cm), eluting with ethyl acetate-light petroleum (1:1) 8 ml fractions). Fractions 8—10 afforded recovered starting material (89) (118 mg). Fractions 11—25 yielded p-nitrobenzyl-3-ethylthio-6β-methyl-7-oxo-1-azabicyclo[3.2.0]hept-2-ene-2-carboxylate (90) (20 mg). The products were identical (tlc analysis; ir spectra) with the previously-prepared samples (example 77).

Example 79

Sodium-3-ethylthio-6β-methyl-7-oxo-1-azabicyclo[3.2.0]hept2-ene-2-carboxylate (91)

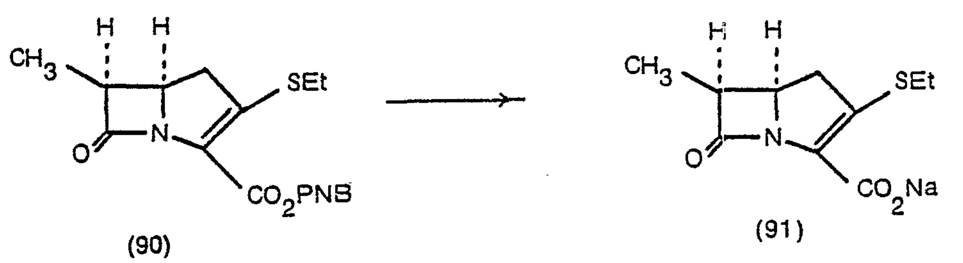

(90) (91)

p-Nitrobenzyl 3-ethylthio-6β-methyl-7-oxo-1-azabicyclo[3.2.0]-hept-2-ene-2-carboxylate (90) (example 77) (31 mg) freshly crystallised from ethyl acetate — light petroleum, in dioxan (4 ml) was added to a suspension of a 5% palladium — carbon catalyst in dioxan-water (2:1) (5 ml) which had been pre-hydrogenated for 20 min. The suspension was shaken in an atmosphere of hydrogen gas for 2.25 h. Sodium hydrogen carbonate (8 mg; 1 equiv) in water (2 ml) was added, and the solution was filtered, evaporated, and extracted with ethyl acetate as previously described (example 72). The aqueous phase was evaporated *in vacuo* to a volume of 3 ml, and applied to a column of 'Biogel P2' (13 x 2 cm). Elution with water (2 ml fractions) afforded the title compound (fractions 5—8) (21% by uv analysis), $\lambda_{max}$ ($H_2O$) 300 nm.

Evaporation of the solvent from an aliquot of the solution, followed by trituration and evaporation from ethanol and toluene gave a solid, $\nu_{max}$ ($CHCl_3$) 1760, 1745 sh, and 1600 $cm^{-1}$.

p-Nitrobenzyl 6β-(2-azidoethyl)-3-ethylthio-7-oxo-1-azabicyclo[3.2.0]hept-2-ene-2-carboxylate.

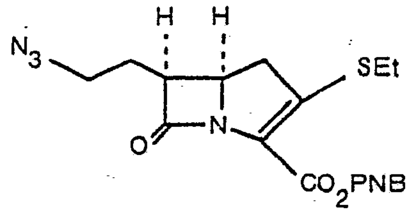

(94)

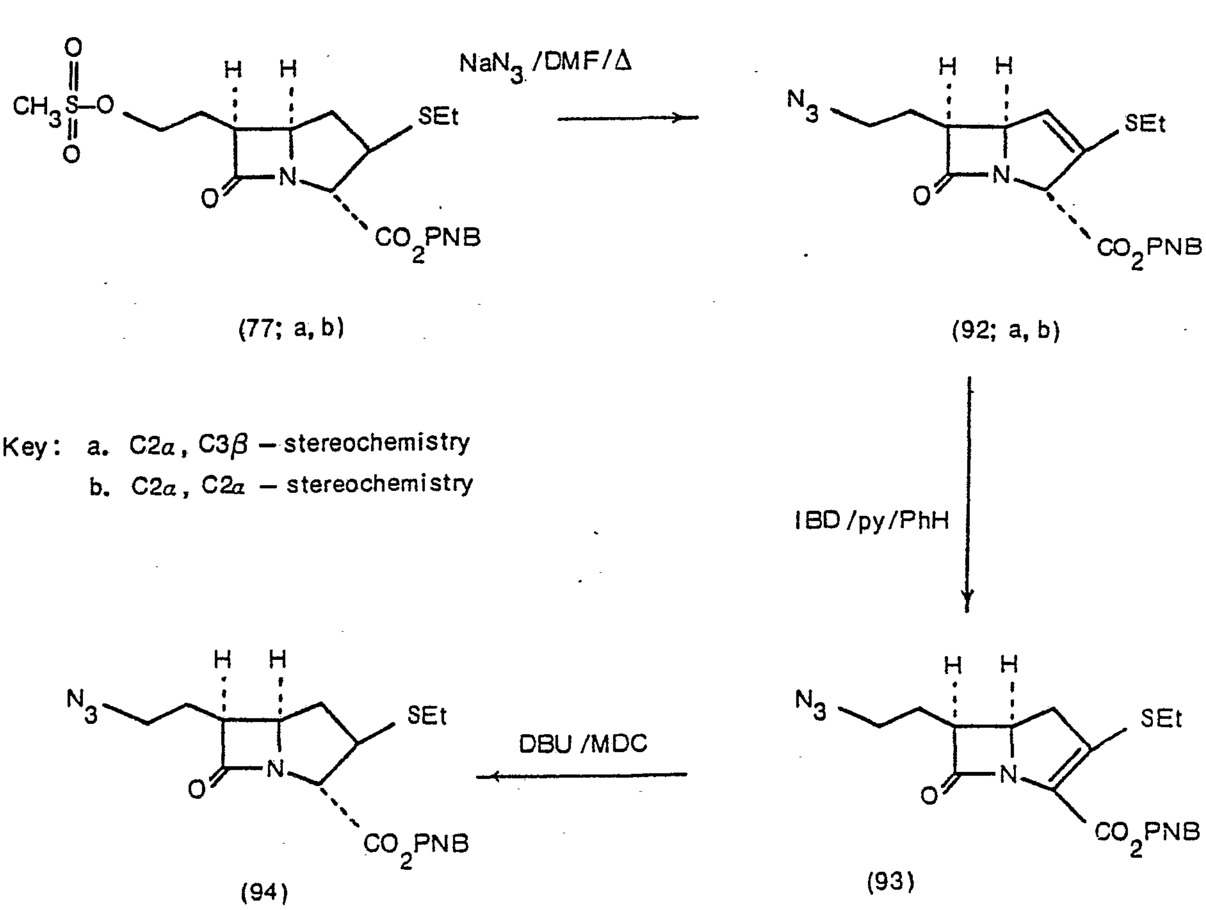


(77; a, b) (92; a, b)

Key: a. C2α, C3β — stereochemistry
b. C2α, C2α — stereochemistry

(94) (93)

Example 80

p-Nitrobenzyl-6β-(2-azidoethyl)-3-ethylthio-7-oxo-1-azabicyclo[3.2.0]heptane-2-carboxylate (92).

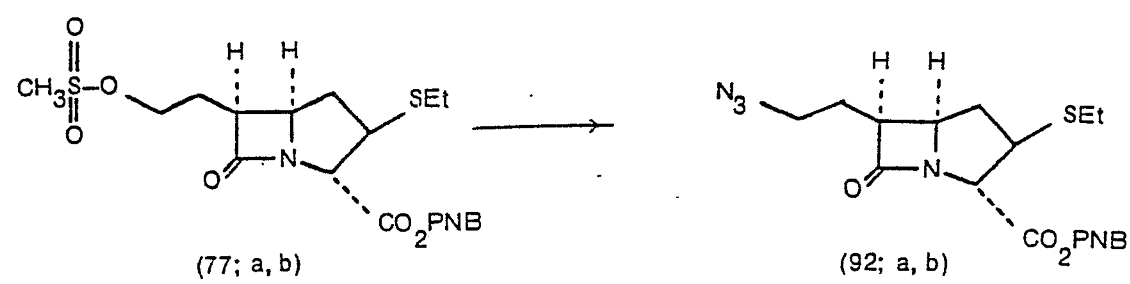

(77; a, b) (92; a, b)

A mixture of the C2α, C3β- (77; a) and C2α, C3α- (92; a) isomers of p-nitrobenzyl-3-ethylthio-6β-(2-methanesulphonyloxyethyl)-7-oxo-1-azabicyclo[3.2.0]heptane-2-carboxylate (example 65) (55 mg) in dimethylformamide was heated with finely-ground sodium azide in an atmosphere of argon at 80° for 50 min. The reaction mixture was diluted with chloroform (10 ml) and extracted with water (2

× 3 ml) and brine (2 × 2 ml). The organic phase was dried ($Na_2SO_4$) and evaporated to a gum (80 mg) which was chromatographed on Kieselgel 60 (<230 mesh) (5 × 2 cm), eluting with ethyl acetate — light petroleum (1:1) (3 ml fractions). Fractions 4—8 gave the C2α,C3β-isomer (92; a) of the title compound as a gum (31 mg; 63%), $\nu_{max}$ ($CHCl_3$) 2110, 1765, 1750, 1610, 1525 and 1350 $cm^{-1}$; δ ($CDCl_3$) 1.24 (3H, t, J 7Hz, $SCH_2CH_3$), 1.6—2.6 (4H, m, C4—$H_2$ + C8—$H_2$), 2.63 (2H, q, J 7Hz, $SCH_2CH_3$), 3.52 (2H, t, J 6Hz, C9—$H_2$), *ca* 3.6 (2H, m, C3—H and C6α—H), 4.25 (1H, m, $W_{\frac{1}{2}}$ 12Hz, C5α—H), 4.78 (1H, d, J 7Hz, C2β—H), 5.33 (2H, s), 7.60 and 8.29 (4H, J 9Hz, AA$^1$ BB$^1$).

Further elution of the column (fractions 10—11) gave the C2α,C3α,- isomer (92; b) of the title compound as a gum (8 mg), $\nu_{max}$ ($CHCl_3$) 2110, 1755, 1610, 1525 and 1350 $cm^{-1}$.

Example 81

p-Nitrobenzyl 6β-(2-azidoethyl)-3-ethylthio-7-oxo-1-azabicyclo[3.2.0]hept-3-ene-2α-carboxylate. (93)

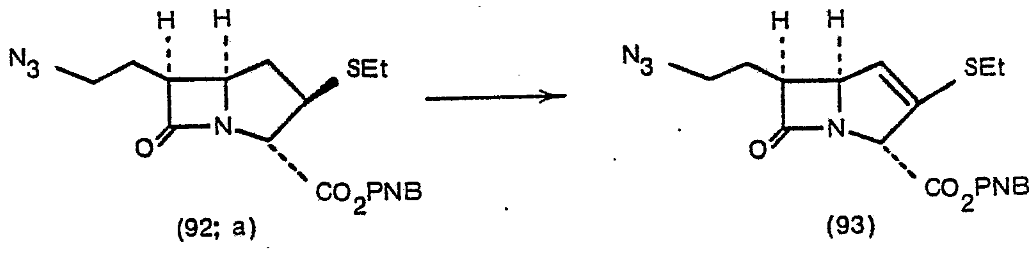

(92; a) (93)

p-Nitrobenzyl 6β-(2-azidoethyl)-3-ethylthio-7-oxo-1-azabicyclo[3.2.0]heptan-2α-carboxylate (92; a) (example 80) in anhydrous benzene was treated successively with pyridine (2 equiv.) and iodobenzene dichloride (1 equiv.). Recovery and chromatography as for (80) (example 69) afforded the title compound (93).

Example 82

p-Nitrobenzyl-6β-(2-azidoethyl)-3-ethylthio-7-oxo-1-azabicyclo[3.2.0]hept-2-ene-2-carboxylate (94).

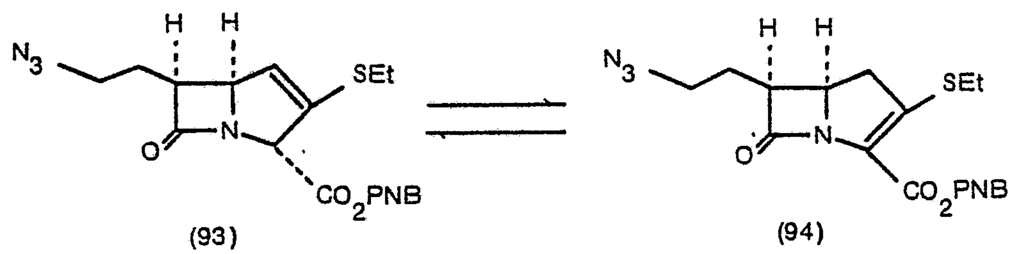

(93) (94)

p-Nitrobenzyl 6β-(2-azidoethyl)-3-ethylthio-7-oxo-1-azabicyclo[3.2.0]hept-3-ene-2α-carboxylate (93) (example 81) in anhydrous dichloromethane was treated with 1,5-diazabicyclo[5.4.0]undec-5-ene- at room temperature in atmosphere of argon from 2.5 h. The equilibrium mixture was chromatographed on Kieselgel 60 (230—400 mesh) as described in example 71 to give the title compound (94).

Example 83

Benzyl 6-benzyl-3-ethylthio-7-oxo-azabicyclo[3.2.0]heptane-2-carboxylate

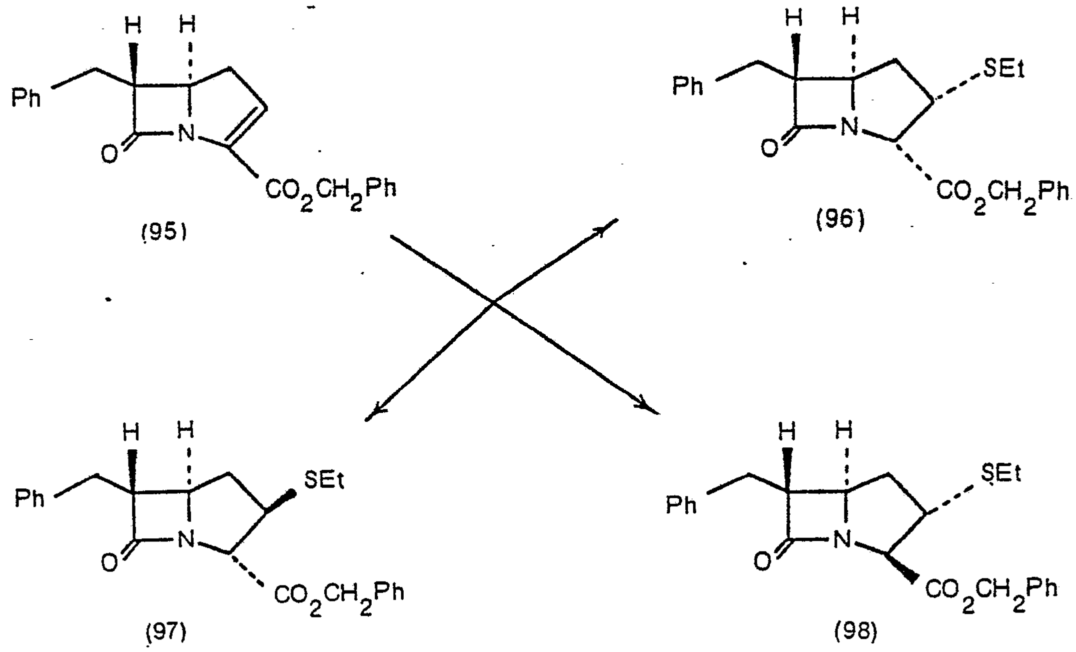

(95) (96) (97) (98)

Benzyl 6-benzyl-7-oxo-1-azabicyclo[3.2.0]hept-2-ene-2-carboxylate (95) (0.42 g) was stirred in dry dimethylformamide (5 ml) at room temperature and treated with ethanethiol (0.12 ml) followed by powdered potassium carbonate (0.087 g). After a period of 1 hour, the solvent was removed under high vacuum at room temperature and the residue was partitioned between ethyl acetate and brine. The organic phase was dried over sodium sulphate, concentrated, and chromatographed on a column of silica gel 60 (<230 mesh) eluting with ethylacetate/light petroleum 3:7 to give the three isomers of benzyl 6-benzyl-3-ethylthio-7-oxo-1-azabicyclo[3.2.0]heptane-2-carboxylate. In order of elution they were the $2\alpha,3\alpha$-isomer (96) (0.054 g); $\nu_{max}$ ($CHCl_3$) 2950, 1765, and 1745 sh $cm^{-1}$; $\tau$ ($CDCl_3$) 2.6—2.9 (10H, m, phenyls), 4.86 (2H, s, $OCH_2$), 5.59 (1H, d, J 5Hz, C2—H), 6.1—6.5 (2H, m, C3—H and C5—H), 6.6—7.2 (3H, m, C6—H and C8—$H_2$), 7.50 (2H, q, J 7Hz, $SCH_2$), 7.55 (1H, dt, J 14 and 7Hz, C4—H), 8.41 (1H, dt, J 14 and 7Hz, C4—H), and 8.84 (3H, t, J 7Hz, $CH_3$) ($M^+$ at m/e 395.1554. $C_{23}H_{25}NO_3S$ requires 395.1553). Next was obtained the $2\alpha,3\beta$-isomer (97) (0.260 g); $\nu_{max}$ ($CHCl_3$) 2960, 1765 and 1750 sh $cm^{-1}$; $\tau$ ($CDCl_3$) 2.6—2.9 (10H, m, phenyls), 4.87 (2H, s, $OCH_2$), 5.26 (1H, d,J 7Hz, C2—H), 6.10 (1H, td, J 6 and 3 Hz, C5—H), 6.52 (1H, td, J 9 and 7Hz, C3—H), 6.7—7.2 (3H, m, C6—H and C8—$H_2$), 7.47 (2H, 9, J 7Hz, $SCH_2$), 7.89 (2H, dd, J9 and 6Hz, C4—Hz) and 8.85 (3H, t, J 7Hz, $CH_3$) ($M^+$ at m/e 395.1553. $C_{23}H_{25}NO_3S$ requires 395.1553). The $2\beta,3\alpha$-isomer (98) was isolated also as a gum (0.112 g); $\nu_{max}$ ($CHCl_3$) 2960, 1770, and 1745 $cm^{-1}$; $\tau$ ($CDCl_3$) 2.6—2.9 (10H, m, phenyls), 4.82 (2H, s, $OCH_2$), 5.94 (1H, d, J 8Hz, C2—H), 6.2—7.2 (5H, m, C3—H, C6—H and C8—$H_2$), 7.51 (2H, q, J 7Hz, $SCH_2$), 7.83 (1H, dt, J 12 and 6Hz, C4—H), 8.10 (1H, td, J 12 and 9Hz, C4—H) and 8.86 (3H, t, J 7Hz, $CH_3$) ($M^+$ at m/e 395.1560, $C_{23}H_{25}NO_3S$ requires 395.1553).

Example 84

Benzyl 6-benzyl-3-ethylthio-7-oxo-1-azabicyclo[3.2.0]hept-3-ene-carboxylate and corresponding hept-2-ene.

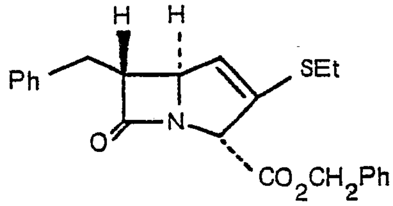

and

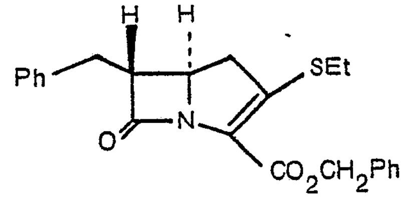

(99) (100)

(i) *From 2α, 3α-isomer*

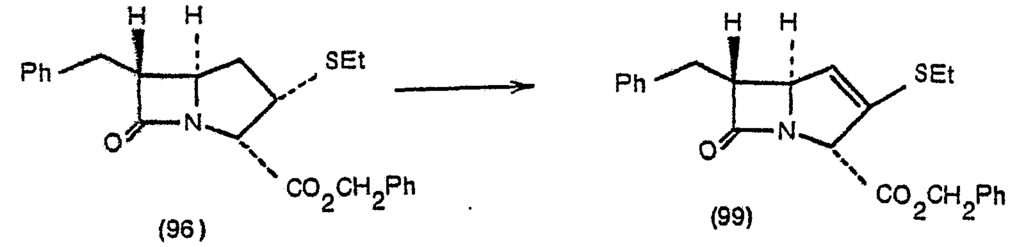

(96) (99)

The 2α,3α-isomer of benzyl 6 benzyl-3-ethylthio-7-oxo-1-azabicyclo[3.2.0]heptane-2-carboxylate (96) (0.048 g) was dissolved in dry benzene (2 ml) and cooled to 5°C with stirring under argon. It was treated with pyridine (0.022 g) followed by iodobenzene dichloride (0.038 g). After 5 mins, it was set aside in a refrigerator for 3 hours. The resulting solution was applied to a column of silica gel (230—400 mesh) and eluted with ethyl acetate/60—80° light petroleum 3:7 to give benzyl 6-benzyl-3-ethylthio-7-oxo-1-azabicyclo[3.2.0]hept-3-ene-2-carboxylate (99) as a colourless gum (0.022 g).

(ii) from 2α,3β-isomer (97).

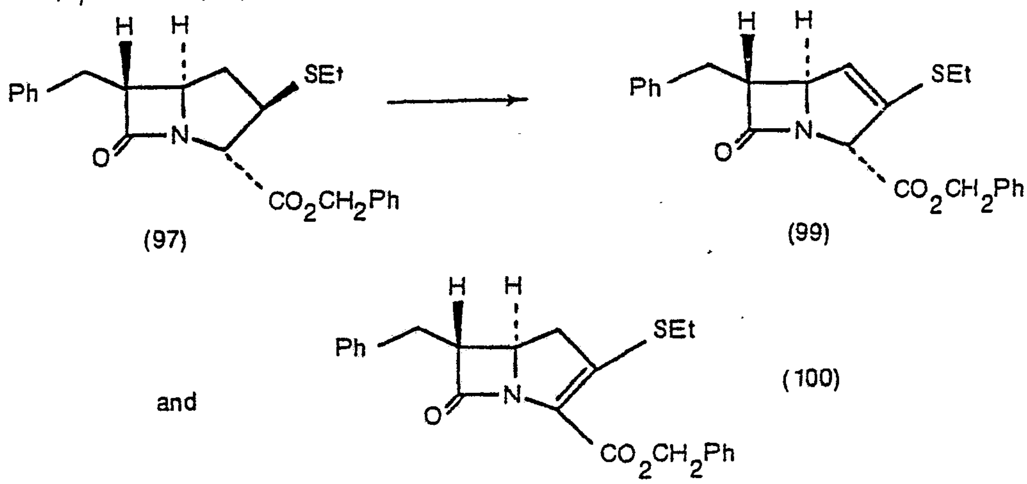

(97) (99)

and (100)

A solution of the 2α,3β-isomer of benzyl 6-benzyl-3-ethylthio-7-oxo-1-azabicyclo[3.2.0]-heptane-2-carboxylate (97) (0.106 g) in dry benzene (4 ml) was treated with pyridine (0.047 g) and iodobenzene dichloride (0.081 g), according to the method described above. Chromatography gave benzyl 6-benzyl-3-ethylthio-7-oxo- -azabicyclo[3.2.0]hept-3-ene-2-carboxylate (99) (0.043 g); $\nu_{max}$ $(CHCl_3)$ 300, 2920, 1770, 1750 and 1570 $cm^{-1}$; $(CDCl_3)$ 2.6—2.9 (10H, m, phenyls), 4.35 (1H, t, J 2Hz, C4—H), 4.85 (2H, s, $OCH_2$), 4.91 (1H, dd, J 3 and 2Hz, C2—H), 5.60 (1H, td, J3 and 2Hz, C5—H), 6.6—7.1 (3H, m, C6—H and C8—$H_2$), 7.27 (2H, q, J 7Hz, $SCH_2$) and 8.78 (3H, t, J 7Hz, $CH_3$) ($M^+$ at m/e 393.1394. $C_{23}H_{23}NO_3S$ requires 393.1398. Continued elution provided benzyl 6-benzyl-3-ethylthio-7-oxo-1-azabicyclo[3.2.0]hept-2-ene-carboxylate (100) (0.007 g).

Example 85

Benzyl 6-benzyl-3-ethylthio-7-oxo-1-azabicyclo[3.2.0]hept-2-ene-2-carboxylate

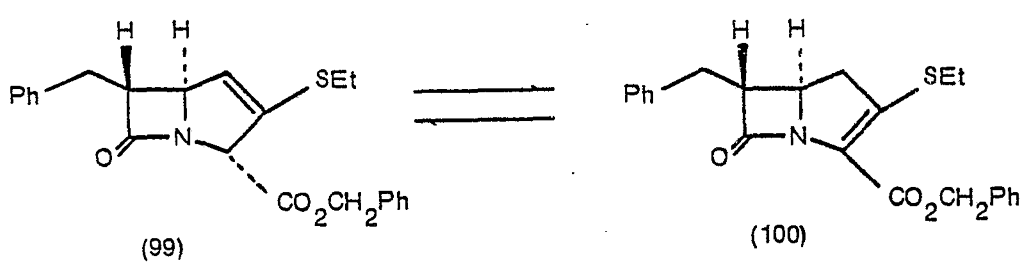

(99) (100)

Benzyl 6-benzyl-3-ethylthio-7-oxo-1-azabicyclo[3.2.0]hept-3-ene-2-carboxylate (99) (0.058 g) was stirred at room temperature under argon in methylene chloride (4 ml) solution. It was treated with 1,5-diazabicyclo[5.4.0]undec-5-ene (0.005 g). Aftrer a period of 5 hours, the solution was concentrated and rapidly chromatographed on a column of silica gel 60 (1:1 mixture of 230—400

mesh and <230 mesh) eluting with ethyl acetate/60—80° light petroleum 3:7. This gave recovered Δ-3 compound (99) (0.043 g) followed by benzyl 6-benzyl-3-ethylthio-7-oxo-1-azabicyclo[3.2.0]hept-2-ene-2-carboxylate (100) (0.010 g); $\lambda_{max}$ (ethanol) 319 nm; $\nu_{max}$ ($CHCl_3$) 3000, 2920, 1780, 1700, 1605 and 1550 $cm^{-1}$; τ ($CDCl_3$), 2.5—3.0 (10H, m, phenyls), 4.63 and 4.81 (2H, ABq, J 12Hz, $OCH_2$), 6.02 (1H, td, J 9 and 3Hz, C5—H), 6.5—7.3 (5H, m, C6—H, C8—$H_2$ and C4—$H_2$), 7.22 (2H, q, J 7Hz, $SCH_2$) and 8.73 (3H, t, J 7Hz, $CH_3$) ($M^+$ at m/e 393.1410. $C_{23}H_{23}NO_3S$ requires 393.1399).

## Example 86

Benzyl-3-(2-acetamidoethylthio)-6-(1-hydroxyethyl)-7-oxo-1-azabicyclo[3.2.0]heptane-2-carboxylate.

i) 4 - Allyl - 3 - (1 - hydroxyethyl) - 1 - (1 - benzyloxycarbonyl - 1 - triphenylphosphoranylidene-methyl)azetidin - 2 - one.

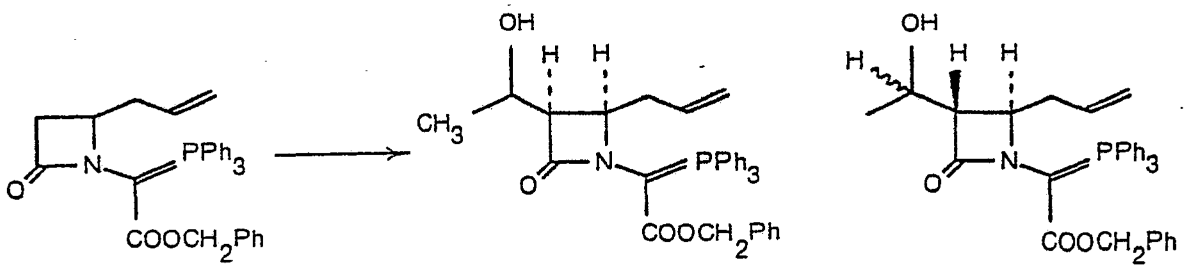

A solution of N-isopropylcyclohexylamine (0.60 g) in dry tetrahydrofuran (10 $cm^3$) was stirred under argon and cooled to −78°. This was treated with a 2.5 M solution of *n*-butyl lithium in *n*-hexane (1.70 $cm^3$). After 10 minutes, a solution of 4-allyl-1-(1-benzyloxycarbonyl-1-triphenyl-phosphoranylidenemethyl) azetidin-2-one (1.00 g) in dry tetrahydrofuran (15 $cm^3$) was added. Five minutes were allowed for the formation of the C(3) carbanion which was quenched by the addition of acetaldehyde (0.54 $cm^3$).

The mixture was stirred under argon for a further ten minutes before it was neutralised with acetic acid (0.56 g). The solvent was evaporated under reduced pressure and the residue chromatographed on silica gel 60 (<230 mesh) eluting with ethyl acetate/cyclohexane mixtures grading from 1:1 to 1:0. This gave a mixture one *cis* and two *trans*-isomers of 4-allyl-3-(1-hydroxyethyl)-1-(1-benzyloxy-carbonyl-1-triphenylphosphoranylidenemethyl)azetidin-2-one (0.71 g, 65%), $\nu_{max}$ ($CHCl_3$) 3000, 1735 and 1620 $cm^{-1}$.

From this mixture may be prepared benzyl 6-(1-hydroxyethyl)-7-oxo-1-azabicyclo[3.2.0]hept-2-ene-2-carboxylate by methods detailed in the incorporated references hereinbefore mentioned.

ii) Benzyl 3-(2-acetamidoethylthio)-6-(1-hydroxyethyl)-7-oxo-1-azabicyclo[3.2.0]heptane-2-carboxylate.

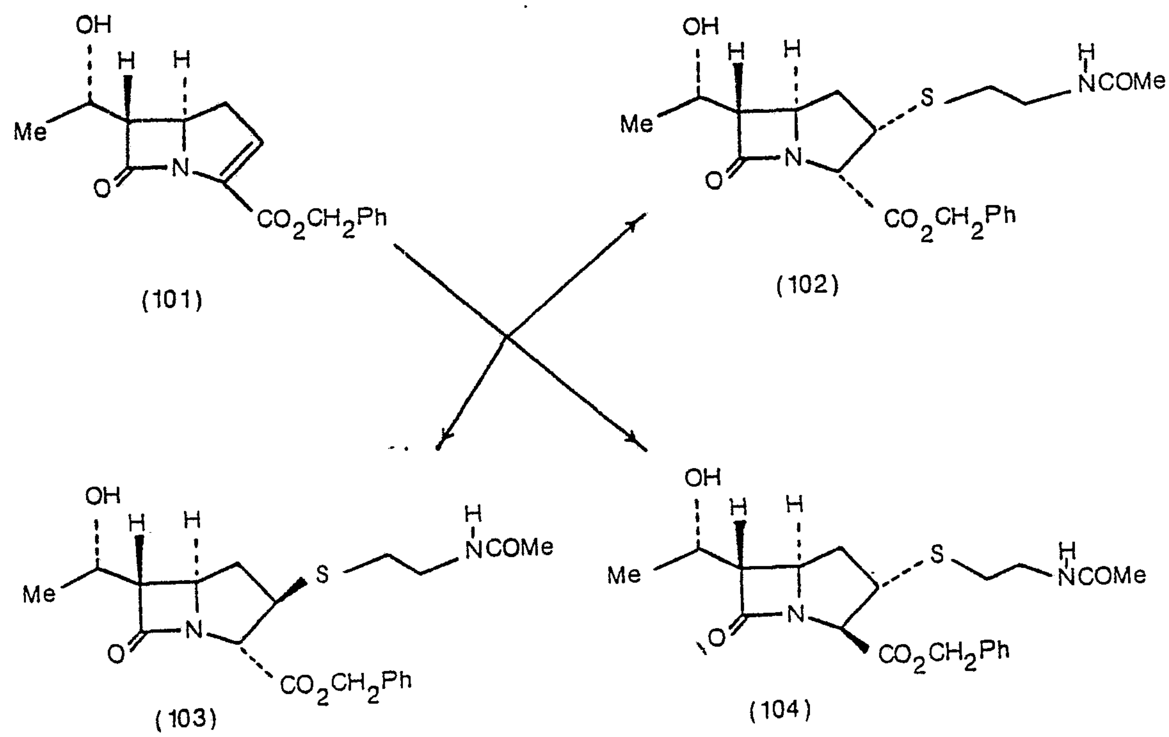

(101)

(102)

(103)

(104)

Benzyl 6-(1-hydroxyethyl)-7-oxo-1-azabicyclo[3.2.0]hept-2-ene-2-carboxylate (101) (0.440 g) was dissolved in dry dimethylformamide (5 ml) and stirred at room temperature under argon. It was treated with 2-acetamidoethanethiol (0.200 g) followed by powdered potassium carbonate (0.106 g). The product was concentrated under vacuum after 1 hour, and the residue taken up in a mixture of ethyl acetate and brine. The organic phase was separated, dried over sodium sulphate and concentrated. Chromatography on silica gel 60 (<230 mesh), eluting with chloroform/ethanol 9:1, provided three isomers of benzyl 3-(2-acetamidoethylthio)-6-(1-hydroxyethyl)-7-oxo-1-azabicyclo 3.2.0 heptane-2-carboxylate. The 2α,3α-isomer (102) was first to be eluted, and was obtained as a chloroform hemi-solvate (0.256 g); m.p. 94—96° (chloroform/-60—80° petroleum ether); $\nu_{max}$ ($CHCl_3$) 3460, 3380, 1760, 1665 and 1515 $cm^{-1}$; τ ($CDCl_3$) 2.66 (5H, s, phenyl), 3.90 (1H, br, NH), 4.83 (2H, s, benzyl $CH_2$), 5.58 (1H, d, J 4Hz, C2—H), 5.7—6.0 (1H, m, C8—H), 6.15 (1H, td, J 6 and 2Hz, C5—H), 6.27 (1H, td, J 6 and 4Hz, C3—H), 6.5—6.8 (2H, m, $NCH_2$), 6.82 (1H, dd, J 5 and 2Hz, C6—H), 7.0—7.6 (4H, m, $SCH_2$, OH and C4—H), 8.05 (3H, s, $COCH_3$), 8.31 (1H, dt, J 14 and 6Hz, C4—H) and 8.68 (8H, d, J 6Hz, $CH_3$—C) (Found: C, 52.7; H, 5.8; N, 6.0%; $M^+$ at m/e 406.1562. $C_{20}H_{26}N_2O_5S$, $\frac{1}{2}$ $CHCl_3$ requires C, 52.8; H, 5.7; N, 6.0% and $M^+$ at m/e 406.1562). Next came the 2α,3β-isomer (103) (0.122 g); $\nu_{max}$ ($CHCl_3$) 3470, 2980, 1765, 1670 and 1515 $cm^{-1}$; τ ($CDCl_3$) 2.66 (5H, s, phenyl), 3.90 (1H, br, NH), 4.83 (2H, s, benzyl $CH_2$), 5.26 (1H, d, J 7Hz, C2—H), 5.7—61. (2H, m, C5—H and C8—H), 6.47 (1H, td, J 9 and 7Hz, C3—H), 6.68 (2H, q, J 6Hz, $NCH_2$), 6.95 (1H, dd, J 5 and 3Hz, C6—H), 7.24 and 7.43 (2H, 2dt, J 12 and 6Hz, $SCH_2$), 7.32 (1H, br, OH), 7.7—8.0 (2H, m, C4—$H_2$), 8.08 (3H, s, $COCH_3$) and 8.71 (3H, d, J 6Hz, C—$CH_3$) ($M^+$ at m/e 406.1542. $C_{20}H_{26}N_2O_5S$ requires 406.1562). Most polar isomer was the 2β,3α-compound (104) (0.049 g); $\nu_{max}$ ($CHCl_3$) 3480, 2980, 1770, 1745, 1675 and 1515 $cm^{-1}$; τ ($CDCl_3$) 2.64 (5H, s, phenyl), 3.97 (1H, br, NH), 4.81 (2H, s, benzyl $CH_2$), 5.89 (1H, d, J 7Hz, C2—H), 5.8—6.0 (1H, m, C8—H), 6.1—6.4 (2H, m, C3—H and C5—H), 6.72 (2H, q, J 6Hz, $NCH_2$), 6.93 (1H, dd, J 5 and 2Hz, C6—H), 7.29 and 7.47 (2H, 2dt, J 12 and 6Hz, $SCH_2$), 7.73 (1H, dt, J 12 and 6Hz, C4—H). 8.10 (3H, s, $COCH_3$), 8.11 (1H, td, J 12 and 10Hz, C4—H) and 8.71 (3H, d, J 6 Hz, C—$CH_3$) ($M^+$ at m/e 406.1561. $C_{20}H_{26}N_2O_5S$ requires 406.1562).

## Example 87

Benzyl 3-(2-acetamidoethylthio)-6-(1-hydroxyethyl)-7-oxo-1-azabicyclo[3.2.0]hept-3-ene-2-carboxylate

(i) *From 2α,3α-isomer (102).*

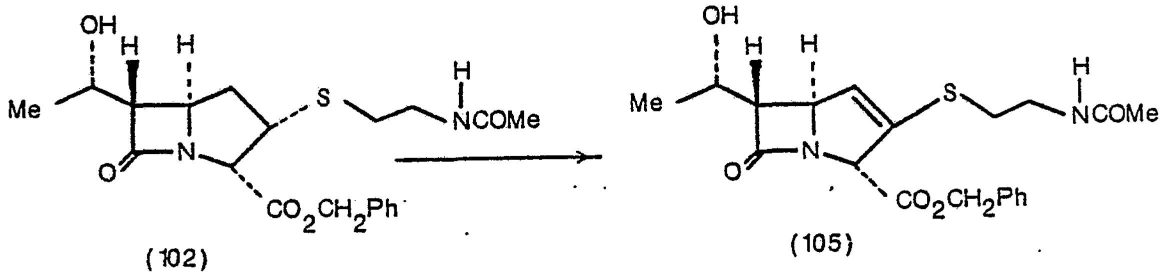

(102) (105)

A solution of the 2α,3α-isomer of benzyl 3-(2-acetamidoethylthio)-6-(1-hydroxyethyl)-7-oxo-1-azabicyclo[3.2.0]heptane-2-carboxylate (102) (0.140 g) in dry tetrahydrofuran (10 ml) was treated under argon with hexamethyldisilazane (1.8 ml) followed by chlorotrimethylsilane (0.6 ml). After 2 hours at room temperature, the reaction mixture was centrifuged and the supernatant solution was concentrated and put under high vacuum for 1$\frac{1}{2}$ hours. The residue was dissolved in benzene (10 ml) and centrifuged again to remove further solid. The benzene solution was stirred at 5° under argon and treated with pyridine (0.081 g) and iodobenzene dichloride (0.100 g). After 18 hours at that temperature, the solution was filtered and concentrated. Residual gum was dissolved in ethyl acetate (10 ml) and stirred vigorously with pH 2 aqueous buffer (10 ml) for 4 hours. The organic phase was separated, washed with aqueous sodium bicarbonate, then brine, and dried over sodium sulphate. Residue was chromatographed on silica gel 60 (<230 mesh) eluting with chloroform/ethanol 9:1 to give benzyl 3-(2-acetamidoethylthio)-6-(1-hydroxyethyl)-7-oxo-1-azabicyclo[3.2.0]hept-3-ene-2-carboxylate (105) (0.060 g); $\nu_{max}$ ($CHCl_3$) 3460, 2980, 1770, 1750 sh, 1670, 1570 and 1510 $cm^{-1}$; π ($CDCl_3$) 2.65 (5H, s, phenyl), 3.88 (1H, br, NH), 4.00 (1H, t, J 2Hz, C4—H), 4.81 (2H, s, benzyl $CH_2$), 4.91 (1H, dd, J 3 and 2Hz, C2—H), 5.46 (1H, ddd, J 3, 3 and 2 Hz, C5—H), 5.81 (1H, br quin. J 6Hz, C8—H), 6.61 (2H, q, J 6Hz, $NCH_2$), 6.85 (1H, dd, J 5 and 3Hz, C6—H), 7.0—7.4 (3H, m, $SCH_2$ and OH), 8.08 (3H, s, $COCH_3$) and 8.66 (3H, d, J 6Hz, C—$CH_3$) ($M^+$ at m/e 404.1388. $C_{20}H_{24}N_2O_5S$ requires 404.1404).

(ii) From $2\alpha,3\beta$-isomer (103).

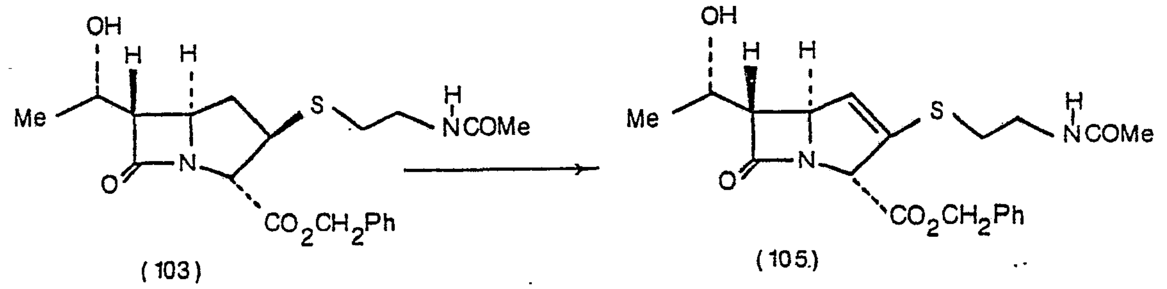

(103) (105)

The $2\alpha,3\beta$-isomer (103) (0.122 g) was subjected to the same set of reactions as described above. This gave 0.078 g of Δ-3 compound (105) identical to the sample prepared in part (i) of this example.

Example 88

Benzyl 3 - (2 - acetamidoethylthio) - 6 - (1 - hydroxyethyl) - 7 - oxo - 1 - azabicyclo[3.2.0]hept - 2 - ene-2 - carboxylate.

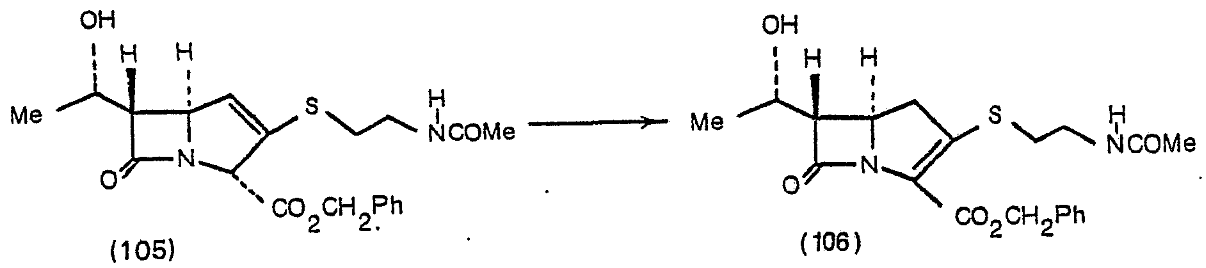

(105) (106)

Benzyl 3 - (2 - acetamidoethylthio - 6 - (1 - hydroxyethyl) - 7 - oxo - 1 - azabicyclo[3.2.0]hept-3-ene-2-carboxylate (05) (0.078 g) was stirred in dry methylene chloride (10 ml) under argon at room temperature and treated with 1.5-diazabicyclo[5.4.0]undec-5-ene (0.08 g). A reaction time of 4 hours was allowed, and the solution was then concentrated and chromatographed in Kieselgel 60 (230 mesh) eluting with chloroform/ethanol 9:1 grading to 4:1. This gave recovered Δ-3 compound (05) (0.051 g) plus benzyl 3-(2-acetamidoethylthio)-6-(1-hydroxyethyl)-7-oxo-1-azabicyclo[3.2.0]hept-2-ene-2-carboxylate (106) (0.006 g); $\lambda_{max}$ (ethanol) 318 nm (10,100); $\lambda_{max}$ ($CHCl_3$) 3470, 3370, 2990, 1780, 1670, 1550 and 1515 $cm^{-1}$; $CDCl_3$) 2.5—2.8 (5H, m, phenyl), 3.95 (1H, br, NH), 4.63 and 4.71 (2H, Abq, J 12Hz, benzyl $CH_2$), 5.7—6.0 (1H, m, C8—H), 5.88 (1H, td, J8 and 3Hz, C5—H), 6.61 (2H, q, J 6Hz, $NCH_2$), 6.76 (2H, d, J 8Hz, C4—$H_2$), covering *ca*, 6.8 (1H, C6—H), 6.9—7.2 (2H, m, $SCH_2$), 7.8 (1H, v, br, CH), 8.04 (3H, s, $COCH_3$) 8.04 (3H, s, $COCH_3$) and 8.65 (3H, d, J 6Hz, C—$CH_3$)-]; ($M^+$ at m/e 404.1398. $C_{20}H_{24}N_2O_5S$ requires 404.1406).

Example 89

p - Bromophenacyl - 6 - (3 - methoxycarbonyl - prop - 2 - ene - 1 - yl) - 3 - ethylthio - 7 - oxo - 1 - aza-bicyclo[3.2.0]heptane - 2 - carboxylates.

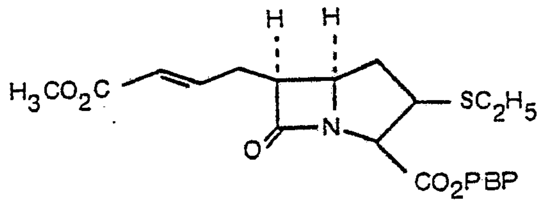

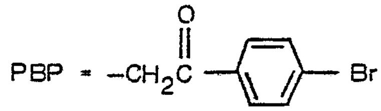

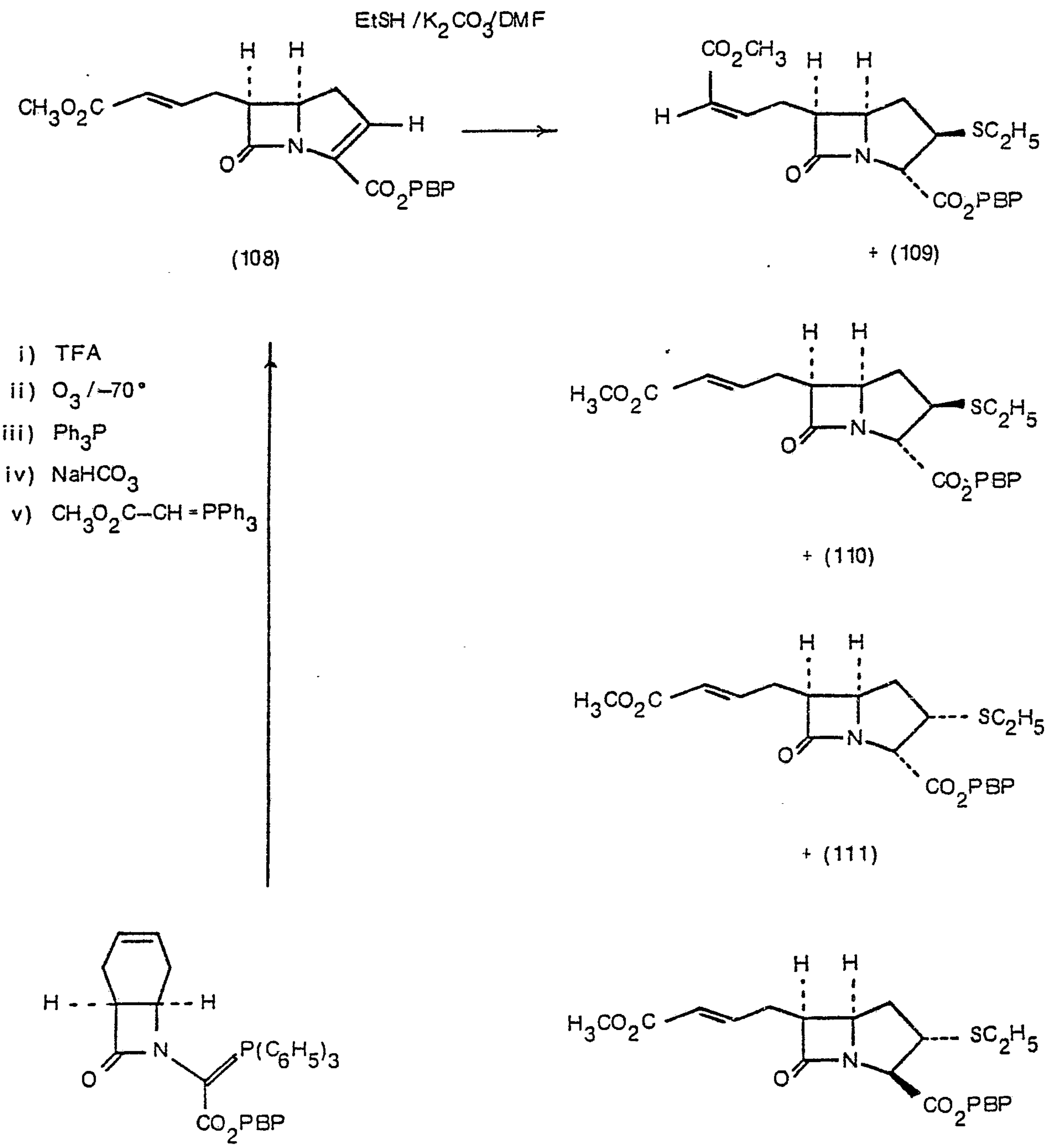


(108) + (109) + (110) + (111) (107) (112)

Example 89

p-Bromophenacyl 6-(3-methoxycarbonyl-prop-2-ene-1-yl)-3-ethylthio-7-oxo-1-azabicyclo[3,2,0]-heptane-2-carboxylates

The phosphorane (107) (5.0 g) in ethyl acetate (400 ml) was treated with trifluoroacetic acid (80 ml) at room temperature for 30 mins. The solution was ozonolysed at −70° (20 mins.) and then treated with triphenylphosphine (2.64 g, 1 equiv.) at this temperature. The mixture was warmed to 0°, saturated aqueous sodium hydrogen carbonate solution (200 ml) was added, and neutralisation completed with addition of solid sodium hydrogen carbonate. The ethyl acetate solution was washed with saturated aqueous sodium chloride solution (3 × 20 ml) and dried ($Na_2SO_4$). Methoxycarbonylmethylene triphenylphosphorane (3.0 g) (1.1 equiv.) was added, and the solution stirred at room temperature for 1 h. The solvent was removed *in vacuo*, and the crude ester (108) in DMF (15 ml) was treated with ethanethiol (0.485 g, 0.585 ml, 1 equiv.) in the presence of finely-ground, anhydrous potassium carbonate (1.08 g, 1 equiv.) for 30 mins. The reaction mixture was diluted with ethyl acetate (150 ml) and washed with saturated sodium chloride solution (3 × 20 ml). Recovery in the usual way afforded a gum which was chromatographed on Kieselgel 60 (Merck, <230 Mesh) (20 × 4 cm), 50 ml fractions).

Elution of the column with ethyl acetate — light petroleum (1:5) (fractions 16—20) gave *p-bromophenacyl 6 - [(Z] - 3 - methoxycarbonyl - prop - 2 - en - 1 - yl] - 3β - ethylthio - 7 - oxo - 1-azabicyclo[3.2.0]heptane-2α-carboxylate* (109) which crystallized from ethylacetate-light petroleum (1:1) as prisms (0.125 g) (3%) m.p. 92° (*Found:* C, 51.4; H, 4.8; N, 2.8; S, 6.4. $C_{22}H_{24}BrNO_6S$ requires C, 51.8; H, 4.7; N, 2.7; S, 6.3%) $\nu_{max}$ ($CHCl_3$) 1765 sh, 1760, 1720 sh, 1710, 1650, 1590 $cm^{-1}$; δ ($CDCl_3$) 1.25 (3H, t, J 7Hz), 2.20 (2H, m, $C_4$—$H_2$), 2.5—3.2 (2H, m), 2.64 (2H, q, J 7Hz), 3.3—3.7 (2H, m, C3α—H + C6α—H), 3.67 (3H, s), 4.18 (1H, m, $W_{\frac{1}{2}}$ 14 Hz, C5α—H), 4.79 (1H, d, J 7Hz, 2β—H), 5.17 (1H, d, J, 16Hz) and 5.39 (1H, d, J 16Hz) (ABq), 5.85 br (1H, d, J 11Hz), 5.22 (1H, ddd, J 11, 8, 7Hz), 7.55 (2H, d, J 8Hz) and 7.74 (2H, d, J 8Hz) (AA'BB'). Irradiation at the frequency of the C2β-proton located the resonance for the C3α-proton at δ 3.5.

Continued elution of the column (ethyl acetate — light petroleum, 1:3) gave a mixture of isomers (110) and (111) (1.912 g). Crystallization (ethyl acetate — light petroleum) afforded *p-bromo-phenacyl 6-[(E) 3-methoxycarbonyl-prop-2-en-1-yl]-3β-*ethylthio-7-oxo-1-azabicyclo[3.2.0]heptane-2*α-carboxylate* (110) as needles (0.648 g) (16%) m.p. 133—134° (Found: C, 51.8; H, 4.5; N, 2.7; S, 6.3. $C_{22}H_{24}BrNO_6S$ requires C, 51.8; H, 4.7; N, 2.7; S, 6.3%) $\nu_{max}$ ($CHCl_3$) 1760, 1720 sh, 1710, 1660, 1590 $cm^{-1}$; $\lambda_{max}$ 218 sh, and 258 nm (ε 9,200); δ ($CDCl_3$) 1.24 (3H, t, J 7Hz), 2.05—2.30 (2H, m, C4—$H_2$), 2.4—2.8 (2H, m) 2.64 (2H, q, J 7Hz), 3.27—3.65 (2H, m, C3α—H and C6α—H), 3.69 (3H, s), 4.05 (1H, m, $W_{\frac{1}{2}}$ 13Hz, C5α—H), 4.80 (1H, d, J 7Hz, C2β—H), 5.20 (1H, d, J 17Hz) and 5.41 (1H, d, J 17Hz) (ABq), 5.83 br (1H, d, J 15Hz), 6.87 (1H, dt, J 15, 6Hz), 7.56 (2H, d, J 8Hz) and 7.4 (2H, d, J 8Hz) (AA'BB').

Rechromatography of the mother liquors gave mixed fractions (0.185 g), together with pure *p-bromophenacyl-6-[(E) 3-methoxycarbonyl-prop-2-en-1-yl]-3α-ethylthio-7-oxo-1-azabicyclo[3.2.0]-heptane-2α-carboxylate (111) as a foam (0.595 g) (15%),* $\nu_{max}$ ($CHCl_3$) 1760 st, 1720, 1710, 1660, 1590 $cm^{-1}$; δ ($CDCl_3$) 1.28 (3H, t, J 7Hz), 1.2—2.3 (1H, m, C4—H), 2.4—2.8 (3H, m), 2.72 (2H, q, J 7Hz), 3.6 br (1H, m, C6α—H), 3.72 (3H, s), 4.1 br (2H, m, C5α—H + C3β—H), 4.46 (1H, d, J 5Hz, C2β—H), 5.20 (1H, d, J 17Hz) and 5.41 (1H, d, J 17Hz), (ABq), 5.83 br (1H, d, J 15Hz), 6.87 (1H, dt, J 15, 6Hz), 7.58 (2H, d, J 8Hz) and 7.72 (2H, d, J 8Hz) AA'BB').

Finally, elution with ethyl acetate — light petroleum (1:1) gave *p-bromophenacyl 6-[(E)3-methoxycarbonyl-pro-2-en-1-yl]-3α-ethylthio-7-oxo-1-azabicyclo[3,2,0]heptane-2β-*carboxylate (112) as a gum (750 mg). Rechromatography followed by trituration with ether gave micro crystals (0.685 g) (17%) m.p. 86—88° (*Found*: C, 51.8; H, 4.7; N, 2.7; S, 6.0. $C_{22}H_{24}BrNO_6S$ requires C, 51.8; H, 4.7; N, 2.7; S, 6.3%) $\nu_{max}$ ($CHCl_3$) 1765, 1750, 1720 sh, 1710, 1660 and 1590 $cm^{-1}$; δ ($CDCl_3$) 1.25 (3H, t, J 7Hz), 2.10 (2H, t, J 9Hz, C4—$H_2$), 2.64 (4H, m), 3.36 (1H, m, $W_{\frac{1}{2}}$ 13Hz, C6α—H), 3.5—4.0 (2H, m, C3β—H + C5α—H), 3.69 (3H,s), 4.23 (1H, d, J 8Hz, C2α—H), 5.25 (1H, d, J 18Hz) and 5.45 (1H, d, J 18Hz) (ABq). 5.82 br (1H, d, J 15Hz), 6.87 (1H, dt, J 15, 6Hz), 7.55 (2H, d, J 8Hz) and 7.75 (2H, d, J 8Hz) (AA'BB').

Total yield of thiol adducts: 2.238 g (56% overall from (108).

Example 90

Sodium 6-(3-methoxycarbonyl-2-propen-1-yl)-3-ethylthio-7-oxo-1-azabicyclo[3.2.0]heptane-2-carboxylate

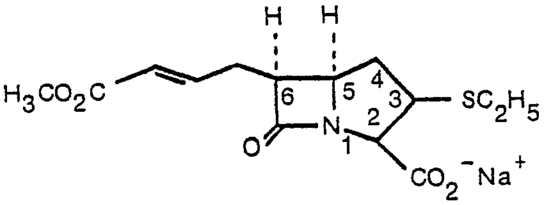

(a) From isomer (110) (example 89)

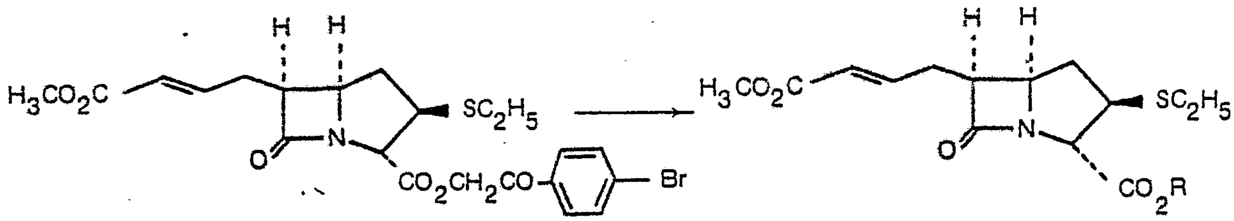

(110)

(113) a, R = Na
b, R = $CH_2C_6H_5$

The *p*-bromophenacyl ester (110) (0.125 g) in an anhydrous dimethylformamide (0.25 ml) was treated with an excess of sodium thiophenoxide (0.020 g; *ca* 1.5 equiv.) at 0° in an atmosphere of argon. The solution was stirred at room temperature for 3.5 h., at which time all the ester (110) had been consumed. Cold ether (10 ml) was added dropwise, and the solution stirred at 0° for 30 minutes. The solution was decanted, and the precipitate washed well with further portions of cold, dry ether, to give the 2α,3β-isomer (113a) (0.074 g) as the dimethylformamide hemi-solvate, m.p. 215—218°. (*Found:* C, 49.8; H, 5.5; H, 5.3%. $C_{14}H_{18}NNaO_5S$. $\frac{1}{2}$ DMF requires C, 50.0; H, 5.8: N, 5.7%). $\nu_{max}$ ($CHCl_3$) 1750, 1720, 1665 (DMF), 1655 sh, 1605 $cm^{-1}$; δ ($D_2O$) 1.09 (3H, t, J 8Hz), 1.98 (2H, t, J 7Hz, C4—$H_2$), 2.3—2.6 (2H, m), 2.53 (2H, q, J 8Hz), 2.71 (s) and 2.87 (s) (together 3H, DMF), 3.60 (3H, s), 3.67 (1H, q, J *ca* 6Hz, C6—H), 4.12 (1H, q, J *ca* 6Hz, C5—H), 4.32 (2H, d, J 7Hz, $Cr_\beta$—H), 5.85 br (1H, d, J 16Hz), 6.89 (H, dt, J 16, 6Hz). 7.84 br (0.5 H, DMF).

The sodium salt (0.02 g) in anhydrous dimethylformamide (0.2 ml), was treated with an excess of benzyl bromide (0.05 ml), overnight. Removal of the solvent *in vacuo* (oil pump, room temperature), chromatography of the residue (0.04 g) on Kieselgel 60 (Merck; <230 mesh), followed by elution with ethyl acetate — petroleum ether (1:4) afforded *benzyl 6-(3-methoxycarbonyl-2-propen-1-yl)-3β-ethylthio-7-oxo-1-azabicyclo[3,2,0]heptane-2α-carboxylate* (113 b) as a gum (0.019 g), $\nu_{max}$ ($CHCl_3$) 1760, 1735, 1720, 1660 $cm^{-1}$; δ ($CDCl_3$) 1.18 (3H, t, J 7Hz), 2.0—2.30 (2H, m, C4—$H_2$), 2.3—2.75 (2H, m, C6 side-chain $CH_2$), 2.56 (2H, q J 7Hz, —SC*H*$CH_3$), 3.2—3.7 (2H, m, C3—H and C6—H), 3.72 (3H, s), *ca.* 4.1 br (1H, m, C5—H), 4.69 (1H, d, J 7Hz, C2—H), 5.15 (2H, s), 5.82 br (1H, d, J 15Hz), 6.85 (1H, dt, J 15, 6Hz), 7.33 (5H, s). (*Found*: m/e 403.1450. $C_{21}H_{25}NO_5S$ requires $M^+$ 403.1453).

(b) From isomer (111) (example 89)

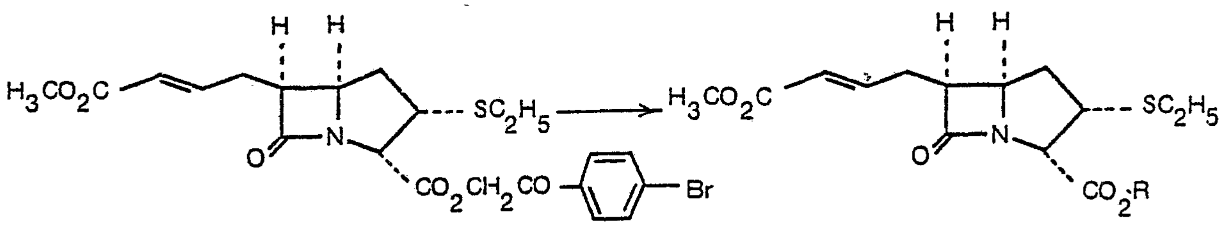

(111)

(114) a, R = Na
b, R = $CH_2C_6H_5$

The *p*-bromophenacyl ester (111) (0.085 g) in anhydrous dimethyl formamide (1.5 ml) was treated with sodium thiophenoxide (0.27 g) as previously described (2.5 h). Precipitation with ether as previously described gave an off-white precipitate of the 2α,3α-sodium salt (114a) (38 mg) $\nu_{max}$ ($CHCl_3$) 1755, 1720, 1600 $cm^{-1}$.

The sodium salt (35 mg) in dimethylformamide (0.5 ml) was treated with an excess of benzyl bromide overnight. Recovery and chromatography in the usual way gave the benzyl ester (124 b) as a gum (45 mg) $\nu_{max}$ ($CHCl_3$) 1765, 1740, 1725, 1655 $cm^{-1}$; δ ($CDCl_3$) 1.17 (3H, t, J 7Hz), 1.4—2.8 (2H, m, C4—$H_2$), 2.4—2.8 (2H, m), 2.55 (2H, q, J 7Hz), *ca.* 3.15 (1H, m, 3β—H), *ca.* 3.55 (1H, m, 6α—H), 3.68 (3H, s), 4.96 (1H, m, C5α—H), 4.27 (1H, d, J 6Hz, C2β—H), 5.14 (2H, s), 5.80 br (1H, d, J 15Hz), 6.84 (1H, m), 7.25 (5H, s) (*Found:* m/e 403.1481. $C_{21}H_{25}NO_5S$ requires $M^+$ 403.1453).

(c) From isomer (112) (example 89)

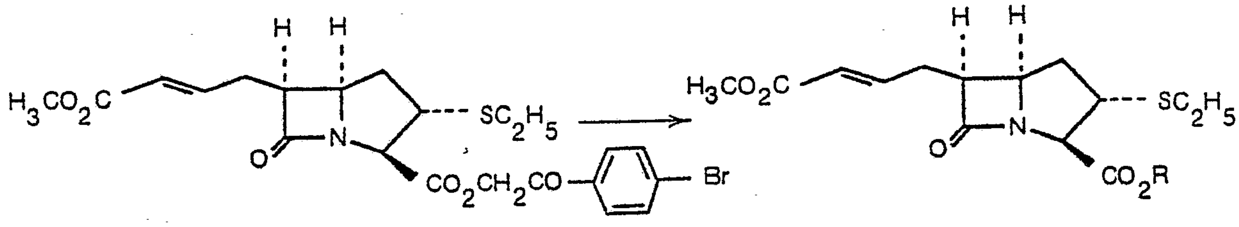

(112)

(115) a, R = Na
b, R = $CH_2C_6H_5$

The *p*-bromophenacyl ester (112) (0.077 g) in anhydrous dimethylformamide (0.5 ml) was treated with an excess of sodium thiophenoxide (0.03 g; 1.5 equiv.) in an atmosphere of argon at 0°. The solution was stirred at room temperature for 2h. Precipitation, and recovery of the crude sodium salt (115a) as before gave gum (0.045 g). $\nu_{max}$ ($CHCl_3$) 1750, 1720, 1610 $cm^{-1}$. Benzylation with benzyl bromode (0.2 ml) in anhydrous dimethylformamide (0.5 ml) overnight as previously described gave an oil (0.080 g) which was chromatographed on Kieselgel. Elution with ethyl acetate — petroleum ether (3:7) gave *benzyl 6-(3-methoxycarbonyl-2-propen-1-yl)-3α-ethylthio-7-oxo-1-azabicyclo[3.2.0]heptane-2β-carboxylate* (115b) as a gum (0.085 g) which crystallised (ethyl acetate — light petroleum) as needles (0.029 g) m.p. 117°, $\nu_{max}$ ($CHCl_3$ 1765, 1720, 1655 $cm^{-1}$; δ ($CDCl_3$) 1.17 (3H, t, J 7Hz), 1.9—2.1 (2H, m, C4—$H_2$), 2.3—2.6 (2H, m), 2.54 (2H, q, J 7Hz), 3.28 (1H, m, C6—H), 3.5—3.9 (2H, m, C3—H and C5—H), 3.67 (3H, s), 4.09 (1H, d, J 8Hz, C2α—H), 5.13 (2H, s), 5.69 (1H, dt, J 15, 1Hz), 6.78 (1H, dt, J 15, 6Hz), and 7.30 (5H, s) (*Found*: C; 62.1; H, 6.5; N, 3.4%; m/e 403.1453. $C_{21}H_{25}NO_5S$ requires C, 62.5; H, 6.2; N, 3.5%; $M^+$ 403.1453).

*Note:*— Deprotection also effective in Z-isomers:—

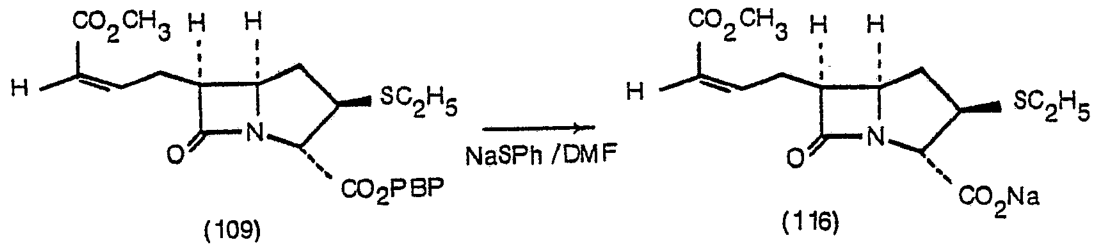


(109) (116)

(d) From isomer (109) example 89

*p*-Bromophenacyl ester (109) (0.072 g) in dimethylformamide (1.0 ml) was treated with sodium thiophenoxide (0.021 g) in the usual way (3h). Precipitation with ether afforded the sodium salt (116), $\nu_{max}$ ($CHCl_3$) 1760, 1720, 1600 $cm^{-1}$.

In all the above deprotections (example 90 a—d), *p*-bromophenacylphenyl sulphide was isolated from the supernatant ether after precipitation of the sodium salts.

Example 91

6-(3-methoxycarbonyl-2-propen-1-yl)-3-ethylthio-7-oxo-1-azabicyclo[3.2.0]heptane-2-carboxylic acids (117)

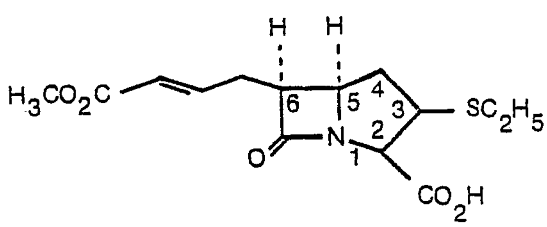

(117)

(23)

(a) From isomer (113a) (example 90)

The sodium salt (0.005 g) in $H_2O$ (0.2 ml) was treated with dilute aqueous citric acid solution and partitioned into ethyl acetate (x4) at pH 6 and 0°. Recovery of the organic phase yielded the 2α, 3β-isomer of the title compound (*ca.* 4 mg), $\nu_{max}$ ($CHCl_3$) 3500—2600, 1760, 1720 sh, 1660 $cm^{-1}$. ($Et_3N$ shift 1755, 1720, 1545 $cm^{-1}$).

(b) From isomer (115a) (example 90)

The sodium salt (0.045 g) was treated in the manner described above. Recovery of the ethyl acetate extracts gave the 2$\beta$, 3$\alpha$-free acid isomer (0.037 g) as a white powder $\nu_{max}$ ($CHCl_3$) 3500—2600, 1760, 1720, 1660 $cm^{-1}$. ($Et_3N$ shift→1760, 1720, 1660 sh, 1620 br); $\delta$ ($CDCl_3$) 1.30 (3H, t, J 7Hz), 2.0—3.0 (6H, m), 3.5—4.0 (2H, m, C3$\beta$—H + C6$\alpha$—H), 3.75 (3H, s), 4.20 (1H, d, J 8Hz, C2$\alpha$—H), 5.95 br (1H, dm, J 15Hz), *ca.* 6.9 (1H, m), 9.2 br (1H).

Example 92

Phthalidyl 6-(3-methoxycarbonyl-prop-2-ene-1-yl)-3-ethylthio-7-oxo-1-azabicyclo[3.2.0]heptane-2-carboxylates (118)

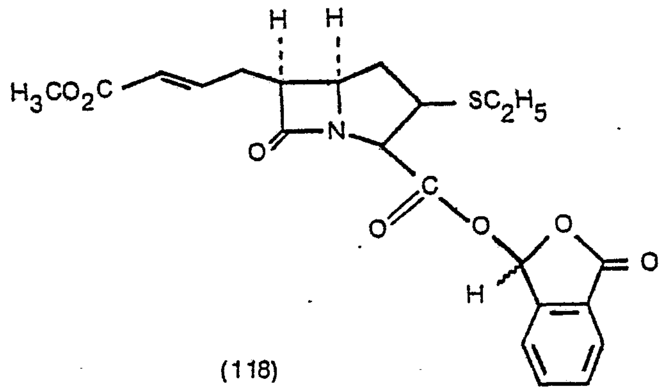

(118)

(a) From p-bromophenacyl ester (110) example (89)

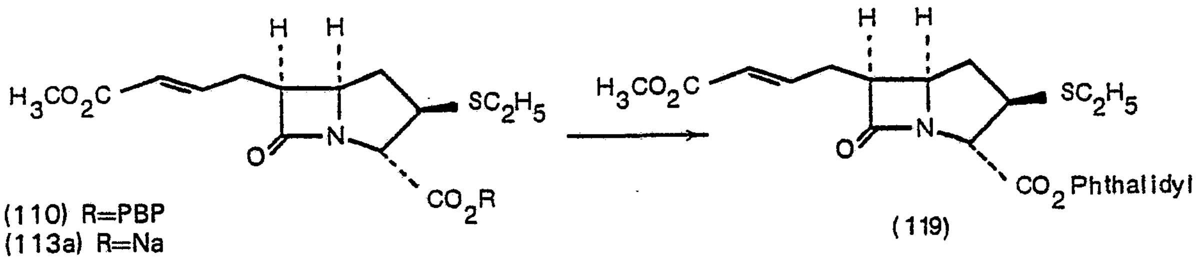

(110) R=PBP
(113a) R=Na

(119)

The C2$\alpha$, C3$\beta$-ester (110) (0.20 g) in dimethylformamide (2 ml, anhydrous) was treated with sodium thiophenoxide (0.055 g) as described above (Example 90). After 6 h, the C2$\alpha$, C3$\beta$-sodium salt (113a) was precipitated with ether in the usual way, and washed with a further portion of cold ether. The purified salt was redissolved in dimethylformamide (2 ml) and treated with bromophthalide (0.084 g) at room temperature in an atmosphere of argon overnight. The solution was diluted with ethyl acetate (50 ml), washed with saturated aqueous sodium chloride solution (3 x 15 ml) and dried ($Na_2SO_4$). Recovery gave an oil which was chromatographed on Kieselgel (5 x 2 mm) [elution with ethyl acetate — light petroleum (3:7)]. Fractions 23—31 afforded an oil which, on trituration with diethyl ether, followed by rapid removal of the solvent *in vacuo* gave a mixture of phthalide epimers of the C2$\alpha$, C3$\beta$-isomer (119) of the title compound as a foam, which crystallised on standing at 0° overnight. The material (0.126) (72% overall from *p*-bromophenacyl ester) had m.p. 48—50° (*Found:* C, 59.1; H, 5.2; N, 3.1; S, 7.2 $C_{22}H_{23}NO_7S$ requires C, 59.3; H, 5.2; N, 3.1; S, 7.2%) $\nu_{max}$ ($CHCl_3$) 1790, 1770, 1750 sh, 1720, 1660 $cm^{-1}$; $\delta$ ($CDCl_3$) 1.14 (t, J 7Hz, minor epimer) and 1.25 (t, J 7Hz, major epimer (together, 3H), 2.0—2.25 (2H, m, C4—$H_2$), 2.3—2.8 (4H, m), 3.46 (1H, m, $W\frac{1}{2}$ 20Hz, C3$\alpha$—H), 3.70 (3H, s), 4.19 (1H, m, $W\frac{1}{2}$ 14Hz, C5$\alpha$—H), 4.73 (d, J 7.5Hz) and 4.75 (d, J 7.5Hz) (together 1H, C2$\beta$—H), 5.85 (1H, dd, J 16, 1Hz), 6.89 (1H, dt, J 16, 6Hz), 7.47 (1H, s), 7.70 br, (3H, s), 7.92 (1H, m); $\lambda_{max}$ (EtOH) 282 nm ($\varepsilon$ 2,240), 2.74 (2500), 228 sh (17,700).

Careful crystallization of the material ($CHCl_3$/EtOAc/$Et_2O$) deposited rosettes of one phthalide epimer m.p. 140—142°.

(b) From p-bromophenacyl ester (111) (example (89)

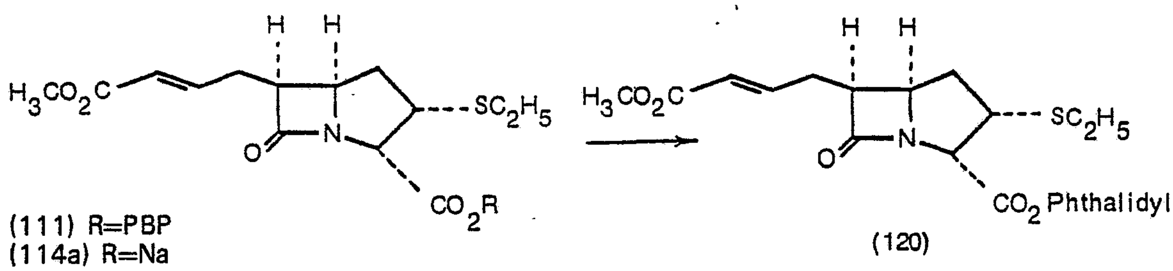

(111) R=PBP
(114a) R=Na

(120)

The C2$\alpha$,C3$\alpha$-ester (111) (0.57 g) in dimethylformamide (3 ml) was treated with sodium thiophenoxide (0.16 g) as described above (4h). The sodium salt (114a) was precipitated and purified in the usual way, redissolved in dimethylformamide (4 ml) and treated with bromophthalide (0.26 g) at room temperature for 5 h. Recovery and chromatography yielded the C2$\alpha$,C3$\alpha$-phthalidyl ester, which was a mixture of phthalide epimers, (120) as a gum (0.29 g) (58% overall from *p*-bromophenacyl ester), $\nu_{max}$ 1790 sh, 1775, 1725, 1660 cm$^{-1}$; $\delta$ ($CDCl_3$) 1.20 (t, J 7Hz) and 1.25 (t, J 7Hz) (together, 3H), 1.5—1.8 (1H, m, C4—H), 2.3—2.75 (4H, m), 3.1—4.15 (4H, m), 3.70 (3H, s), 4.33 (1H, d, J 6Hz, C2$\beta$—H), 5.83 br (1H, d, J 16Hz), 6.87 (1H, dt, J 16, 6Hz), 7.40 (1H, s), 7.5—8.0 (4H, m).

(c) From p-bromophenacyl ester (112) (example 89)

The C2$\beta$,C3$\alpha$-ester (112) (0.2 g) in dimethylformamide was treated with sodium thiophenoxide (0.055 g) (3h). Purification of the sodium salt (115a) was followed by treatment with bromophthalide (0.1 g) in dimethylformamide in the usual way. Recovery and chromatography gave phthalide epimers of (121) the C2$\beta$,C3$\alpha$-isomer of the title compound (0.092 g) (53%), $\nu_{max}$ ($CDCl_3$) 1785, 1770, 1740 sh, 1720, 1660 cm$^{-1}$; $\delta$ ($CDCl_3$) 1.24 (3H, t, J 7Hz), 1.8—2.7 (6H, m), 3.3 (1H, m $W_{\frac{1}{2}}$ 10Hz, C6$\alpha$H), 3.67 (3H, s), 3.8 (1H, m $W_{\frac{1}{2}}$ 10Hz, C5$\alpha$—H), 4.08 (d, J 5Hz), and 4.17 (d, J 5Hz), (together 1H, C2$\alpha$—H), 5.69 (dt, J 15, 1Hz) and 5.78 (dt, J 15, 1Hz) (together 1H), 6.90 (1H, m, $W_{\frac{1}{2}}$ 20Hz), 7.43 (s) and 7.46 (s) (together, 1H), 7.5—7.9 (4H, m).

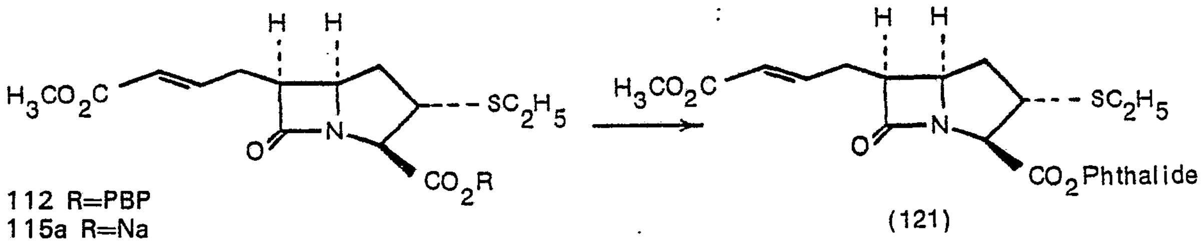

112 R=PBP
115a R=Na

(121)

Example 93

Isomerisation of C2$\beta$,C3$\alpha$-series to C2$\alpha$,C3$\alpha$-substituted esters

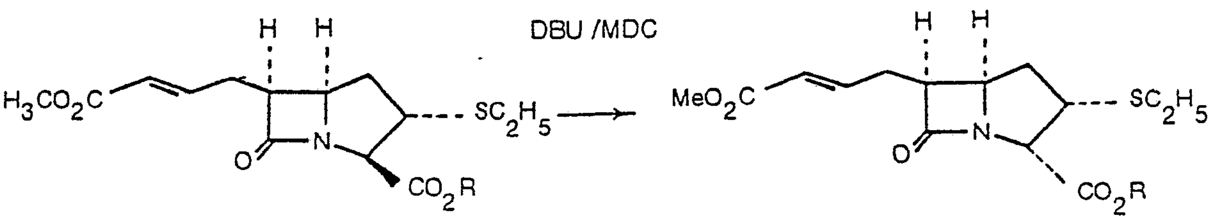


R = PBP (112) → (111)
R = Phthalidyl (121) → (120)

(a) p-bromophenacyl ester

Ester (112) (0.005 g) in anhydrous methylene chloride (0.2 ml) was treated with 1,5-diazabicyclo[5.4.0]undec-5-ene (DBU) (0.001 g) in methylene chloride (0.1 ml) at 0° in an atmosphere of argon. The solution was warmed to room temperature and stirred for 4 h. The solution was diluted with methylene chloride, washed with saturated sodium chloride solution (3 × 1 ml) and dried ($Na_2SO_4$). Recovery gave a gum (4 mg), identical (i.r. spectrum, t.l.c. analysis) with the previous samples of C2$\alpha$,C3$\alpha$-ester (111) (example (89).

(b) Phthalidyl ester

Ester (121) (0.005 g) was treated with DBU as described above (example a). Recovery gave the

C2α,C3α-phthalide ester (120), identical with authentic material (example 92) (i.r. spectrum, t.l.c. analysis).

Examples 94 and 95

3-Ethylthio-6-(3-methoxycarbonyl-prop-2-en-1-yl)-7-oxo-1-azabicyclo[3.2.0]hept-2-ene-2-carboxylic esters

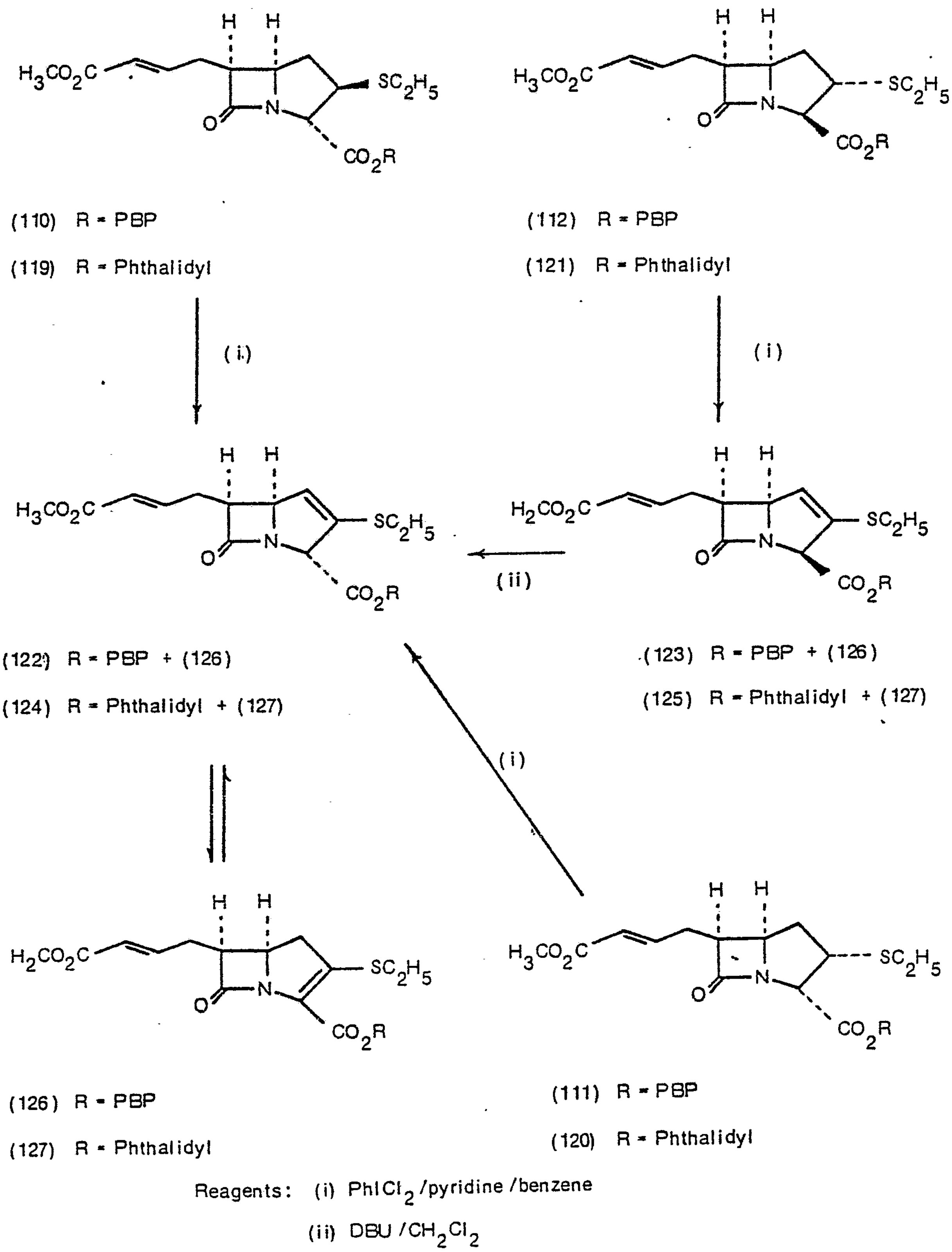


(110) R = PBP

(119) R = Phthalidyl

(112) R = PBP

(121) R = Phthalidyl

(122) R = PBP + (126)

(124) R = Phthalidyl + (127)

(123) R = PBP + (126)

(125) R = Phthalidyl + (127)

(126) R = PBP

(127) R = Phthalidyl

(111) R = PBP

(120) R = Phthalidyl

Example 94

p-bromophenacyl series

(a) Reaction of (110) with IBD/pyridine under anhydrous conditions

The C2$\alpha$,C3$\beta$-ester (110) (0.070 g) in anhydrous benzene (2 ml) was treated with pyridine (0.022 g) (2 equivalents), followed by iodobenzene dichloride (IBD) (0.039 g) (1 equivalent) in at atmosphere of argon at 0—5° for 3 h. The reaction mixture was chromatographed on Kieselgel (8 × 2 cm; 1:1 mixture of >230 mesh and 230—400 mesh grades) eluting with ethyl acetate — light petroleum (1:1) (6 ml fractions).

Fractions 3—6 [Rf. 0.65, eluting with ethyl acetate — light petroleum (7:3)] afforded *p-bromophenacyl 3-ethylthio-6-(3-methoxycarbonyl-prop-2-en-1-yl)-7-oxo-1-azabicyclo[3.2.0]hept-3-ene-2α-carboxylate* (122) as a gum (0.048 g) (69%). $\nu_{max}$ ($CHCl_3$) 1775, 1760, 1715 br, 1660, 1595 $cm^{-1}$; $\delta$ ($CDCl_3$) 1.32 (3H, t, J 7Hz), 1.8—2.5 (2H, m), 2.86 (2H, q, J 7Hz), 3.67 br (1H, m, C6$\alpha$—H), 3.72 (3H, s), 4.75 (1H, m, $W_{\frac{1}{2}}$ 10Hz, C5$\alpha$—H), 5.20 (1H, d, J 3Hz, C4—H), 5.31 (2H, s), 5.67 (1H, d, J 2Hz, C2$\beta$—H), 5.86 (1H, d, J 16Hz), 6.93 (1H, dt, J16, 7Hz), 7.61 (2H, d, J 9Hz) and 7.76 (2H, d, J 9Hz) (AA'BB').

Fractions 8—12 [Rf. 0.53] gave *p-bromophenacyl 3-ethylthio-6-(3-methoxycarbonyl-prop-2-en-1-yl)-7-oxo-1-azabicyclo[3.2.0]hept-2-ene-2-carboxylate* (126) (0.015 g) (21%) which crystallized from ethyl acetate — diethyl ether — light petroleum as needles m.p. 147—150° (*Found:* C, 51.9; H, 4.0; N, 3.1. $C_{22}H_{22}BrNO_6S$ requires C, 52.0; H, 4.4; N, 2.8%), $\nu_{max}$ ($CHCl_3$) 1780, 1735 sh, 1730, 1720 sh, 1660, 1590 $cm^{-1}$; $\lambda_{max}$ (EtOH) 318 nm ($\varepsilon$ 4,500) 256 (9,100), 210; $\delta$ ($CDCl_3$) 1.33 (3H, t, J 7Hz), 2.3—3.05 (4H, m), 2.38 (2H, q, J 7Hz), 3.74 (3H, s) and 3.75 (1H, m, $W_{\frac{1}{2}}$ 10Hz, 6$\alpha$—H), 4.37 (1H, m, $W_{\frac{1}{2}}$ 13Hz, C5$\alpha$—H), 5.23 (2H, d, J 16Hz) and 5.51 (2H, d, J 16Hz) (ABq), 5.84 br (1H, d, J 16Hz), 6.91 (1H, dt, J 16, 7Hz), 7.57 (2H, d, J 9Hz), and 7.78 (2H, d, J 9Hz) (AA BB ).

(b) Isomerization of the $\Delta^3$ compound (122) to the $\Delta^2$ ester (126)

*p*-Bromophenacyl ester (122) (0.025 g) in methylene chloride (2 ml, anhydrous) was treated with DBU (0.003 g) in an atmosphere of argon at 0—25° until equilibrium had been established (4h). Recovery and chromatography on Kieselgel (2.5 × 1 cm) (230—400 mesh), eluting with ethyl acetate — light petroleum (2:3) (1 ml fractions) gave (fractions 5—18) unchanged (122) (0.015 g), followed by the $\Delta^2$-isomer (126) (fractions 19, 20) (0.004 g), identical (i.r., u.v. spectra; t.l.c. analysis) with the material obtained in example 94a.

Example 95

Phthalidyl series

(a) Reaction of (119) with IBD/pyridine under anhydrous conditions

The C2$\alpha$,C3$\beta$-phthalidyl ester (119) (0.070 g) in benzene (2 ml) was treated with pyridine (0.025 g) (2 equivalents) and iodobenzene dichloride (0.049 g) (1 equivalent) as described above (example 94) (2.5 h). Recovery and chromatography gave (fractions 8—15) (Rf. 0.50) *phthalidyl 3-ethylthio-6-(3-methoxycarbonylprop-2-en-1-yl)-7-oxo-azabicyclo[3.2.0]hept-3-ene-2α-carboxylate* (124) as a foam (36 mg) (52%) $\nu_{max}$ ($CHCl_3$) 1785, 1775 sh, 1720, 1660, 1600, 1570 $cm^{-1}$; $\delta$ ($CDCl_3$) 1.26 (3H, cpx m), 2.2—2.6 (2H, m), 2.79 (2H, q, J 7Hz), 3.65 (1H, m, $W_{\frac{1}{2}}$ 7Hz, C6$\alpha$—H), 3.69 (3H, s), 4.71 (1H, m, $W_{\frac{1}{2}}$ 7Hz, C5$\alpha$—H), 5.06 (1H, dd, J3 2Hz, C2$\beta$—H), 5.62 (1H, q, J 2Hz, C4—H), 5.82 (dt, J 16, 1Hz), 6.88 (dt, J 16, 6Hz), 7.38 (1H, s), 7.5—7.8 (3H, m) and 7.8—7.95 (1H, m); irradiation at $\delta$ 2.47 simplified the multiplet due to the C6$\alpha$-proton centred at $\delta$ 3.65, and irradiation at the frequency of the C6$\alpha$-proton-sharpened the C5$\alpha$-proton resonance centred at $\delta$ 4.71. The material crystallised slowly (ex EtOAc/$Et_2O$) m.p. 82—87°. (*Found:* C, 58.5; H, 5.1; N, 2.9. $C_{22}H_{21}NSO_7$. $\frac{1}{2}$ $H_2O$ requires , 58.4; H, 4.9; N, 3.1%).

Fractions 19—29 (Rf. 0.36) afforded *phthalidyl 3-ethylthio-6-(3-methoxycarbonyl-prop-2-en-1-yl)-7-oxo-1-azabicyclo[3.2.0]hept-2-ene-2-carboxylate* (127) as a gum (5 mg) (7%) $\nu_{max}$ ($CHCl_3$) 1785, 1775, 1740, 1720 sh, 1660 $cm^{-1}$; $\lambda_{max}$ (EtOH) 325 nm ($\varepsilon$ 3500), 280 (2500), 272 (2500). Crystallisation (chloroform — light petroleum) gave a powder m.p. 60—64°; $\delta$ ($CDCl_3$) 1.35 (3H, t, J 7Hz, 2.5—3.1 (3H, m, C4—$H_2$) + S$CH_2CH_3$ + C6 — side chain $CH_2$), 3.72 (3H, s), 3.75 (1H, m $W_{\frac{1}{2}}$ 10Hz, C6$\alpha$—H), 4.35 (1H, m, $W_{\frac{1}{2}}$ 12.5Hz, C5$\alpha$—H), 5.85 br (1H, d, J 16Hz), 6.88 (1H, dt, J 16, 7Hz), 7.36 (1H, s), 7.5—7.8 (3H, m) and 7.8—7.95 (1H, m) (*Found:* C, 59.3; H, 4.5; N, 2.8. $C_{22}H_{21}NSO_7$ requires C, 59.7; H, 4.8; N, 3.2%).

(b) Isomerisation of the $\Delta^3$ compound (124) to the $\Delta^2$-isomer (127)

The phthalidyl ester (124) (0.005 g) in methylene chloride was treated with DBU as described in exmaple 94b. Recovery and chromatography afforded (127) identical (i.r., u.v. spectra; t.l.c. analysis) with the material previously prepared (example 95a).

(c) Reaction of (120) with IBD/pyridine under anhydrous conditions

The C2$\alpha$,C3$\alpha$-phthalidyl ester (120) in benzene was treated with IBD and pyridine as described

under example 95a. The reaction gave the same product (124) that was obtained from the C2$\alpha$, C3$\beta$-isomer (119) (*cf* example 95a).

(d) Reaction of (121) with IDB/pyridine under anhydrous conditions

The C2$\beta$-C3$\alpha$-phthalidyl ester (121) in benzene was treated with IBD and pyridine as described above. The reaction afforded *phthalidyl 3-ethylthio-6-(3-methoxycarbonyl-prop-2-en-1-yl)-7-oxo-1-azabicyclo[3.2.0]hept-3-ene-2$\beta$-carboxylate* (125), together with smaller amounts of the $\Delta^2$-isomer (125).

Isomerisation of the $\Delta^3$ compound (125)

The C2$\beta$—$\Delta^3$-phthalidyl ester (125) in methylene chloride was treated with DBU as described in example (95a). to give an equilibrium mixture with the C2$\alpha$—$\Delta^2$-ester (127).

## Example 96

Preparation of (—) and (+) p-nitrobenzyl 7-oxo-1-azabicyclo[3.2.]hept-2-ene-2-carboxylate

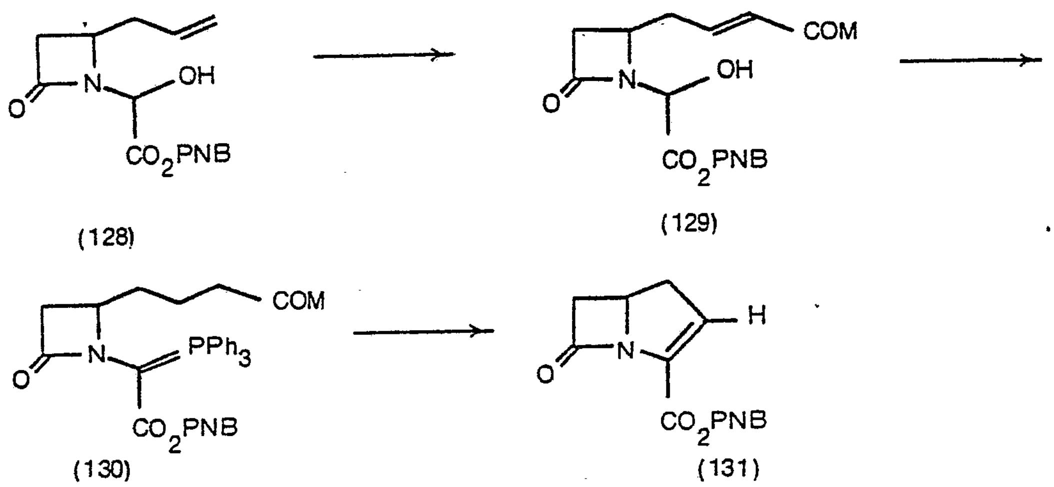

(128) (129)

(130) (131)

[(—) and (+)] isomers

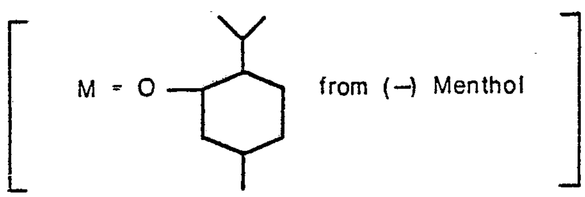


The glyoxylate (128) (8 g) was dissolved in ethyl acetate (100 ml); the solution was cooled to —70° and ozone passed through until the solution just became blue. The excess ozone was then removed by blowing a stream of argon through the solution. Triphenylphosphine (7.2 g) in the minimum volume of ethyl acetate was added to the reaction, while continuing to blow argon through the solution. After allowing the reaction temperature to rise to 0° solid (—) carbomenthoxymethylenetriphenylphosphorane (12.7 g) was added and the reaction stirred for 0.5 hour, when room temperature had been attained.

The solution was evaporated to dryness and chromatographed on silica gel (finer than 230 mesh) made up in 3/7 ethyl acetate/60°—80° petroleum ether. The column was initially eluted with 3/7 ethyl acetate/60°—80° petroleum ether (6 × 100 ml, fractions 1 to 6). Elution was continued using 1/1 ethyl acetate/60°80° petroleum ether. Fractions 7 to 11 were of 100 ml volume; fractions 12 to 71 of 20 ml volume. The product (129), which exists as a mixture of 4 diastereoisomers, was detected in fractions 19 to 56 (T.L.C. analysis).

Fractions 30 to 56 were combined and evaporated to dryness to give a gum (2.3 g), which was crystallised from ethyl acetate/60°—80° petroleum ether to give crystalline material (129) (A) (1.12 g) and mother liquors (129) (b) (1.1 g).

(—) p-Nitrobenzyl 7-oxo-1-azabicyclo[3.2.0]hept-2-ene-2-carboxylate

The crystalline product (129) (A) (1.12 g) was recrystallised from $CHCl_3$ — ether to give purer material (0.66 g), m.p. 112—115°, $\nu_{max}$ ($CHCl_3$) 3550, 1765, 1770, 1710, 1660, 1605, 1520 and 1350 cm$^{-1}$; $\delta$ ($CDCl_3$) 0.70—2.60 (18H, m), 2.67 (1H, dd, J 16 and 3Hz), 3.10 (1H, dd, J 16 and 6Hz), 3.90—4.13 (1H, m), 4.30 (1H, exch. $D_2O$), 4.74 (1H, dt, J 9 and 6Hz), 5.33 (2H, s), 5.40 (1H, slightly

broadened, collapsing to sharp singlet on $D_2O$ exch.), 5.85 (1H, d, J 16Hz), 6.80 (1H, dt, J 16 and 7Hz), 7.55 (2H, d, J 9Hz), 8.23 (2H, d, J 9Hz).

The above alcohol (0.56 g) in tetrahydrofuran (20 ml) was treated (at −20°) with 2,6 lutidine (0.24 g) and thionyl chloride (0.27 g). After 20 minutes the solution was filtered, the filtrate evaporated to dryness from toluene and the residue dissolved in dioxan (20 ml). Triphenylphosphine (0.58 g) and 2,6 lutidine (0.24 g) were added and the reaction mixture stirred at room temperature overnight. The solution was filtered, evaporated to dryness and dissolved in ethyl acetate (50 ml), which was washed with 0.1 N HCl (20 ml) and then water. The dried ($MgSO_4$) organic phase was evaporated to dryness and chromatographed on silica gel (finer than 230 mesh), eluting with 3/7 ethyl acetate/60°—80° and then ethyl acetate to give the product (130) (0.63 g), $[\alpha]_D^{22°}$ − 9.7° (C, 1.0 $CHCl_3$). This phosphorane (0.6 g) in ethyl acetate (12 ml) and trifluoroacetic acid (3 ml) was cooled to −70° and treated with ozone until slightly blue. Excess ozone was removed with a stream of argon, when triphenylphosphine (0.3 g) in ethyl acetate was added. The solution was removed from the cold bath and at *ca* 0° neutralized by stirring vigorously with a saturated soluton of aqueous sodium bicarbonate (5%). The organic phase was separated, dried ($MgSO_4$), evaporated to small volume and chromatographed rapidly on silica gel (230—400 Mesh ASTM). Elution with 1/1 ethyl acetate — 60°—80° petroleum ether gave (—) p-nitrobenzyl 7-oxo-1-azabicyclo[3.2.0]hept-2-ene-2-carboxylate (131) as a yellow solid (87 mg) after trituration with ether, $[\alpha]_D^{22°}$ − 42.12° (C, 0.6 $CHCl_3$).

(+) p-Nitrobenzyl 7-oxo-1-azabicyclo[3.2.0]hept-2-ene-2-carboxylate

The mother liquors (129) (B) (1.02 g) were treated with 2,6 lutidine (0.44 g) and thionyl chloride (0.48 g) as described for the crystalline isomer; the chloride was similarly treated with triphenylphosphine (1.06 g) and 2,6 lutidine (0.44 g) to give the phosphorane (130) (1.14 g), $[\alpha]_D^{24°}$ − 31.8° (C 1.81 $CHCl_3$). This phosphorane (0.935 g) was dissolved in ethyl acetate (20 ml) and trifluoroacetic acid (5 ml) and cyclised as for the previous isomer using triphenylphosphine (0.47 g) to reduce the ozonide. The product after chromatography was a light yellow solid (94 mg), $[\alpha]_D$ + 23.2° (C, 0.6 $CHCl_3$).

The preparation of (—) carbomenthoxymethylenetriphenylphosphorane

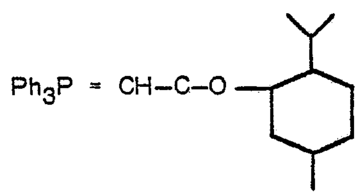

(—) Menthol (50 g) was dissolved in methylene chloride (100 ml) and treated with N,N-dimethylaniline (49 ml). The solution was cooled (ice-bath) and bromoacetyl bromide (33.5 ml) was added dropwise over 0.5 hour. The solution was washed with sodium bicarbonate solution and then water. The organic phase was dried and evaporated to give (—) menthylbromoacetate (78 g), $[\alpha]_D^{20}$ − 64.5° (C, 2.5 $CHCl_3$) as an oil. The foregoing ester (60 g) was dissolved in dioxan (100 ml) and stirred with triphenylphosphine (57 g) at 50° overnight. The solution was evaporated to dryness and the product crystallised by trituration with ether to give (—) carbomenthoxymethyl triphenylphosphonium bromide (115 g), m.p. 100—103°, $[\alpha]_D^{20°}$ − 26.8° (C, 2.5 $CHCl_3$). This bromide (50 g) was dissolved in chloroform (200 ml) and stirred rapidly while adding a saturated solution of sodium bicarbonate (200 ml). The organic phase was separated and shaken vigorously twice more with saturated bicarbonate solution (2 × 200 ml). The organic phase was dried and evaporated and the residue crystallised from ether to give the title compound (28 g), m.p. 159—160°, $[\alpha]_D^{20°}$ − 8.36 (C, 2.5).

Example 97

(—) p-Nitrobenzyl 3-ethylthio-7-oxo-1-azabicyclo[3.2.0]hept-2-ene-2-carboxylate

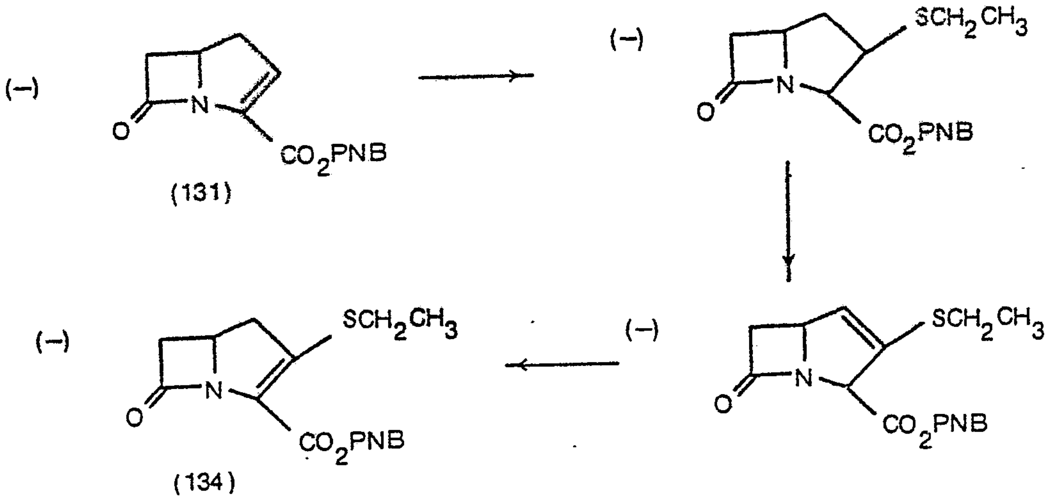
(−) (131) (−) (132) (−) (133) (−) (134)

(—) p-Nitrobenzyl 7-oxo-1-azabicyclo[3.2.0]hept-2-ene-2-carboxylate (131) $[\alpha]_D$ − 42°, prepared as in Example 96 (75 mg) was treated with ethanethiol (18 mg) on dry dimethylformamide (1 ml). Potassium carbonate (3.6 mg) was then added and the mixture stirred at ambient temperature for 30 minutes. The yellow solution was diluted with ethyl acetate, washed with water then brine, dried over magnesium sulphate, filtered and evaporated to an oil. This oil, taken up in toluene was chromatographed on silica gel (200—300 mesh, 5 g). Elution with 30—40% ethyl acetate in petroleum ether (60°—80°) gave three stereoisomers of p-nitrobenzyl 3-ethylthio-7-oxo-1-azabicyclo[3.2.0]heptane-2-carboxylate (132) (70 mg).

The two less polar isomers (1:1 ethyl acetate: petroleum ether (60°—80°)) were not separated and the mixture as a gum (50 mg) was treated with dry pyridine (27 mg) in dry benzene (3 ml). The clear solution obtained was cooled, under argon with stirring to its freezing point, iodobenzene dichloride (47 mg) was added to give a clear solution which was stood in the refrigerator (+ 8°). After 10 minutes a solid had precipitated. After 1 hour the reaction solution has decanted off and chromatographed on silica gel (230—400 Mesh, 2 x 4 cm). Elution with 40% ethyl acetate in petroleum ether (60°—80°) gave (—) p-nitrobenzyl 3-ethylthio-7-oxo-1-azabicyclo[3.2.0]hept-3-ene-2-carboxylate (133) as a gum (20 mg), $[\alpha]_D^{25°}$ − 42° (C, 1 in chloroform).

This gum (18 mg) was treated with D.B.U. (2.4 mg) in dry methylene chloride (0.75 ml). After 1.75 hours the yellow solution was chromatographed on Florisil (200—300 M, 1 x 4 cm). Elution with 50% ethyl acetate in petroleum ether (60°—80°) gave recovered starting material (133) as a gum (10 mg), $[\alpha]_D^{25°}$ − 37.5° (C, 0.67 in chloroform) followed by (—) p-nitrobenzyl 3-ethylthio-7-oxo-1-azabicyclo[3.2.0]hept-2-ene-2-carboxylate (134) as yellow crystals (5.3 mg) (ex ether) m.p. 107—118°, $\lambda_{max}$ (ethanol) 320 and 267 nm. $[\alpha]_D^{25°}$ − 21.2° (C, 0.35 in chloroform).

Example 98

(+) p-Nitrobenzyl 3-ethylthio-7-oxo-1-azabicyclo[3.2.0]hept-2-ene-2-carboxylate

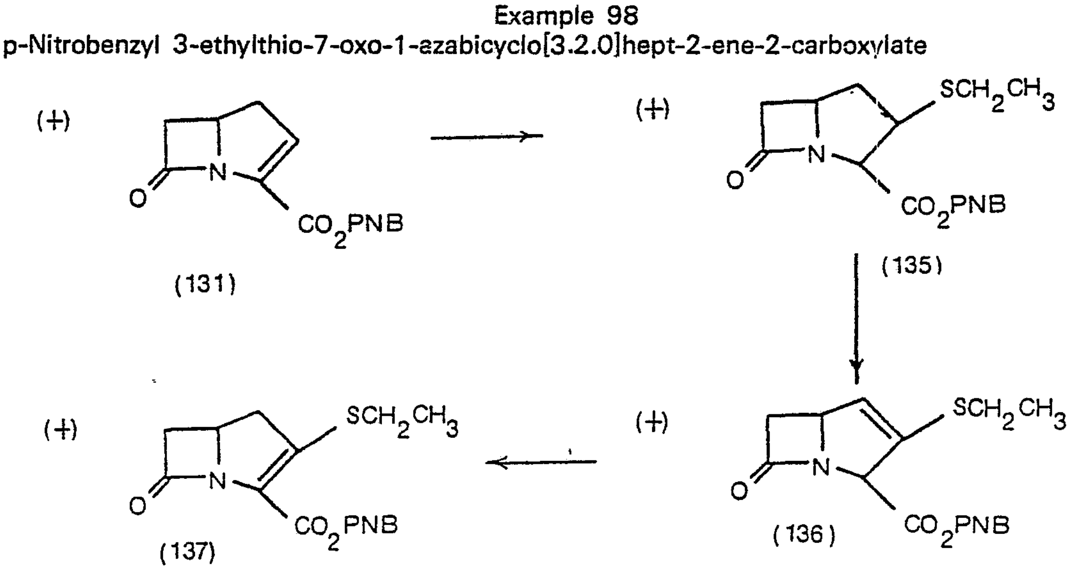
(+) (131) (+) (135) (+) (136) (+) (137)

+ p-Nitrobenzyl 7-oxo-1-azabicyclo[3.2.0]hept-2-ene-2-carboxylate (131), $[\alpha]_D$ + 23° prepared as in Example 96 (85 mg) was treated with ethanethiol (27.5 mg) in dry dimethylformamide (1 ml). To the stirred mixture was added potassium carbonate (4 mg). After 1 hour work up and chromatography as Example 97 gave three stereoisomers of p-nitrobenzyl 3-ethylthio-7-oxo-1-azabicyclo[3.2.0]heptane-2-carboxylate (135), (76 mg).

The two less polar isomers (1:1 ethylacetate: petroleum ether (60°—80°)) were not separated and the mixture obtained as a gum (55 mg), which was treated with dry pyridine (30 mg) in dry benzene (3 ml). The solution obtained was cooled under argon with stirring to its freezing point then iodobenzene dichloride (52 mg) was added to give a clear solution which was stood at +8° for 1.5 hours. A solid was precipitated. Work up and chromatography as in Exmaple (97) gave (+) p-nitrobenzyl 3-ethylthio-7-oxo-1-azabicyclo[3.2.0]hept-3-ene-2-carboxylate (136) as a gum (17.5 mg), $[\alpha]_D^{25°}$ + 26° (C, 0.875 in chloroform).

This gum (17 mg) was treated with D.B.U. (2.2 mg) in dry methylene chloride (0.7 ml) for 1.75 hours. Chromatography of the yellow reaction solution on florisil (200—300 Mesh, 1 × 4 cm), eluting with 50% ethyl acetate in petroleum ether (60°—80°) gave recovered starting material (136) (10 mg) $[\alpha]_D^{25°}$ + 19.8° (C, 0.67 in chloroform) followed by (+) p-nitrobenzyl 3-ethylthio-7-oxo-1-azabicyclo-[3.2.0]hept-2-ene-carboxylate (137) as yellow crystals (4.5 mg) (ex ether) m.p. 120—125°, $\lambda_{max}$ (ethanol) 320 and 267 nm, $[\alpha]_D^{25°}$ + 10° (c, 0.3 in chloroform).

## Example 99

(±)

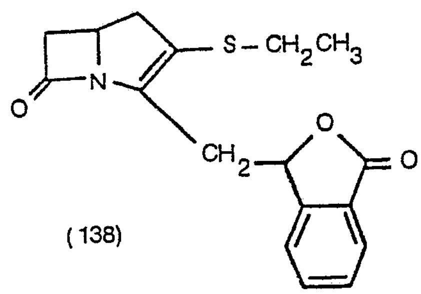

(138)

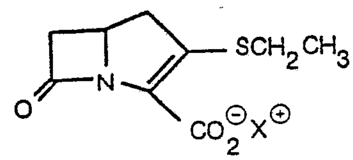

(139)

$X^{\oplus}$ is a counter-ion

(±) Phthalidyl 3-ethylthio-7-oxo-1-azabicyclo[3.2.0]hept-2-ene-2-carboxylate (138) was treated with mouse plasma at a concentration of 165 $\mu$gml$^{-1}$.

Aliquots (20 $\mu$l) were removed at certain time intervals and assayed by h.p.l.c. using a $C_{18}$ reversed phase column (Waters G8 Microbondapak) eluting with 0.05 M ammonium phosphate buffer (pH 4.7) containing 5% acetonitrile, and monitoring at 300 nm with a u.v. detector. It was found that the preferred enantiomer of 3-ethylthio-7-oxo-1-azabicyclo[3.2.0]hept-2-ene-2-carboxylate (139) had a retention time in this system of 4.6 minutes with a flow rate of 3 ml min.$^{-1}$.

Demonstration of Effectiveness

Antibacterial Activity (MIC in μg/ml) of (+), (–) and (±)-p-nitrobenzyl 3-ethylthio-7-oxo-1-azabicyclo-[3.2.0]hept-2-ene-2-carboxylate

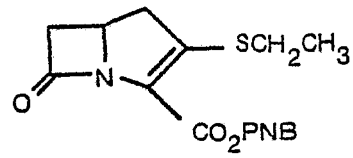

(±) $[\alpha]_D$ 0°

(–) $[\alpha]_D$ – 21°

(+) $[\alpha]_D$ + 10°

| Organism | (±) | (–) | (+) |
|---|---|---|---|
| Citrobacter freundii E8 | 50 | >50 | 25 |
| Escherichia coli ESS | 12.5 | 25 | 5.0 |
| Escherichia coli NCTC 10418 | 50 | >50 | 25 |
| Escherichia coli 0111 | >50 | >50 | 50 |
| Escherichia coli ATCC 27166 | 12.5 | 25 | 12.5 |
| Klebsiella aerogenes A | 25 | 25 | 12.5 |
| Salmonella typhimurium CT10 | 50 | >50 | 50 |
| Shigella sonnei MB11967 | >50 | >50 | 50 |
| Branhamella catarrhalis 1502 | 1.2 | 2.5 | 0.5 |
| Bacillus subtilis ATCC 6633 | 25 | 50 | 25 |
| Staphylococcus aureus Oxford | >50 | >50 | >50 |
| Streptococcus pneumoniae CN33 | 1.2 | 2.5 | 0.5 |
| Streptococcus pyogenes CN10 | 1.2 | 2.5 | 1.2 |

In a standard test, the MIC's of the following compounds were obtained

| Organism | Compounds of Example No: 23 | 82 | 79 | 42 | 75 | 15 | 9 | 47 | 7 | 37 | 32 |
|---|---|---|---|---|---|---|---|---|---|---|---|
| Citrobacter freundii E8 | 1.6 | 3.1 | 6.2 | 1.6 | 25 | 3.1 | 12.5 | 25 | 2.5 | 1.6 | 3.1 |
| Enterobacter cloacoae N1 | — | 12.5 | 6.2 | 3.1 | 12.5 | 3.1 | — | 12.5 | 2.5 | 1.6 | 6.2 |
| Escherichia coli O111 | 1.6 | 1.6 | 6.2 | 3.1 | 25 | 3.1 | 12.5 | 6.2 | 2.5 | 0.8 | 3.1 |
| Escherichia coli JT39 | 6.2 | 12.5 | 6.2 | 6.2 | 50 | 1.6 | >50 | 50 | 50 | 6.2 | 6.2 |
| Klebsiella aerogenes A | 1.6 | 1.6 | 3.1 | 3.1 | 25 | 1.6 | 12.5 | 6.2 | 1.0 | 0.4 | 1.6 |
| Proteus mirabilis C977 | 12.5 | 12.5 | 6.2 | 25 | 25 | 25 | 25 | 50 | 10 | 1.6 | 6.2 |
| Proteus morganii I580 | 25 | 12.5 | 25 | 25 | 25 | 25 | 25 | 50 | 10 | 12.5 | 50 |
| Proteus rettgeri WM16 | 25 | 6.2 | 25 | 25 | 25 | 25 | >50 | >50 | 50 | 12.5 | 50 |
| Proteus vulgaris W091 | 25 | 12.5 | 25 | 25 | 50 | 25 | >50 | >50 | 50 | 12.5 | 50 |
| *Pseudomonas aeruginosa* A | >50 | >50 | >50 | >50 | >50 | 6.2 | >50 | >50 | >100 | >50 | >100 |
| Salmonella typhimurium CT10 | 3.1 | 3.1 | 3.1 | 3.1 | 12.5 | 3.1 | 12.5 | 6.2 | 2.5 | 0.8 | 3.1 |
| Serratia marcescens US20 | 25 | 12.5 | 12.5 | 3.1 | 50 | 6.2 | 50 | 50 | 10 | 6.2 | 12.5 |
| Shigella sonnei MB 11967 | — | 3.1 | 3.1 | 3.1 | 12.5 | 3.1 | 12.5 | 6.2 | 5.2 | 0.8 | 3.1 |
| Bacillus subtilis A | 3.1 | 1.6 | 6.2 | 1.6 | 5.0 | 0.8 | 25 | 1.6 | 1.0 | 0.8 | 1.6 |
| Staphylococcus aureus Oxford | 12.5 | 0.8 | 3.1 | 6.2 | 5.0 | 0.8 | >50 | 6.2 | 5.0 | 1.6 | 6.2 |
| Staphyloccus aureus Russell | 50 | 1.6 | 6.2 | 12.5 | 12.5 | >3.1 | >50 | 6.2 | 10 | 12.5 | 25 |
| Staphylococcus aureus 1517 | >50 | 50 | >50 | >50 | >50 | — | >50 | >50 | >100 | >50 | >50 |
| Streptococcus faecalis I | >50 | 50 | >50 | >50 | >50 | 25 | >50 | >50 | 100 | 25 | 50 |
| Streptococcus pneumoniae CN33 | ⩽0.2 | NG | 0.8 | ⩽0.2 | 1.2 | 0.2 | — | ⩽0.2 | 1.0 | ⩽0.2 | ⩽0.2 |
| Streptococcus pyogenes CN10 | 0.8 | NG | 3.1 | 0.8 | 5.0 | 0.4 | 12.5 | 1.6 | 1.0 | ⩽0.2 | 1.6 |

**Claims for the contracting States: BE CH DE FR GB NL SE**

1. A compound of the formula (II):

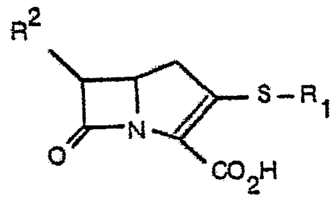

(II)

or a salt or ester thereof wherein $R_1$ is a lower alkyl group, an aralkyl group or is a lower alkyl group substituted on other than the carbon atom to which the sulphur atom is attached by a $CO_2R_3$, $OR_4$, $N(R_5)COR_6$, $N(R_5)CO_2R_6$ or $N(R_5)R_7$ group or by a $CO_2R_3$ and a $N(R_5)COR_6$ group; wherein $R^3$ is a group such that $CO_2R_3$ is a carboxylic acid group or a salt or lower alkyl or benzyl ester thereof; $R_4$ is a hydrogen atom or a lower alkyl, lower acyl, benzyloxycarbonyl or p-nitrobenzyloxycarbonyl group or a $CONR_8R_9$ group, $R_5$ is a hydrogen atom or a lower alkyl group; $R_6$ is a lower alkyl, benzyl or p-nitrobenzyl group; $R_7$ is a hydrogen atom or a lower alkyl, aralkyl, benzhydryl or trityl group; $R_8$ is a hydrogen atom or a lower alkyl or phenyl group; $R_9$ is a hydrogen atom or a lower alkyl group; and $R_2$ is a hydrogen atom or a an azido lower alkyl group or a group $CR_{10}R_{11}R_{12}$ wherein $R_{10}$ is a hydrogen atom or a hydroxyl group; $R_{11}$ is a hydrogen atom or a lower alkyl group; and $R_{12}$ is a hydrogen atom, loweracyloxyloweralkyl group, hydroxyloweralkyl group, lower alkyl group, a benzyl group, a phenyl group or is joined to $R_{11}$ to form part of a $C_{5-7}$ carbocyclic ring or is a group of the formula $CH(OH)R_{13}$ or CHX wherein $R_{13}$ is a hydrogen atom or lower alkyl group and X is an oxygen atom or a $C_{14}R_{15}$ group where $R_{14}$ is a hydrogen atom or a lower alkyl, phenyl, CN,$CO_2R_{16}$ or $CO.R_{16}$ group where $R_{16}$ is a lower alkyl, phenyl or benzyl group and $R_{15}$ is a hydrogen atom or a lower alkyl group or is joined to $R_{14}$ to form part of a $C_{5-7}$ carbocyclic ring; wherein "lower alkyl" means a group of up to 4 carbon atoms, and "aralkyl" means a group of up to 4 carbon atoms substituted by phenyl, fluorophenyl, chlorophenyl, bromophenyl, methoxyphenyl, methylphenyl, nitrophenyl or aminophenyl: with the proviso that the following compounds are excluded: wherein $R_2$ is 1-hydroxyethyl or acylated derivatives thereof or hydroxymethyl and $R_1$ is methyl or amino alkyl of up to 3 carbon atoms: wherein $R_2$ is 1-hydroxyethyl and $R_1$ is benzyl, $NH_2(CH_2)_4$—, $NH_2C(CH_3)_2CH_2$—, $NH_2CH_2C(CH_3)_2$— or N-substituted aminoethyl: wherein $R_2$ is hydroxymethyl and $R_1$ is $NH_2C(CH_3)_2CH_2$—; wherein $R_2$ is $PhCH_2CH(OH)$— or ethyl and $R_1$ is aminoethyl: and wherein $R_2$ is isopropyl and $R_1$ is 2-acetamidoethyl.

2. A compound according to claim 1 wherein $R_1$ is a lower alkyl group.

3. A compound according to claim 2 wherein $R_1$ is an ethyl group.

4. A compound according to claim 1 wherein $R_1$ is a $CH_2CH_2NHCOCH_3$ group.

5. A compound according to claim 1 wherein $R_1$ is a $CH_2CH_2OCONHCH_3$ group.

6. A compound according to claim 1 wherein $R_1$ is a lower alkyl group substituted on other than the carbon atom to which the sulphur atom is attached by a $OR_4$ group wherein the lower alkyl group is an ethyl group and $R_4$ is a hydrogen atom or a methyl or acetyl group.

7. A compound according to any of claims 1 to 6 wherein $R_2$ is a hydrogen atom.

8. A compound according to any of claims 1 to 6 wherein $R_2$ is a group $CR_{10}R_{11}R_{12}$ wherein $R_{10}$, $R_{11}$ and $R_{12}$ are as defined in claim 1.

9. A compound according to claim 8 wherein the $CR_{10}R_{11}R_{12}$ group is a $C(OH)(R_{19})R_{20}$ moiety wherein $R_{19}$ is a hydrogen atom or a lower alkyl group and $R_{20}$ is a hydrogen atom or a lower alkyl group.

10. A compound according to claim 9 wherein the $C(OH)(R_{19})R_{20}$ moiety is a $CH(OH)CH_3$ group.

11. A compound according to any of claims 1 to 10 in the form of a pharmaceutically acceptable salts.

12. A compound according to any of claims 1 to 10 wherein the ester group is of the sub-formulae (a), (b), (c) or (d):

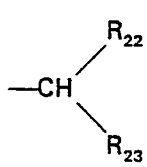 (a) 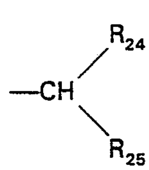 (b) 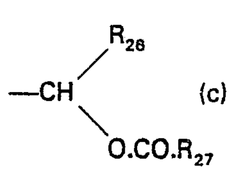 (c) 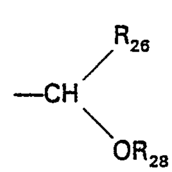 (d)

wherein $R_{22}$ is a hydrogen atom or an alkyl, alkenyl or alkynyl group of up to 3 carbon atoms; $R_{23}$ is a hydrogen atom or a methyl group; $R_{24}$ is a phenyl group or a phenyl group substituted by a fluorine, chlorine or bromine atom or a nitro, methyl or methoxyl group; $R_{25}$ is a hydrogen atom or a phenyl group or a phenyl group substituted by a fluorine, chlorine or bromine atom or a nitro, methyl or methoxyl group; $R_{26}$ is a hydrogen atom or a methyl group and $R_{27}$ is a lower alkyl, phenyl or

lower alkoxy group or $R_{26}$ is joined to $R_{27}$ to form a phthalidyl group; and $R_{28}$ is a lower alkyl, phenyl, chlorophenyl or nitrophenyl group; or $CH(R_{22})R_{23}$ is a phenacyl or p-bromophenacyl group.

13. A compound according to any of claims 1 to 10 in the form of its p-nitrobenzyl ester.

14. A compound according to any of claims 1 to 10 in the form of its phthalidyl ester.

15. A pharmaceutical composition which comprises a compound according to any of claims 1 to 14 and a pharmaceutically acceptable carrier.

16. A pharmaceuticaly composition according to claim 15 which also comprises a penicillin or cephalosporin.

17. A compound according to any of claims 1 to 16 when used as an antibacterial agent.

18. A process for the preparation of a compound according to any of claims 1 to 14 which process comprises the base catalysed isomerism of the double bond in an ester of a compound of the formula (VIII):

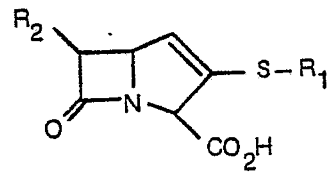

(VIII)

and thereafter if desired converting a cleavable ester of formula (II) into the compound of the formula (II) or a salt thereof.

19. 3-Ethylthio-7-oxo-1 azabicyclo[3.2.0]hept-2-ene-2-carboxylic acid or a pharmaceutically acceptable salt or cleavable ester thereof.

20. A pharmaceutically acceptable salt of 3-ethylthio-7-oxo-1-azabicyclo[3.2.0]hept-2-ene-2-carboxylic acid according to claim 19.

21. Phthalidyl 3-ethylthio-7-oxo-1-azabicyclo[3.2.0]hept-2-ene-2-carboxylate according to claim 19.

22. A compound according to any of claims 7, 19, 20 and 21 in form of the isomer having the same stereochemistry at C5 as (+)-p-nitrobenzyl 3-ethylthio-7-oxo-1-azabicyclo[3.2.0]hept-2-ene-2-carboxylate.

**Claims for the Contracting States: IT**

1. A compound of the formula (II):

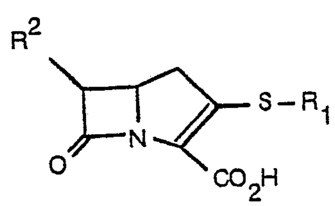

(II)

or a salt or ester thereof wherein $R_1$ is a lower alkyl group, an aralkyl group or is a lower alkyl group substituted on other than the carbon atom to which the sulphur atom is attached by a $CO_2R_3$, $OR_4$, $N(R_5)COR_6$, $N(R_5)CO_2R_6$ or $N(R_5)R_7$ group or by a $CO_2R_3$ and a $N(R_5)COR_6$ group; wherein $R^3$ is a group such that $CO_2R_3$ is a carboxylic acid group or a salt or lower alkyl or benzyl ester thereof; $R_4$ is a hydrogen atom or a lower alkyl lower acyl, benzyloxycarbonyl or p-nitrobenzyloxycarbonyl group or a $CONR_8R_9$ group, $R_5$ is a hydrogen atom or a lower alkyl group; $R_6$ is a lower alkyl, benzyl or p-nitrobenzyl group; $R_7$ is a hydrogen atom or a lower alkyl aralkyl, benzhydryl or trityl group; $R_8$ is a hydrogen atom or a lower alkyl or phenyl group; $R_9$ is a hydrogen atom or a lower alkyl group; and $R_2$ is a hydrogen atom or an azido lower alkyl group or a group $CR_{10}R_{11}R_{12}$ wherein $R_{10}$ is a hydrogen atom or a hydroxyl group; $R_{11}$ is a hydrogen atom or a lower alkyl group; and $R_{12}$ is a hydrogen atom, loweracyloxyloweralkyl group, hydroxyloweralkyl group, lower alkyl group, a benzyl group, a phenyl group or is joined to $R_{11}$ to form part of a $C_{5-7}$ carbocyclic ring or is a group of the formula $CH(OH)R_{13}$ or CHX wherein $R_{13}$ is a hydrogen atom or lower alkyl group and X is an oxygen atom or a $C_{14}R_{15}$ group where $R_{14}$ is a hydrogen atom or a lower alkyl, phenyl CN, $CO_2R_{16}$ or $CO.R_{16}$ group where $R_{16}$ is a lower alkyl, phenyl or benzyl group and $R_{15}$ is a hydrogen atom or a lower alkyl group or is joined to $R_{14}$ to form part of a $C_{5-7}$ carbocyclic ring; wherein "lower alkyl" means a group of up to 4 carbon atoms, and "aralkyl" means a group of up to 4 carbon atoms substituted by phenyl, fluorophenyl, chlorophenyl, bromophenyl, methoxyphenyl, methylphenyl, nitrophenyl or aminophenyl: except compounds wherein $R_2$ is 1-hydroxyethyl or acylated derivatives thereof and $R_1$ is aminoethyl or an N-substituted derivative thereof; and except compounds wherein $R_2$ is ethyl and $R_1$ is $CH_2CH_2NH_2$ and except compounds wherein $R_2$ is isopropyl and $R_1$ is 2-acetamidoethyl.

2. A compound according to claim 1 wherein $R_1$ is a lower alkyl group.

3. A compound according to claim 2 wherein $R_1$ is an ethyl group.

4. A compound according to claim 1 wherein $R_1$ is a $CH_2CH_2NHCOCH_3$ group.

5. A compound according to claim 1 wherein $R_1$ is a $CH_2CH_2OCONHCH_3$ group.

6. A compound according to claim 1 wherein $R_1$ is a lower alkyl group substituted on other than the carbon atom to which the sulphur atom is attached by a $OR_4$ group wherein the lower alkyl group is an ethyl group and $R_4$ is a hydrogen atom or a methyl or acetyl group.

7. A compound according to any of claims 1 to 6 wherein $R_2$ is a hydrogen atom.

8. A compound according to any of claims 1 to 6 wherein $R_2$ is a group $CR_{10}R_{11}R_{12}$ wherein $R_{10}$, $R_{11}$ and $R_{12}$ are as defined in claim 1.

9. A compound according to claim 8 wherein the $CR_{10}R_{11}R_{12}$ group is a $C(OH)(R_{19})R_{20}$ moiety wherein $R_{19}$ is a hydrogen atom or a lower alkyl group and $R_{20}$ is a hydrogen atom or a lower alkyl group.

10. A compound according to claim 9 wherein the $C(OH)(R_{19})R_{20}$ moiety is a $CH(OH)CH_3$ group.

11. A compound according to any of claims 1 to 10 in the form of a pharmaceutically acceptable salt.

12. A compound according to any of claims 1 to 10 wherein the ester group is of the sub-formulae (a), (b), (c) or (d):

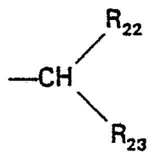 (a) 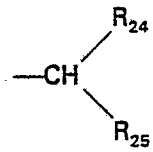 (b) 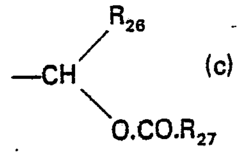 (c) 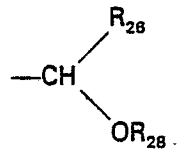 (d)

wherein $R_{22}$ is a hydrogen atom or an alkyl, alkenyl or alkynyl group of up to 3 carbon atoms; $R_{23}$ is a hydrogen atom or a methyl group; $R_{24}$ is a phenyl group or a phenyl group substituted by a fluorine, chlorine or bromine atom or a nitro, methyl or methoxyl group; $R_{25}$ is a hydrogen atom or a phenyl group or a phenyl group substituted by a fluorine, chlorine or bromine atom or a nitro, methyl or methoxyl group; $R_{26}$ is a hydrogen atom or a methyl group and $R_{27}$ is a lower alkyl, phenyl or lower alkoxy group or $R_{26}$ is joined to $R_{27}$ to form a phthalidyl group; and $R_{28}$ is a lower alkyl, phenyl, chlorophenyl or nitrophenyl group; or $CH(R_{22})R_{23}$ is a phenacyl or p-bromophenacyl group.

13. A compound according to any of claims 1 to 10 in the form of its p-nitrobenzyl ester.

14. A compound according to any of claims 1 to 10 in the form of its phthalidyl ester.

15. A pharmaceutical composition which comprises a compound according to any of claims 1 to 14 and a pharmaceutically acceptable carrier.

16. A pharmaceutical composition according to claim 15 which also comprises a penicillin or cephalosporin.

17. A compound according to any of claims 1 to 16 when used as an antibacterial agent.

18. A process for the preparation of a compound according to any of claims 1 to 14 which process comprises the base catalysed isomerism of the double bond in an ester of a compound of the formula (VIII):

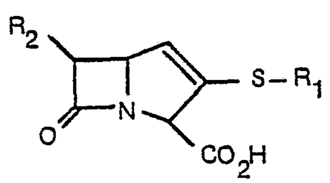

(VIII)

and thereafter if desired converting a cleavable set of formula (II) into the compound of the formula (II) or a salt thereof.

19. 3-Ethylthio-7-oxo-1 azabicyclo[3.2.0]hept-2-ene-2-carboxylic acid or a pharmaceutically acceptable salt or cleavable ester thereof.

20. A pharmaceutically acceptable salt of 3-ethylthio-7-oxo-1-azabicyclo[3.2.0]hept-2-ene-2-carboxylic acid according to claim 19.

21. Phthalidyl 3-ethylthio-7-oxo-1-azabicyclo[3.2.0]hept-2-ene-2-carboxylate according to claim 19.

22. A compound according to any of claims 7, 19, 20 and 21 in form of the isomer having the same stereochemistry at C5 as (+)-p-nitrobenzyl 3-ethylthio-7-oxo-1-azabicyclo[3.2.0]hept-2-ene-2-carboxylate.

**Patentansprüche für die Vertragsstaaten: BE CH DE FR GB NL SE**

1. Eine Verbindung der Formel (II):

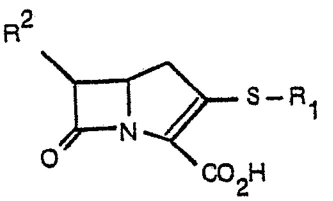

(II)

oder ein Salz oder Ester derselben, in der $R_1$ ein niedere Alkylgruppe, eine Arylalkylgruppe oder eine niedere Alkylgruppe ist, die an einem anderen Kohlenstoffatom, als das, an das das Schwefelatom gebunden ist, mit einer $CO_2R_3$—, $OR_4$—, $N(R_5)COR_6$—, $N(R_5)CO_2R_6$— oder $N(R_5)R_7$-Gruppe oder mit einer $CO_2R_3$— und einer $N(R_5)COR_6$-Gruppe substituiert ist, wobei $R_3$ eine solche Gruppe ist, daß $CO_2R_3$ eine Carbonsäuregruppe oder ein Salz oder ein niederer Alkyl- oder Benzylester derselben ist, $R_4$ ein Wasserstoffatom oder eine niedere Alkyl-, niedere Acyl-, Benzyloxycarbonyl- oder p-Nitrobenzyloxycarbonylgruppe oder eine $CONR_8R_9$-Gruppe ist, $R_5$ ein Wasserstoffatom oder eine niedere Alkylgruppe ist, $R_6$ eine niedere Alkyl-, Benzyl- oder p-Nitrobenzylgruppe ist, $R_7$ ein Wasserstoffatom oder eine nieder Alkyl-, Arylalkyl-, Benzhydryl- oder Tritylgruppe ist, $R_8$ ein Wasserstoffatom oder eine niedere Alkyl- oder Phenylgruppe ist; $R_9$ ein Wasserstoffatom oder eine niedere Alkylgruppe ist; und $R_2$ ein Wasserstoffatom oder eine Azidonieder-alkylgruppe oder eine Gruppe $CR_{10}R_{11}R_{12}$ ist, wobei $R_{10}$ ein Wasserstoffatom oder eine Hydroxylgruppe ist; $R_{11}$ ein Wasserstoffatom oder eine niedere Alkylgruppe ist; und $R_{12}$ ein Wasserstoffatom, eine Nieder-acyloxy-nieder-alkylgruppe, eine niedere Hydroxyalkylgruppe, niedere Alkylgruppe, eine Benzylgruppe, eine Phenylgruppe ist, oder an $R_{11}$ zu einem Rest eines $C_{5-7}$-carbocyclischen Rings gebunden ist, oder eine Gruppe der Formel $CH(OH)R_{13}$ oder CHX ist, in der $R_{13}$ ein Wasserstoffatom oder eine niedere Alkylgruppe ist und X ein Sauerstoffatom oder eine $CR_{14}R_{15}$-Gruppe ist, in der $R_{14}$ ein Wasserstoffatom oder eine niedere Alkyl-, Phenyl-, CN—, $CO_2R_{16}$— oder $COR_{16}$-Gruppe ist, wobei $R_{16}$ eine niedere Alkyl-, Phenyl- oder Benzylgruppe ist und $R_{15}$ ein Wasserstoffatom oder eine niedere Alkylgruppe ist, oder an $R_{14}$ zu einem $C_{5-7}$-carbocyclischen Ring gebunden ist, wobei "Niederalkyl" eine Gruppe mit bis zu 4 Kohlenstoffatom bedeutet und "Aralalkyl" eine Gruppe mit bis zu 4 Kohlenstoffatomen bedeutet, die mit Phenyl, Fluorphenyl, Chlorphenyl, Bromphenyl, Methoxyphenyl, Methylphenyl, Nitrophenyl oder Aminophenyl substituiert ist, mit der Maßgabe, daß folgende Verbindungen ausgeschlossen sind: wenn $R_2$ 1-Hydroxyäthyl oder acylierte Derivate davon oder Hydroxymethyl ist und $R_1$ eine Methyl-oder Aminoalkylgruppe mit bis zu 3 Kohlenstoffatomen ist; wenn $R_2$ eine 1-Hydroxyäthylgruppe und $R_1$ die Benzyl-, $NH_2(CH_2)_4$—, $NH_2C(CH_3)_2CH_2$—, $NH_2CH_2C(CH_3)_2$— oder N-substituierte Aminoäthylgruppe ist; wenn $R_2$ die Hydroxymethylgruppe und $R_1$ die $NH_2C(CH_3)_2CH_2$—Gruppe ist; wenn $R_2$ die $PhCH_2CH(OH)$— oder Äthylgruppe und $R_1$ die Aminoäthylgruppe ist; und wenn $R_2$ die Isopropylgruppe und $R_1$ die 2-Acetamidoäthylgruppe ist.

2. Eine Verbindung gemäß Anspruch 1, in der $R_1$ eine niedere Alkylgruppe ist.

3. Eine Verbindung gemäß Anspruch 2, in der $R_1$ eine Äthylgruppe ist.

4. Eine Verbindung gemäß Anspruch 1, in der $R_1$ eine $CH_2CH_2NHCOCH_3$-Gruppe ist.

5. Eine Verbindung gemäß Anspruch 1, in der $R_1$ eine $CH_2CH_2OCONHCH_3$-Gruppe ist.

6. Eine Verbindung gemäß Anspruch 1, in der $R_1$ eine niedere Alkylgruppe ist, die an einem anderen Kohlenstoffatom als das, an das das Schwefelatom gebunden ist, mit einer $OR_4$-Gruppe substituiert ist, in der die niedere Alkylgruppe eine Äthylgruppe und $R_4$ ein Wasserstoffatom oder eine Methyl- oder Acetylgruppe ist.

7. Eine Verbindung gemäß einem der Ansprüche 1 bis 6, in der $R_2$ ein Wasserstoffatom ist.

8. Eine Verbindung gemäß einem der Ansprüche 1 bis 6, in der $R_2$ eine Gruppe $CR_{10}R_{11}R_{12}$ ist, in der $R_{10}$, $R_{11}$ und $R_{12}$ wie in Anspruch 1 definiert sind.

9. Eine Verbindung gemäß Anspruch 8, in der die $CR_{10}R_{11}R_{12}$-Gruppe ein $C(OH)(R_{19})R_{20}$-Rest ist, in dem $R_{19}$ ein Wasserstoffatom oder eine niedere Alkylgruppe ist und $R_{20}$ ein Wasserstoffatom oder eine niedere Alkylgruppe ist.

10. Eine Verbindung gemäß Anspruch 9, in der der $C(OH)(R_{19})R_{20}$-Rest eine $CH(OH)CH_3$-Gruppe ist.

11. Eine Verbindung gemäß einem der Ansprüche 1 bis 10, in Form eines pharmakologisch verträglichen Salzes.

12. Eine Verbindung gemäß einem der Ansprüche 1 bis 10, in der die Estergruppe die Unterformel (a), (b), (c) oder (d) hat:

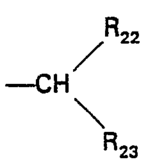 (a) 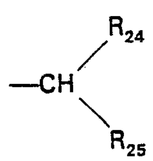 (b) 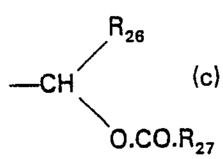 (c) 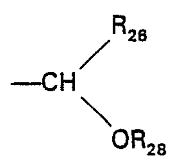 (d)

in denen $R_{22}$ ein Wasserstoffatom oder eine Alkyl-, Alkenyl- oder Alkinylgruppe mit bis zu 3 Kohlenstoffatomen ist, $R_{23}$ ein Wasserstoffatom oder eine Methylgruppe ist; $R_{24}$ eine Phenylgruppe oder eine Phenylgruppe ist, die mit einem Fluor-, Chlor- oder Bromatom oder mit einer Nitro-, Methyl- oder Methoxygruppe substituiert ist; $R_{25}$ ein Wasserstoffatom oder eine Phenylgruppe oder eine Phenylgruppe ist, die mit einem Fluor-, Chlor- oder Bromatom oder mit einer Nitro-, Methyl- oder Methoxygruppe substituiert ist; $R_{26}$ ein Wasserstoffatom oder eine Methylgruppe ist und $R_{27}$ eine niedere Alkyl-, Phenyl- oder niedere Alkoxygruppe ist, oder $R_{26}$ und $R_{27}$ zu einer Phthalidylgruppe verbunden sind; und $R_{28}$ eine niedere Alkyl-, Phenyl-, Chlorphenyl- oder Nitrophenylgruppe ist; oder $CH(R_{22})R_{23}$ eine Phenacyl- oder p-Bromphenacyl-Gruppe ist.

13. Eine Verbindung gemäß einem der Ansprüche 1 bis 10 in Form ihres p-Nitrobenzylesters.

14. Eine Verbindung gemäß einem der Ansprüche 1 bis 10 in Form ihres Phthalidylesters.

15. Eine pharmazeutische Zusammensetzung, die eine Verbindung gemäß einem der Ansprüche 1 bis 14 und einen pharmakologisch verträglichen Träger enthält.

16. Eine pharmazeutische Zusammensetzung gemäß Anspruch 15, die auch ein Penicillin oder Cephalosporin enthält.

17. Eine Verbindung gemäß einem der Ansprüche 1 bis 16, wenn als antibakterielles Mittel verwendet.

18. Eine Verfahren zur Herstellung einer Verbindung gemäß einem der Ansprüche 1 bis 14, umfassend die basenkatalysierte Isomerisierung der Doppelbindung in einem Ester einer Verbindung der Formel (VIII):

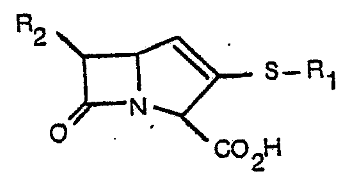

(VIII)

und anschließend, wenn erwünscht, das Umwandeln eines spaltbaren Esters der Formel (II) in die Verbindung der Formel (II) oder ein Salz derselben.

19. 3-Äthylthio-7-oxo-1-azabicyclo[3.2.0]hept-2-en-2-carbonsäure oder ein pharmakologisch verträgliches Salz oder ein spaltbarer Ester derselben.

20. Ein pharmakologisch verträgliches Salz von 3-Äthylthio-7-oxo-1-azabicyclo[3.2.0]hept-2-en-2-carbonsäure gemäß Anspruch 19.

21. 3-Äthylthio-7-oxo-1-azabicyclo[3.2.0]hept-2-en-2-carbonsäure-phthalidylester gemäß Anspruch 19.

22. Eine Verbindung gemäß einem der Ansprüche 7, 19, 20 und 21 in Form des Isomeren mit der gleichen Stereochemie am C-5 wie (+)-3-Äthylthio-7-oxo-1-azabicyclo[3.2.0]hept-2-en-2-carbonsäure-p-nitrobenzylester.

**Patentansprüche für den Vertragsstaat: IT**

1. Eine Verbindung der Formel (II):

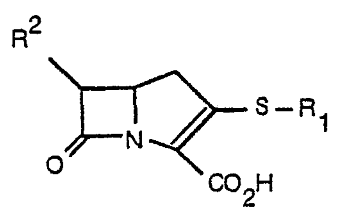

(II)

oder ein Salz oder Ester derselben, in der $R_1$ eine niedere Alkylgruppe, eine Arylalkylgruppe oder eine niedere Alkylgruppe ist, die an einem anderen Kohlenstoffatom, als das, an das das Schwefelatom gebunden ist, mit einer $CO_2R_3$—, $OR_4$—, $N(R_5)COR_6$—, $N(R_5)CO_2R_6$— oder $N(R_5)R_7$-Gruppe oder mit einer $CO_2R_3$— und einer $N(R_5)COR_6$-Gruppe substituiert ist, wobei $R_3$ eine solche Gruppe ist, daß $CO_2R_3$ eine Carbonsäuregruppe oder ein Salz oder ein niederer Alkyl- oder Benzylester derselben ist, $R_4$ ein Wasserstoffatom oder eine niedere Alkyl-, niedere Acyl-, Benzyloxycarbonyl- oder p-Nitrobenzyloxycarbonylgruppe oder eine $CONR_8R_9$-Gruppe ist, $R_5$ ein Wasserstoffatom oder eine niedere Alkylgruppe ist, $R_6$ eine niedere Alkyl-, Benzyl- oder p-Nitrobenzylgruppe ist, $R_7$ ein Wasserstoffatom oder eine niedere Alkyl-, Arylalkyl-, Benzhydryl- oder Tritylgruppe ist, $R_8$ ein Wasserstoffatom oder eine niedere Alkyl- oder Phenylgruppe ist; $R_9$ ein Wasserstoffatom oder eine niedere Alkylgruppe ist; und $R_2$ ein Wasserstoffatom oder eine Azidonieder-alkylgruppe oder eine Gruppe $CR_{10}R_{11}R_{12}$ ist, wobei $R_{10}$ ein Wasserstoffatom oder eine Hydroxylgruppe ist; $R_{11}$ ein

Wasserstoffatom oder eine niedere Alkylgruppe ist; und $R_{12}$ ein Wasserstoffatom, eine Nieder-acyloxy-nieder-alkylgruppe, eine niedere Hydroxyalkylgruppe, niedere Alkylgruppe, eine Benzylgruppe, eine Phenylgruppe ist, oder an $R_{11}$ zu einem Rest eines $C_{5-7}$-carbocyclischen Rings gebunden ist, oder eine Gruppe der Formel $CH(OH)R_{13}$ oder CHX ist, in der $R_{13}$ ein Wasserstoffatom oder eine niedere Alkylgruppe ist und X ein Sauerstoffatom oder eine $CR_{14}R_{15}$-Gruppe ist, in der $R_{14}$ ein Wasserstoffatom oder eine niedere Alkyl-, Phenyl-, CN—, $CO_2R_{16}$- oder $COR_{16}$-Gruppe ist, wobei $R_{16}$ eine niedere Alkyl-, Phenyl- oder Benzylgruppe ist und $R_{15}$ ein Wasserstoffatom oder eine niedere Alkylgruppe ist, oder an $R_{14}$ zu einem $C_{5-7}$-carbocyclischen Ring gebunden ist, wobei "Niederalkyl" eine Gruppe mit bis zu 4 Kohlenstoffatomen bedeutet und "Arylalkyl" eine Gruppe mit bis zu 4 Kohlenstoffatomen bedeutet, die mit Phenyl, Fluorphenyl, Chlorphenyl, Bromphenyl, Methoxyphenyl, Methylphenyl, Nitrophenyl oder Aminophenyl substituiert ist, ausgenommen die Verbindungen, in denen $R_2$ eine 1-Hydroxyäthylgruppe oder acylierte Derivate derselben ist und $R_1$ eine Aminoäthylgruppe oder ein N-substituiertes Derivat derselben ist; und ausgenommen Verbindungen, in denen $R_2$ die Äthylgruppe und $R_1$ $CH_2CH_2NH_2$ ist und ausgenommen die Verbindungen, in denen $R_2$ die Isopropylgruppe ist und $R_1$ die 2-Acetamidoäthylgruppe ist.

2. Eine Verbindung gemäß Anspruch 1, in der $R_1$ eine niedere Alkylgruppe ist.

3. Eine Verbindung gemäß Anspruch 2, in der $R_1$ eine Äthylgruppe ist.

4. Eine Verbindung gemäß Anspruch 1, in der $R_1$ eine $CH_2CH_2NHCOCH_3$-Gruppe ist.

5. Eine Verbindung gemäß Anspruch 1, in der $R_1$ eine $CH_2CH_2OCONHCH_3$-Gruppe ist.

6. Eine Verbindung gemäß Anspruch 1, in der $R_1$ eine niedere Alkylgruppe ist, die an einem anderen Kohlenstoffatom als das, an das das Schwefelatom gebunden ist, mit einer $OR_4$-Gruppe substituiert ist, in der die niedere Alkylgruppe eine Äthylgruppe und $R_4$ ein Wasserstoffatom oder eine Methyl- oder Acetylgruppe ist.

7. Eine Verbindung gemäß einem der Ansprüche 1 bis 6, in der $R_2$ ein Wasserstoffatom ist.

8. Eine Verbindung gemäß einem der Ansprüche 1 bis 6, in der $R_2$ eine Gruppe $CR_{10}R_{11}R_{12}$ ist, in der $R_{10}$, $R_{11}$ und $R_{12}$ wie in Anspruch 1 definiert sind.

9. Eine Verbindung gemäß Anspruch 8, in der die $CR_{10}R_{11}R_{12}$-Gruppe ein $C(OH)(R_{19})R_{20}$-Rest ist, in dem $R_{19}$ ein Wasserstoffatom oder eine niedere Alkylgruppe ist und $R_{20}$ ein Wasserstoffatom oder eine niedere Alkylgruppe ist.

10. Eine Verbindung gemäß Anspruch 9, in der der $C(OH)(R_{19})R_{20}$-Rest eine $CH(OH)CH_3$-Gruppe ist.

11. Eine Verbindung gemäß einem der Ansprüche 1 bis 10, in Form eines pharmakologisch verträglichen Salzes.

12. Eine Verbindung gemäß einem der Ansprüche 1 bis 10, in der die Estergruppe die Unterformel (a), (b), (c) oder (d) hat:

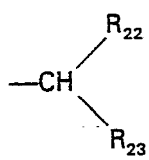 (a) 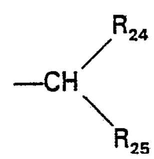 (b) 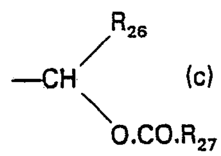 (c) 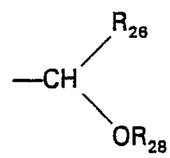 (d)

in denen $R_{22}$ ein Wasserstoffatom oder eine Alkyl-, Alkenyl- oder Alkinylgruppe mit bis zu 3 Kohlenstoffatomen ist, $R_{23}$ ein Wasserstoffatom oder eine Methylgruppe ist; $R_{24}$ eine Phenylgruppe oder eine Phenylgruppe ist, die mit einem Fluor-, Chlor- oder Bromatom oder mit einer Nitro-, Methyl- oder Methoxygruppe substituiert ist; $R_{25}$ ein Wasserstoffatom oder eine Phenylgruppe oder eine Phenylgruppe ist, die mit einem Fluor-, Chlor- oder Bromatom oder mit einer Nitro-, Methyl- oder Methoxygruppe substituiert ist; $R_{26}$ ein Wasserstoffatom oder eine Methylgruppe ist und $R_{27}$ eine niedere Alkyl-, Phenyl- oder niedere Alkoxygruppe ist, oder $R_{26}$ und $R_{27}$ zu einer Phthalidylgruppe verbunden sind; und $R_{28}$ eine niedere Alkyl-, Phenyl-, Chlorphenyl- oder Nitrophenylgruppe ist; oder $CH(R_{22})R_{23}$ eine Phenacyl- oder p-Bromphenacyl-Gruppe ist.

13. Eine Verbindung gemäß einem der Ansprüche 1 bis 10 in Form ihres p-Nitrobenzylesters.

14. Eine Verbindung gemäß einem der Ansprüche 1 bis 10 in Form ihres Phthalidylesters.

15. Eine pharmazeutische Zusammensetzung, die eine Verbindung gemäß einem der Ansprüche 1 bis 14 und einen pharmakologisch verträglichen Träger enthält.

16. Eine pharmazeutische Zusammensetzung gemäß Anspruch 15, die auch ein Penicillin oder Cephalosporin enthält.

17. Eine Verbindung gemäß einem der Ansprüche 1 bis 16, wenn als antibakterielles Mittel versendet.

18. Eine Verfahren zur Herstellung einer Verbindung gemäß einem der Ansprüche 1 bis 14, umfassend die basenkatalysierte Isomerisierung der Doppelbindung in einem Ester einer Verbindung der Formel (VIII):

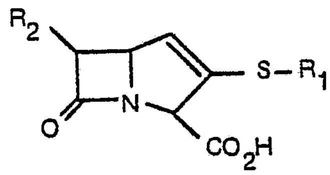

(VIII)

und anschließend, wenn erwünscht, das Umwandeln eines spaltbaren Esters der Formel (II) in die Verbindung der Formel (II) oder ein Salz derselben.

19. 3-Äthylthio-7-oxo-1-azabicyclo[3.2.0]hept-2-en-2-carbonsäure oder ein pharmakologisch verträgliches Salz oder ein spaltbarer Ester derselben.

20. Ein pharmakologisch verträgliches Salz von 3-Äthylthio-7-oxo-1-azabicyclo[3.2.0]hept-2-en-2-carbonsäure gemäß Anspruch 19.

21. 3-Äthylthio-7-oxo-1-azabicyclo[3.2.0]hept-2-en-2-carbonsäure-phthalidylester gemäß Anspruch 19.

22. Eine Verbindung gemäß einem der Ansprüche 7, 19, 20 und 21 in Form des Isomeren mit der gleichen Stereochemie am C-5 wie (+)-3-Äthylthio-7-oxo-1-azabicyclo[3.2.0]hept-2-en-2-carbonsäure-p-nitrobenzylester.

**Revendications pour les Etats contractants: BE CH DE FR GB NL SE**

1. Composé de formule (II):—

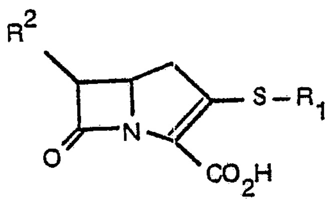

(II)

ou sel ou ester de ce composé, formule dans laquelle $R_1$ est un groupe alcoyle inférieur, un groupe aralcoyle ou est un groupe alcoyle inférieur substitué sur un atome de carbone autre que celui auquel l'atome de soufre est rattaché par un groupe $CO_2R_3$, $OR_4$, $N(R_5)COR_6$, $N(R_5)CO_2R_6$ ou $N(R_5)R_7$, ou par un groupe $CO_2R_3$ et un groupe $N(R_5)COR_6$; où $R_3$ est un groupe tel que $CO_2R_3$ soit un groupe acide carboxylique ou un sel ou ester d'alcoyle inférieur ou de benzyle de de celui-ci; $R_4$ est un atome d'hydrogène ou un groupe alcoyle inférieur, acyle inférieur, benzyloxycarbonyle ou p-nitrobenzyloxycarbonyle ou un groupe $CONR_8R_9$; $R_5$ est un atome d'hydrogène ou un groupe alcoyle inférieur; $R_6$ est un groupe alcoyle inférieur, benzyle ou p-nitrobenzyle; $R_7$ est un atome d'hydrogène ou un groupe alcoyle inférieur, aralcoyle, benzhydryle ou trityle; $R_8$ est un atome d'hydrogène ou un groupe alcoyle inférieur ou phényle; $R_9$ est un atome d'hydrogène ou un groupe alcoyle inférieur; et $R_2$ est un atome d'hydrogène ou un groupe azido-alcoyle inférieur ou un groupe $CR_{10}R_{11}R_{12}$ dans lequel $R_{10}$ est un atome d'hydrogène ou un groupe hydroxy; $R_{11}$ est un atome d'hydrogène ou un groupe alcoyle inférieur; et $R_{12}$ est un atome d'hydrogène ou un groupe acyl (inférieur)-oxy-alcoyle(inférieur), un groupe hydroxyalcoyle inférieur, un groupe alcoyle inférieur, un groupe benzyle, un groupe phényle ou est réuni à $R_{11}$ pour faire partie d'un carbocycle en $C_{5-7}$ ou est un groupe de formule $CH(OH)R_{13}$ ou CHX dans lequel $R_{13}$ est un atome d'hydrogène ou un groupe alcoyle inférieur et X est un atome d'oxygène ou un groupe $CR_{14}R_{15}$ dans lequel $R_{14}$ est un atome d'hydrogène ou un groupe alcoyle inférieur, phényle CN, $CO_2R_{16}$ ou $CO.R_{16}$; où $R_{16}$ est un groupe alcoyle inférieur, phényle ou benzyle et $R_{15}$ est un atome d'hydrogène ou un groupe alcoyle inférieur ou est réuni à $R_{14}$ pour faire partie d'un carbocycle en $C_{5-7}$: ou "alcoyle inférieur" signifie un groupe ayant jusqu'à 4 atomes de carbone et "aralcoyle" désigne un groupe ayant jusqu'à 4 atomes de carbone substitué par un groupe phényle, fluorophényle, chlorophényle, bromophényle, méthoxyphényle, méthylphényle, nitrophényle ou aminophényle; à la condition que les composés suivants soient exclus; ceux dans lesquels $R_2$ est un groupe 1-hydroxyéthyle ou des dérivés acylés de celui-ci ou hydroxyméthyle et $R_1$ est un groupe méthyle ou amino-alcoyle ayant jusqu'à 3 atomes de carbone; ceux dans lesquels $R_2$ est un groupe hydroxyéthyle et $R_1$ est un groupe benzyle, $NH_2(CH_2)_4$—, $NH_2C(CH_3)_2CH_2$—, $NH_2CH_2C(CH_3)_2$— ou amino-éthyle substitué à l'azote; ceux dans lesquels $R_2$ est un groupe hydroxyméthyle et $R_1$ est $NH_2C(CH_3)_2CH_2$—; ceux dans lesquels $R_2$ est $PhCH_2CH(OH)$— ou éthyle et $R_1$ est un groupe aminoéthyle; et ceux dans lesquels $R_2$ est un groupe isopropyle et $R_1$ est un groupe 2-acétamidoéthyle.

2. Composé suivant la revendication 1 dans lequel $R_1$ est un groupe alcoyle inférieur.

3. Composé suivant la revendication 2 dans lequel $R_1$ est un groupe éthyle.

4. Composé suivant la revendication 1 dans lequel $R_1$ est un groupe $CH_2CH_2NHCOCH_3$.

5. Composé suivant la revendication 1 dans lequel $R_1$ est un groupe $CH_2CH_2OCONHCH_3$.

6. Composé suivant la revendication 1 dans lequel $R_1$ est un groupe alcoyle inférieur substitué sur un atome de carbone autre que celui auquel l'atome de soufre est rattaché par un groupe $OR_4$ dans lequel le groupe alcoyle inférieur est un groupe éthyle et $R_4$ est un atome d'hydrogène ou un groupe méthyle ou acétyle.

7. Composé suivant l'une quelconque des revendications 1 à 6, dans lequel $R_2$ est un atome d'hydrogène.

8. Composé suivant l'une quelconque des revendications 1 à 6, dans lequel $R_2$ est un groupe $CR_{10}R_{11}R_{12}$, ou $R_{10}$, $R_{11}$ et $R_{12}$ sont tels que définis dans la revendication 1.

9. Composé suivant la revendication 8, dans lequel le groupe $CR_{10}R_{11}R_{12}$ est une fraction molaire $C(OH)(R_{19})R_{20}$ dans laquelle $R_{19}$ est un atome d'hydrogène ou un groupe alcoyle inférieur et $R_{20}$ est un atome d'hydrogène ou un groupe alcoyle inférieur.

10. Composé suivant la revendication 9, dans lequel la fraction molaire $C(OH)(R_{19})R_{20}$ est un groupe $CH(OH)CH_3$.

11. Composé suivant l'une quelconque des revendications 1 à 10 sous la forme d'un sel pharmaceutiquement acceptable.

12. Composé suivant l'une quelconque des revendications 1 à 10 dans lequel le groupe ester correspond à la sous-formule (a), (b), (c) ou (d):—

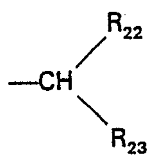 (a) 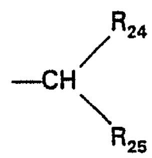 (b) 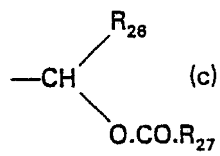 (c) 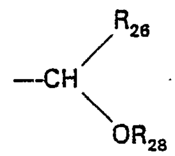 (d)

dans lesquelles $R_{22}$ est un atome d'hydrogène ou un groupe alcoyle, alcényle ou alcynyle ayant jusqu'à 3 atomes de carbone; $R_{23}$ est un atome d'hydrogène ou un groupe méthyle; $R_{24}$ est un groupe phényle ou un groupe phényle substitué par un atome de fluor, de chlore ou de brome ou par un groupe nitro, méthyle ou méthoxy; $R_{25}$ est un atome d'hydrogène ou un groupe phényle ou bien un groupe phényle substitué par un atome de fluor, de chlore ou de brome ou par un groupe nitro, méthyle ou méthoxy; $R_{26}$ est un atome d'hydrogène ou un groupe méthyle; et $R_{27}$ est un groupe alcoyle inférieur, phényle ou alcoxy inférieur, ou bien $R_{26}$ est réuni à $R_{27}$ pour former un groupe phtalidyle; et $R_{28}$ est un groupe alcoyle inférieur, phényle, chlorophényle ou nitrophényle; ou bien $CH(R_{22})R_{23}$ est un groupe phénacyle ou p-bromophénacyle.

13. Composé suivant l'une quelconque des revendications 1 à 10 sous la forme de son ester p-nitrobenzylique.

14. Composé suivant l'une quelconque des revendications 1 à 10 sous la forme de son ester phtalidylique.

15. Composition pharmaceutique renfermant un composé suivant l'une quelconque des revendications 1 à 14 et un excipient pharmaceutiquement acceptable.

16. Composition pharmaceutique suivant la revendication 15 renfermant également une pénicilline ou une céphalosporine.

17. Composé suivant l'une quelconque des revendications 1 à 16 lorsqu'il est utilisé comme agent antibactérien.

18. Procédé pour la préparation d'un composé suivant l'une quelconque des revendications 1 à 14 ce procédé consistant à effectuer l'isomérisme catalysé par une base de la double liaison dans un ester d'un composé de formule (VIII):

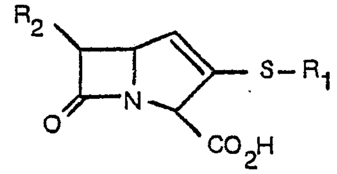

(VIII)

puis si désiré à convertir l'ester dissociable de formule (II) en composé de formule (II) ou en un sel de celui-ci.

19. Acide 3-éthylthio-7-oxo-1-azabicyclo[3.2.0]hept-2-ène-2-carboxylique ou sel pharmaceutiquement acceptable ou ester dissociable de celui-ci.

20. Sel pharmaceutiquement acceptable d'acide 3-éthylthio-7-oxo-1-azabicyclo[3.2.0]hept-2-ène 2-carboxylique suivant la revendication 19.

21. 3-Ethylthio-7-oxo-1-azabicyclo[3.2.0]hept-2-ène-2-carboxylate de phtalidyle suivant la revendication 19.

22. Composé suivant l'une quelconque des revendications 7, 19, 20 et 21 sous la forme de l'isomère ayant la même stéréochimie en C5 que le (+) 3-éthylthio-7-oxo-1-azabicyclo[3.2.0]hept-2-ène-2-carboxylate de p-nitrobenzyle.

**Revendications pour l'Etat contractant: IT**

1. Composé de formule (II):—

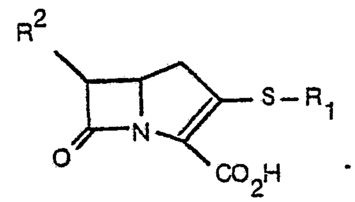

(II)

ou sel ou ester de ce composé, formule dans laquelle $R_1$ est un groupe alcoyle inférieur, un groupe aralcoyle ou est un groupe alcoyle inférieur substitué sur un atome de carbone autre que celui auquel l'atome de soufre est rattaché par un groupe $CO_2R_3$, $OR_4$, $N(R_5)COR_6$, $N(R_5)CO_2R_6$ ou $N(R_5)R_7$, ou par un groupe $CO_2R_3$ et un groupe $N(R_5)COR_6$; où $R_3$ est un groupe tel que $CO_2R_3$ soit un groupe acide carboxylique ou un sel ou ester d'alcoyle inférieur ou de benzyle de celui-ci; $R_4$ est un atome d'hydrogène ou un groupe alcoyle inférieur, acyle inférieur, benzyloxycarbonyle ou p-nitrobenzyloxycarbonyle ou un groupe $CONR_8R_9$; $R_5$ est un atome d'hydrogène ou un groupe alcoyle inférieur; $R_6$ est un groupe alcoyle inférieur, benzyle ou p-nitrobenzyle; $R_7$ est un atome d'hydrogène ou un groupe alcoyle inférieur, aralcoyle, benzhydryle ou trityle; $R_8$ est un atome d'hydrogène ou un groupe alcoyle inférieur ou phényle; $R_9$ est un atome d'hydrogène ou un groupe alcoyle inférieur; et $R_2$ est un atome d'hydrogène ou un groupe azido-alcoyle inférieur ou un groupe $CR_{10}R_{11}R_{12}$ dans lequel $R_{10}$ est un atome d'hydrogène ou un groupe hydroxy; $R_{11}$ est un atome d'hydrogène ou un groupe alcoyle inférieur; et $R_{12}$ est un atome d'hydrogène ou un groupe acyl (inférieur)-oxy-alcoyle (inférieur), un groupe hydroxyalcoyle inférieur, un groupe alcoyle inférieur, un groupe benzyle, un groupe phényle ou est réuni à $R_{11}$ pour faire partie d'un carbocycle en $C_{5-7}$ ou est un groupe de formule $CH(OH)R_{13}$ ou CHX dans lequel $R_{13}$ est un atome d'hydrogène ou un groupe alcoyle inférieur et X est un atome d'oxygène ou un groupe $CR_{14}R_{15}$ dans lequel $R_{14}$ est un atome d'hydrogène ou un groupe alcoyle inférieur, phényle CN, $CO_2R_{16}$ ou $CO.R_{16}$, où $R_{16}$ est un groupe alcoyle inférieur, phényle ou benzyle et $R_{15}$ est un atome d'hydrogène ou un groupe alcoyle inférieur ou est réuni à $R_{14}$ pour faire partie d'un carbocycle en $C_{5-7}$; ou "alcoyle inférieur" signifie un groupe ayant jusqu'à 4 atomes de carbone et "aralcoyle" désigne un groupe ayant jusqu'à 4 atomes de carbone substitué par un groupe phényle, fluorophényle, chlorophényle, bromophényle, méthoxyphényle, méthylphényle, nitrophényle ou aminophényle; sauf les composés dans lesquels $R_2$ est un groupe 1-hydroxyéthyle ou des dérivés acylés de celui-ci et $R_1$ est un groupe aminoéthyle ou un dérivé substitué à l'azote de celui-ci; et sauf les composés dans lesquels $R_2$ est un groupe éthyle et $R_1$ est un groupe $CH_2CH_2NH_2$; et sauf les composés dans lesquels $R_2$ est un groupe isopropyle et $R_1$ est un groupe 2-acétamidoéthyle.

2. Composé suivant la revendication 1 dans lequel $R_1$ est un groupe alcoyle inférieur.

3. Composé suivant la revendication 2 dans lequel $R_1$ est un groupe éthyle.

4. Composé suivant la revendication 1 dans lequel $R_1$ est un groupe $CH_2CH_2NHCOCH_3$.

5. Composé suivant la revendication 1 dans lequel $R_1$ est un groupe $CH_2CH_2OCONHCH_3$.

6. Composé suivant la revendication 1 dans lequel $R_1$ est un groupe alcoyle inférieur substitué sur un atome de carbone autre que celui auquel l'atome de soufre est rattaché par un groupe $OR_4$ dans lequel le groupe alcoyle inférieur est un groupe éthyle et $R_4$ est un atome d'hydrogène ou un groupe méthyle ou acétyle.

7. Composé suivant l'une quelconque des revendications 1 à 6, dans lequel $R_2$ est un atome d'hydrogène.

8. Composé suivant l'une quelconque des revendications 1 à 6, dans lequel $R_7$ est un groupe $CR_{10}R_{11}R_{12}$, où $R_{10}$, $R_{11}$ et $R_{12}$ sont tels que définis dans la revendication 1.

9. Composé suivant la revendication 8, dans lequel le groupe $CR_{10}R_{11}R_{12}$ est une fraction molaire $C(OH)(R_{19})R_{20}$ dans laquelle $R_{19}$ est un atome d'hydrogène ou un groupe alcoyle inférieur et $R_{20}$ est un atome d'hydrogène ou un groupe alcoyle inférieur.

10. Composé suivant la revendication 9, dans lequel la fraction molaire $C(OH)(R_{19})R_{20}$ est un groupe $CH(OH)CH_3$.

11. Composé suivant l'une quelconque des revendications 1 à 10 sous la forme d'un sel pharmaceutiquement acceptable.

12. Composé suivant l'une quelconque des revendications 1 à 10 dans lequel le groupe ester correspond à la sous-formule (a), (b), (c) ou (d):—

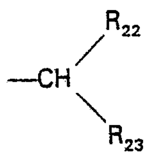 (a) 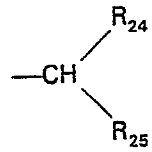 (b) 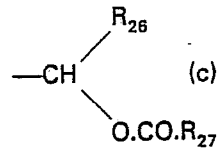 (c) 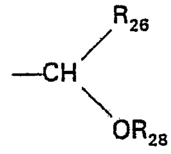 (d)

dans lesquelles $R_{22}$ est un atome d'hydrogène ou un groupe alcoyle, alcényle ou alcynyle ayant jusqu'à 3 atomes de carbone; $R_{23}$ est un atome d'hydrogène ou un groupe méthyle; $R_{24}$ est un groupe phényle ou un groupe phényle substitué par un atome de fluor, de chlore ou de brome ou par un groupe nitro, méthyle ou méthoxy; $R_{25}$ est un atome d'hydrogène ou un groupe phényle ou bien un groupe phényle substitué par un atome de fluor, de chlore ou de brome ou par un groupe nitro, méthyle ou méthoxy; $R_{26}$ est un atome d'hydrogène ou un groupe méthyle; et $R_{27}$ est un groupe alcoyle inférieur, phényle ou alcoxy inférieur, ou bien $R_{26}$ est réuni à $R_{27}$ pour former un groupe phtalidyle; et $R_{28}$ est un groupe alcoyle inférieur, phényle, chlorophényle ou nitrophényle; ou bien $CH(R_{22})R_{23}$ est un groupe phénacyle ou p-bromophénacyle.

13. Composé suivant l'une quelconque des revendications 1 à 10 sous la forme de son ester p-nitrobenzylique.

14. Composé suivant l'une quelconque des revendications 1 à 10 sous la forme de son ester phtalidylique.

15. Composition pharmaceutique renfermant un composé suivant l'une quelconque des revendications 1 à 14 et un excipient pharmaceutiquement acceptable.

16. Composition pharmaceutique suivant la revendication 15 renfermant également une pénicilline ou une céphalosporine.

17. Composé suivant l'une quelconque des revendications 1 à 16 lorsqu'il est utilisé comme agent antibactérien.

18. Procédé pour la préparation d'un composé suivant l'une quelconque des revendications 1 à 14, ce procédé consistant à effectuer l'isomérisme catalysé par une base de la double liaison dans un ester d'un composé de formule (VIII):

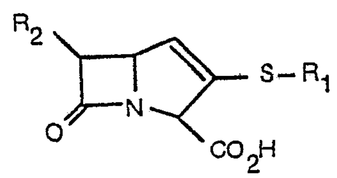

(VIII)

puis si désiré à convertir l'ester dissociable de formule (II) en composé de formule (II) ou en un sel de celui-ci.

19. Acide 3-éthylthio-7-oxo-1-azabicyclo[3.2.0]hept-2-ène-2-carboxylique ou sel pharmaceutiquement acceptable ou ester dissociable de celui-ci.

20. Sel pharmaceutiquement acceptable d'acide 3-éthylthio-7-oxo-1-azabicyclo[3.2.0]hept-2-ène 2-carboxylique suivant la revendication 19.

21. 3-Ethylthio-7-oxo-1-azabicyclo[3.2.0]hept-2-ène-2-carboxylate de phtalidyle suivant la revendication 19.

22. Composé suivant l'une quelconque des revendications 7, 19, 20 et 21 sous la forme de l'isomère ayant la même stéréochimie en C5 que le (+) 3-éthylthio-7-oxo-1-azabicyclo[3.2.0]hept-2-ène-2-carboxylate de p-nitrobenzyle.